# EUROPEAN PATENT SPECIFICATION

(11) **EP 3 844 151 B1**
(45) Date of publication and mention of the grant of the patent: **23.08.2023**
(21) Application number: 19855183.0
(22) Date of filing: 29.08.2019
(51) Int. Cl.: C07D 487/04, A61K 31/519, A61P 35/00

(54) **KRAS G12C INHIBITORS**
KRAS-G12C-INHIBITOREN
INHIBITEURS DE KRAS G12C

(30) Priority: 31.08.2018 US 201862725449 P
(43) Date of publication of application: 07.07.2021
(73) Proprietor: Mirati Therapeutics, Inc., San Diego, CA 92121 (US); Array BioPharma Inc., Boulder, CO 80301 (US)
(72) Inventor: MARX, Matthew, Arnold, San Diego, CA 92127 (US); BLAKE, James, F., Boulder, CO 80301 (US); FELL, Jay, Bradford, Boulder, CO 80301 (US); FISCHER, John, P., Boulder, CO 80301 (US); MEJIA, Macedonio, J., Boulder, CO 80301 (US)
(74) Representative: Elkington and Fife LLP
(86) International application number: PCT/US2019/048713
(87) International publication number: WO 2020/047192

(56) References cited:
- WO-A1-2017/201161
- CN-A- 112 430 234
- US-A1- 2018 072 723
- US-A1- 2018 155 348
- US-A1- 2019 144 444
- US-B2- 9 828 363
- HAWRYLUK et al.: "Discovery and synthesis of 6,7,8,9-tetrahydro-5H-pyrimido-[4,5-d]azep ines as novel TRPV1 antagonists", Bioorganic & Medicinal Chemistry Letters, vol. 20, no. 23 1 December 2010 (2010-12-01), XP027459374, DOI: 10.1016/j.bmcl.2010.09.023 Retrieved from the Internet: URL:https://www.sciencedirect.com/science/ article/pii/S0960894XlO013144?via%3Dihub [retrieved on 2019-10-14]

## Description

### FIELD OF THE INVENTION

The present invention relates to compounds that inhibit KRas G12C. In particular, the present invention relates to compounds that irreversibly inhibit the activity of KRas G12C, pharmaceutical compositions comprising the compounds, methods of use therefor and medical uses therefor.

### BACKGROUND OF THE INVENTION

Kirsten Rat Sarcoma 2 Viral Oncogene Homolog ("KRas") is a small GTPase and a member of the Ras family of oncogenes. KRas serves a molecular switch cycling between inactive (GDP-bound) and active (GTP-bound) states to transduce upstream cellular signals received from multiple tyrosine kinases to downstream effectors to regulate a wide variety of processes, including cellular proliferation (e.g., see Alamgeer et al., (2013) Current Opin Pharmcol. 13:394-401).

The role of activated KRas in malignancy was observed over thirty years ago (e.g., see Santos et al., (1984) Science 223:661-664). Aberrant expression of KRas accounts for up to 20% of all cancers and oncogenic KRas mutations that stabilize GTP binding and lead to constitutive activation of KRas and downstream signaling have been reported in 25 -30% of lung adenocarcinomas. (e.g., see Samatar and Poulikakos (2014) Nat Rev Drug Disc 13(12): 928-942 doi: 10.103 8/nrd428). Single nucleotide substitutions that result in missense mutations at codons 12 and 13 of the KRas primary amino acid sequence comprise approximately 40% of these KRas driver mutations in lung adenocarcinoma, with a G12C transversion being the most common activating mutation (e.g., see Dogan et al., (2012) Clin Cancer Res. 18(22):6169-6177, published online 2012 Sep 26. doi: 10.1158/1078-0432.CCR-11-3265).

The well-known role of KRAs in malignancy and the discovery of these frequent mutations in KRas in various tumor types made KRas a highly attractable target of the pharmaceutical industry for cancer therapy. Notwithstanding thirty years of large scale discovery efforts to develop inhibitors of KRas for treating cancer, no KRas inhibitor has demonstrated sufficient safety and/or efficacy to obtain regulatory approval (e.g., see McCormick (2015) Clin Cancer Res. 21 (8):1797-1801).

Despite many failed efforts to target KRas, compounds that inhibit KRas activity are still highly desirable and under investigation, including those that disrupt effectors such as guanine nucleotide exchange factors (e.g., see Sun et al., (2012) Agnew Chem Int Ed Engl. 51(25):6140-6143 doi: 10.1002/anie201201358) as well target KRas G12C (e.g., see Ostrem et al., (2013) Nature 503:548-551). Clearly there remains a continued interest and effort to develop inhibitors of KRas, particularly inhibitors of activating KRas mutants, including KRas G12C.
WO 2017/201161 relates to compounds that inhibit KRas G12C, in particular compounds that irreversibly inhibit the activity of KRas G12C, pharmaceutical compositions comprising the compounds and methods of use therefor.
CN 112430234 relates to a KRAS G12C protein inhibitor, a preparation method and application thereof. The compound can be used as an efficient KRAS G12C protein inhibitor and has various pharmacological activities of resisting tumors, proliferative diseases, inflammation, autoimmune diseases and the like.

Thus, there is a need to develop new KRas G12C inhibitors that demonstrate sufficient efficacy, stability and/or safety for treating KRas G12C-mediated cancer. The compounds and compositions of the present invention advantageously overcome one or more of the previous shortcomings by providing selective KRas G12C inhibitors.

### SUMMARY OF THE INVENTION

In one aspect of the invention, compounds are provided that inhibit KRas G12C activity. In certain embodiments, the compounds are represented by formula (I): 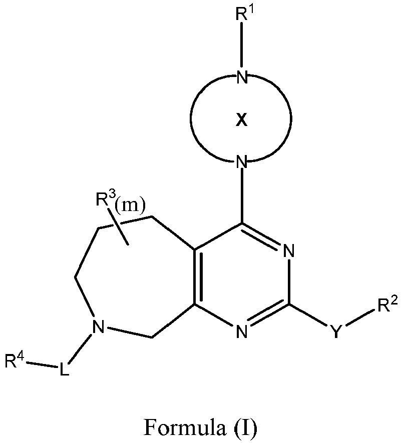 or a pharmaceutically acceptable salt thereof, wherein:
R¹-X is: 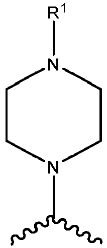 wherein the piperazinyl ring is optionally substituted with R⁸;
Y is a bond, O, S or NR⁵;
R¹ is -C(O)C(R^{A}) 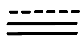 C(R^{B})ₚ or -SO₂C(R^{A}) 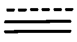 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
Z is C1 - C4 alkylene;
each R³ is independently C1 - C3 alkyl, oxo, or haloalkyl;
L is a bond, -C(O)-, or C1 - C3 alkylene;
R⁴ is aryl or heteroaryl, wherein the aryl or heteroaryl may be optionally substituted with one or more R⁶ or R⁷;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, -C(O)N(R⁵)₂,-N(R⁵)₂, wherein the C1 - C3 alkyl may be optionally substituted with cyano, halogen, -OR⁵,-N(R⁵)₂, or heteroaryl
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, -C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, -NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
m is 0, 1, 2 or 3;
p is one or two; and wherein,
when 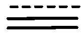 is a triple bond then R^{A} is absent, R^{B} is present and p equals one,
or when 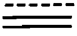 is a double bond then R^{A} is present, R^{B} is present and p equals two, or R^{A}, R^{B} and the carbon atoms to which they are attached form a 5-8 membered partially saturated cycloalkyl optionally substituted with one or more R⁷,
   wherein
   amino is -NH₂,
   acyl is -C(O)CH₃,
   alkyl is a straight or branched chain aliphatic group having from 1 to 12 carbon atoms,
   haloalkyl is an alkyl chain in which one or more hydrogens has been replaced by a halogen, alkoxy is -OC1 - C6 alkyl,
   cycloalkyl is a saturated or partially unsaturated cyclic hydrocarbon group having 3 to 12 carbon atoms,
   heteroalkyl is an alkyl group wherein one or more carbon atoms in the chain are replaced by a heteroatom selected from the group consisting of O, S, and N,
   hydroxyalkyl is -alkyl-OH,
   dihydroxyalkyl is an alkyl group wherein two carbon atoms are each substituted with a hydroxyl group,
   alkylaminylalkyl is -alkyl-NR^{x}-alkyl wherein R^{x} is hydrogen,
   dialkylaminyl is N(R^{y})₂ wherein each R^{y} is Cl - C3 alkyl,
   dialkylaminylalkyl is -alkyl-N(R^{y})₂ wherein each R^{y} is Cl - C4 alkyl,
   aryl is a C₆-C₁₄ aromatic moiety comprising one to three aromatic rings,
   aralkyl is a group comprising an aryl group covalently linked to an alkyl group,
   heterocyclyl is a ring structure having from 3 to 12 atoms wherein one or more atoms are selected from the group consisting of N, O, and S, the remainder of the ring atoms being carbon, and excluding compounds having adjacent annular O and/or S atoms,
   heterocyclylalkyl is a heterocyclyl group linked to the remaining portion of the molecule via an alkyl linker,
   heteroaryl is a group having 5 to 14 ring atoms, having 6, 10, or 14 p electrons shared in a cyclic array, and having, in addition to carbon atoms, from one to three heteroatoms per ring selected from the group consisting of N, O, and S, and
   heteroarylalkyl is a group comprising a heteroaryl group covalently linked to an alkyl group wherein the radical is on the alkyl group, and excluding compounds having adjacent annular O and/or S atoms.

Also included are compounds of Formula I having the Formula I-A: 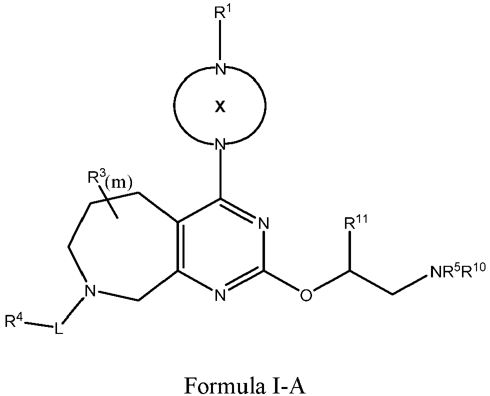 wherein R¹, R³, R⁴, R⁵, R¹⁰, Land m are as defined for Formula I, R¹¹ is hydrogen, C1 - C3 alkyl or hydroxyalkyl, and X is a piperazinyl ring which is optionally substituted with R⁸ wherein R⁸ is as defined for Formula I.

Also included are compounds of Formula I having the Formula I-B: 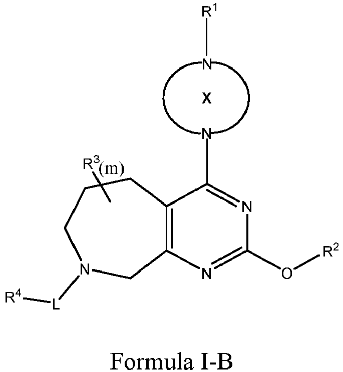 where R¹, R³, R⁴, R⁸, L and m are as defined for Formula I, R² is heterocyclylalkyl optionally substituted with one or more R⁹, and X is a piperazinyl ring which is optionally substituted with R⁸, where R⁸ is as defined for Formula I.

In another aspect of the invention, pharmaceutical compositions are provided comprising a therapeutically effective amount of a compound of the present invention or a pharmaceutically acceptable salt thereof and a pharmaceutically acceptable excipient.

In yet another aspect of the invention, provided herein is an in vitro method for inhibiting KRas G12C activity in a in a cell, comprising contacting the cell with a compound of Formula I, Formula I-A, Formula 1-B. In a further aspect of the invention, provided herein is a compound of Formula (I), Formula I-A or Formula I-B, pharmaceutically acceptable salts thereof or pharmaceutical compositions containing the compound of Formula (I), Formula I-A or Formula I-B, or pharmaceutically acceptable salt thereof for use in a method for inhibiting KRas G12C activity in a cell, comprising contacting the cell in which inhibition of KRas G12C activity is desired with an effective amount of the compound of Formula (I), Formula I-A or Formula I-B, pharmaceutically acceptable salts thereof or pharmaceutical compositions containing the compound of Formula (I), Formula I-A or Formula I-B, or pharmaceutically acceptable salt thereof

Also provided herein is an in vitro method of inhibiting cell proliferation, the method comprising contacting a cell with an effective amount of a compound of Formula I, Formula I-A, Formula 1-B, or a pharmaceutically acceptable salt thereof, or a pharmaceutical composition thereof as defined herein. Also provided herein is a compound of Formula I, Formula I-A, Formula 1-B, or a pharmaceutically acceptable salt thereof, or a pharmaceutical composition thereof as defined herein, for use in a method of inhibiting cell proliferation, the method comprising contacting a cell with an effective amount of the compound of Formula I, Formula I-A, Formula 1-B, or the pharmaceutically acceptable salt thereof, or the pharmaceutical composition thereof as defined herein.

Also provided is a compound of Formula (I), Formula I-A or Formula I-B, or a pharmaceutically acceptable salt thereof, alone or combined with a pharmaceutically acceptable carrier, excipient or diluents for use in a method for treating cancer in a patient comprising administering a therapeutically effective amount of a compound or pharmaceutical composition of the present invention or a pharmaceutically acceptable salt thereof to a patient in need thereof.

Also provided herein is a compound of Formula (I), Formula I-A or Formula I-B, or a pharmaceutically acceptable salt thereof, alone or combined with a pharmaceutically acceptable carrier, excipient or diluents for use in a method of treating a KRas G12C -associated disease or disorder in a patient in need of such treatment, the method comprising administering to the patient a therapeutically effective amount of a compound of Formula I, Formula I-A, Formula 1-B, or a pharmaceutically acceptable salt or solvate thereof, or a pharmaceutical composition thereof as defined herein.

Also provided herein is a compound of Formula I, Formula I-A, Formula 1-B, or a pharmaceutically acceptable salt or solvate thereof, or a pharmaceutical composition thereof as defined herein for use in therapy.

Also provided herein is a compound of Formula I, Formula I-A, Formula 1-B,or a pharmaceutically acceptable salt or solvate thereof or a pharmaceutical composition thereof as defined herein for use in the treatment of cancer.

Also provided herein is a compound of Formula I, Formula I-A, Formula 1-B,or a pharmaceutically acceptable salt or solvate thereof for use in the inhibition of KRas G12C.

Also provided herein is a compound of Formula I, Formula I-A, Formula 1-B, or a pharmaceutically acceptable salt or solvate thereof or a pharmaceutical composition thereof as defined herein, for use in the treatment of a KRas G12C-associated disease or disorder.

Also provided herein is the use of a compound of Formula I, Formula I-A, Formula 1-B, or a pharmaceutically acceptable salt or solvate thereof, as defined herein in the manufacture of a medicament for the treatment of cancer.

Also provided herein is a use of a compound of Formula I, Formula I-A, Formula 1-B, or a pharmaceutically acceptable salt or solvate thereof, as defined herein in the manufacture of a medicament for the inhibition of activity of KRas G12C.

Also provided herein is the use of a compound of Formula I, Formula I-A, Formula 1-B, or a pharmaceutically acceptable salt or solvate thereof, as defined herein, in the manufacture of a medicament for the treatment of a KRas G12C-associated disease or disorder.

Also provided herein is a compound of Formula (I), Formula I-A or Formula I-B or a pharmaceutically acceptable salt thereof, or a pharmaceutical composition thereof, for use in a method for treating cancer in a patient in need thereof, the method comprising (a) determining that the cancer is associated with a KRas G12C mutation (e.g., a KRas G12C-associated cancer); and (b) administering to the patient a therapeutically effective amount of a compound of Formula I, Formula I-A, Formula 1-B, or the pharmaceutically acceptable salt thereof, or the pharmaceutical composition thereof.

Also provided herein is a process for preparing a compound of Formula I, Formula I-A, Formula 1-B, or a pharmaceutically acceptable salt or solvate thereof.

Also provided herein is a compound of Formula I, Formula I-A, Formula 1-B, or a pharmaceutically acceptable salt thereof obtained by a process of preparing the compound as defined herein.

### DETAILED DESCRIPTION OF THE INVENTION

The present invention relates to inhibitors of KRas G12C. In particular, the present invention relates to compounds that irreversibly inhibit the activity of KRas G12C, pharmaceutical compositions comprising a therapeutically effective amount of the compounds, methods of use therefor and medical uses therefor.

### DEFINITIONS

Unless defined otherwise, all technical and scientific terms used herein have the same meaning as is commonly understood by one of skill in the art to which this invention belongs.

As used herein, "KRas G12C" refers to a mutant form of a mammalian KRas protein that contains an amino acid substitution of a cysteine for a glycine at amino acid position 12. The assignment of amino acid codon and residue positions for human KRas is based on the amino acid sequence identified by UniProtKB/Swiss-Prot P01116: Variant p.Gly12Cys.

As used herein, a "KRas G12C inhibitor" refers to compounds of the present invention that are represented by Formula (I) as described herein. These compounds are capable of negatively modulating or inhibiting all or a portion of the enzymatic activity of KRas G12C. The KRas G12C inhibitors of the present invention interact with and irreversibly bind to KRas G12C by forming a covalent adduct with the sulfhydryl side chain of the cysteine residue at position 12 resulting in the inhibition of the enzymatic activity of KRas G12C.

A "KRas G12C-associated disease or disorder" as used herein refers to diseases or disorders associated with or mediated by or having a KRas G12C mutation. A non-limiting example of a KRas G12C-associated disease or disorder is a KRas G12C-associated cancer.

As used herein, the term "subject," "individual," or "patient," used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the subject has been identified or diagnosed as having a cancer having a KRas G12C mutation (e.g., as determined using a regulatory agency-approved, e.g., FDA-approved, assay or kit). In some embodiments, the subject has a tumor that is positive for a KRas G12C mutation (e.g., as determined using a regulatory agency-approved assay or kit). The subject can be a subject with a tumor(s) that is positive for a KRas G12C mutation (e.g., identified as positive using a regulatory agency-approved, e.g., FDA-approved, assay or kit). The subject can be a subject whose tumors have a KRas G12C mutation (e.g., where the tumor is identified as such using a regulatory agency-approved, e.g., FDA-approved, kit or assay). In some embodiments, the subject is suspected of having a KRas G12C gene-associated cancer. In some embodiments, the subject has a clinical record indicating that the subject has a tumor that has a KRas G12C mutation (and optionally the clinical record indicates that the subject should be treated with any of the compositions provided herein).

In some embodiments of any of the methods or uses described herein, an assay is used to determine whether the patient has KRas G12C mutation using a sample (e.g., a biological sample or a biopsy sample (e.g., a paraffin-embedded biopsy sample) from a patient (e.g., a patient suspected of having a KRas G12C-associated cancer, a patient having one or more symptoms of a KRas G12C-associated cancer, and/or a patient that has an increased risk of developing a KRas G12C-associated cancer) can include, for example, next generation sequencing, immunohistochemistry, fluorescence microscopy, break apart FISH analysis, Southern blotting, Western blotting, FACS analysis, Northern blotting, and PCR-based amplification (e.g., RT-PCR and quantitative real-time RT-PCR). As is well-known in the art, the assays are typically performed, e.g., with at least one labelled nucleic acid probe or at least one labelled antibody or antigen-binding fragment thereof.

The term "regulatory agency" is a country's agency for the approval of the medical use of pharmaceutical agents with the country. For example, a non-limiting example of a regulatory agency is the U.S. Food and Drug Administration (PDA).

The term "amino" refers to -NH₂;

The term "acyl" refers to -C(O)CH₃.

The term "alkyl" as employed herein refers to straight and branched chain aliphatic groups having from 1 to 12 carbon atoms, 1-8 carbon atoms 1-6 carbon atoms, or 1-3 carbon atoms which is optionally substituted with one, two or three substituents. Examples of alkyl groups include, without limitation, methyl, ethyl, propyl, isopropyl, butyl, isobutyl, sec-butyl, tert-butyl, pentyl, and hexyl.

The term "haloalkyl" refers to an alkyl chain in which one or more hydrogen has been replaced by a halogen. Examples of haloalkyls are trifluoromethyl, difluoromethyl and fluoromethyl.

The term "haloalkyloxy" refers to -O-haloalkyl.

An "alkylene," group is an alkyl group, as defined hereinabove, that is positioned between and serves to connect two other chemical groups. Exemplary alkylene groups include, without limitation, methylene, ethylene, propylene, and butylene.

The term "alkoxy" refers to -OC1 - C6 alkyl.

The term "cycloalkyl" as employed herein includes saturated and partially unsaturated cyclic hydrocarbon groups having 3 to 12 carbons, for example 3 to 8 carbons, and as a further example 3 to 6 carbons, wherein the cycloalkyl group additionally is optionally substituted. Examples of cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroalkyl" refers to an alkyl group, as defined hereinabove, wherein one or more carbon atoms in the chain are replaced by a heteroatom selected from the group consisting of O, S, and N.

As used herein, the term "hydroxyalkyl" refers to -alkyl-OH.

The term "dihydroxyalkyl" refers to an alkyl group as defined herein wherein two carbon atoms are each substituted with a hydroxyl group.

The term "alkylaminyl" refers to -NR^{x}-alkyl, wherein R^{x} is hydrogen. In one embodiment, R^{x} is hydrogen.

The term "dialkylaminyl" refers to -N(R^{y})₂, wherein each R^{y} is C1 - C3 alkyl.

The term "alkylaminylalkyl" refers to -alkyl-NR^{x}-alkyl, wherein R^{x} is hydrogen. In one embodiment, R^{x} is hydrogen.

The term "dialkylaminylalkyl" refers to -alkyl-N(R^{y})₂, wherein each R^{y} is C1 - C4 alkyl, wherein the alkyl of the-alkyl-N(R^{y})₂ may be optionally substituted with hydroxy or hydroxy alkyl.

An "aryl" group is a C₆-C₁₄ aromatic moiety comprising one to three aromatic rings, which is optionally substituted. As one embodiment, the aryl group is a C₆-C₁₀ aryl group. Examples of aryl groups include, without limitation, phenyl, naphthyl, anthracenyl, fluorenyl, and dihydrobenzofuranyl.

An "aralkyl" or "arylalkyl" group comprises an aryl group covalently linked to an alkyl group, either of which may independently be optionally substituted or unsubstituted. An example of an aralkyl group is (C₁- C₆)alkyl(C₆-C₁₀)aryl, including, without limitation, benzyl, phenethyl, and naphthylmethyl. An example of a substituted aralkyl is wherein the alkyl group is substituted with hydroxyalkyl.

A "heterocyclyl" or "heterocyclic" group is a ring structure having from about 3 to about 12 atoms, for example 4 to 8 atoms, wherein one or more atoms are selected from the group consisting of N, O, and S, the remainder of the ring atoms being carbon. The heterocyclyl may be a monocyclic, a bicyclic, a spirocyclic or a bridged ring system. The heterocyclic group is optionally substituted with R⁷ on carbon or nitrogen at one or more positions, wherein R⁷ is as defined for Formula I. The heterocyclic group is also independently optionally substituted on nitrogen with alkyl, aryl, aralkyl, alkylcarbonyl, alkylsulfonyl, arylcarbonyl, arylsulfonyl, alkoxycarbonyl, aralkoxycarbonyl, or on sulfur with oxo or lower alkyl. Examples of heterocyclic groups include, without limitation, epoxy, azetidinyl, aziridinyl, tetrahydrofuranyl, tetrahydropyranyl, pyrrolidinyl, pyrrolidinonyl, piperidinyl, piperazinyl, imidazolidinyl, thiazolidinyl, dithianyl, trithianyl, dioxolanyl, oxazolidinyl, oxazolidinonyl, decahydroquinolinyl, piperidonyl, 4-piperidinonyl, thiomorpholinyl, thiomorpholinyl 1,1 dioxide, morpholinyl, oxazepanyl, azabicyclohexanes, azabicycloheptanes and oxa azabiocycloheptanes. Specifically excluded from the scope of this term are compounds having adjacent annular O and/or S atoms.

The term "heterocyclylalkyl" refers to a heterocyclyl group as defined herein linked to the remaining portion of the molecule via an alkyl linker, wherein the alkyl linker of the heterocyclylalkyl may be optionally substituted with hydroxy or hydroxyalkyl.

As used herein, the term "heteroaryl" refers to groups having 5 to 14 ring atoms, preferably 5, 6, 9, or 10 ring atoms; having 6, 10, or 14 π electrons shared in a cyclic array; and having, in addition to carbon atoms, from one to three heteroatoms per ring selected from the group consisting of N, O, and S. Examples of heteroaryl groups include acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, furanyl, furazanyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolinyl, 2H-pyrrolyl, pyrrolyl, quinazolinyl, quinolinyl, 4H-quinolizinyl, quinoxalinyl, quinuclidinyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

A "heteroarylalkyl" group comprises a heteroaryl group covalently linked to an alkyl group, wherein the radical is on the alkyl group, either of which is independently optionally substituted or unsubstituted. Examples of heteroarylalkyl groups include a heteroaryl group having 5, 6, 9, or 10 ring atoms bonded to a C1-C6 alkyl group. Examples of heteroaralkyl groups include pyridylmethyl, pyridylethyl, pyrrolylmethyl, pyrrolylethyl, imidazolylmethyl, imidazolylethyl, thiazolylmethyl, thiazolylethyl, benzimidazolylmethyl, benzimidazolylethyl quinazolinylmethyl, quinolinylmethyl, quinolinylethyl, benzofuranylmethyl, indolinylethyl isoquinolinylmethyl, isoinodylmethyl, cinnolinylmethyl, and benzothiophenylethyl. Specifically excluded from the scope of this term are compounds having adjacent annular O and/or S atoms.

As used herein, "an effective amount" of a compound is an amount that is sufficient to negatively modulate or inhibit the activity of KRas G12C. Such amount may be administered as a single dosage or may be administered according to a regimen, whereby it is effective.

As used herein, a "therapeutically effective amount" of a compound is an amount that is sufficient to ameliorate, or in some manner reduce a symptom or stop or reverse progression of a condition, or negatively modulate or inhibit the activity of KRas G12C. Such amount may be administered as a single dosage or may be administered according to a regimen, whereby it is effective.

As used herein, treatment means any manner in which the symptoms or pathology of a condition, disorder or disease are ameliorated or otherwise beneficially altered. Treatment also encompasses any pharmaceutical use of the compositions herein.

As used herein, amelioration of the symptoms of a particular disorder by administration of a particular pharmaceutical composition refers to any lessening, whether permanent or temporary, lasting or transient that can be attributed to or associated with administration of the composition.

### COMPOUNDS

In one aspect of the invention, compounds are provided represented by formula (I): 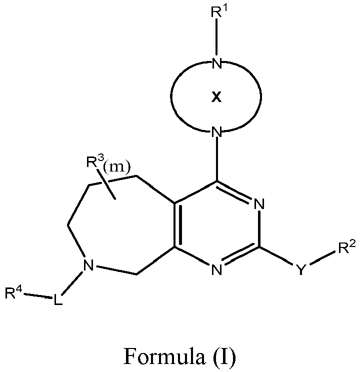 or a pharmaceutically acceptable salt thereof, wherein:
R¹-X is: 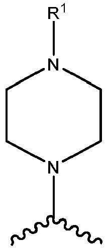 wherein the piperazinyl ring is optionally substituted with R⁸;
Y is a bond, O, S or NR⁵;
R¹ is -C(O)C(R^{A}) 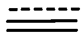 C(R^{B})ₚ or -SO₂C(R^{A}) 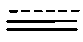 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
Z is C1 - C4 alkylene;
each R³ is independently C1 - C3 alkyl, halogen or -OR⁵;
L is a bond, -C(O)-, or C1 - C3 alkylene;
R⁴ is aryl or heteroaryl, wherein the aryl or heteroaryl may be optionally substituted with one or more R⁶ or R⁷;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, -C(O)N(R⁵)₂,-N(R⁵)₂, wherein the C1 - C3 alkyl may be optionally substituted with cyano, halogen, -OR⁵,-N(R⁵)₂, or heteroaryl;
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R^{A} is absent, hydrogen, or C1 - C3 alkyl;
each R^{B} is independently hydrogen, C1 - C3 alkyl, alkylaminylalkyl, dialkylaminylalkyl or heterocyclylalkyl;
m is 0, 1, 2 or 3;
p is one or two; and wherein,
when 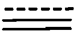 is a triple bond then R^{A} is absent, R^{B} is present and p equals one;
or when 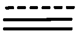 is a double bond then R^{A} is present, R^{B} is present and p equals two, or R^{A}, R^{B} and the carbon atoms to which they are attached form a 5-8 membered partially saturated cycloalkyl optionally substituted with one or more R⁷,
   wherein
   amino is -NH₂,
   acyl is -C(O)CH₃,
   alkyl is a straight or branched chain aliphatic group having from 1 to 12 carbon atoms,
   haloalkyl is an alkyl chain in which one or more hydrogens has been replaced by a halogen, alkoxy is -OC1 - C6 alkyl,
   cycloalkyl is a saturated or partially unsaturated cyclic hydrocarbon group having 3 to 12 carbon atoms,
   heteroalkyl is an alkyl group wherein one or more carbon atoms in the chain are replaced by a heteroatom selected from the group consisting of O, S, and N,
   hydroxyalkyl is -alkyl-OH,
   dihydroxyalkyl is an alkyl group wherein two carbon atoms are each substituted with a hydroxyl group,
   alkylaminylalkyl is -alkyl-NR^{x}-alkyl wherein R^{x} is hydrogen,
   dialkylaminyl is N(R^{y})₂ wherein each R^{y} is Cl - C3 alkyl,
   dialkylaminylalkyl is -alkyl-N(R^{y})₂ wherein each R^{y} is Cl - C4 alkyl,
   aryl is a C₆-C₁₄ aromatic moiety comprising one to three aromatic rings,
   aralkyl is a group comprising an aryl group covalently linked to an alkyl group,
   heterocyclyl is a ring structure having from 3 to 12 atoms wherein one or more atoms are selected from the group consisting of N, O, and S, the remainder of the ring atoms being carbon, and excluding compounds having adjacent annular O and/or S atoms,
   heterocyclylalkyl is a heterocyclyl group linked to the remaining portion of the molecule via an alkyl linker,
   heteroaryl is a group having 5 to 14 ring atoms, having 6, 10, or 14 p electrons shared in a cyclic array, and having, in addition to carbon atoms, from one to three heteroatoms per ring selected from the group consisting of N, O, and S, and
   heteroarylalkyl is a group comprising a heteroaryl group covalently linked to an alkyl group wherein the radical is on the alkyl group, and excluding compounds having adjacent annular O and/or S atoms.

In the present invention, R¹-X is: 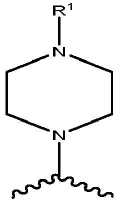 wherein R¹ is as defined for Formula I and the piperazinyl ring is optionally substituted with R⁸, where R⁸ is as defined for Formula I. In certain embodiments, R⁸ is C1 - C3 alkyl wherein the alkyl is optionally substituted with cyano or OR⁵, or -C(O)N(R⁵)₂, wherein each R⁵ is independently hydrogen or C1 - C3 alkyl.

In particular embodiments, R¹ is -C(O)C(R^{A}) 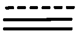 C(R^{B})ₚ where R^{A}, R^{B} and p are as defined for Formula I. In one embodiment, R¹ is -C(O)C(R^{A}) 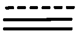 C(R^{B})ₚ, wherein 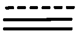 is a triple bond and R^{A} is absent, p is one and R^{B} is hydroxyalkyl.

In one embodiment, R¹ is -C(O)C(R^{A}) 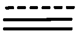 C(R^{B})ₚ, wherein 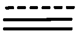 is a double bond and R^{A} is hydrogen or C1 - C3 alkyl, p is two and at least one R^{B} is deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂,-NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷. In one embodiment, when 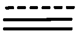 is a double bond, the double bond is in the E configuration. In one embodiment, the double bond is in the Z configuration.

In certain embodiments, one R^{B} is heterocyclylalkyl substituted with one or more substituents independently selected from halogen, hydroxyl, alkoxy or C1 - C3 alkyl and the other R^{B} is hydrogen. In one embodiment, the heterocyclyl portion of the heterocyclylalkyl is azetidinyl substituted with a halogen. In certain embodiments, the halogen is fluorine. In one embodiment, the heterocyclyl portion of the heterocyclylalkyl is pyrrolidinyl substituted with one or more halogen. In certain embodiments, the halogen-substituted pyrrolidinyl is fluoropyrrolidinyl or difluorpyrrolidinyl.

In certain embodiments, one R^{B} is halogen and the other R^{B} is hydrogen. In one embodiment, the halogen is chlorine.

In certain embodiments, one R^{B} is haloalkyl and the other R^{B} is hydrogen. In one embodiment, the haloalkyl is chloromethyl, fluoromethyl, difluoromethyl or trifluoromethyl.

In certain embodiments, one R^{B} is heteroalkyl and the other R^{B} is hydrogen. In one embodiment, the heteroalkyl is methoxymethyl.

In ceratin embodiments, one R^{B} is -ZNR⁵R¹¹, wherein Z is methylene, R⁵ is methyl and R¹¹ is trifluoromethyl or 2,2,2-trifluoroethyl, and the other R^{B} is hydrogen.

In certain embodiments, one R^{B} is hydroxyalkyl and the other R^{B} is hydrogen.

In certain embodiments, one R^{B} is heteroaryl optionally substituted with one or more R⁷ and the other R^{B} is hydrogen. In one embodiment, the heteroaryl is pyrrolyl, imidazolyl, pyrazolyl, triazolyl, pyridyl, pyridazinyl, pyrimidinyl, pyrazinyl or triazinyl, each substituted with one or more R⁷.

In certain embodiments, one R^{B} is heteroarylalkyl optionally substituted with one or more R⁷, and the other R^{B} is hydrogen. In one embodiment, the heteroaryl portion of the heteroarylalkyl is pyrrolyl, imidazolyl, pyrazolyl, triazolyl, pyridyl, pyridazinyl, pyrimidinyl, pyrazinyl or triazinyl, each optionally substituted with one or more R⁷. In one embodiment, the one or more R⁷ is C1 - C3 alkyl.

In certain embodiments, one R^{B} is -C(O)N(R⁵)₂ and the other R^{B} is hydrogen. In one embodiment, each R⁵ is hydrogen. In one embodiment, each R⁵ is C1 - C3 alkyl.

In certain embodiments, one R^{B} is -NHC(O)C1 - C3 alkyl or -CH₂NHC(O)C1 - C3 alkyl and the other R^{B} is hydrogen. In one embodiment, the C1 - C3 alkyl is methyl.

In one embodiment, R¹ is -C(O)C(R^{A}) =C(R^{B})ₚ, wherein R^{A} is deuterium, cyano, halogen, C1 - C-3 alkyl, haloalkyl, heteroalkyl, -C(O)N(R⁵)₂, or hydroxyalkyl, p is two, each R^{B} is hydrogen. In one embodiment, R^{A} is halogen. In one embodiment, the halogen is fluorine or chlorine. In one embodiment, R^{A} is haloalkyl. In one embodiment, the haloalkyl is trifluoromethyl. In one embodiment, R^{A} is cyano. In one embodiment, R^{A} is heteroalkyl. In one embodiment, the heteroalkyl is methoxy. In one embodiment, R^{A} is hydroxyalkyl.

In one embodiment, R¹ is -C(O)C(R^{A}) 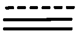 C(R^{B})ₚ, wherein 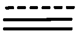 is a double bond and R^{A} is deuterium, p is two and at least one R^{B} is deuterium.

In one embodiment, R¹ is -C(O)C(R^{A}) 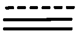 C(R^{B})ₚ, wherein 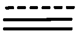 is a double bond and p is two, one R^{B} is hydrogen and R^{A} and one R^{B} and the carbon atoms to which they are attached form a 5-8 membered partially saturated cycloalkyl substituted with oxo.

In one embodiment, R¹ is -C(O)C(R^{A}) 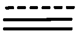 C(R^{B})ₚ, wherein 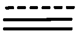 is a double bond and p is two, one R^{B} is hydrogen, the second R^{B} is dialkylaminylalkyl, and R^{A} is halogen.

In one embodiment, Y is O or NR⁵ and R² is selected from the group consisting of alkyl, hydroxyalkyl, dihydroxyalkyl, alkylaminylalkyl, dialkylaminylalkyl, heterocyclyl, heterocyclylalkyl, and heteroaryl. In one embodiment, Y is O and R² is hydroxyalkyl, dihydroxyalkyl, alkylaminylalkyl, or dialkylaminylalkyl, wherein the alkylaminylalkyl or dialkylaminylalkyl is optionally substituted with one or more R⁹. In one embodiment, the optionally substituted alkylaminylalkyl or dialkylaminylalkyl is independently selected from methylaminylpropan-2-yl, dimethylaminylethyl, methylethylaminylethyl, dimethylaminylpropanyl, dimethylaminylpropan-2-yl, dimethylaminylbutanyl, dimethylaminylbutan-2-yl, 2-dimethylaminylpropanol, or diethylaminylethyl. In one embodiment, Y is O or NR⁵ and R² is heterocyclyl or heterocyclylalkyl optionally substituted with one or more R⁹. Nonlimiting examples of one or more R⁹ when R² is heterocyclyl or heterocyclylalkyl include C1 - C3 alkyl, acyl, oxo, cyano, alkoxy, cycloalkyl, cycloalkylmethyl, halogen, and hydroxyl. Nonlimiting examples of R² heterocyclyls optionally substituted with one or more R⁹ include azetidinyl, C1-C3alkyl-substituted azetidinyl (e.g., methylazetidinyl), halo-substituted azetidinyl (e.g., difluoroazetidinyl), tetrahydropyran, pyrrolidinyl, C1-C3 alkyl-substituted pyrrolidinyl (e.g., methylpyrrolidinyl, dimethylpyrrolidinyl, and isopropylpyrrolidinyl), cycloalkylalkylpyrrolidinyl, hydroxypyrrolindinyl, halo-substituted pyrrolidinyl (e.g., fluoropyrrolidinyl and difluoropyrrolidinyl), methoxyethylpyrrolidinyl, (N-methyl)methoxypyrrolidinyl, piperazinyl, dimethylaminylpyrrolidinyl, morpholinyl, methylmorpholinyl, 1,4-oxazepanyl, piperdinyl, C1-C3 alkyl-substituted piperidinyl (e.g., methylpiperidinyl), acylpiperdinyl, cyanopiperdinyl, cycloalkylpiperdinyl, halopiperdinyl (e.g., fluoropiperdinyl), dihalopiperdinyl (e.g., difluoropiperdinyl), alkoxypiperdinyl, pyrrolidonyl, piperidonyl, thiomorpholinyl-1,1-dioxide, 3-azabicyclo[3.1.0]hexanyl, oxa-5-azabicyclo[2.2.1]heptan-5-yl, and azabicyclo[2.2.1]heptan-2-yl.

In one embodiment, Y is O and R² is heteroarylalkyl optionally substituted with one or more R⁹. In one embodiment, the heteroaryl portion of the heteroarylalkyl is pyridinyl.

In one embodiment, Y is O and R² is -ZR⁵R¹⁰. In one embodiment, R⁵ is C1 - C3 alkyl and R¹⁰ is independently selected from acyl, hydroxyalkyl or alkoxy.

In one embodiment, Y is a bond and R² is hydrogen, heterocyclyl or aryl, wherein said heterocyclyl and aryl are optionally substituted with one or more R⁹.

In one embodiment, Y is a bond and R² is hydrogen.

In one embodiment, Y is a bond and R² is heterocyclyl optionally substituted with one or more R⁹. In one embodiment, Y is a bond and R² is heterocyclyl optionally substituted with methyl, halogen or dimethylamino. Nonlimiting examples of R² heterocyclyls include azetidinyl, piperidinyl, piperazinyl, morpholinyl, and pyrrolidinyl.

In one embodiment, Y is a bond and R² is aryl optionally substituted with one or more R⁹. In one embodiment, the aryl is phenyl substituted with heterocyclylalkyl.

In certain other embodiments, R⁴ is aryl. In one embodiment, R⁴ is selected from the group consisting of phenyl and naphthyl and is optionally substituted with one or more R⁶ or R⁷. Examples of R⁷ substituents include halogen, hydroxyl, C1- C6 alkyl (e.g., C1- C3 alkyl), cycloalkyl, haloalkyl, Q-haloalkyl, amino, cyano, hydroxyalkyl and alkoxy. In one embodiment, the aryl is phenyl substituted with one or more R⁷ groups independently selected from halogen, hydroxyl, C1- C3 alkyl, haloalkyl, Q-haloalkyl, and alkoxy. In one embodiment, the aryl is phenyl substituted with one or more R⁷ groups independently selected from halogen, haloalkyl, methyl, isopropyl, methoxy, Q-haloalkyl and hydroxyl. In one embodiment, the aryl is phenyl substituted with one or more R⁷ groups independently selected from methyl, trifluoromethyl, 2,2,2-trifluoroethyl, hydroxyl, trifluoromethoxy, hydroxyl, fluoro, chloro, isopropyl, cyclopropyl and trifluoromethylthio. In one embodiment, the aryl is phenyl substituted with one to three R⁷ groups independently selected from hydroxyl, fluorine and chlorine. In one embodiment, the aryl is phenyl substituted with hydroxyl and C1 - C3 alkyl or two C1 - C3 alkyl. In one embodiment, the aryl is phenyl substituted with Q-haloalkyl and hydroxyl or fluorine.

In one embodiment, R⁴ is aryl wherein aryl is naphthyl optionally substituted with one or more R⁷. In one embodiment, the aryl is naphthyl substituted with one or more R⁷ groups independently selected from halogen, hydroxyl, C1- C3 alkyl, haloalkyl, hydroxyalkyl, Q-haloalkyl, and alkoxy. In one embodiment, the aryl is naphthyl substituted with one or more R⁷ groups independently selected from halogen, haloalkyl, methyl, isopropyl, methoxy, Q-haloalkyl, hydroxymethyl and hydroxyl. In one embodiment, R⁴ is naphthyl optionally substituted with one or more R⁷ substituents independently selected from halogen, C1 - C3 alkyl, haloalkyl and hydroxyalkyl. In one embodiment, R⁴ is naphthyl optionally substituted with one to three R⁷ substituents independently selected from methyl, isopropyl, chloro, fluoro, and trifluoromethyl.

In one embodiment, the aryl is naphthyl optionally substituted with one or more halogen. In one embodiment, the aryl is naphthyl substituted with hydroxyl and trifluoromethyl or C1 - C3alkyl. In one embodiment, the aryl is naphthyl substituted with hydroxyl.

In one embodiment, R⁴ is heteroaryl optionally substituted with one or more R⁷. In one embodiment, R⁴ is heteroaryl optionally substituted with one or more R⁷ independently selected from halogen, hydroxyl, C1- C3 alkyl, haloalkyl, Q-haloalkyl, alkoxy and amino. In one embodiments, R⁴ is indoyl, indazolyl, quinolinyl, isoquinolinyl, pyridinyl or benzo[d]thiazolyl optionally substituted with one or more R⁷. In one embodiment, R⁴ is indoyl, indazolyl, quinolinyl, isoquinolinyl, pyridinyl or benzo[d]thiazolyl optionally substituted with one or more R⁷ independently selected from halogen, hydroxyl, C1- C3 alkyl, haloalkyl, Q-haloalkyl, alkoxy and amino. In one embodiment, R⁴ is indazolyl or quinolinyl optionally substituted with C1-C3 alkyl.

In yet other embodiments, R⁴ is heteroaryl, optionally an indoyl or an indazolyl, each of which may be substituted with one or more R⁷. In one embodiment, R⁴ is heteroaryl optionally substituted with one or more R⁷ substituents independently selected from the group consisting of halogen, hydroxyl, C1- C3 alkyl, haloalkyl, Q-haloalkyl and alkoxy. In one embodiment, the R⁴ heteroaryl is indazolyl optionally substituted with one or two R⁷ independently selected from alkoxy, haloalkyl, and C1-C6 alkyl. In other embodiments, the R⁴ heteroaryl is a quinolinyl or isoquinolinyl, each optionally substituted with one or more R⁷. In one embodiment, the R⁴ heteroaryl is a quinolinyl or isoquinolinyl, each optionally substituted with one or more R⁷ independently selected from amino, hydroxyl, C1 - C3 alkyl, and hydroxyl. In one embodiment, the R⁴ heteroaryl is a quinolinyl or isoquinolinyl, each optionally substituted with R⁷ selected from hydroxyl and amino. In one embodiment, the R⁴ heteroaryl is a pyridinyl optionally substituted with one or more R⁷. In one embodiment, the R⁴ heteroaryl is pyridinyl optionally substituted with one or more R⁷ independently selected from C1 - C3 alkyl, halogen and haloalkyl. In other embodiments, the R⁴ heteroaryl is benzo[d]thiazolyl optionally substituted with one or more R⁷, such as hydroxyl, one or two C1 - C3 alkyl, or hydroxyl and one or two C1 - C3 alkyl. In one embodiment, the R⁴ heteroaryl is indolyl optionally substituted with one or more R⁷. In one embodiment, the R⁴ heteroaryl is indolyl optionally substituted with one or two R⁷ independently selected from hydroxyl and C1 - C3alkyl.

In one embodiment, L is a bond.

In one embodiment, R³ is C1 - C3 alkyl. In one embodiment, the C1 - C3 alkyl is methyl.

In one embodiment, R³ is halogen. In one embodiment, the halogen is fluorine or chlorine.

In one embodiment, R⁸ is heteroalkyl, C2-C4 alkynyl or C1 - C3 alkyl optionally substituted with -OR⁵, cyano or heteroaryl. In one embodiment, R⁸ is methyl, cyanomethyl, methoxymethyl, hydroxymethyl. In one embodiment, R⁸ is methyl. In one embodiment, R⁸ is cyanomethyl. In one embodiment, R⁸ is hydroxymethyl.

In one embodiment, Formula I includes compounds having the Formula I-A: 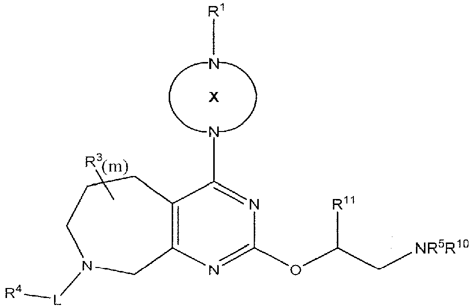 wherein R¹, R³, R⁴, R⁵, R¹⁰, L and m are as defined for Formula I, R¹¹ is hydrogen, methyl or hydroxyalkyl, and X is a piperazinyl ring which is optionally substituted with R⁸ wherein R⁸ is as defined for Formula I. In one embodiment, L is a bond. In one embodiment, R⁴ is aryl or heteroaryl, each of which is optionally substituted with one or more R⁶ or R⁷. In one embodiment, R⁴ is aryl or heteroaryl, each of which is optionally substituted with one or more R⁷. In one embodiment, each R⁷ is independently selected from hydroxyl, amino, halogen, C1-C3 alkyl, haloalkyl, Q-haloalkyl, cycloalkyl and alkoxy. In one embodiment, R⁵ and R¹⁰ are each C1 - C3 alkyl. In one embodiment, the aryl is phenyl substituted with one or more R⁷ groups independently selected from halogen, hydroxyl, C1 - C3 alkyl, haloalkyl, Q-haloalkyl, and alkoxy. In one embodiment, the aryl is phenyl substituted with one or more R⁷ groups independently selected from halogen, haloalkyl, methyl, isopropyl, methoxy, Q-haloalkyl and hydroxyl. In one embodiment, the aryl is phenyl substituted with one or more R⁷ groups independently selected from methyl, trifluoromethyl, 2,2,2-trifluoroethyl, hydroxyl, trifluoromethoxy, hydroxyl, fluoro, chloro, isopropyl, cyclopropyl and trifluoromethylthio. In one embodiment, the aryl is phenyl substituted with one to three R⁷ groups independently selected from hydroxyl, fluorine and chlorine. In one embodiment, the aryl is phenyl substituted with hydroxyl and C1 - C3 alkyl or two C1 - C3 alkyl. In one embodiment, the aryl is phenyl substituted with Q-haloalkyl and hydroxyl or fluorine. In one embodiment, the aryl is naphthyl substituted with one or more R⁷ groups independently selected from halogen, hydroxyl, C1- C3 alkyl, haloalkyl, Q-haloalkyl, and alkoxy. In one embodiment, the aryl is naphthyl substituted with one or more R⁷ groups independently selected from halogen, haloalkyl, methyl, isopropyl, methoxy, Q-haloalkyl and hydroxyl. In one embodiment, R⁴ is naphthyl optionally substituted with one or more R⁷ substituents independently selected from hydroxyl, halogen, C1 - C3 alkyl, amino, and haloalkyl. In one embodiment, R⁴ is naphthyl optionally substituted with one to three R⁷ substituents independently selected from difluoromethyl, methyl, hydroxyl, amino, fluoro, and chloro. In one embodiment, the aryl is naphthyl optionally substituted with one or more halogen. In one embodiment, the aryl is naphthyl substituted with hydroxyl and trifluoromethyl or C1 - C3alkyl. In one embodiment, the aryl is naphthyl substituted with hydroxyl. In one embodiment, R⁴ is heteroaryl, wherein the heteroaryl is indazolyl optionally substituted with one or two R⁷ independently selected from alkoxy, haloalkyl, and C1-C6 alkyl. In one embodiment, R⁴ is heteroaryl, wherein the heteroaryl is quinolinyl or isoquinolinyl, each optionally substituted with one or more R⁷. In one embodiment, R⁴ is heteroaryl, wherein the heteroaryl is quinolinyl or isoquinolinyl, each optionally substituted with one or more R⁷ independently selected from amino, hydroxyl, C1 - C3alkyl, and hydroxyl. In one embodiment, the R⁴ heteroaryl is a pyridinyl optionally substituted with one or more R⁷. In one embodiment, the R⁴ heteroaryl is pyridinyl optionally substituted with one or more R⁷ independently selected from C1 - C3 alkyl, halogen and haloalkyl. In one embodiment, the R⁴ heteroaryl is benzo[d]thiazolyl optionally substituted with one or more R⁷, such as hydroxyl, one or two C1 - C3 alkyl, or hydroxyl and one or two C1 - C3 alkyl. In one embodiment, the R⁴ heteroaryl is indolyl optionally substituted with one or more R⁷. In one embodiment, the R⁴ heteroaryl is indolyl optionally substituted with one or two R⁷ independently selected from hydroxyl and C1 - C3alkyl. In one embodiment, R¹¹ is methyl. In one embodiment, the piperazinyl ring is unsubstituted. In one embodiment, the piperazinyl ring is substituted with R⁸. In one embodiment, R⁸ is C1 - C3 alkyl optionally substituted with cyano or hydroxyl. In one embodiment, R⁸ is methyl, cyanomethyl or hydroxymethyl. In one embodiment, R⁸ is methyl. In one embodiment, R⁸ is cyanomethyl. In one embodiment, R⁸ is hydroxymethyl. In another embodiment, R⁵ and R¹⁰ are each C1 - C3 alkyl, R¹¹ is methyl, R⁸ is methyl, cyanomethyl or hydroxymethyl, L is a bond, and R⁴ is aryl or heteroaryl, each optionally substituted with one or more R⁶ or R⁷.

In particular embodiments, R¹ is -C(O)C(R^{A}) 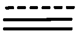 C(R^{B})ₚ where R^{A}, R^{B} and p are as defined for Formula I. In one embodiment, R¹ is -C(O)C(R^{A}) 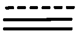 C(R^{B})ₚ, wherein 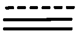 is a triple bond and R^{A} is absent, p is one and R^{B} is hydroxyalkyl.

In one embodiment, R¹ is -C(O)C(R^{A}) 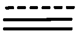 C(R^{B})ₚ, wherein 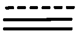 is a double bond and R^{A} is hydrogen or C1 - C3 alkyl, p is two and at least one R^{B} is deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂,-NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷. In one embodiment, when 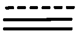 is a double bond, the double bond is in the E configuration. In one embodiment, the double bond is in the Z configuration.

In certain embodiments, one R^{B} is heterocyclylalkyl substituted with one or more substituents independently selected from halogen, hydroxyl, alkoxy or C1 - C3 alkyl and the other R^{B} is hydrogen. In one embodiment, the heterocyclyl portion of the heterocyclylalkyl is azetidinyl substituted with a halogen. In certain embodiments, the halogen is fluorine. In one embodiment, the heterocyclyl portion of the heterocyclylalkyl is pyrrolidinyl substituted with one or more halogen. In certain embodiments, the halogen-substituted pyrrolidinyl is fluoropyrrolidinyl or difluorpyrrolidinyl.

In certain embodiments, one R^{B} is halogen and the other R^{B} is hydrogen. In one embodiment, the halogen is chlorine.

In certain embodiments, one R^{B} is haloalkyl and the other R^{B} is hydrogen. In one embodiment, the haloalkyl is chloromethyl, fluoromethyl, difluoromethyl or trifluoromethyl.

In certain embodiments, one R^{B} is heteroalkyl and the other R^{B} is hydrogen. In one embodiment, the heteroalkyl is methoxymethyl.

In ceratin embodiments, one R^{B} is -ZNR⁵R¹¹, wherein Z is methylene, R⁵ is methyl and R¹¹ is trifluoromethyl or 2,2,2-trifluoroethyl, and the other R^{B} is hydrogen.

In certain embodiments, one R^{B} is hydroxyalkyl and the other R^{B} is hydrogen.

In certain embodiments, one R^{B} is heteroaryl optionally substituted with one or more R⁷ and the other R^{B} is hydrogen. In one embodiment, the heteroaryl is pyrrolyl, imidazolyl, pyrazolyl, triazolyl, pyridyl, pyridazinyl, pyrimidinyl, pyrazinyl or triazinyl, each substituted with one or more R⁷.

In certain embodiments, one R^{B} is heteroarylalkyl optionally substituted with one or more R⁷, and the other R^{B} is hydrogen. In one embodiment, the heteroaryl portion of the heteroarylalkyl is pyrrolyl, imidazolyl, pyrazolyl, triazolyl, pyridyl, pyridazinyl, pyrimidinyl, pyrazinyl or triazinyl, each optionally substituted with one or more R⁷. In one embodiment, the one or more R⁷ is C1 - C3 alkyl.

In certain embodiments, one R^{B} is -C(O)N(R⁵)₂ and the other R^{B} is hydrogen. In one embodiment, each R⁵ is hydrogen. In one embodiment, each R⁵ is C1 - C3 alkyl.

In certain embodiments, one R^{B} is -NHC(O)C1 - C3 alkyl or -CH₂NHC(O)C1 - C3 alkyl and the other R^{B} is hydrogen. In one embodiment, the C1 - C3 alkyl is methyl.

In one embodiment, R¹ is -C(O)C(R^{A}) =C(R^{B})ₚ, wherein R^{A} is deuterium, cyano, halogen, C1 - C-3 alkyl, haloalkyl, heteroalkyl, -C(O)N(R⁵)₂, or hydroxyalkyl, p is two, each R^{B} is hydrogen. In one embodiment, R^{A} is halogen. In one embodiment, the halogen is fluorine or chlorine. In one embodiment, R^{A} is haloalkyl. In one embodiment, the haloalkyl is trifluoromethyl. In one embodiment, R^{A} is cyano. In one embodiment, R^{A} is heteroalkyl. In one embodiment, the heteroalkyl is methoxy. In one embodiment, R^{A} is hydroxyalkyl.

In one embodiment, R¹ is -C(O)C(R^{A}) 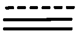 C(R^{B})ₚ, wherein 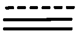 is a double bond and R^{A} is deuterium, p is two and at least one R^{B} is deuterium.

In one embodiment, R¹ is -C(O)C(R^{A}) 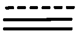 C(R^{B})ₚ, wherein 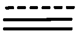 is a double bond and p is two, one R^{B} is hydrogen and R^{A} and one R^{B} and the carbon atoms to which they are attached form a 5-8 membered partially saturated cycloalkyl substituted with oxo.

In one embodiment, R¹ is -C(O)C(R^{A}) 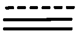 C(R^{B})ₚ, wherein 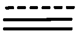 is a double bond and p is two, one R^{B} is hydrogen, the second R^{B} is dialkylaminylalkyl, and R^{A} is halogen.

In one embodiment, Formula I includes compounds having the Formula I-B: 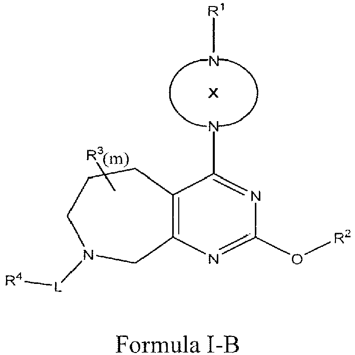 and R¹, R³, R⁴, R⁹, L and m are as defined for Formula I, R² is heterocyclylalkyl optionally substituted with one or more R⁹, and X is a piperazinyl ring which is optionally substituted with R⁸, where R⁸ is as defined for Formula I. In one embodiment, the heterocyclyl portion of the R² heterocyclylalkyl is a monocyclic, bicyclic, or bridged ring system having one or two ring heteroatoms independently selected from N and O. In one embodiment, R² heterocyclyl is pyrrolidinyl, piperidinyl, piperazinyl, morpholinyl, 1,4-oxazepanyl, thiomorpholinyl-1,1-dioxide, 3-azabicyclo[3.1.0]hexanyl, 2-oxa-5-azabicyclo[2.2.1]heptan-5-yl, and azabicyclo[2.2.1]heptan-2-yl, optionally substituted with one or more R⁹. In one embodiment, each R⁹ is selected from acyl, oxo, halogen, cyano, C1 - C3 alkyl, alkoxy, hydroxyalkyl, heteroalkyl, cycloalkyl, aralkyl and dialkylamidoalkyl. In one embodiment, L is a bond. In one embodiment, R⁴ is aryl or heteroaryl, each of which is optionally substituted with one or more R⁶ or R⁷. In one embodiment, R⁴ is aryl or heteroaryl, each of which is optionally substituted with one or more R⁷. In one embodiment, each R⁷ is independently selected from hydroxyl, amino, halogen, C1-C3 alkyl, haloalkyl, Q-haloalkyl, cycloalkyl and alkoxy. In one embodiment, the aryl is phenyl substituted with one or more R⁷ groups independently selected from halogen, hydroxyl, C1- C3 alkyl, haloalkyl, Q-haloalkyl, and alkoxy. In one embodiment, the aryl is phenyl substituted with one or more R⁷ groups independently selected from halogen, haloalkyl, methyl, isopropyl, methoxy, Q-haloalkyl and hydroxyl. In one embodiment, the aryl is phenyl substituted with one or more R⁷ groups independently selected from methyl, trifluoromethyl, 2,2,2-trifluoroethyl, hydroxyl, trifluoromethoxy, hydroxyl, fluoro, chloro, isopropyl, cyclopropyl and trifluoromethylthio. In one embodiment, the aryl is phenyl substituted with one to three R⁷ groups independently selected from hydroxyl, fluorine and chlorine. In one embodiment, the aryl is phenyl substituted with hydroxyl and C1 - C3 alkyl or two C1 - C3 alkyl. In one embodiment, the aryl is phenyl substituted with Q-haloalkyl and hydroxyl or fluorine. In one embodiment, the aryl is naphthyl substituted with one or more R⁷ groups independently selected from halogen, hydroxyl, C1- C3 alkyl, haloalkyl, Q-haloalkyl, and alkoxy. In one embodiment, the aryl is naphthyl substituted with one or more R⁷ groups independently selected from halogen, haloalkyl, methyl, isopropyl, methoxy, Q-haloalkyl and hydroxyl. In one embodiment, R⁴ is naphthyl optionally substituted with one or more R⁷ substituents independently selected from hydroxyl, halogen, C1 - C3 alkyl, amino, and haloalkyl. In one embodiment, R⁴ is naphthyl optionally substituted with one to three R⁷ substituents independently selected from difluoromethyl, methyl, hydroxyl, amino, fluoro, and chloro. In one embodiment, the aryl is naphthyl optionally substituted with one or more halogen. In one embodiment, the aryl is naphthyl substituted with hydroxyl and trifluoromethyl or C1 - C3alkyl. In one embodiment, the aryl is naphthyl substituted with hydroxyl. In one embodiment, R⁴ is heteroaryl, wherein the heteroaryl is indazolyl optionally substituted with one or two R⁷ independently selected from alkoxy, haloalkyl, and C1-C6 alkyl. In one embodiment, R⁴ is heteroaryl, wherein the heteroaryl is quinolinyl or isoquinolinyl, each optionally substituted with one or more R⁷. In one embodiment, R⁴ is heteroaryl, wherein the heteroaryl is quinolinyl or isoquinolinyl, each optionally substituted with one or more R⁷ independently selected from amino, hydroxyl, C1-C3 alkyl, and hydroxyl. In one embodiment, the R⁴ heteroaryl is a pyridinyl optionally substituted with one or more R⁷. In one embodiment, the R⁴ heteroaryl is pyridinyl optionally substituted with one or more R⁷ independently selected from C1 - C3 alkyl, halogen and haloalkyl. In one embodiment, the R⁴ heteroaryl is benzo[d]thiazolyl optionally substituted with one or more R⁷, such as hydroxyl, one or two C1 - C3 alkyl, or hydroxyl and one or two C1 - C3 alkyl. In one embodiment, the R⁴ heteroaryl is indolyl optionally substituted with one or more R⁷. In one embodiment, the R⁴ heteroaryl is indolyl optionally substituted with one or two R⁷ independently selected from hydroxyl and C1 - C3 alkyl. In one embodiment, R¹¹ is methyl. In one embodiment, the piperazinyl ring is unsubstituted. In one embodiment, the piperazinyl ring is substituted with R⁸. In one embodiment, the piperazinyl ring is unsubstituted. In one embodiment, the piperazinyl ring is substituted with R⁸. In one embodiment, R⁸ is C1 - C3 alkyl optionally substituted with cyano, hydroxyl or methoxy. In one embodiment, R⁸ is methyl, cyanomethyl, hydroxymethyl or methoxymethyl.

In particular embodiments, R¹ is -C(O)C(R^{A}) 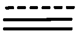 C(R^{B})ₚ where R^{A}, R^{B} and p are as defined for Formula I. In one embodiment, R¹ is -C(O)C(R^{A}) 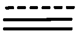 C(R^{B})ₚ, wherein 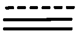 is a triple bond and R^{A} is absent, p is one and R^{B} is hydroxyalkyl.

In one embodiment, R¹ is -C(O)C(R^{A}) 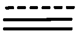 C(R^{B})ₚ, wherein 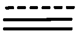 is a double bond and R^{A} is hydrogen or C1 - C3 alkyl, p is two and at least one R^{B} is deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂,-NHC(O)C1 - C3 alkyl, -CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷. In one embodiment, when 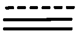 is a double bond, the double bond is in the E configuration. In one embodiment, the double bond is in the Z configuration.

In certain embodiments, one R^{B} is heterocyclylalkyl substituted with one or more substituents independently selected from halogen, hydroxyl, alkoxy or C1 - C3 alkyl and the other R^{B} is hydrogen. In one embodiment, the heterocyclyl portion of the heterocyclylalkyl is azetidinyl substituted with a halogen. In certain embodiments, the halogen is fluorine. In one embodiment, the heterocyclyl portion of the heterocyclylalkyl is pyrrolidinyl substituted with one or more halogen. In certain embodiments, the halogen-substituted pyrrolidinyl is fluoropyrrolidinyl or difluorpyrrolidinyl.

In certain embodiments, one R^{B} is halogen and the other R^{B} is hydrogen. In one embodiment, the halogen is chlorine.

In certain embodiments, one R^{B} is haloalkyl and the other R^{B} is hydrogen. In one embodiment, the haloalkyl is chloromethyl, fluoromethyl, difluoromethyl or trifluoromethyl.

In certain embodiments, one R^{B} is heteroalkyl and the other R^{B} is hydrogen. In one embodiment, the heteroalkyl is methoxymethyl.

In ceratin embodiments, one R^{B} is -ZNR⁵R¹¹, wherein Z is methylene, R⁵ is methyl and R¹¹ is trifluoromethyl or 2,2,2-trifluoroethyl, and the other R^{B} is hydrogen.

In certain embodiments, one R^{B} is hydroxyalkyl and the other R^{B} is hydrogen.

In certain embodiments, one R^{B} is heteroaryl optionally substituted with one or more R⁷ and the other R^{B} is hydrogen. In one embodiment, the heteroaryl is pyrrolyl, imidazolyl, pyrazolyl, triazolyl, pyridyl, pyridazinyl, pyrimidinyl, pyrazinyl or triazinyl, each substituted with one or more R⁷.

In certain embodiments, one R^{B} is heteroarylalkyl optionally substituted with one or more R⁷, and the other R^{B} is hydrogen. In one embodiment, the heteroaryl portion of the heteroarylalkyl is pyrrolyl, imidazolyl, pyrazolyl, triazolyl, pyridyl, pyridazinyl, pyrimidinyl, pyrazinyl or triazinyl, each optionally substituted with one or more R⁷. In one embodiment, the one or more R⁷ is C1 - C3 alkyl.

In certain embodiments, one R^{B} is -C(O)N(R⁵)₂ and the other R^{B} is hydrogen. In one embodiment, each R⁵ is hydrogen. In one embodiment, each R⁵ is C1 - C3 alkyl.

In certain embodiments, one R^{B} is -NHC(O)C1 - C3 alkyl or -CH₂NHC(O)C1 - C3 alkyl and the other R^{B} is hydrogen. In one embodiment, the C1 - C3 alkyl is methyl.

In one embodiment, R¹ is -C(O)C(R^{A}) =C(R^{B})ₚ, wherein R^{A} is deuterium, cyano, halogen, C1 - C-3 alkyl, haloalkyl, heteroalkyl, -C(O)N(R⁵)₂, or hydroxyalkyl, p is two, each R^{B} is hydrogen. In one embodiment, R^{A} is halogen. In one embodiment, the halogen is fluorine or chlorine. In one embodiment, R^{A} is haloalkyl. In one embodiment, the haloalkyl is trifluoromethyl. In one embodiment, R^{A} is cyano. In one embodiment, R^{A} is heteroalkyl. In one embodiment, the heteroalkyl is methoxy. In one embodiment, R^{A} is hydroxyalkyl.

In one embodiment, R¹ is -C(O)C(R^{A}) 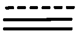 C(R^{B})ₚ, wherein 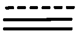 is a double bond and R^{A} is deuterium, p is two and at least one R^{B} is deuterium.

In one embodiment, R¹ is -C(O)C(R^{A}) 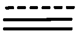 C(R^{B})ₚ, wherein 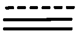 is a double bond and p is two, one R^{B} is hydrogen and R^{A} and one R^{B} and the carbon atoms to which they are attached form a 5-8 membered partially saturated cycloalkyl substituted with oxo.

In one embodiment, R¹ is -C(O)C(R^{A}) 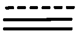 C(R^{B})ₚ, wherein 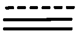 is a double bond and p is two, one R^{B} is hydrogen, the second R^{B} is dialkylaminylalkyl, and R^{A} is halogen.

In one embodiment of Formula I, R² is selected from the group consisting of hydroxyalkyl, dialkylaminylalkyl, heterocyclyl and heterocyclylalkyl, wherein each of the heterocyclyl or heterocyclylalkyl are independently optionally substituted with R⁹. In another embodiment, R² is heterocyclyl and heterocyclylalkyl, wherein each of the heterocyclyl or heterocyclylalkyl are independently optionally substituted with one or more R⁹. In certain embodiments, R² is dialkylaminylalkyl optionally substituted with one or more R⁹. Non-limiting examples include dimethylaminylethyl, dimethylaminylpropanyl, dimethylaminylpropan-2-yl, dimethylaminylbutanyl, dimethylaminylbutan-2-yl, 2-dimethylaminylpropanol, or diethylaminylethyl.

In one embodiment, Y is O and R² is selected from the group consisting of hydroxyalkyl, dialkylaminylalkyl, heterocyclyl, heterocyclylalkyl, and -ZR⁵R¹⁰, wherein R⁵ and R¹⁰ are as defined for Formula I.

In one embodiment, Y is O and R² is selected from the group consisting of hydroxyalkyl, dialkylaminylalkyl, heterocyclyl and heterocyclylalkyl, wherein each of the heterocyclyl or heterocyclylalkyl are independently optionally substituted with R⁹. In another embodiment, R² is heterocyclyl and heterocyclylalkyl, wherein each of the heterocyclyl or heterocyclylalkyl are independently optionally substituted with one or more R⁹. Non-limiting examples of R⁹ include acyl, oxo, halogen, cyano, C1 - C6 alkyl, alkoxy, hydroxyalkyl, heteroalkyl, cycloalkyl, aralkyl or dialkylamidoalkyl. In certain embodiments, R² is dialkylaminylalkyl optionally substituted with one or more R⁹. Non-limiting examples include dimethylaminylethyl, dimethylaminylpropanyl, dimethylaminylpropan-2-yl, dimethylaminylbutanyl, dimethylaminylbutan-2-yl, 2-dimethylaminylpropanol, or diethylaminylethyl.

In one embodiment of Formula I, R⁴ is aryl optionally substituted with one or more R⁶ or R⁷. In one embodiment, R⁴ is phenyl or naphthyl optionally substituted with one or more R⁶ or R⁷. In one embodiment, R⁴ is phenyl or naphthyl optionally substituted with one or more R⁷. In one embodiment, R⁴ is phenyl or naphthyl optionally substituted with one or more R⁷ substituents independently selected from halogen, hydroxyl, C1 - C3alkyl, cycloalkyl, alkoxy, haloalkyl, or Q-haloalkyl wherein Q is O or S. In one embodiment, R⁴ is phenyl or naphthyl optionally substituted with one or more R⁷ substituents independently selected from methyl, trifluoromethyl, hydroxyl, trifluoromethoxy, hydroxyl, fluoro, chloro, isopropyl, cyclopropyl and methylthio.

In one embodiment, R⁴ is isoquinolinyl which is optionally substituted with amino.

Nonlimiting examples of compounds of Formula (I), Formula I-A and Formula I-B are selected from the group consisting of: 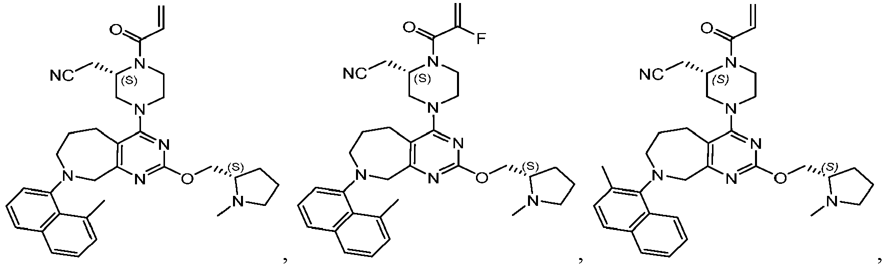 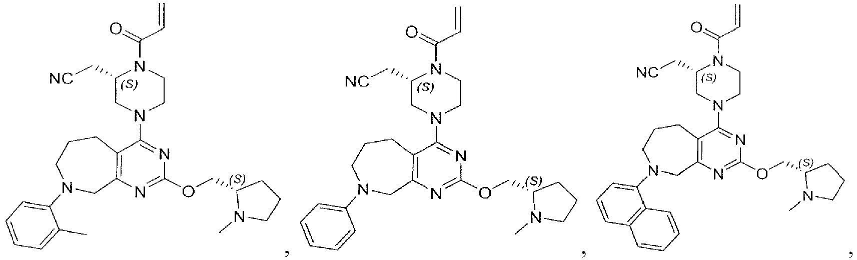 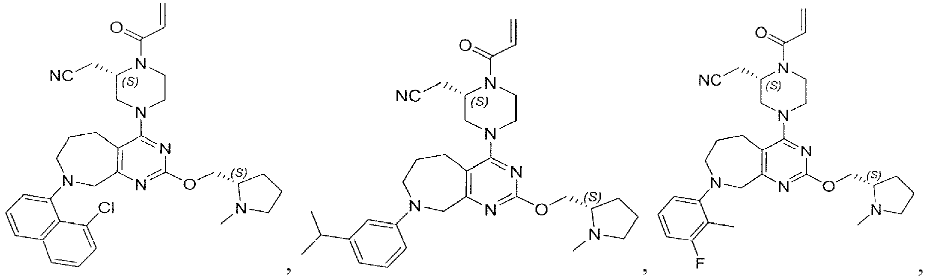 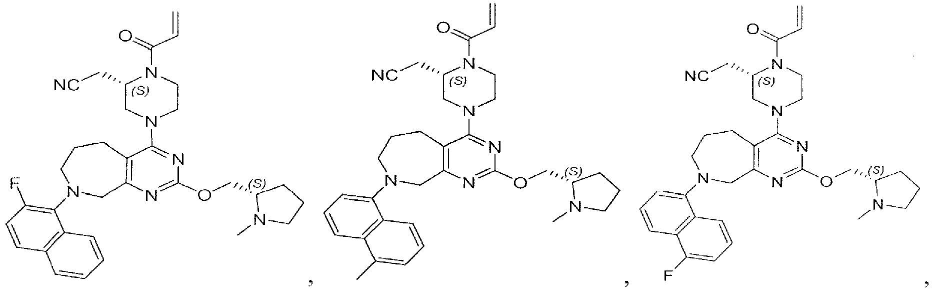 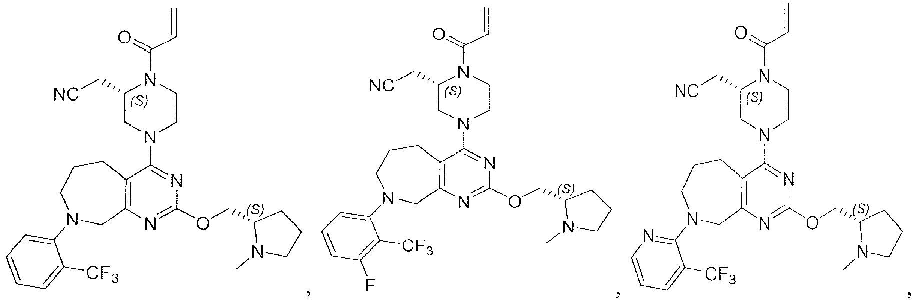 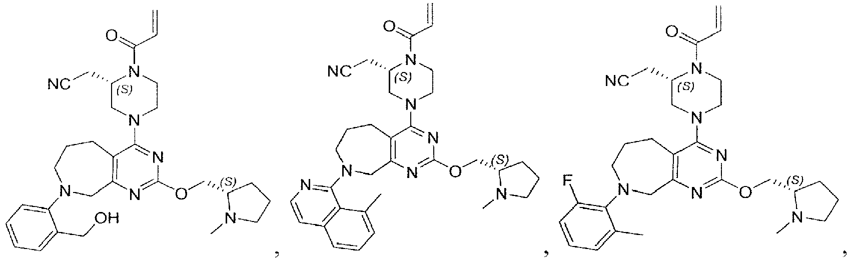 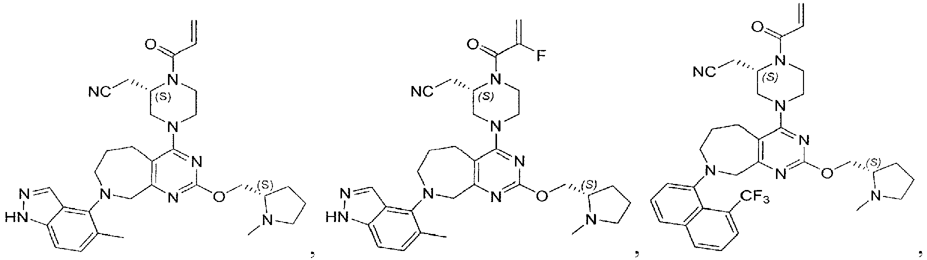 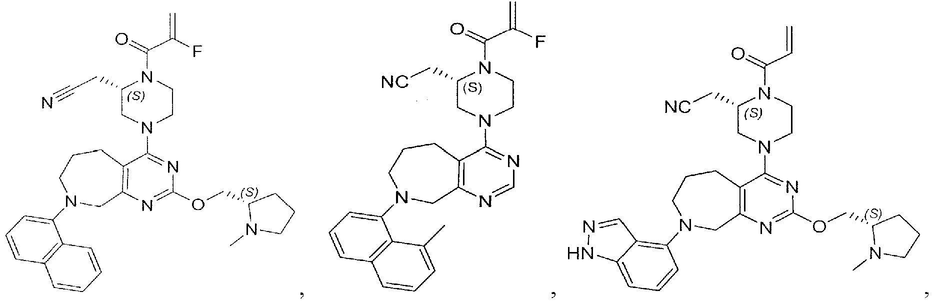 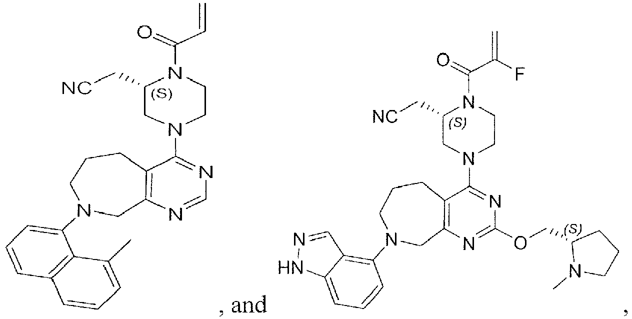 and pharmaceutically acceptable salts thereof.

In one embodiment, the compounds of Formula I include trifluoroacetic acid salts of the above compounds. The compounds of Formula (I), Formula I-A, Formula I-B, may be formulated into pharmaceutical compositions.

### PHARMACEUTICAL COMPOSITIONS

In another aspect, the invention provides pharmaceutical compositions comprising a KRas G12C inhibitor according to the invention and a pharmaceutically acceptable carrier, excipient, or diluent. Compounds of the invention may be formulated by any method well known in the art and may be prepared for administration by any route, including, without limitation, parenteral, oral, sublingual, transdermal, topical, intranasal, intratracheal, or intrarectal. In certain embodiments, compounds of the invention are administered intravenously in a hospital setting. In one embodiment, administration may be by the oral route.

The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, compositions according to the invention may contain, in addition to the inhibitor, diluents, fillers, salts, buffers, stabilizers, solubilizers, and other materials well known in the art. The preparation of pharmaceutically acceptable formulations is described in, e.g., Remington's Pharmaceutical Sciences, 18th Edition, ed. A. Gennaro, Mack Publishing Co., Easton, Pa., 1990.

As used herein, the term pharmaceutically acceptable salt refers to salts that retain the desired biological activity of the above-identified compounds and exhibit minimal or no undesired toxicological effects. Examples of such salts include, but are not limited to acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. The compounds can also be administered as pharmaceutically acceptable quaternary salts known by those skilled in the art, which specifically include the quaternary ammonium salt of the formula --NR+Z-, wherein R is hydrogen, alkyl, or benzyl, and Z is a counterion, including chloride, bromide, iodide, --O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The active compound is included in the pharmaceutically acceptable carrier or diluent in an amount sufficient to deliver to a patient a therapeutically effective amount without causing serious toxic effects in the patient treated. In one embodiment, a dose of the active compound for all of the above-mentioned conditions is in the range from about 0.01 to 300 mg/kg, for example 0.1 to 100 mg/kg per day, and as a further example 0.5 to about 25 mg per kilogram body weight of the recipient per day. A typical topical dosage will range from 0.01-3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

The pharmaceutical compositions comprising compounds of the present invention may be used in the methods of use described herein.

### METHODS OF USE AND MEDICAL USES

In yet another aspect, the invention provides for an in vitro method for inhibiting KRas G12C activity in a cell, comprising contacting the cell in which inhibition of KRas G12C activity is desired with an effective amount of a compound of Formula (I), Formula I-A, or Formula I-B, pharmaceutically acceptable salts thereof or pharmaceutical compositions containing the compound or pharmaceutically acceptable salt thereof. In a further aspect, the invention provides a compound of Formula (I), Formula I-A or Formula I-B, pharmaceutically acceptable salts thereof or pharmaceutical compositions containing the compound of Formula (I), Formula I-A or Formula I-B, or pharmaceutically acceptable salt thereof for use in a method for inhibiting KRas G12C activity in a cell, comprising contacting the cell in which inhibition of KRas G12C activity is desired with an effective amount of the compound of Formula (I), Formula I-A or Formula I-B, pharmaceutically acceptable salts thereof or pharmaceutical compositions containing the compound of Formula (I), Formula I-A or Formula I-B, or pharmaceutically acceptable salt thereof.

As used herein, the term "contacting" refers to the bringing together of indicated moieties in an in vitro system or an in vivo system. For example, "contacting" a KRas G12C with a compound provided herein includes the administration of a compound provided herein to an individual or patient, such as a human, having KRas G12C, as well as, for example, introducing a compound provided herein into a sample containing a cellular or purified preparation containing the KRas G12C.

In one embodiment, a cell in which inhibition of KRas G12C activity is desired is contacted with an effective amount of a compound of Formula (I), Formula I-A, or Formula I-B, to negatively modulate the activity of KRas G12C. In other embodiments, a therapeutically effective amount of pharmaceutically acceptable salt or pharmaceutical compositions containing the compound of Formula (I), Formula I-A, or Formula I-B, may be used.

By negatively modulating the activity of KRas G12C, the methods and compounds for use described herein are designed to inhibit undesired cellular proliferation resulting from enhanced KRas G12C activity within the cell. The cells may be contacted in a single dose or multiple doses in accordance with a particular treatment regimen to effect the desired negative modulation of KRas G12C. The degree of covalent modification of KRas G12C may be monitored in vitro using well known methods, including those described in Example A below. In addition, the inhibitory activity of exemplary compounds in cells may be monitored, for example, by measuring the inhibition of KRas G12C activity of the amount of phosphylated ERK, including those described in Example B below, to assess the effectiveness of treatment and dosages may be adjusted accordingly by the attending medical practitioner.

In another aspect, a compound of Formula (I), Formula I-A or Formula I-B, or a pharmaceutically acceptable salt thereof, alone or combined with a pharmaceutically acceptable carrier, excipient or diluents for use in a method of treating cancer in a patient in need thereof, comprising administering to said patient a therapeutically effective amount of the compound of Formula (I), Formula I-A, or Formula I-B, pharmaceutically acceptable salts thereof or pharmaceutical compositions comprising the compound or pharmaceutically acceptable salts thereof are provided.

The compositions provided herein may be used for the treatment of a KRas G12C-associated cancer in a patient in need thereof, comprising administering to said patient a therapeutically effective amount of a compound of Formula (I), Formula I-A, or Formula I-B, pharmaceutically acceptable salts thereof or pharmaceutical compositions comprising the compound or pharmaceutically acceptable salts thereof. In one embodiment, the KRas G12C-associated cancer is lung cancer.

The compositions provided herein may be used for the treatment of a wide variety of cancers including tumors such as lung, prostate, breast, brain, skin, cervical carcinomas, testicular carcinomas, etc. More particularly, cancers that may be treated by the compositions and methods of the invention include, but are not limited to tumor types such as astrocytic, breast, cervical, colorectal, endometrial, esophageal, gastric, head and neck, hepatocellular, laryngeal, lung, oral, ovarian, prostate and thyroid carcinomas and sarcomas. More specifically, these compounds can be used to treat: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma. In certain embodiments, the cancer is non-small cell lung cancer.

The concentration and route of administration to the patient will vary depending on the cancer to be treated. The compounds, pharmaceutically acceptable salts thereof and pharmaceutical compositions comprising such compounds and salts also may be co-administered with other anti-neoplastic compounds, e.g., chemotherapy, or used in combination with other treatments, such as radiation or surgical intervention, either as an adjuvant prior to surgery or post-operatively.

Also provided herein is a compound of Formula I, Formula I-A, Formula 1-B, or a pharmaceutically acceptable salt or solvate thereof, or a pharmaceutical composition thereof as defined herein for use in therapy.

Also provided herein is a compound of Formula I, Formula I-A, Formula 1-B, or a pharmaceutically acceptable salt or solvate thereof or a pharmaceutical composition thereof as defined herein for use in the treatment of cancer.

Also provided herein is a compound of Formula I, Formula I-A, Formula 1-B, or a pharmaceutically acceptable salt or solvate thereof for use in the inhibition of KRas G12C.

Also provided herein is a compound of Formula I, Formula I-A, Formula 1-B, or a pharmaceutically acceptable salt or solvate thereof or a pharmaceutical composition thereof as defined herein, for use in the treatment of a KRas G12C-associated disease or disorder.

Also provided herein is the use of a compound of Formula I, Formula I-A, Formula 1-B, or a pharmaceutically acceptable salt or solvate thereof, as defined herein in the manufacture of a medicament for the treatment of cancer.

Also provided herein is a use of a compound of Formula I, Formula I-A, Formula 1-B, or a pharmaceutically acceptable salt or solvate thereof, as defined herein in the manufacture of a medicament for the inhibition of activity of KRas G12C.

Also provided herein is the use of a compound of Formula I, Formula I-A, Formula 1-B, or a pharmaceutically acceptable salt or solvate thereof, as defined herein, in the manufacture of a medicament for the treatment of a KRas G12C-associated disease or disorder.

Also provided herein is a compound of Formula (I), Formula I-A or Formula I-B or a pharmaceutically acceptable salt thereof, or a pharmaceutical composition thereof, for use in a method for treating cancer in a patient in need thereof, the method comprising (a) determining that cancer is associated with a KRas G12C mutation (e.g., a KRas G12C-associated cancer) (e.g., as determined using a regulatory agency-approved, e.g., FDA-approved, assay or kit); and (b) administering to the patient a therapeutically effective amount of the compound of Formula I, Formula I-A, Formula 1-B, or the pharmaceutically acceptable salt thereof, or the pharmaceutical composition thereof.

One skilled in the art will recognize that, both in vivo and in vitro trials using suitable, known and generally accepted cell and/or animal models are predictive of the ability of a test compound to treat or prevent a given disorder.

One skilled in the art will further recognize that human clinical trials including first-inhuman, dose ranging and efficacy trials, in healthy patients and/or those suffering from a given disorder, may be completed according to methods well known in the clinical and medical arts.

### REACTION SCHEMES AND EXAMPLES

The compounds of the present invention may be prepared from commercially available reagents using the synthetic methods and reaction schemes described herein, or using other reagents and conventional methods well known to those skilled in the art.

For instance, compounds of the present invention may be prepared according to the General Reaction Schemes I & II.

### GENERAL REACTION SCHEMES

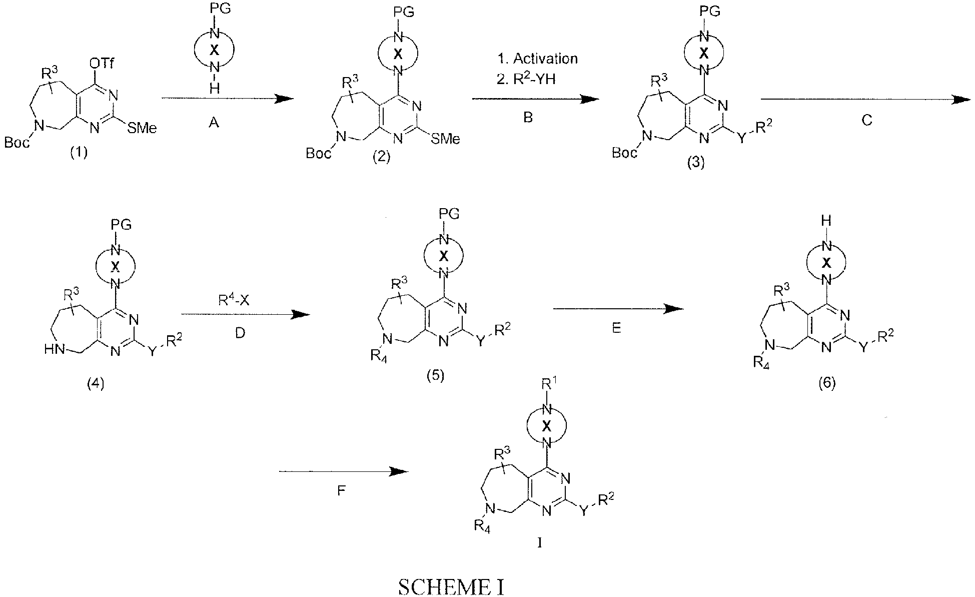

Compounds of Formula I wherein L is a bond, -Y-R² is other than hydrogen and R⁴ is aryl or heteroaryl can be prepared according to Scheme I. In step A, an appropriately functionalized dihydropyridopyrimidine (6) is coupled to a heterocycle containing one nucleophilic amine species, with the other bound to a protecting group to provide compound (7). This coupling proceeds in a solvent such as dimethylacetamide in the presence of a base such as triethylamine or Hunig's base. In step B, the substituent -Y-R² is introduced by substitution of the activated sulfoxide by a nucleophile, for example (S)-1-(dimethylamino-propan-2-ol in a polar solvent such as dioxane to provide compound (8). In step C, the Boc group is removed using conditions known in the art, for example with trifluoroacetic acid in a solvent such as dichloromethane to provide compound (9). In step D, the substituent R⁴ is introduced with a palladium coupling, using a suitable functionalized aryl or heteroaryl system, for example an aryl triflate, in the presence of a palladium catalyst such as Pd₂DBA₃/BINAP in a solvent such as toluene with a base such as sodium tert-butoxide to provide compound (10). In step E, the protecting group of ring X is removed, for example hydrogenolysis by Pd/C in the presence of H₂ in a polar solvent such as EtOH/THF to provide compound (11). In step F, R¹ is introduced to provide a compound of Formula I, for example by treating with an acid chloride having the formula C1-C(O)C(R^{A}) 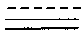 C(R^{B})ₚ or C1-SO₂C(R^{A}) 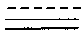 C(R^{B})ₚ, or an anhydride having the formula C(R^{B})ₚ 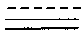 C(R^{A})C(O)OC(O)C(R^{A}) 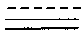 C(R^{B})ₚ, where R^{A}, R^{B} and p are as defined for Formula I. For example, in the case where R¹ is an acryloyl group, this reaction proceeds, for example, in a solvent such as methylene chloride in the presence of acryloyl chloride acryloyl anhydride and a base such as Hunig's base. In some cases, the species R⁴ and R² may also contain protecting groups, which can be removed at a subsequent step in the synthetic sequence.

Compounds (1), (2), (3), (4), (5) and (6) as shown and described above for Scheme I are useful as intermediates for preparing compounds of Formula I, Formula I-A or Formula I-B and are provided as further aspects of the invention.

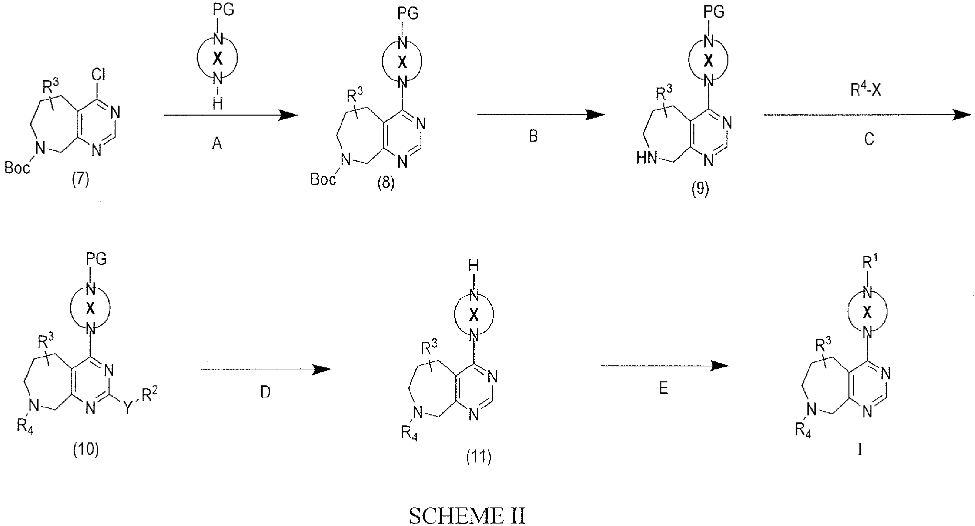

Compounds of Formula I wherein L and Y are bonds, R² is hydrogen and R⁴ is aryl or heteroaryl can be prepared according to Scheme II. In step A, an appropriately functionalized bicycle (1) is coupled to a heterocycle containing one nucleophilic amine species, with the other bound to a protecting group to provide compound (2). This coupling proceeds in a solvent such as dichloromethane in the presence of a base such as triethylamine or Hunig's base. In step B, the Boc group of compound (2) is removed using conditions known in the art, for example with trifluoroacetic acid in a solvent such as dichloromethane, to provide compound (3). In step C, the substituent R⁴ is introduced with a palladium coupling, using a suitable functionalized aryl or heteroaryl system, for example an aryl triflate, in the presence of a palladium catalyst such as Pd₂DBA₃/Xantphos in a solvent such as toluene with a base such as sodium tert-butoxide to provide compound (4). In step D, the protecting group of ring X compound (4) is removed, for example hydrogenolysis by Pd/C in the presence of H₂ in a polar solvent such as EtOH/THF to provide compound (5). In the final step, E, R¹ is introduced to provide a compound of Formula 1, for example by treating with an acid chloride having the formula C1-C(O)C(R^{A}) 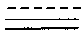 C(R^{B})ₚ or C1-SO₂C(R^{A}) 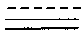 C(R^{B})ₚ, or an anhydride having the formula C(R^{B})ₚ 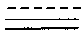 C(R^{A})C(O)OC(O)C(R^{A}) 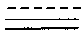 C(R^{B})ₚ, where R^{A}, R^{B} and p are as defined for Formula I. For example, in the case where R¹ is an acryloyl group, this reaction proceeds, for example, in a solvent such as methylene chloride in the presence of acryloyl chloride or an acryloyl anhydride and a base such as Hunig's base. In some cases, the species R⁴ will also contain a protecting group, which can be removed at a subsequent step in the synthetic sequence.

Compounds (7), (8), (9), (10) and (11) as shown and described above for Scheme II are useful as intermediates for preparing compounds of Formula I and are provided as further aspects of the invention.

Accordingly, also provide is a process for preparing a compound of Formula I, comprising: for a compound of Formula I where -Y-R² is other than hydrogen, reacting a compound of formula 5 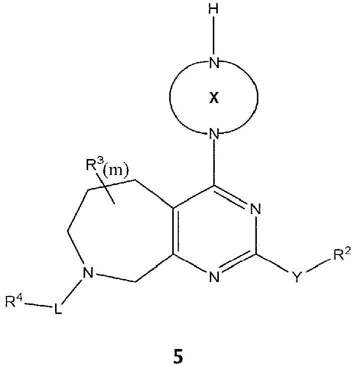
where X, R³, R⁴, L and m are as defined for Formula I with an acid chloride having the formula C1-C(O)C(R^{A}) 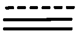 C(R^{B})ₚ or C1-SO₂C(R^{A}) 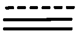 C(R^{B})ₚ or an anhydride having the formula C(R^{B})ₚ 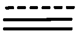 C(R^{A})C(O)OC(O)C(R^{A}) 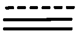 C(R^{B})ₚ, where R^{A}, R^{B} and p are as defined for Formula I, in the presence of a base; and
optionally forming a salt thereof.

The compounds of the present invention may have one or more chiral center and may be synthesized as stereoisomeric mixtures, isomers of identical constitution that differ in the arrangement of their atoms in space. The compounds may be used as mixtures or the individual components/isomers may be separated using commercially available reagents and conventional methods for isolation of stereoisomers and enantiomers well-known to those skilled in the art, e.g., using CHIRALPAK^{®} (Sigma-Aldrich) or CHIRALCEL^{®} (Diacel Corp) chiral chromatographic HPLC columns according to the manufacturer's instructions. Alternatively, compounds of the present invention may be synthesized using optically pure, chiral reagents and intermediates to prepare individual isomers or enantiomers. Unless otherwise indicated, all chiral (enantiomeric and diastereomeric) and racemic forms are within the scope of the invention. Unless otherwise indicated, whenever the specification, including the claims, refers to compounds of the invention, the term "compound" is to be understood to encompass all chiral (enantiomeric and diastereomeric) and racemic forms.

The following Examples are intended to illustrate further certain embodiments of the invention and are not intended to limit the scope of the invention.

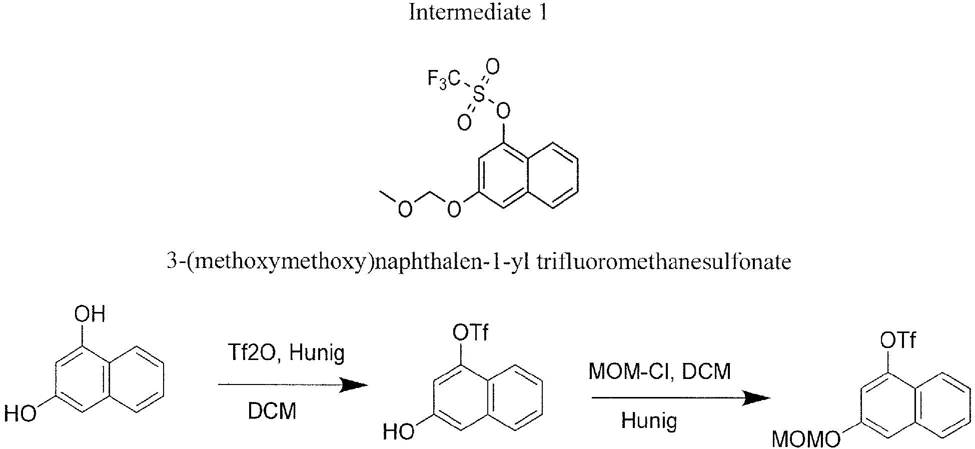

3-Hydroxynaphthalen-1-yl trifluoromethanesulfonate (13.101 g, 44.831 mmol) was dissolved in dichloromethane (100 mL) and stirred at 0 °C. To this solution was added chloro(methoxy)methane (3.7456 ml, 49.315 mmol) and Hunig's base (11.745 mL, 67.247 mmol). The reaction was stirred at 0 °C for 4 hrs. The reaction was partitioned with 1M HCl and washed with saturated sodium bicarbonate. The combined organic layers were dried over magnesium sulfate and concentrated under vacuum. The concentrated material was loaded onto a 120 g RediSep^{®} gold silica gel column with dichloromethane and purified by normal phase chromatography (CombiFlash^{®}, 0%-20% ethyl acetate/hexanes as the eluent) to give 3-(methoxymethoxy)naphthalen-1-yl trifluoromethanesulfonate (11.785 g, 35.045 mmol, 78.171 % yield).

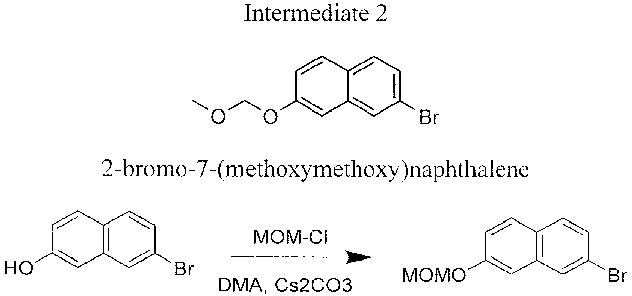

To a solution of 7-bromonaphthalen-2-ol (2.0 g, 9.0 mmol) in dimethyl acetamide (40 mL) was added chloro(methoxy)methane (1.4 g, 18 mmol) and cesium carbonate (5.8 g, 18 mmol) and the reaction mixture was stirred overnight at room temperature. The reaction was diluted with water and the aqueous layer washed with ethyl acetate. The combined organic layers were washed with water and brine, dried over magnesium sulfate and concentrated under vacuum. The crude material was purified by normal phase chromatography using 5-50% ethyl acetate/hexanes as the eluent to give 2-bromo-7-(methoxymethoxy)naphthalene (1.0 g, 3.7 mmol, 42 % yield).

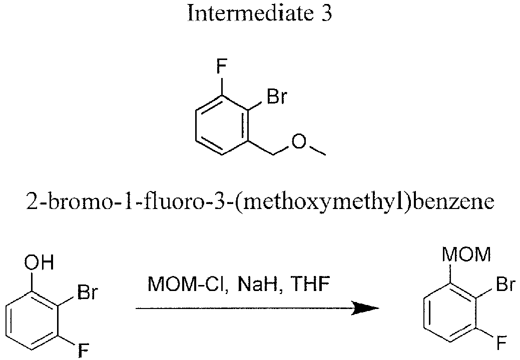

To a stirred solution of 2-bromo-3-fluorophenol (1422 mg, 7.445 mmol) in 22 mL tetrahydrofuran at room temperature under nitrogen was added NaH (327.6 mg, 8.190 mmol) neat as a solid portion wise. After 15 minutes, a solution had formed. Chloro(methoxy)methane (678.6 µL, 8.934 mmol) was added by syringe. After stirring for 2 hours, the reaction was quenched with saturated ammonium chloride solution and then partitioned between ethyl acetate (30 mL) and water (30 mL). The combined organic layers were isolated, washed with brine, dried over MgSO₄, filtered and concentrated. The crude product was loaded in a minimum of dichloromethane onto a 40 gram RediSep^{®} column pre-wet with hexanes and eluted with an ethyl acetate/hexanes gradient (0% to 20% ethyl acetate). Fractions containing the product were combined and concentrated to provide the product as a clear oil (1.45g, 83%).

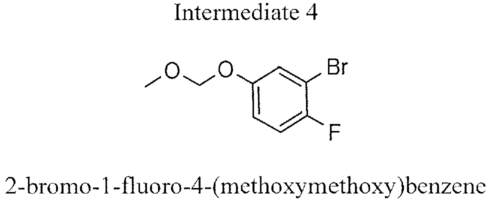

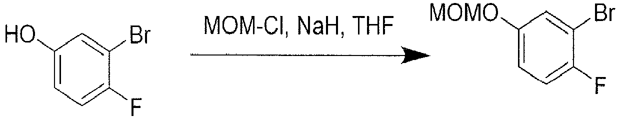

To a stirred solution of 3-bromo-4-fluorophenol (327 mg, 1.71 mmol) in 5.1 mL tetrahydrofuran at room temperature under nitrogen was added NaH (75.3 mg, 1.88 mmol) neat as a solid portion wise. After 15 minutes, a solution had formed. Chloro(methoxy)methane (156 µL, 2.05 mmol) was added by syringe. After stirring for 2 hours, the reaction was quenched with saturated ammonium chloride solution and partitioned between ethyl acetate and water. The combined organic layers were washed with brine, dried over MgSO₄, filtered and concentrated. The crude product was loaded in a minimum of dichloromethane onto a 24 gram RediSep^{®} column pre-wet with hexanes and eluted with an ethyl acetate/hexanes gradient (0% to 20% ethyl acetate). Fractions containing the product were combined and concentrated to provide the product as a clear oil (120 mg, 29.8%)

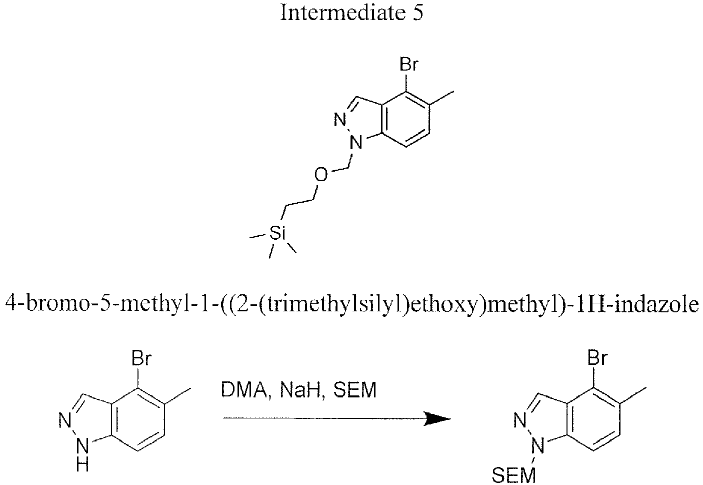

To a solution of 4-bromo-5-methyl-1H-indazole (0.7 g, 3.3 mmol) in dimethyl acetamide (30 mL) cooled to 0 °C was added NaH (0.19 g, 4.6 mmol) in portions and the reaction mixture was purged with nitrogen. The reaction was stirred for 20 minutes, and then (2-(chloromethoxy)ethyl)trimethylsilane (0.83 g, 5.0 mmol) was added and the reaction was stirred for 2 hours while warming to room temperature. The reaction was quenched by pouring into water and the aqueous layer was extracted into ethyl acetate. The combined organic layers were washed with water and brine, dried over MgSO₄ and concentrated under vacuum. The crude material was purified by chromatography using 10-50% ethyl acetate/hexanes as the eluent to give 4-bromo-5-methyl-1-((2-(trimethylsilyl)ethoxy)methyl)-1H-indazole (0.87 g, 79%).

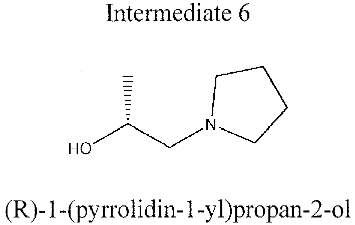

In a sealed tube, R-(+)-Propylene oxide (3.69 mL, 52.7 mmol) was cooled to -78°C and then sparged with anhydrous dimethyl amine for a few minutes. The reaction mixture was heated to 70°C for 16 hours. The reaction was cooled and concentrated in vacuo for 20 minutes to provide (R)-1-(pyrrolidin-1-yl)propan-2-ol (5.35 g, 41.4 mmol, 98.2% yield).

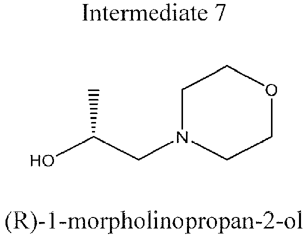

In a sealed tube, R-(+)-Propylene oxide (2.111 mL, 30.13 mmol) and morpholine (1.490 mL, 17.22 mmol) were heated to 70°C for 20 hours. The reaction was cooled and concentrated in vacuo to provide (R)-1-morpholinopropan-2-ol (2.47 g, 17.01 mmol, 98.80 % yield).

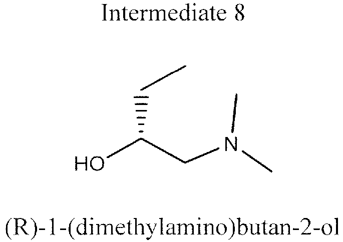

In a sealed tube, R-(+)-Propylene oxide (4.00 g, 55.5 mmol) and dimethylamine (1.00 g, 22.2 mmol), were heated to 65°C for 18 hours. The reaction was cooled and concentrated in vacuo. The resulting residue was purified by silica gel (0-12% MeOH in DCM) to provide (R)-1-(dimethylamino)butan-2-ol (1.38 g, 11.8 mmol, 53.1 % yield).

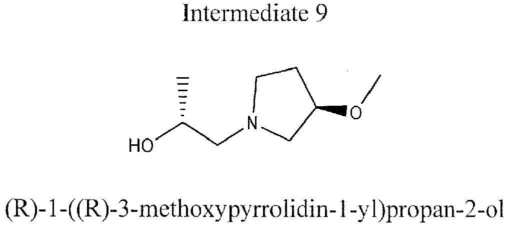

In a sealed tube, (R)-3-methoxypyrrolidine hydrochloride (1.00 g, 7.27 mmol), TEA (2.03 mL, 14.5 mmol) and R-(+)-Propylene oxide (1.27 mL, 18.2 mmol) were heated to 65°C for 18 hours. The reaction was cooled and concentrated in vacuo. The resulting residue was purified by silica gel (0-12% MeOH in DCM) to provide (R)-1-((R)-3-methoxypyrrolidin-1-yl)propan-2-ol (775 mg, 4.87 mmol, 67.0 % yield).

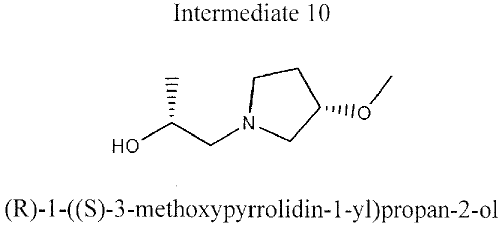

In a sealed tube, (S)-3-methoxypyrrolidine hydrochloride (1.00 g, 7.27 mmol), TEA (2.03 mL, 14.5 mmol) and R-(+)-Propylene oxide (1.27 mL, 18.2 mmol) were heated to 65°C for 18 hours. The reaction was cooled and concentrated in vacuo. The resulting residue was purified by silica gel (0-12% MeOH in DCM) to provide (R)-1-((S)-3-methoxypyrrolidin-1-yl)propan-2-ol (781 mg, 4.90 mmol, 67.5 % yield)

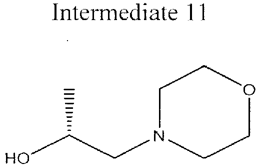

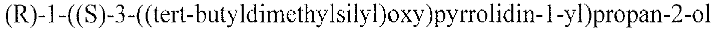

In a sealed tube, R-(+)-Propylene oxide (0.609 mL, 8.69 mmol) and (S)-3-((tertbutyldimethylsilyl)oxy)pyrrolidine (1.00 g, 4.97 mmol) were heated to 70°C for 20 hours. The reaction was cooled and concentrated in vacuo to provide (R)-1-((S)-3-((tertbutyldimethylsilyl)oxy)pyrrolidin-1-yl)propan-2-ol (1.29 g, 4.20 mmol, 84.6 % yield).

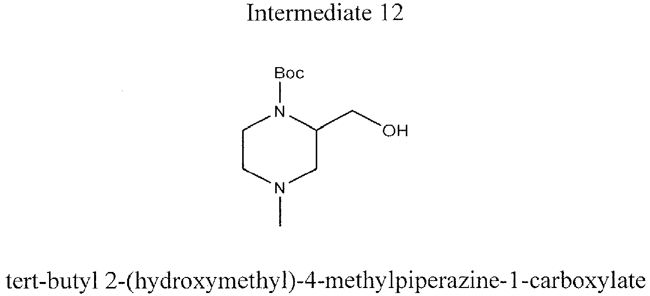

To a suspension of lithium chloride (246 mg, 5.81 mmol) and Lithium Borohydride (126 mg, 5.81 mmol) in ethanol (9 mL), at 0°C under nitrogen, a solution of 1-(tert-butyl) 2-methyl 4-methylpiperazine-1,2-dicarboxylate (750 mg, 2.90 mmol) in dry THF (6 mL) was added dropwise. The reaction was stirred overnight forming a white precipitate. The precipitate was filtered and washed with ethanol. The combined filtrate and organic extracts were concentrated to provide a white residue which was extracted with ethyl acetate. The combined organic layers were washed with saturated sodium chloride solution, dried over sodium sulfate and concentrated in vacuo. The residue was purified by chromatography with isocratic 10% MeOH in DCM with 0.2% NH₄OH to provide tert-butyl 2-(hydroxymethyl)-4-methylpiperazine-1-carboxylate (104 mg, 0.452 mmol, 15.6 % yield).

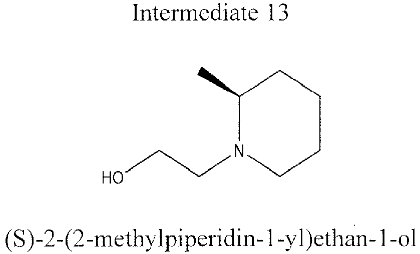

A mixture of (S)-2-methylpiperidine (100 mg, 1.01 mmol), 2-bromoethanol (78 µL, 139 mg, 1.11 mmol, 1.1 eq.), sodium iodide (151 mg, 1 eq.), potassium carbonate (418 mg, 3 eq.) and acetonitrile (1 mL) in a 4-mL vial was purged with nitrogen, sealed and stirred at room temperature for 2 days. The reaction mixture was partitioned between diethyl ether (15 mL) and water (2 mL). The ether layer was washed with brine (2 mL), acidified with TFA and dried under high vacuum for 2 days. The residue was washed with ether (3 mL), diluted with water (0.5 mL) and 10M NaOH was added (0.2 mL). The layers were separated and the upper layer was carefully dried over NaOH. The ether solution was evaporated under nitrogen to yield crude (S)-2-(2-methylpiperidin-1-yl)ethan-1-ol (100 mg, 0.698 mmol, 69.24% yield) as colorless oil.

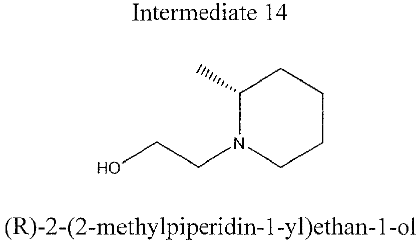

Synthesized according to the method of Intermediate 13, using (R)-2-methylpiperidine (99 mg, 1 mmol) in place of (S)-2-methylpiperidine.

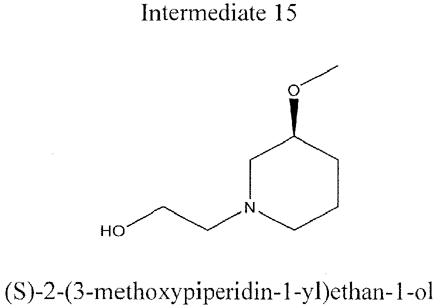

Synthesized according to the method of Intermediate 13, using (S)-3-methoxypiperidine (173 mg, 1.50 mmol) in place of (S)-2-methylpiperidine.

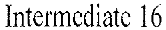

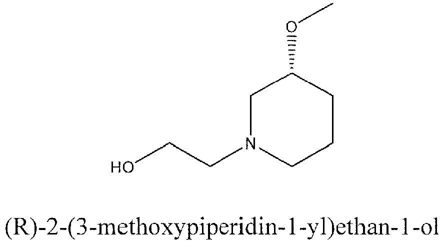

Synthesized according to the method of Intermediate 13, using R-3-methoxypiperidine (173 mg, 1.50 mmol) in place of (S)-2-methylpiperidine.

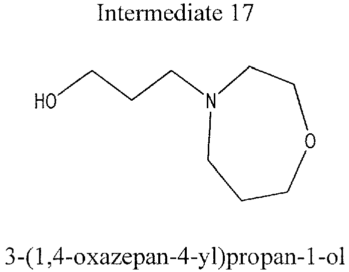

To a vial was added homomorpholine (0.250 g, 2.472 mmol), Acetonitrile (4.943 mL, 2.472 mmol) and 3-Bromo-1-propanol (0.2459 mL, 2.719 mmol). Potassium carbonate (0.6832 g, 4.943 mmol) was added and the mixture was warmed to 50 °C and stirred for 6 hours. The mixture was cooled to ambient temperature, diluted with DCM, filtered and the collected solids were washed with DCM. The filtrate was concentrated in vacuo and the crude oil was purified via column chromatography (Biotage Isolera, 12g Isco RediSep Gold, 10-20% MeOH/DCM with 0.2% NH₄OH) to afford 3-(1,4-oxazepan-4-yl)propan-1-ol (0.272 g, 1.708 mmol) as a colorless oil.

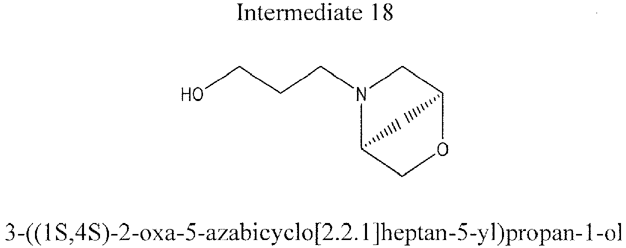

Synthesized according to the method of Intermediate 17, using (1 S,4S)-2-Oxa-5-azabicyclo[2.2.1]heptane (0.250 g, 2.522 mmol) in place of homomorpholine.

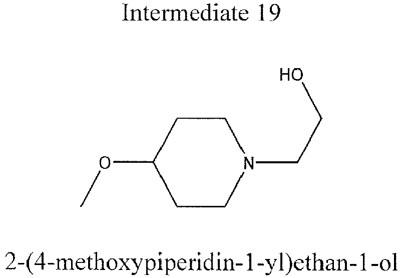

Synthesized according to the method of Intermediate 13, using 4-methoxypiperidine (173 mg, 1.50 mmol) in place of (S)-2-methylpiperidine.

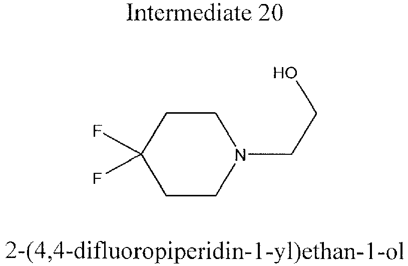

Synthesized according to the method of Intermediate 13, using 4,4-difluoropiperidine hydrochloride (173 mg, 1.50 mmol) in place of (S)-2-methylpiperidine.

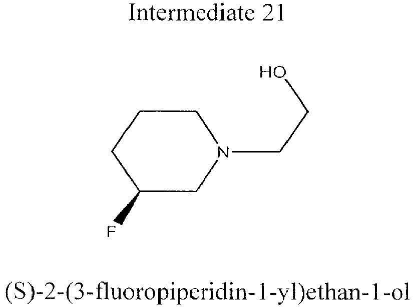

Synthesized according to the method of Intermediate 13, using S-3-fluoropiperidine hydrochloride (209 mg, 1.50 mmol) in place of (S)-2-methylpiperidine.

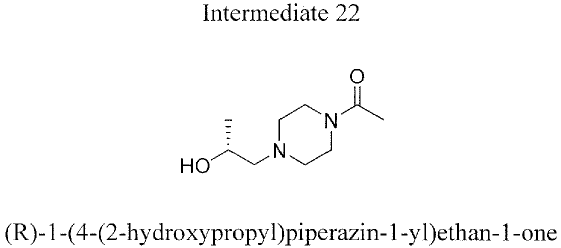

Step A: 1-[4-[(2*R*)-2-hydroxypropyl]piperazin-1-yl]ethanone: (2*R*)-2-methyloxirane (1.00 g, 17.2 mmol, 1.20 mL, 1.00 *eq*) and 1-piperazin-1-ylethanone (8.00 g, 62.4 mmol, 3.62 *eq*) were taken up into a microwave tube. The sealed tube was heated at 150 °C for 1 hour under microwave. The mixture was dissolved in DCM (80.0 mL), added (Boc)₂O (3.62 eq,13.6 g) and stirred at 20 °C for 1 hour. The residue was purified by column chromatography (DCM/MeOH 100/1 to 10/1) to give 1-[4-[(2R)-2-hydroxypropyl]piperazin-1-yl]ethanone (3.80 g, 13.5 mmol, 78.2 % yield, 66.0 % purity) as a yellow oil.

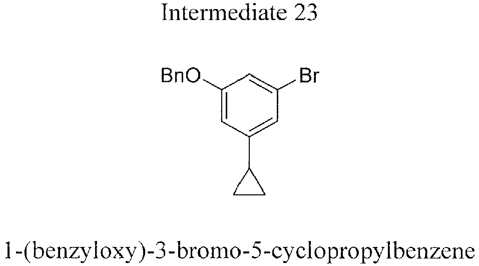

Step A: 1-benzyloxy-3,5-dibromo-benzene: To a mixture of 3,5-dibromophenol (1.50 g, 5.95 mmol, 1.00 *eq*) and K₂CO₃ (2.47 g, 17.9 mmol, 3.00 *eq*) in MeCN (30.0 mL) was added benzyl bromide (1.07 g, 6.25 mmol, 742 µL, 1.05 *eq*)*,* the reaction mixture was stirred at 80 °C for 2 hours. The reaction mixture was filtered and concentrated. The residue was purified by column chromatography (SiO₂, Petroleum ether/Ethyl acetate = 1:1 to give 1-benzyloxy-3,5-dibromobenzene (1.60 g, 4.68 mmol, 78.6 % yield) as colorless oil.

Step B: 1-benzyloxy-3-bromo-5-cyclopropylbenzene: To a mixture of 1-benzyloxy-3,5-dibromobenzene (1.20 g, 3.51 mmol, 1.00 *eq*) and cyclopropylboronic acid (392 mg, 4.56 mmol, 1.30 *eq*) in H₂O (4.00 mL) and dioxane (20.0 mL) was added Pd(dppf)Cl₂ (513 mg, 702 µmol, 0.20 *eq*) and Cs₂CO₃ (2.29 g, 7.02 mmol, 2.00 *eq*)*.* The reaction mixture was stirred at 90 °C for 12 hours under N₂. The reaction mixture was added to water (20 mL) and extracted with ethyl acetate (2 × 15 mL). The combined organic layers were dried over Na₂SO₄, filtered and concentrated. The residue was purified by column chromatography (SiO₂, Petroleum ether/Ethyl acetate = 1:1 to give 1-benzyloxy-3-bromo-5-cyclopropyl -benzene (270 mg, 890 µmol, 25.4 % yield) as colorless oil.

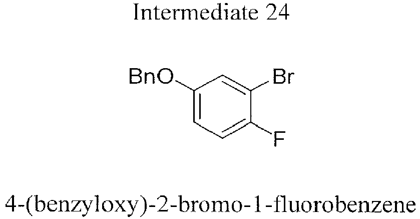

To a solution of 3-bromo-4-fluorophenol (4.00 g, 20.9 mmol, 1.00 *eq*) and K₂CO₃ (8.68 g, 62.8 mmol, 3.00 *eq*) in ACN (80.0 mL) was added benzyl bromide (3.65 g, 21.4 mmol, 2.54 mL, 1.02 *eq*) and the reaction mixture was stirred at 60 °C for 2 hrs. The reaction mixture was filtered and concentrated in vacuum. The residue was purified by silica gel chromatography (petroleum ether:ethyl acetate; gradient from 1:0 to 10:1) to give 4-benzyloxy-2-bromo-1-fluoro-benzene (5.02 g, 17.0 mmol, 81.0 % yield, 95 % purity) was obtained as white solid.

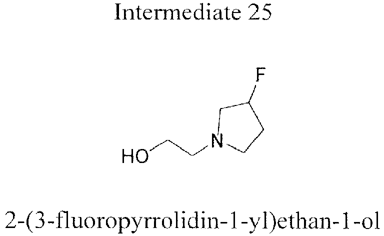

Step A: *tert*-butyl 3-fluoropyrrolidine-1-carboxylate: To a solution of *tert*-butyl 3-hydroxypyrrolidine-1-carboxylate (10.0 g, 53.4 mmol, 1.00 *eq*) in DCM (150.00 mL) was added diethylaminosulfur trifluoride (DAST) (12.9 g, 80.1 mmol, 10.6 mL, 1.50 *eq*) at -40 °C under a nitrogen atmosphere. After stirring at - 40 °C for 2 hours, the mixture was warmed to 20 °C and stirred for 16 hours. The mixture was poured into 5% aqueous sodium bicarbonate (200 mL) and extracted with dichloromethane (2 × 100 mL). The organic layer was dried over sodium sulfate, filtered and concentrated under vacuum. The residue was purified by column chromatography over silica gel (petroleum ether/ethyl acetate 100: 1 to 5:1). The desired fractions were collected and concentrated under vacuum to give *tert-*butyl 3-fluoropyrrolidine-1-carboxylate (4.30 g, 22.7 mmol, 42.6 % yield) as a colorless oil. ¹H NMR (400 MHz, Chloroform-d) δ = 5.27 (t, *J* = 3.6 Hz, 0.5H), 5.13 (t, *J* = 3.6 Hz, 0.5H), 3.77 - 3.38 (m, 4H), 2.26 - 2.15 (m, 1H), 2.08 - 1.85 (m, 1H), 1.46 (s, 9H).

Step B: 3-fluoropyrrolidine: To a solution of tert-butyl 3-fluoropyrrolidine-1-carboxylate (4.30 g, 22.7 mmol, 1.00 *eq*) in DCM (50.00 mL) was added HCl/dioxane (4 M, 35.0 mL, 6.16 *eq*) dropwise at 0 °C. The mixture was warmed to 20 °C and stirred for 1 hour. The mixture was concentrated under vacuum. The residue was triturated with diisopropyl ether (20 mL) and the precipitate was filtered and dried under vacuum to provide 3-fluoropyrrolidine (2.70 g, 21.5 mmol, 94.6 % yield, HCl) as a white solid. ¹H NMR (400 MHz, Methanol-d₄) δ = 5.51 (t, *J* = 3.6 Hz, 0.5H), 5.38 (t, *J*=3.6 Hz, 1H), 3.66 - 3.27 (m, 5H), 2.45 - 2.12 (m, 2H).

Step C: methyl 2-(3-fluoropyrrolidin-1-yl)acetate: A suspension of 3-fluoropyrrolidine (2.70 g, 21.5 mmol, 1.00 *eq,* HCl) in DCM (27.00 mL) was cooled to 0° C. Triethylamine (5.44 g, 53.8 mmol, 7.45 mL, 2.50 *eq*) and methyl 2-bromoacetate (3.62 g, 23.7 mmol, 2.23 mL, 1.10 *eq*) were added and the reaction mixture was stirred at 20 °C for 16 h. The reaction mixture was diluted with CH₂Cl₂ (100 mL) and water (50 mL). The organic layer was washed with 5% aqueous citric acid solution (1 × 50 mL). To the water layer, saturated aqueous sodium carbonate solution was added (20 mL) and extracted with ethyl acetate (3 × 100 mL). The combined organic layers were dried over sodium sulfate and concentrated in vacuo to give methyl 2-(3-fluoropyrrolidin-1-yl)acetate (2.20 g, 13.7 mmol, 63.5 % yield). ¹H NMR (400 MHz, Chloroform-d) δ = 5.22 - 5.02 (m, 1H), 3.66 (s, 3H), 3.35 (s, 2H), 3.07 - 2.93 (m, 1H), 2.91 - 2.77 (m, 2H), 2.67 (dt, *J* = 5.2, 8.4 Hz, 1H), 2.21 - 1.93 (m, 2H).

Step D: 2-(3-fluoropyrrolidin-1-yl)ethanol: To a solution of LiAlH₄ (706 mg, 18.6 mmol, 1.50 *eq*) in THF (20 mL) was added a solution of methyl 2-(3-fluoropyrrolidin-1-yl)acetate (2.00 g, 12.4 mmol, 1.00 *eq*) in THF (10 mL) dropwise at 0 °C. The mixture was warmed up to 20 °C and stirred for 3 hours. The mixture was quenched with saturated aqueous sodium sulfate solution (1 mL). The mixture was filtered and the filtrate was concentrated under vacuum. The product was purified by silica gel chromatography using 5% MeOH in DMC. The desired fractions were collected and concentrated under vacuum to give 2-(3-fluoropyrrolidin-1-yl)ethanol (1.20 g, 9.01 mmol, 72.6 % yield) as a colorless oil. ¹H NMR (400 MHz, Chloroform-d) δ = 5.28 - 5.05 (m, 1H), 3.68 - 3.61 (m, 2H), 2.99 - 2.73 (m, 4H), 2.72 - 2.67 (m, 2H), 2.58 - 2.45 (m, 1H), 2.28 - 1.97 (m, 2H).

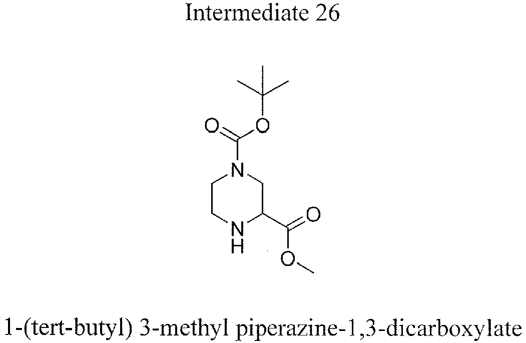

Step A: methyl piperazine-2-carboxylate: To a mixture of 1-tert-butyl 2-methyl piperazine-1,2-dicarboxylate (5.0 g, 22.6 mmol, 1.00 eq) in MeOH (50.0 mL) was added HCl/dioxane (4.0 M, 134 mL). The reaction mixture was degassed and purged with nitrogen 3 times, and the mixture was stirred at 25 °C for 12 hours under a nitrogen atmosphere. The reaction mixture was concentrated under reduced pressure to dryness to give methyl piperazine-2-carboxylate (4.89 g, 2HCl, crude) as a white solid, which was used directly in the next step without further purification.

Step B: 1-(tert-butyl) 3-methyl piperazine-1,3-dicarboxylate: To a solution of methyl piperazine-2-carboxylate (4.30 g, crude) and TEA (8.02 g, 79.2 mmol, 11.0 mL) in MeOH (50.0 mL) was added di-tert-butyl dicarbonate (4.32 g, 19.8 mmol, 4.55 mL). After stirring at 25 °C for 12 hours, the reaction mixture was filtered and concentrated under reduced pressure to dryness. The residue was purified by column chromatography (SiO₂, DCM / MeOH = 1:0 to 20:1) to give 1-(tert-butyl) 3-methyl piperazine-1,3-dicarboxylate (4.80 g, 19.7 mmol, two steps, 99.0 % yield) as a colorless oil.¹H NMR (400 MHz, chloroform-d) δ = 4.10 - 3.85 (m, 1H), 3.73 (s, 3H), 3.71 - 3.65 (m, 1H), 3.47 - 3.38 (m, 1H), 3.10 - 2.98 (m, 211), 2.78 - 2.66 (m, 1H), 2.17 (s, 1H), 1.46 (s, 9H).

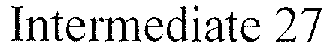

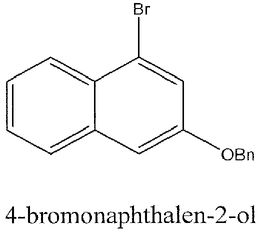

Step A: 2,4-dibromonaphthalen-1-amine: To a solution of Br₂ (246 g, 1.54 mol, 79.3 mL, 2.18 *eq*) in AcOH (750 mL) was added a solution of naphthalen-1-amine (101 g, 705 mmol, 99.0 mL, 1.00 *eq*) in AcOH (500 mL) at ambient temperature, and the reaction was stirred at 70 °C for 1 hour. The reaction mixture was cooled at room temperature and filtered. The filter cake was washed with AcOH (300 mL), then added to 20 % aqueous of NaOH (1.2 L). The mixture was stirred for 20 min and filtered. The isolated solid was washed with water (1 L) and dried under vacuum to provide 2,4-dibromonaphthalen-1-amine (200 g, 664 mmol, 94.2% yield) as gray solid. **ESI** MS m/z 301. 9 [M+H]⁺.

Step B: 4-bromo-1-diazonio-naphthalen-2-olate: To a solution of 2,4-dibromonaphthalen-1-amine (60.0 g, 199 mmol, 1.00 *eq*) in AcOH (900 mL) and propionic acid (150 mL) was added NaNO₂ (16.5 g, 239 mmol, 13.0 mL, 1.20 *eq*) portionwise at 5-8 °C over 30 min, and then the reaction mixture was stirred at 5-8 °C for 30 min. The reaction mixture was poured into ice-water (4000 mL), and the resulting solid was collected and washed with water (2 × 50 mL) to provide 4-bromo-1-diazonio-naphthalen-2-olate (150 g, wet crude) as gray solid which was used directly in the next step. ¹H NMR (400 MHz, CDCl₃) δ 8.12 - 8.10 (d, *J*=8.4 Hz, 1H), 7.62 - 7.58 (t, *J*=7.6 Hz, 1H), 7.41 - 7.37 (t, *J*=7.6 Hz, 1H), 7.31 - 7.29 (d, *J*=8.0 Hz, 1H), 7.20 (s, 1H).

Step C: 4-bromonaphthalen-2-ol: To a solution of 4-bromo-1-diazonio-naphthalen-2-olate (100 g, 402 mmol, 1.00 *eq*) in EtOH (2.00 L) was added portionwise NaBH₄ (30.4 g, 803 mmol, 2.00 *eq*) at 13-15 °C over 1 h, and the reaction mixture was stirred at 15-18 °C for 3 hrs. The reaction was filtered and concentrated to dryness. The residue was dissolved in DCM (1000 mL) and washed with water (500 mL × 2). The organic phase was dried over Na₂SO₄ and concentrated to dryness. The residue was purified by silica gel column chromatograph, eluting with diethyl ether/ethyl acetate (60:1 to 10:1). The isolated product was further purified by reverse phase HPLC to provide 4-bromonaphthalen-2-ol (40.0 g, 139 mmol, 17.3 % yield, 77.4% purity) as a gray solid. ¹H NMR (400 MHz, CDCl₃) δ 8.07 - 8.05 (d, *J*=8.0 Hz, 1H), 7.60 - 7.58 (d, *J*=7.6 Hz, 1H), 7.41 - 7.36 (m, 3H), 7.07 (s, 1H).

Step D: 3-benzyloxy-1-bromo-naphthalene: A mixture of 4-bromonaphthalen-2-ol (30.0 g, 134 mmol, 1.00 *eq*), benzyl bromide (25.3 g, 148 mmol, 17.6 mL, 1.10 *eq*) and K₂CO₃ (55.7 g, 403 mmol, 3.00 *eq*) in MeCN (500 mL) was heated at 80 °C for 1 hr. The reaction mixture was filtered and concentrated to dryness. The residue was purified by silica gel column chromatography, eluting with diethyl ether/ethyl acetate (100:1 to 60:1) to provide 3-benzyloxy-1-bromo-naphthalene (40.0 g, 128 mmol, 95 % yield) as yellow oil. ¹H NMR (400 MHz, CDCl₃) δ 8.19 - 8.17 (d, *J*=8.0 Hz, 1H), 7.75 - 7.32 (d, *J*=8.8 Hz, 1H), 7.64 - 7.63 (d, *J*=2.4 Hz,1H), 7.52 - 7.37 (m, 7H), 7.23 - 7.21 (d, *J*=2.0 Hz,1H), 5.2 (s, 2H).

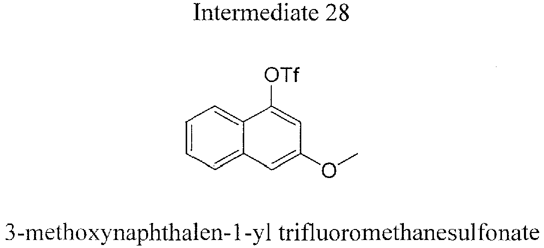

Step A: 3-methoxynaphthalen-1-ol : To a solution of naphthalene-1,3-diol (3.00 g, 18.7 mmol, 1.00 eq) in MeOH (60.0 mL) was added HCl/MeOH (4 M, 60.0 mL, 12.8 eq) at 0 °C. The mixture was stirred at 25 °C for 60 hours. The solvent was removed under vacuum. The residue was purified by silica gel chromatography (diethyl ether:ethyl acctate=10:1 to 5:1) to give 3-methoxynaphthalen-1-ol (2.10 g, 12.1 mmol, 64.4% yield) as a brown solid. ¹H NMR (400 MHz, CDCl₃-d₆) δ = 8.10 - 8.08 (d, *J*=8.4 Hz, 1H).7.73 - 7.71 (d, *J*=8.4 Hz, 1H), 7.47 - 7.45(m, 1H), 7.38 - 7.35(m, 1H), 6.80 - 6.79 (d, *J*=2.0 Hz, 1H), 6.56 - 6.55 (d, *J*=2.4 Hz, 1H), 3.92 (s, 3H).

Step B: (3-methoxy-1-naphthyl) trifluoromethanesulfonate: To a solution of 3-methoxynaphthalen-1-ol (2.10 g, 12.0 mmol, 1.00 eq) in DCM (40.0 mL) was added DIEA (7.79 g, 60.3 mmol, 10.5 mL, 5.00 eq) and trifluoromethanesulfonic anhydride (5.10 g, 18.1 mmol, 2.98 mL, 1.50 eq) at 0 °C. The mixture was stirred at 25 °C for 1 hour. The mixture was diluted with DCM (30 mL) and water (10 mL) and extracted with DCM (20 mL). The combined organic layers were washed with brine (5 mL), dried over Na₂SO₄ and concentrated under vacuum. The residue was purified by silica gel chromatography (diethyl ether:ethyl acetate=20:1 to 10 :1) to give (3-methoxy-1-naphthyl) trifluoromethanesulfonate (3.00 g, 8.52 mmol, 70.7 % yield, 87.0 % purity) as a brown oil. **ESI** MS m/z 307.1 [M+H]⁺.

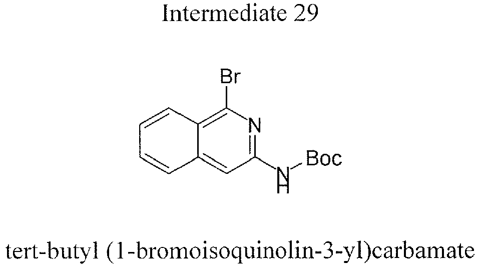

Step A: A mixture of 1-bromoisoquinolin-3-amine (400 mg, 1.79 mmol, 1.00 eq) and tert-butoxycarbonyl tert-butyl carbonate (3.91 g, 17.9 mmol, 4.12 mL, 10.0 eq) was stirred at 70 °C for 16 hours. The residue was purified by column chromatography (SiO₂, diethyl ether/ethyl acetate = 5:1) to give *tert*-butyl N-(1-bromo-3-isoquinolyl) carbamate (400 mg, 1.24 mmol, 69.2 % yield) as a yellow solid. ESI MS m/z 322.1, 324.1 [M+H]⁺ .

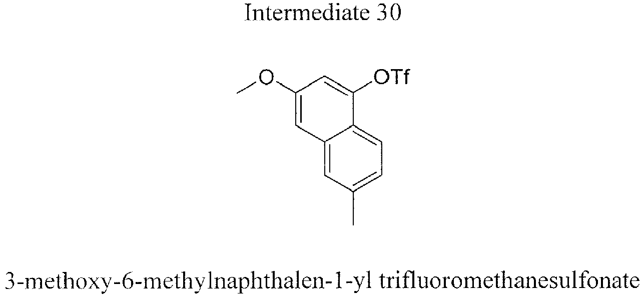

Step A: 3-methoxynaphthalen-1-ol: To a solution of naphthalene-1,3-diol (40.0 g, 250 mmol, 1.00 *eq*) in MeOH (800 mL) was added HCl (4 M, 750 mL, 12.0 *eq,* 4 M in MeOH) at 0 °C. The mixture was warmed up to 18 °C and stirred for 30 hours. The mixture was concentrated under vacuum. The residue was purified by column chromatography over silica gel (petroleum ether/ethyl acetate 100/1 to 1/1). The desired fractions were collected and concentrated under vacuum to give 3-methoxynaphthalen-1-ol (17.7 g, 96.5 mmol, 38.6 % yield, 95 % purity) as a red oil. ¹H NMR (400MHz, Chloroform-d) δ = 8.17 (d, *J*= 8.4 Hz, 1H), 7.74 (d, *J*= 8.0 Hz, 1H), 7.50 (ddd,*J*= 1.2, 6.8, 8.0 Hz, 1H), 7.38 (ddd, *J*=1.2, 6.8, 8.0 Hz, 1H), 6.81 (d, 7=2.0 Hz, 1H), 6.76 (br s, 1H), 6.62 (d, *J*=2.4 Hz, 1H), 3.91 (s, 3H).

Step B: *tert*-butyl-[(3-methoxy-l-naphthyl)oxy]-dimethyl-silane: To a solution of 3-methoxynaphthalen-1-ol (20.0 g, 115 mmol, 1.00 *eq*) and imidazole (23.5 g, 344 mmol, 3.00 *eq*) in THF (400 mL) was added TBSCl (26.0 g, 172 mmol, 21.1 mL, 1.50 *eq*) dropwise at 0 °C. The mixture was warmed up to 25 °C and stirred for 16 hours. The mixture was diluted with petroleum ether (600 mL) and ethyl acetate (200 mL), and then washed with water (1 × 200 mL) and brine (1 × 200 mL). The separated organic layer was dried over sodium sulfate, filtered and concentrated under vacuum. The residue was purified by column chromatography over silica gel (petroleum ether/ethyl acetate 100/1 to 10/1). *tert*-butyl-[(3-methoxy-1-naphthyl)oxy]-dimethylsilane (28.0 g, 97.1 mmol, 84.6 % yield) was obtained as a colorless oil. ¹H NMR (400MHz, Chloroform-d) δ = 8.01 (d, *J* = 8.4 Hz, 1H), 7.61 (d, *J* = 8.0 Hz, 1H), 7.35 (dt, *J* = 1.2, 7.6 Hz, 1H), 7.24 (dt, *J* = 1.2, 7.6 Hz, 1H), 6.71 (d, *J=* 2.0 Hz, 1H), 6.48 (d, *J* = 2.4 Hz, 1H), 3.82 (s, 3H), 1.02 (s, 9H), 0.23 (s, 6H).

Step C: *tert*-butyl-[[3-methoxy-6-(4,4,5,5-tetramethyl-1,3,2-dioxaborolan-2-yl)-1-naphthyl]oxy]-dimethyl-silane and *tert*-butyl((3-methoxy-7-(4,4,5,5-tetramethyl-1,3,2-dioxaborolan-2-yl)naphthalen-1 -yl)oxy)dimethylsilane: A mixture of *tert*-butyl-[(3-methoxy-1-naphthyl) oxy]-dimethyl-silane (26.0 g, 90.1 mmol, 1.00 *eq),* 4,4,5,5-tetramethyl-2-(4,4,5,5-tetramethyl-1,3,2-dioxaborolan-2-yl)-1,3,2-dioxaborolane (45.8 g, 180 mmol, 2.00 *eq*)*,* (1*Z,*5*Z*)-cycloocta-1,5-diene;2,4-dimethyl-BT,AHbicyclo[1.1.0]butane (2.39 g, 3.61 mmol, 0.04 *eq*) and 4-*tert*-butyl-2-(4-*tert*-butyl-2-pyridyl)pyridine (1.45 g, 5.41 mmol, 0.06 *eq*) in hexane (500 mL) was stirred at 100 °C under nitrogen atmosphere for 16 hours. The mixture was diluted with water (500 mL) and ethyl acetate (1000 mL). The separated organic layer was washed with brine (1 × 500 mL), dried over sodium sulfate, filtered and concentrated under vacuum. The residue was purified by column chromatography over silica gel (petroleum etehr/ethyl acetate 100/1 to 10/1). The desired fractions were collected and concentrated under vacuum to give a mixture of *tert*-butyl-[[3-methoxy-6-(4,4,5,5-tetramethyl-1,3,2-dioxaborolan-2-yl)-1-naphthyl]oxy]-dimethyl-silane and *tert*-butyl((3-methoxy-7-(4,4,5,5-tetramethyl-1,3,2- dioxaborolan-2-yl)naphthalen-1-yl)oxy)dimethylsilane (38.0 g, 85.3 mmol, 94.6 % yield, 93 % purity) as a light yellow oil. **ESI** MS m/z 415.5 [M+H]⁺

Step D: 8-[*tert*-butyl(dimethyl)silyl]oxy-6-methoxy- naphthalen-2-ol: To a solution of mixture (36.0 g, 86.9 mmol, 1.00 *eq*) of *tert-*butyl-[[3-methoxy-6-(4,4,5,5- tetramethyl-1,3,2-dioxaborolan-2-yl)-1- naphthyl]oxy]-dimethyl-silanc and *tert*-butyl((3-methoxy-7-(4,4,5,5-tetramethyl-1,3, 2-dioxaborolan-2-yl)naphthalen-1-yl)oxy)dimethylsilanein in acetone (400 mL) was added a solution of Oxone (58.7 g, 95.6 mmol, 1.10 *eq*) in H₂O (400 mL) at 0 °C. The mixture was stirred at 0 °C for 1 hour. The mixture was quenched with 5% aqueous sodium thiosulfate solution (50 mL) and extracted with ethyl acetate (2 × 300 mL). The extracts were combined and washed with water (1 × 200 mL), brine (1 × 200 mL), dried over magnesium sulfate, filtered and the filtrate was concentrated under vacuum. The residue was purified by column chromatography over silica gel (petroleum ether/ethyl acetate 200/1 to 20/1). The desired fractions were collected and concentrated under vacuum to give 8-[*tert*-butyl(dimethyl)silyl]oxy-6-methoxy- naphthalen-2-ol (9.00 g, 28.4 mmol, 32.7 % yield, 96 % purity) as a colorless oil and 5-[*tert*-butyl(dimethyl)silyl]oxy-7-methoxy-naphthalen-2-ol (9.00 g, 29.0 mmol, 33.4 % yield, 98 % purity) as a white solid. **ESI** MS m/z 305.2 [M+H]⁺

Step E: [5-[*tert*-butyl(dimethyl)silyl]oxy-7-methoxy-2-naphthyl] trifluoromethanesulfonate: To a solution of 5-[*tert*-butyl(dimethyl)silyl]oxy-7- methoxynaphthalen-2-ol (11.0 g, 36.1 mmol, 1.00 *eq*) and DIEA (14.0 g, 108 mmol, 18.9 mL, 3.00 *eq*) in DCM (150 mL) was added Tf₂O (12.2 g, 43.4 mmol, 7.15 mL, 1.20 *eq*) dropwise at - 40 °C. The mixture was stirred for 1 hour. The mixture was diluted with dichloromethane (200 mL) and washed with water (1 × 200 mL) and brine (1 × 200 mL). The separated organic layer was dried over sodium sulfate, filtered and concentrated under vacuum. The residue was purified by column chromatography over silica gel (petroleum ether/ethyl acetate 100/1 to 10/1). The desired fractions were collected and concentrated under vacuum to give [5*-*[*tert-*butyl(dimethyl)silyl]oxy-7-methoxy-2-naphthyl] trifluoromethanesulfonate (13.0 g, 29.8 mmol, 82.4 % yield, 100 % purity) as a white solid. **ESI** MS m/z 436.9 [M+H]⁺

Step F: *tert*-butyl-[(3-methoxy-6-methyl-1-naphthyl)oxy]-dimethyl-silane: To a solution of [5-[*tert*-butyl(dimethyl)silyl]oxy-7-methoxy-2- naphthyl]trifluoromethanesulfonate (12.5 g, 28.6 mmol, 1.00 eq) and K₂CO₃ (11.9 g, 85.9 mmol, 3.00 eq) in dioxane (160 mL) was added Pd(PPh₃)₄ (3.31 g, 2.86 mmol, 0.10 eq) and trimethylboroxine (14.4 g, 57.3 mmol, 16.0 mL, 2.00 eq) under nitrogen atmosphere. The reaction was heated to 100 °C for 16 hours. The mixture was diluted with ethyl acetate (200 mL) and then washed with water (1 × 200 mL) and brine (1 × 200 mL). The separated organic layer was dried over sodium sulfate, filtered and concentrated under vacuum. The residue was purified by column chromatography over silica gel (petroleum ether/ethyl acetate 100/1 to 5/1). The desired fractions were collected and concentrated under vacuum to give *tert*-butyl-[(3-methoxy-6-methyl-1-naphthyl)oxy]-dimethyl-silane (8.00 g, 24.6 mmol, 85.9 % yield, 93 % purity) as a colorless oil as red solid. **ESI** MS m/z 303.2 [M+H]⁺

Step G: 3-methoxy-6-methyl-naphthalen-1-ol: To a solution of *tert*-butyl-[(3-methoxy-6-methyl-1-naphthyl) oxy]-dimethyl-silane (8.00 g, 26.5 mmol, 1.00 eq) in THF (100 mL) was added TBAF (10.4 g, 39.7 mmol, 1.50 eq) at 0 °C. The mixture was stirred at 0 °C for 3 hours. The mixture was diluted with water (100 mL) and ethyl acetate (200 mL). The separated organic layer was washed with brine (1 × 100 mL), dried over sodium sulfate, filtered and concentrated under vacuum. The residue was purified by column chromatography over silica gel (petroleum ether/ethyl acetate 50/1 to 5/1). The desired fractions were collected and concentrated under vacuum to give 3-methoxy-6-methyl-naphthalen-1-ol (4.70 g, 25.0 mmol, 94.4 % yield) as a red solid. **ESI** MS m/z 188.4 [M+H]⁺

Step H: 3-methoxy-6-methyl-1-naphthyl trifluoromethanesulfonate: To a solution of 3-methoxy-6-methyl-naphthalen-1-ol (4.70 g, 25.0 mmol, 1.00 eq) and DIEA (9.68 g, 74.9 mmol, 13.1 mL, 3.00 eq) in DCM (3.00 mL) was added TfzO (8.45 g, 30.0 mmol, 4.94 mL, 1.20 eq) dropwise at - 40 °C. The mixture was stirred for 1 hour. The mixture was diluted with dichloromethane (200 mL) and washed with water (1 × 200 mL) and brine (1 × 200 mL). The separated organic layer was dried over sodium sulfate, filtered and concentrated under vacuum. The residue was purified by column chromatography over silica gel (petroleum ether/ethyl acetate 100/1 to 10/1). 3-methoxy-6-methyl-1-naphthyl trifluoromethanesulfonate (7.70 g, 24.0 mmol, 96.2 % yield, 99.9 % purity) was obtained as a colorless oil. **ESI** MS m/z 320.7 [M+H]⁺.

The following intermediates were prepared according to the preparation for Intermediate 3, substituting the appropriate phenol for 2-bromo-3-fluorophenol.

| Intermediate No. | Structure | Name |
|---|---|---|
| | | |
| Intermediate 31 | 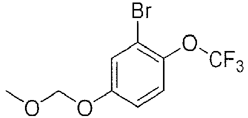 | 2-bromo-4-(methoxymethoxy)-1-(trifluoromethoxy)benzene |
| Intermediate 32 | 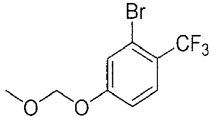 | 2-bromo-4-(methoxymethoxy)-1 - (trifluoromethyl)benzene |
| Intermediate 33 | 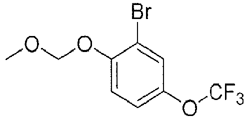 | 2-bromo-1-(methoxymethoxy)-4-(trifluoromethoxy)benzene |
| Intermediate 34 | 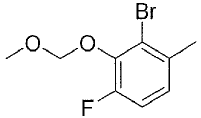 | 2-bromo-4-fluoro-3-(methoxymethoxy)-1-methylbenzene |
| Intermediate 35 | 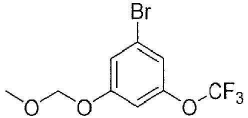 | 1 -bromo-3-(methoxymethoxy)-5-(trifluoromethoxy)benzene |
| Intermediate 36 | 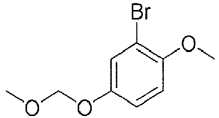 | 2-bromo-1-methoxy-4-(methoxymethoxy)benzene |
| Intermediate 37 | 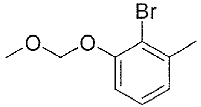 | 2-bromo-1-(methoxymethoxy)-3-methylbenzene |
| Intermediate 38 | 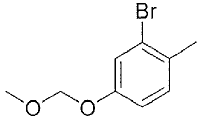 | 2-bromo-4-(methoxymethoxy)-1-methylbenzene |
| Intermediate 39 | 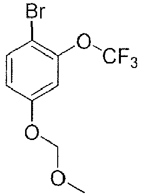 | 1-bromo-4-(methoxymethoxy)-2-(trifluoromethoxy)benzene |

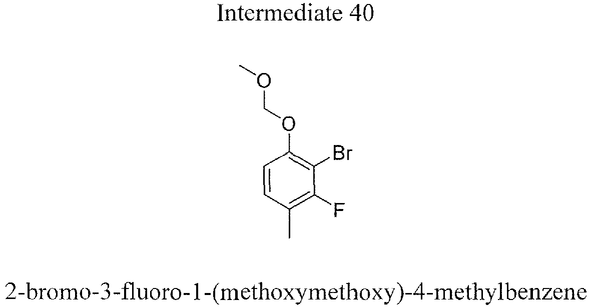

Step 1: 3-fluoro-4-methylphenol (1.016 g, 8.055 mmol) was placed in Cs₂ (3.9 mL, 64.44 mmol) and was cooled to 0°C. Br₂ (0.4150 mL, 8.055 mmol) was added and the mixture was stirred at room temperature for 2 hrs. 10% Na₂S₂O₂ was added and the mixture was extracted with DCM. The organic layers were combined, dried and filtered to provide 2-bromo-3-fluoro-4-methylphenol (1.389 g, 6.775 mmol, 84.10 % yield) which was used directly in the next step.

Step 2: 2-bromo-3-fluoro-1-(methoxymethoxy)-4-methylbenzene was prepared according to the procedure for Intermediate 8 using 2-bromo-3-fluoro-4-methylphenol in place of 2-bromo-3-fluorophenol.

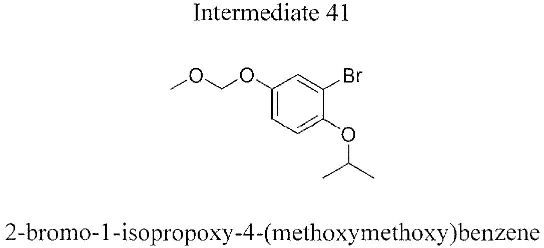

Step 1: 4-isopropoxyphenol (1.00 g, 6.57 mmol) and TEA (1.83 mL, 13.1 mmol) were placed in DCM (25 mL). Acetyl chloride (7.56 mL, 7.56 mmol) was added dropwise and the reaction was stirred at room temperature for 2hr. Water was added and the mixture was extracted with DCM. The organic layer was dried, filtered and concentrated to provide 4-isopropoxyphenyl acetate (1.24 g, 6.38 mmol, 97.2 % yield) which was directly in the next step.

Step2: 4-Isopropoxyphenyl acetate (1.24 g, 6.585 mmol) was placed in ACN (20 mL) and N-bromosuccinimide (1.173 g, 6.590 mmol) was added. The mixture was stirred for 18 hr. Water was added and the mixture was extracted with ether. The organic layers were combined, dried, and concentrated to provide 3-bromo-4-isopropoxyphenyl acetate (1.584 g, 5.800 mmol, 88.00 % yield) which was directly in the next step.

Step 3: 3-Bromo-4-isopropoxyphenyl acetate (500 mg, 1.83 mmol) was placed in MeOH (7 mL). A solution of KOH (111 mg, 1.98 mmol) in water (2 mL) was added to mixture and was stirred for 1 hr at room temperature. The reaction mixture was adjusted to pH 3 by the addition of 1N HCl. The mixture was extracted with DCM. The extracts were combined, dried, filtered and concentrated to provide crude 3-bromo-4-isopropoxyphenol which was used directly the next reaction.

Step 4: 2-Bromo-1-isopropoxy-4-(methoxymethoxy)benzene was prepared according to the procedure for Intermediate 8 using 3-bromo-4-isopropoxyphenol in place of 2-bromo-3-fluorophenol

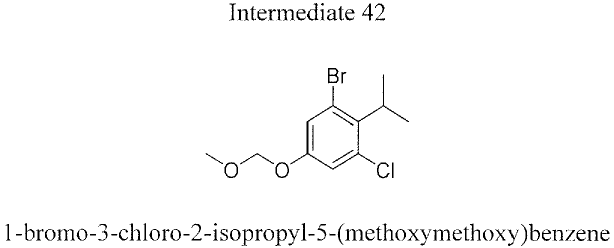

Step 1: 1-bromo-3-chloro-2-isopropyl-5-methoxybenzenc (952 mg, 3.61 mmol) was placed in DCM (3 mL) and was cooled to 0°C. BBr3 (9030 µL, 9.03 mmol) was added and the reaction was stirred at 0°C for 2 hr. Water was added and the mixture was extracted with DCM. The extracts were combined and concentrated. The resulting residue was purified by silica gel (0-20% EtOAc in hexane) to provide 3-bromo-5-chloro-4-isopropylphenol (575 mg, 2.30 mmol, 63.8 % yield)

Step 2: 1-bromo-3-chloro-2-isopropyl-5-(methoxymethoxy)benzene was prepared according to the procedure for Intermediate 8 using 3-bromo-5-chloro-4-isopropylphenol in place of 2-

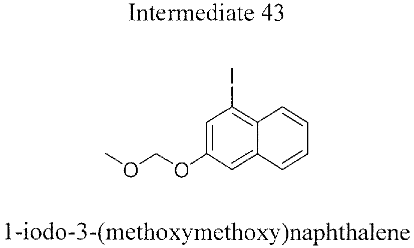

To a solution of 4-iodonaphthalen-2-ol (0.80 g, 3.0 mmol) in DCM (20 mL) was added N-ethyl-N-isopropylpropan-2-amine (1.1 mL, 5.9 mmol) and chloro(methoxy)methane (0.29 g, 3.6 mmol) and the reaction stirred at room temperature for 4 hours, with additional chloro(methoxy)methane (0.15 g) being added after 2 hours. The reaction was washed with brine and concentrated *in vacuo.* The material was purified by chromatography using a gradient of 0 to 10% EtOAc/hexanes as the eluent to give 1-iodo-3-(methoxymethoxy)naphthalene (0.80 g, 2.5 mmol, 86 % yield).

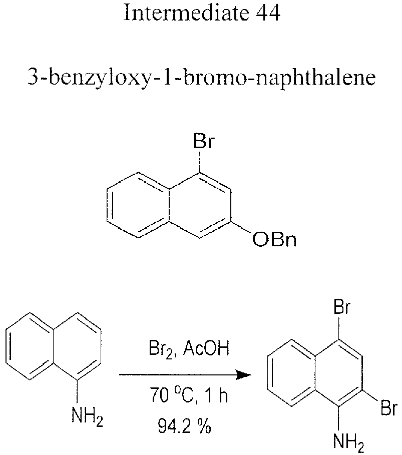

Step A: 2,4-dibromonaphthalen-1-amine: To a solution of Br₂ (246 g, 1.54 mol, 79.3 mL) in AcOH (750 mL) was added a solution of naphthalen-1-amine (101 g, 705 mmol, 99.0 mL) in AcOH (500 mL) at room temperature and the reaction stirred at 70 °C for 1 hour. The reaction mixture was cooled to room temperature and filtered. The filter cake was washed with AcOH (300 mL). The solid was next suspended in 20 % aqueous of NaOH (1.2 L). The mixture was stirred for 20 minutes and filtered. The solid was washed with water (1 L) and dried under vacuum to give 2,4-dibromonaphthalen-1-amine (200 g, 664 mmol, 94.2% yield) as gray solid. ES+APCI MS m/z 301.9 [M+H]⁺.

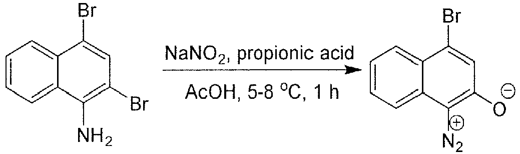

Step B: 4-bromo-1 -diazonio-naphthalen-2-olate: To a solution of 2,4-dibromonaphthalen-1-amine (60.0 g, 199 mmol) in AcOH (900 mL) and propionic acid (150 mL) was added NaNO₂ (16.5 g, 239 mmol, 13.0 mL) portionwise at 5-8 °C over 30 minutes and the reaction mixture stirred at 5-8 °C for 30 minutes. The reaction mixture was poured into ice-water (4000 mL), the slurry filtered and the solid washed with water (2 × 50 mL) to give 4-bromo-1-diazonionaphthalen-2-olate (150 g, wet crude) which was used crude in the next step immediately. ¹H NMR (400 MHz, CDCl₃) δ 8.12 - 8.10 (d, *J*=8.4 Hz, 1H), 7.62 - 7.58 (t, *J*=7.6 Hz, 1H), 7.41 - 7.37 (t, *J*=7.6 Hz, 1H), 7.31 - 7.29 (d, *J*=8.0 Hz, 1H), 7.20 (s, 1H).

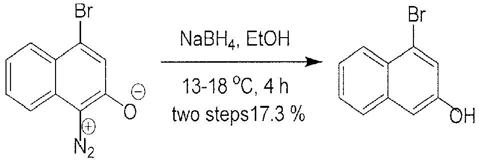

Step C: 4-bromonaphthalen-2-ol: To a solution of 4-bromo-1-diazonio-naphthalen-2-olate (100 g, 402 mmol) in EtOH (2.00 L) was added portion-wise NaBH₄ (30.4 g, 803 mmol) at 13-15 °C over 1 hour and the reaction stirred at 15-18 °C for 3 hours. The reaction was filtered and concentrated to dryness. The residue was dissolved in DCM (1000 mL) and washed with water (500 mL × 2). The organics were dried over Na₂SO₄ and concentrated to dryness. The residue was purified by chromtography eluting with petroleum ether/EtOAc (60/1 → 10/1) and material re-purified by reverse phase HPLC to give 4-bromonaphthalen-2-ol (40.0 g, 139 mmol, 17.3 % yield, 77.4% purity) as a gray solid. ¹H NMR (400 MHz, CDCl₃) δ 8.07 - 8.05 (d, *J*=8.0 Hz, 1H), 7.60 - 7.58 (d, *J*=7.6 Hz, 1H), 7.41 - 7.36 (m, 3H), 7.07 (s, 1H).

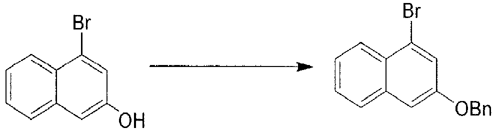

Step D: 3-benzyloxy-1-bromo-naphthalene: A mixture of 4-bromonaphthalen-2-ol (30.0 g, 134 mmol), BnBr (25.3 g, 148 mmol, 17.6 mL) and K₂CO₃ (55.7 g, 403 mmol) in MeCN (500 mL) was heated at 80 °C for 1 hr. The reaction mixture was filtered and concentrated to dryness. The residue was purified by silica gel column eluting with PE/EA (100/1 to 60/1) to give 3-benzyloxy-1-bromo-naphthalene (40.0 g, 128 mmol, 95 % yield). ¹H NMR (400 MHz, CDCl₃) δ 8.19 - 8.17 (d, *J*=8.0 Hz, 1H), 7.75 - 7.32 (d, *J*=8.8 Hz, 1H), 7.64 - 7.63 (d, *J*=2.4 Hz,1H), 7.52 - 7.37 (m, 7H), 7.23 - 7.21 (d, *J*=2.0 Hz,1H), 5.2 (s, 2H).

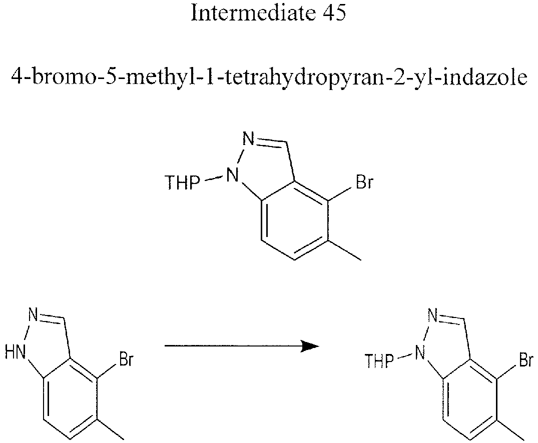

Step A: 4-bromo-5-methyl-1-tetrahydropyran-2-yl-indazole: To a mixture of 4-bromo-5-methyl-1*H*-indazole (3 g, 14.2 mmol) and 3,4-dihydro-2*H*-pyran (2.39 g, 28.4 mmol, 2.60 mL) in DCM (30 mL) was added TsOH^{∗}H₂O (270 mg, 1.42 mmol) and the mixture stirred at 15 °C for 2 hours. After completion, the reaction mixture was concentrated under vacuum and the residue purified by column chromatography using 5→20& EtOAc/Petroleum Ether as eluent to give 4-bromo-5-methyl-1-tetrahydropyran-2-yl-indazole (4 g, 13.6 mmol, 95.3% yield) as white solid. ¹H NMR (400 MHz, chloroform-d) δ 8.01 (s, 1H), 7.47 (d, *J*=8.4 Hz, 1H), 7.25 (d, *J*=8.4 Hz, 1H), 5.70 (dd, *J*=2.8, 9.2 Hz, 1H), 4.05 - 3.96 (m, 1H), 3.79 - 3.70 (m, 1H), 2.66 - 2.44 (m, 4H), 2.25 - 2.04 (m, 2H), 1.84 - 1.56 (m, 3H).

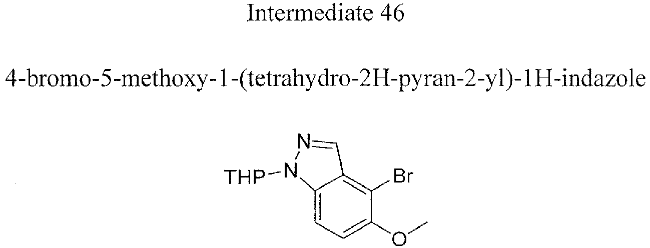

4-bromo-5-methoxy-1-(tetrahydro-2H-pyran-2-yl)-1H-indazole was prepared following Intermediate 51 substituting 4-bromo-5-methoxy-1H-indazole for 4-bromo-5-methyl-1*H-*indazole in Step A. ¹H NMR (400 MHz, chloroform-d) δ 8.00 (s, 1H), 7.53 (d, *J*=9.2 Hz, 1H), 7.16 (d, *J*=9.2 Hz, 1H), 5.70 (dd, *J*=2.8, 9.2 Hz, 1H), 4.04 - 3.98 (m, 1H), 3.96 (s, 3H), 2.55 - 2.49 (m, 1H), 2.23 - 2.05 (m, 2H), 1.83 - 1.69 (m, 3H).

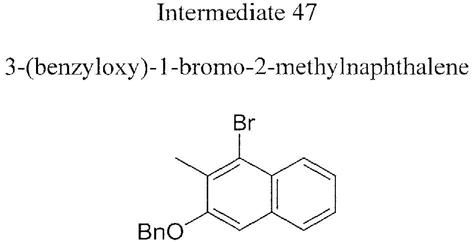

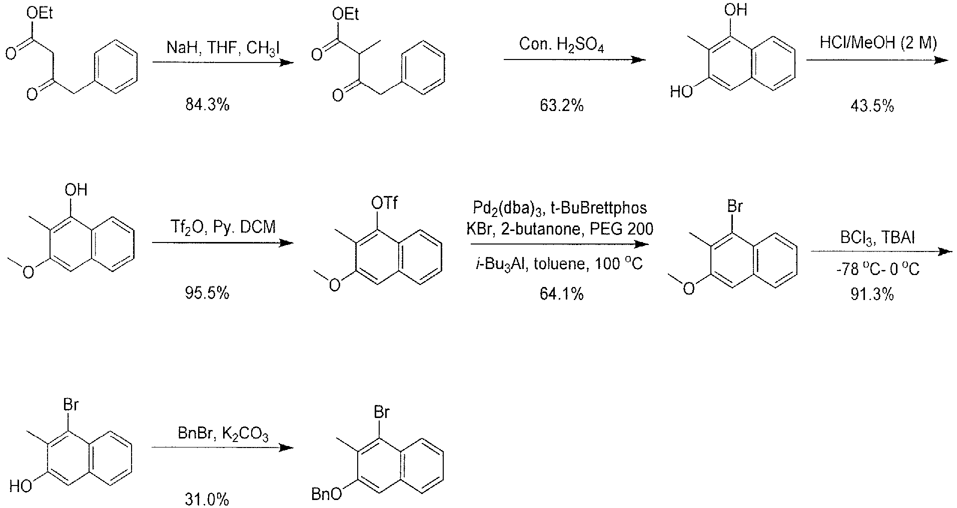

Step A: ethyl 2-methyl-3-oxo-4-phenyl-butanoate. To a dried 250 ml three-necked flask was added ethyl 3-oxo-4-phenyl-butanoate (4.00 g, 19.4 mmol.), THF (50.0 mL), sodium hydride (931 mg, 23.3 mmol) and the reaction stirred for 0.5 hours at 0°C. A solution of methyl iodide (3.03 g, 21.3) was next added drop-wise. After addition was completed, the reaction mixture was warmed to 20 °C and stirred for two hours at 20°C. The reaction mixture was quenched by addition of water (10.0 mL) at 20 °C and then diluted with ethyl acetate (20.0 mL) and the layers separated. The aqueous layer was next extracted with ethyl acetate (20.0 mL × 3). The combined organic layers were washed with brine (30.0 mL), dried over anhydrous sodium sulfate, filtered and concentrated under reduced pressure to give a residue. The residue was purified by column chromatography (SiO2, Petroleum ether : Ethyl acetate 20:1 to 10:1) to give ethyl 2-methyl-3-oxo-4-phenyl-butanoate (3.60 g, 16.3 mmol, 84.3% yield) as a colorless oil. ¹H NMR (400 MHz, CDCl₃) δ = 7.38 - 7.28 (m, 3H), 7.25 - 7.19 (m, 2H), 4.22 - 4.15 (m, 2H), 3.87 (d, *J* = 2.0 Hz, 2H), 3.65 (q, *J* = 7.2 Hz, 1H), 1.34 (d, *J* = 7.2 Hz, 3H), 1.30 - 1.26 (m, 3H).

Step B: 2-methylnaphthalene-1,3-diol. A solution of ethyl 2-methyl-3-oxo-4-phenylbutanoate (3.60 g, 16.3 mmol) in concentrated sulfuric acid (19.9 g, 203 mmol) was stirred at 15 °C for 12 hours. The reaction mixture was poured into ice-water (30.0 mL) and the resulting solid collected by filtration and dried under vacuum to afford 2-methylnaphthalene-1,3-diol (1.80 g, 10.3 mmol, 63.2% yield) as a red solid. ¹H NMR (400 MHz, CDCl₃) δ = 8.02 (d, *J* = 8.0 Hz, 1H), 7.65 - 7.54 (m, 1H), 7.41 (t, *J* = 7.2 Hz, 1H), 7.36 - 7.31 (m, 1H), 6.80 (s, 1H), 4.29 - 4.20 (s, 2H), 2.41 - 2.24 (s, 3H).

Step C: 3-methoxy-2-methyl-naphthalen-1-ol. 2-methylnaphthalene-1,3-diol (1.70 g, 9.76 mmol) was added to HCl/MeOH (2 M, 35.0 mL) and the result mixture was stirred at 30 °C for 3 days. The reaction was concentrated in vacuo and the residue purified by Prep-TLC (Petroleum ether : Ethyl acetate 1:1) to give 3-methoxy-2-methyl-naphthalen-1-ol (800 mg, 4.25 mmol, 43.5% yield) as a white solid. ¹H NMR (400 MHz, CDCl₃) δ = 8.02 (d, *J* = 8.4 Hz, 1H), 7.69 (d, *J* = 8.4 Hz, HI), 7.44 - 7.38 (m, 1H), 7.37 - 7.31 (m, 1H), 6.79 (s, 1H), 5.14 (s, 1H), 3.94 (s, 3H), 2.29 (s, 3H).

Step D: (3-methoxy-2-methyl-1-naphthyl)trifluoromethanesulfonate. To a mixture of 3-methoxy-2-methyl-naphthalen-1-ol (800 mg, 4.25 mmol.) and pyridine (504 mg, 6.38 mmol) in DCM (10.0 mL) was added trifluoroacetic anhydride (1.44 g, 5.10 mmol) dropwise at 0°C under N₂ atmosphere. The mixture was warmed to 20°C and stirred for an additional 5 hours. The solvent was removed under vacuum and the residue purified by Prep-TLC (Petroleum ether : Ethyl acetate 1:1) to give (3-methoxy-2-methyl-1-naphthyl)trifluoromethanesulfonate (1.30 g, 4.06 mmol, 95.5% yield) as a white solid. ¹H NMR (400 MHz, CDCl₃) δ = 7.97 (d, *J* = 7.6 Hz, 1H), 7.79 - 7.74 (m, 1H), 7.52 - 7.43 (m, 2H), 7.14 (s, 1H), 3.99 (s, 3H), 2.42 (s, 3H)

Step E: 1-bromo-3-methoxy-2-methyl-naphthalene : In a sealed tube was added (3-methoxy-2-methyl-1-naphthyl)trifluoromethanesulfonate (466 mg, 1.45 mmol), t-Bu-Brettphos (154 mg, 290 µmol), potassium bromide (259 mg, 2.17 mmol), PEG-200 (175 mg), 2-butanone (157 mg, 2.17 mmol) and Pd₂(dba)₃ (133 mg, 145 µmol) in toluene (10.0 mL) and the mixture de-gassed with N2 for 5 minutes. Next, triisobutylaluminum (431 mg, 2.17 mmol) was added drop-wise at 20 °C. The mixture was heated to 100 °C for 24 hrs. The reaction mixture was poured into water (30.0 mL) and the aqueous layer extracted with ethyl acetate (20.0 mL × 3). The combined organics were washed with brine (30.0 mL), dried over anhydrous sodium sulfate and concentrated in vacuo to give a residue which was pre-purified by column chromatography (Petroleum ether:Ethyl acetate 10:1) and then by Prep-TLC (Petroleum ether : Ethyl acetate 10:1) to give 1-bromo-3-methoxy-2-methyl-naphthalene (700 mg, 2.79 mmol, 64.1% yield) as a white solid. ¹H NMR (400 MHz, CDCl₃) δ = 8.26 - 8.17 (m, 1H), 7.73 - 7.69 (m, 1H), 7.47 - 7.40 (m, 2H), 7.09 (s, 1H), 3.98 - 3.95 (m, 311), 2.56 (s, 3H).

Step F: 4-bromo-3-methyl-naphthalen-2-ol: To a solution of 1-bromo-3-methoxy-2-methyl-naphthalene (580 mg, 2.31 mmol) and tetrabutylammonium iodide (2.13 g, 5.78 mmol) in DCM (11.0 mL) cooled to -78 °C was added a solution of BCl₃ (1 M, 5.78 mL) dropwise over a period of 10 minutes while under N₂. The reaction mixture was warmed to 0 °C and stirred for 2 hours at room temperature. Next the solvent was removed under vacuum and the residue was purified by Prep-TLC (Petroleum ether : Ethyl acetate 5:1) to give 4-bromo-3-methyl-naphthalen-2-ol (500 mg, 2.11 mmol, 91.3% yield) as a white solid. ¹H NMR (400 MHz, CDCl₃) δ = 8.26 - 8.15 (m, 1H), 7.63 (dd, *J* = 3.6, 6.0 Hz, 1H), 7.45 - 7.38 (m, 2H), 7.11 (s, 1H), 5.09 (s, 1H), 2.60 (s, 3H), 1.56 (s, 3H).

Step G: 3-benzyloxy-1-bromo-2-methyl-naphthalene. To a mixture of 4-bromo-3-methyl-naphthalen-2-ol (265 mg, 1.12 mmol) and benzyl bromide (201 mg, 1.18 mmol) in acetonitrile (3.00 mL) was added potassium carbonate (310 mg, 2.24 mmol) in one portion at 20 °C under N₂. The mixture was next stirred at 60°C for two hours. The solvent was removed under vacuum and the residue purified by Prep-TLC (Petroleum ether : Ethyl acetate 5:1) to give the 3-benzyloxy-1-bromo-2-methyl-naphthalene (250 mg, 695 µmol, 31.0% yield, 91.0% purity) as a white solid. ES+APCI MS m/z 327.0, 329.0 [M+H]⁺.

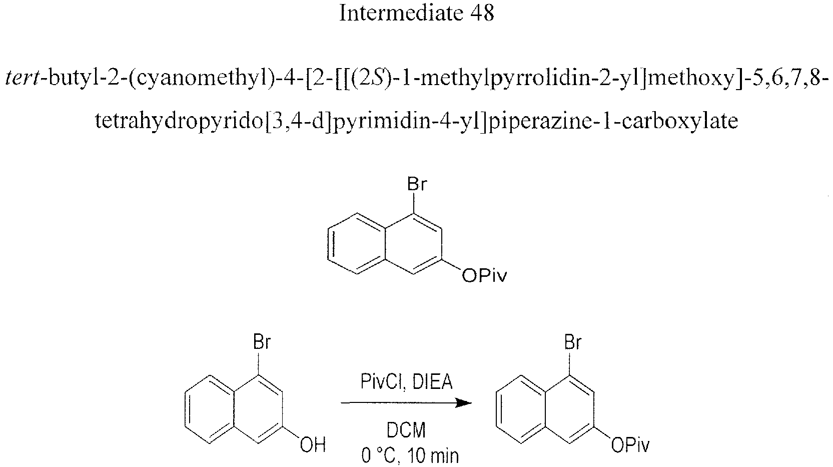

Step A: (4-bromo-2-naphthyl) 2,2-dimethylpropanoate. To a solution of 4-bromonaphthalen-2-ol (10 g, 44.8 mmol) and TEA (9.07 g, 89.7 mmol) in DCM (200 mL) was added 2,2-dimethylpropanoyl chloride (8.11 g, 67.2 mmol) at 0°C. The reaction mixture was stirred at 0 °C for 10 min. T reaction mixture was quenched by addition of water (50 mL) and the layers separated. The organic layer was washed with brine (30 mL), dried over Na₂SO₄ filtered and concentrated under vacuum. The residue was purified by silica gel chromatography (PE: EA =1:0 to 100:1) to give (4-bromo-2-naphthyl) 2,2-dimethylpropanoate (9 g, 29.3 mmol, 65.4% yield) as a red oil. ¹H NMR (400MHz, CHLOROFORM-d) δ = 8.22 (d, *J*=8.0 Hz, 1H), 7.83 - 7.77 (m, 1H), 7.63 - 7.49 (m, 4H), 1.41 (s, 9H).

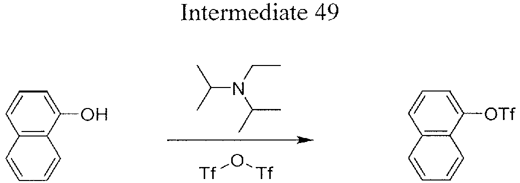

Naphthalen-1-yl trifluoromethanesulfonate. alpha-Naphthol (4 g, 27.74 mmol) was dissolved in DCM (200 mL) in a 3 neck flask. The reaction was cooled to 10°C in a water bath. N-ethyl-N-isopropylpropan-2-amine (4.846 ml, 27.74 mmol) and trifluoromethanesulfonic anhydride (4.668 ml, 27.74 mmol) were added to the solution dropwise. The reaction was stirred at 10°C for 2 hours. TLC (25% EtOAc, UV vis) showed reaction complete. The organics were with water (2X) and brine (2X). The organics were dried over MgSO4 and concentrated in vacuo. The concentrate was purified using normal phase chromatography on the CombiFlash (0%-12% EtOAc:Hexanes). All fractions containing clean product were combined and conentrated in vacuo to give naphthalen-1-yl trifluoromethanesulfonate (6.77 g, 24.51 mmol, 88.34 % yield).

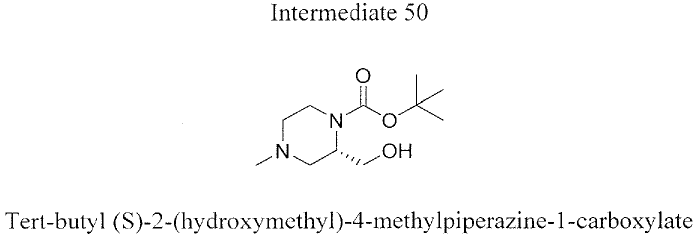

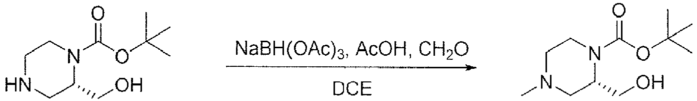

To a solution of (S)-1-Boc-2-hydroxymethylpiperazine (1.0 g, 4.62 mmol) in DCE (92.47 ml, 4.624 mmol) was added formaldehyde (3.474 ml, 46.24 mmol) (37% in water) followed by sodium triacetoxyborohydride (4.9 g, 23.12 mmol). The mixture was stirred vigorously at room temperature for 2.5hours. The mixture was treated with saturated sodium bicarbonate (30 mL), stirred for 10 min then extracted with DCM (3 × 10 mL). The combined organic phases were dried over sodium sulfate, filtered and concentrated. ES+APCI MS m/z 231.1 [M+H]⁺.

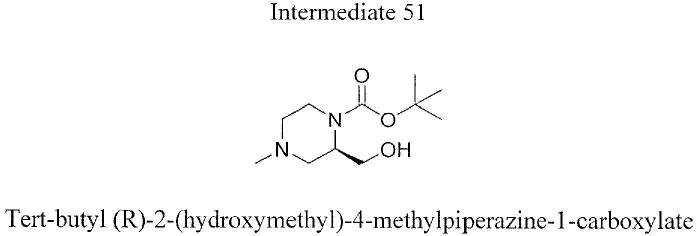

Title compound was prepared as in Intermediate 57, substituting tert-butyl (R)-2-(hydroxymethyl)piperazine-1-carboxylate for (S)-1-Boc-2-hydroxymethylpiperazine. ES+APCI MS m/z 231.1 [M+H]⁺

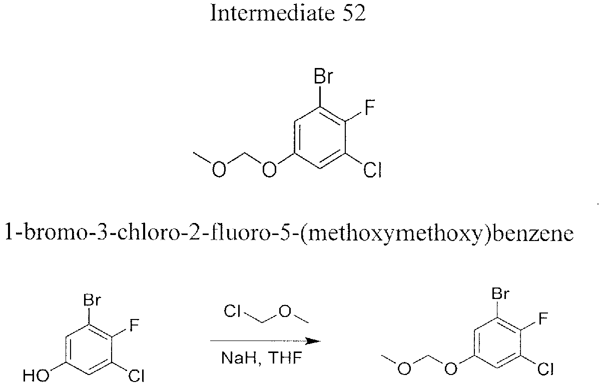

To a round bottom flask was added THF (8.87 ml, 4.44 mmol) followed by sodium hydride, 60 % dispersion in mineral oil (0.213 g, 5.32 mmol). The mixture was cooled to 0 °C then 3-bromo-5-chloro-4-fluorophenol (1.0 g, 4.44 mmol) was added portionwise. Once the bubbling had ceased the resulting dark mixture was stirred at 0 °C for 30 min. Then chloromethyl methyl ether (0.421 ml, 5.54 mmol) was added and the mixture was warmed to ambient temperature where it was stirred for 2 hr. A saturated aqueous ammonium chloride solution was added and the mixture was extracted with DCM. The organic layer was dried over sodium sulfate, filtered and concentrated. Crude material was chromatographed (0-15% EtOAc in hexanes) to provide product as clear oil.

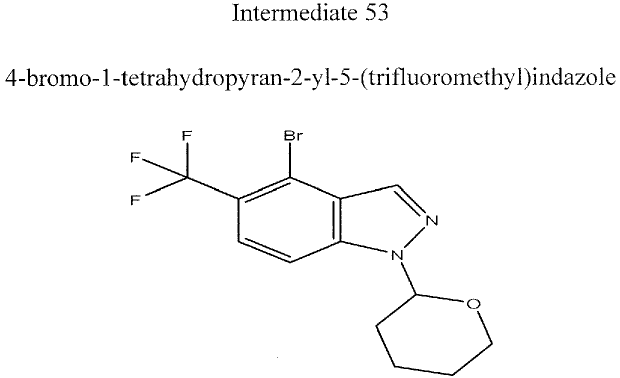

Step A: 4-bromo-1-tetrahydropyran-2-yl-5-(trifluoromethyl)indazole: To a solution of 4-bromo-5-(trifluoromethyl)-1H-indazole (500 mg, 1.89 mmol, 1 *eq)* in DCM (10 mL) was added 3,4-dihydro-2H-pyran (476 mg, 5.66 mmol, 517 uL, 3 *eq)* and TsOH·H₂O (35.9 mg, 188 µmol, 0.1 *eq).* The mixture was stirred at 15 °C for 1 hour. The mixture was concentrated. The residue was purified by column chromatography (SiO₂, PE:EA=10:1 to 1:1) to give 4-bromo-1-tetrahydropyran-2-yl-5-(trifluoromethyl)indazole (480 mg, 1.37 mmol, 72.9% yield) as yellow oil.¹H NMR (400 MHz, chloroform-d) δ 8.20 (s, 1H), 7.69 - 7.63 (m, 2H), 5.70 (dd, *J*=2.8, 8.8 Hz, 1H), 4.05 - 3.96 (m, 1H), 3.79 - 3.70 (m, 1H), 2.56 - 2.50 (m, 1H), 2.27 - 2.04 (m, 2H), 1.80 - 1.74 (m, 2H), 1.60 - 1.54 (m, 1H).

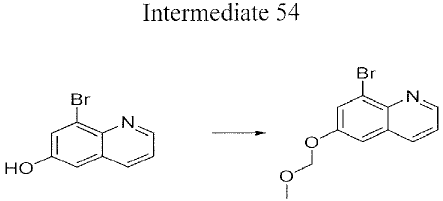

8-bromo-6-(methoxymethoxy)quinoline: A stirred suspension of 8-bromoquinolin-6-ol (1.00 g, 4.46 mmol) in DCM (20 mL) was cooled to 0°C and diisopropylethylamine (1.2 mL, 6.7 mmol, 1.5 eq.) was added followed by chloro(methoxy)methane (0.41 mL, 5.4 mmol, 1.2 eq.) dropwise and the reaction mixture was warmed to room temperature overnight. Concentrated aqueous ammonia (0.5 mL, ~5 mmol) was next added and the resulted mixture was stirred for 1 hour at room temperature. The mixture was evaporated in vacuo and chromatographed on silica gel, Redisep 40g, using 20% EtOAc/hexane as eluent to give a colorless powder (0.52 g, 44%). ES+APCI MS m/z 268.0, [M+H]⁺.

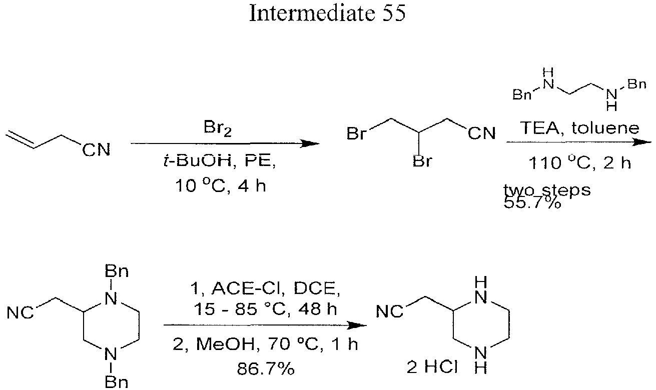

To a solution of but-3-enenitrile (80.0 g, 1.19 mol, 96.4 mL, 1.00 eq) in tert-butanol (130 mL) and petroleum ether (480 mL) was added a solution of Br₂ (191 g, 1.19 mol, 61.5 mL, 1.00 eq) in *tert*-butanol (130 mL). The mixture was stirred at 10 °C for 4 hours. The mixture was used into next step without any workup.

To the above mixture (274 mL) was added a solution of *N*,*N*'-dibenzylethane-1,2-diamine (160 g, 445 mmol, 157 mL, 2 HOAc) and Et₃N (178 g, 1.76 mol, 245 mL) in toluene (300 mL). After was stirred at 110 °C for 2 hours, the mixture was filtered and the filtrate was concentrated under vacuum. The residue was purified by column chromatography (SiO₂, petroleum ether/ethyl acetate = 3/1) to give 2-(1, 4-dibenzylpiperazin-2-yl)acetonitrile (75.0 g, 246 mmol, two steps 55.7 % yield) as a yellow solid. LCMS [ESI, M+1]: 306.

¹H NMR (400MHz, chloroform-d) δ = 7.37 - 7.23 (m, 10H), 3.80 (d, *J* = 13.2 Hz, 1H), 3.60 - 3.42 (m, 3H), 3.06 - 2.96 (m, 1H), 2.95 - 2.83 (m, 1H), 2.69 - 2.53 (m, 4H), 2.52 - 2.35 (m, 3H).

To a solution of 2-(1,4-dibenzylpiperazin-2-yl)acetonitrile (160 g, 524 mmol, 1.00 eq) in dichloroethane (1.50 L) was added 1-chloroethyl carbonochloridate (300 g, 2.10 mol, 4.00 eq) at 15 °C. After stirred at 85 °C for 48 h, the mixture was concentrated under vacuum. The residue was then taken up into methanol (1.50 L) and heated to reflux for 1 hour. The mixture was concentrated. The solid was treated with methyl tert-butyl ether (1.00 L), 2-piperazin-2-ylacetonitrile (Intermediate 62, 90.0 g, 454 mmol, 86.7 % yield, 2HCl) was obtained as a white solid and used for next step without further purification.

¹H NMR (400MHz, DMSO-d6) δ = 10.19 (br s, 2H), 4.01 - 3.73 (m, 1H), 3.69 - 3.41 (m, 4H), 3.32 (dt, *J* = 2.8, 13.2 Hz, 1H), 3.27 - 3.10 (m, 3H).

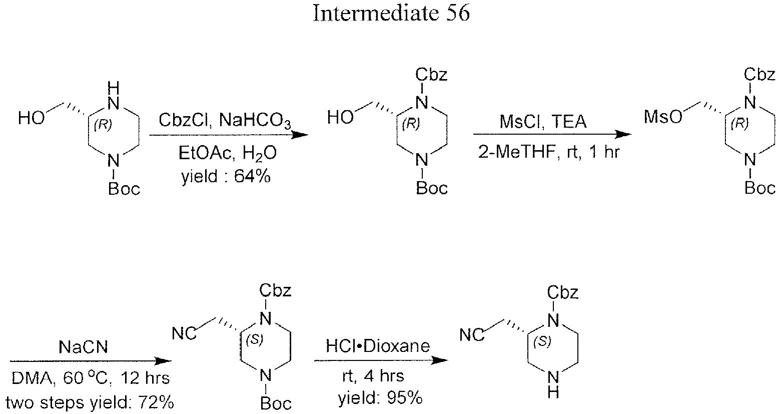

To a solution of *tert*-butyl (3*R*)-3-(hydroxymethyl)piperazine-1-carboxylate (80.0 g, 370 mmol, 1.0 *eq*) in Ethyl acetate (1400 mL) was added NaHCO₃ (93.2 g, 1.11 mol, 43.2 mL, 3.0 *eq*)*,* H₂O (700 mL) and benzyl carbonochloridate (82.0 g, 481 mmol, 68.4 mL, 1.30 *eq*)*.* The mixture was stirred at 25 °C for 12 hour. After completion, the organic phase was separated, washed with water (500 mL × 2) dried over Na₂SO₄ and filtered. The solvent was removed under vacuum to give a residue. The residue was purified by column chromatography (SiO₂, Petroleum ether/Ethyl acetate=40/1 to 1/1). The product 1-benzyl 4-*tert*-butyl (2R)-2-(hydroxymethyl)piperazine-1,4-dicarboxylate (85.0 g, 235 mmol, 64% yield, 96% purity) was obtained as a yellow oil. LCMS [ESI, M-99]: 251.

To a solution of 1-benzyl 4-tert-butyl (2*R*)-2-(hydroxymethyl)piperazine-1,4-dicarboxylate (20.0 g, 57.1 mmol, 1.0 *eq*) in 2-Methyltetrahydrofuran (240 mL) was added TEA (17.3 g, 171.23 mmol, 23.8 mL, 3.0 *eq*) and methanesulfonyl chloride (7.74 g, 67.6 mmol, 5.23 mL, 1.18 *eq*)*.* The mixture was stirred at 20 °C for 1 hour. The reaction mixture was quenched by addition H₂O 150 mL at 20 °C. The reaction mixture was extracted with Ethyl acetate (300 mL × 2). The organic layers were washed with H₂O (100 mL), dried over Na₂SO₄, and filtered. The solvent was removed under vacuum. 1-benzyl 4-*t*ert-butyl (2*R*)-2-(methylsulfonyloxymethyl)piperazine-1,4-dicarboxylate (22.0 g, crude) was obtained as a yellow oil. The crude product was used directly to the next step without further purification.

To a solution of 1-benzyl 4-tert-butyl (2*R*)-2-(methylsulfonyloxymethyl)piperazine-1,4-dicarboxylate (22.0 g, 51.3 mmol) in DMA (150 mL) was added NaCN (10.4 g, 211 mmol). The mixture was stirred at 60 °C for 12 hour. The solvent was removed under vacuum to give a oil residue. The residue was diluted with H₂O (40.0 mL) and extracted with Ethyl acetate (50.0 mL × 3). The combined organic layers were washed with saturated brine (80.0 mL), dried over Na₂SO₄, filtered and concentrated under reduced pressure to give a residue. The residue was purified by column chromatography (SiO₂, Petroleum ether/Ethyl acetate=40/1 to 5:1) The product 1-benzyl 4-tert-butyl (2*S*)-2-(cyanomethyl)piperazine-1,4-dicarboxylate (18.5 g, 46.4 mmol, two steps yield 72%) was obtained as a yellow oil. LCMS [ESI, M+1]: 360.

To a solution of 1-benzyl 4-tert-butyl (2*S*)-2-(cyanomethyl)piperazine-1,4-dicarboxylate (18.5 g, 43.3 mmol, 1.00 eq) in dioxane (40.0 mL) was added HCl•dioxane (4 M, 54.1 mL, 5.0 eq). The mixture was stirred at 20 °C for 1 hour. Then the reaction mixture was added NaHCO₃ to pH>7, and concentrated under reduced pressure to remove dioxane. The residue was diluted with H₂O (50.0 mL) and extracted with Ethyl acetate (50.0 mL × 3). The combined organic layers were washed with H₂O (20.0 mL), dried over Na₂SO₄, filtered and concentrated under reduced pressure to give a residue. The product benzyl (2*S*)-2-(cyanomethyl)piperazine-1-carboxylate (Intermediate 63, 11.5 g, 91.8% purity, 95% yield) was obtained as a yellow oil. LCMS [ESI, M+1]: 260.

¹H NMR (400MHz, CHLOROFORM-d) δ = 7.37 - 7.31 (m, 5H), 5.14 (s, 2H), 4.49 (br, s, 1H), 3.93 (br, s, 1H), 3.07 - 2.81 (m, 5H), 2.78 - 2.54 (m, 2H).

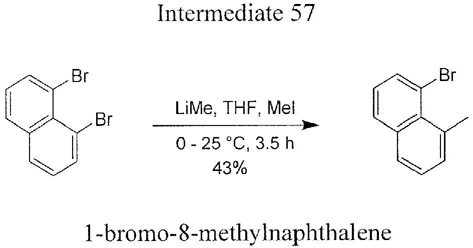

Step A: 1-bromo-8-methyl-naphthalene. To a solution of 1,8-dibromonaphthalene (1 g, 3.50 mmol, 1 *eq*) in THF (20 mL) was added MeLi (1.6 M in diethyl ether, 2.62 mL, 1.2 *eq*) at 0°C dropwise. After stirring for 30 minutes at 0°C, iodomethane (3.38 g, 23.8 mmol, 1.48 mL, 6.81 *eq*) was added dropwise. The mixture was warmed up to 25°C and stirred for another 3 hours. The reaction mixture was quenched with water (20 mL) and extracted with ethyl acetate (20 mL × 3). The combined organic layers were washed with brine (20 mL), dried over Na₂SO₄, filtered and concentrated under reduced pressure to give a residue. The residue was purified by prep-HPLC (column: Phenomenex Gemini C18 250^{∗}50mm^{∗} 10 um; mobile phase: [water (0.05% ammonium hydroxide v/v) - ACN]; B%: 45% - 70%, 28 MIN; 40% min). Ttitle compound 1-bromo-8-methyl-naphthalene (340 mg, 1.49 mmol, 43% yield, 97% purity) was obtained as a yellow solid after lyophilisation.

¹H NMR (400MHz, chloroform-d) δ = 7.75 (dd, *J* = 0.8, 7.2 Hz, 1H), 7.69 (dd, *J* = 0.8, 8.0 Hz, 1H), 7.66 - 7.59 (m, 1H), 7.30 - 7.22 (m, 2H), 7.13 (t, *J* = 8.0 Hz, 1H), 3.05 (s, 3H).

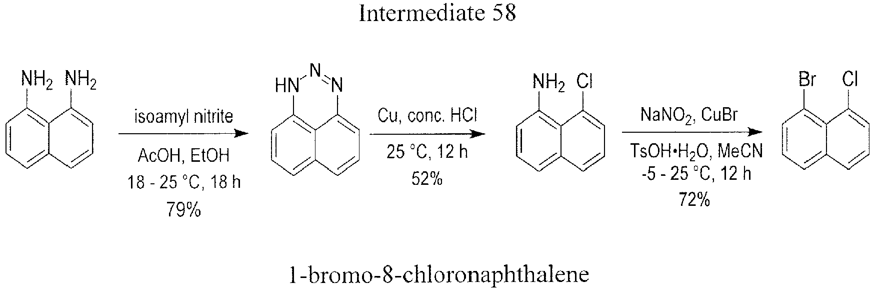

Step A: 1*H*-naphtho[1,8-de][1,2,3]triazine. To a solution of naphthalene-1,8-diamine (100 g, 632 mmol, 1 *eq*) in AcOH (200 mL) and EtOH (1000 mL) was added isoamyl nitrite (72.6 g, 619 mmol, 83.4 mL, 0.98 *eq*) dropwise over a period of 2 h with temperature controlled between 18 and 21 °C under a cold-water bath. After the addition, the resulting red suspension was stirred at 25 °C for 16 hours. The solid was collected by filtration, washed with ethanol (2 × 500 mL) and dried under vacuum. Compound 1*H*-naphtho[1,8-de][1,2,3]triazine (84 g, 496 mmol, 79% yield) was obtained as a red crystalline solid and directly used next step without purification. LCMS [ESI, M+1]: 170.

Step B: 8-chloronaphthalen-1-amine. To a solution of 1*H-*naphtho[1,8-de][1,2,3]triazine (84 g, 496 mmol, 1 *eq*) in HCl (1.5 L) was added Cu (2.10 g, 33.1 mmol, 234 uL, 0.0665 *eq*)*.* The mixture was stirred at 25 °C for 12 hours. The resulting mixture was diluted with water (500 mL) and heated at 85 °C for 30 mins. The resulting almost clear aqueous solution was filtered, cooled, treated with aqueous ammonia (until blue to litmus paper) and the solution was extracted with ether acetate (2 × 1000 mL). The combined extracts were dried over Na₂SO₄, filtered and concentrated under vacuum. The residue was purified by column chromatography (SiO₂, Petroleum ether/Ethyl acetate = 200/1 to 5/1). Compound 8-chloronaphthalen-1-amine (57 g, 259 mmol, 52% yield, 81% purity) was obtained as a red solid. LCMS [ESI, M+1]: 178.

Step C: 1-bromo-8-chloro-naphthalene. To a solution of 8-chloronaphthalen-1-amine (57 g, 320 mmol, 1 *eq*) and TsOH•H₂O (219 g, 1.16 mol, 3.6 *eq*) in MeCN (1000 mL) was added a solution of NaNO₂ (39.8 g, 577 mmol, 1.8 *eq*) and CuBr (138 g, 963 mmol, 29.3 mL, 3 *eq*) in H₂O (120 mL) at - 5 °C, then the reaction mixture was stirred at 25 °C for 12 hours. The reaction mixture was added saturated Na₂SO₃ solution (100 mL) and stirred for 15 mins, then extracted with ethyl acetate (1000 mL×3). The combined organic layers were washed with brine (500 mL), dried over Na₂SO₄, filtered and concentrated under reduced pressure to give a residue. The residue was purified by column chromatography (SiO₂, Petroleum ether). Title compound 1-bromo-8-chloro-naphthalene (56 g, 229 mmol, 72% yield, 99% purity) was obtained as white solid.

¹H NMR (400MHz, chloroform-d) δ = 7.93 (dd, *J* = 1.2, 7.6 Hz, 1H), 7.82 (dd, *J* = 1.2, 8.4, 1H), 7.79 (dd, *J* = 1.2, 8.4, 1H), 7.67 (dd. *J* = 1.2, 7.6 Hz, 1H), 7.37 (t, *J* = 8.0 Hz, 1H), 7.28 (t, *J* = 8.0 Hz, 1H).

### EXAMPLE 1

### 2-[(2S)-4-[8-(8-methyl-1-naphthyl)-2-[[(2S)-1-Methylpyrrolidin-2-yl]methoxy]-5,6,7,9-tetrahydropyrimido[4,5-c]azepin-4-yl]-1-prop-2-enoyl-piperazin-2-yl]acetonitrile

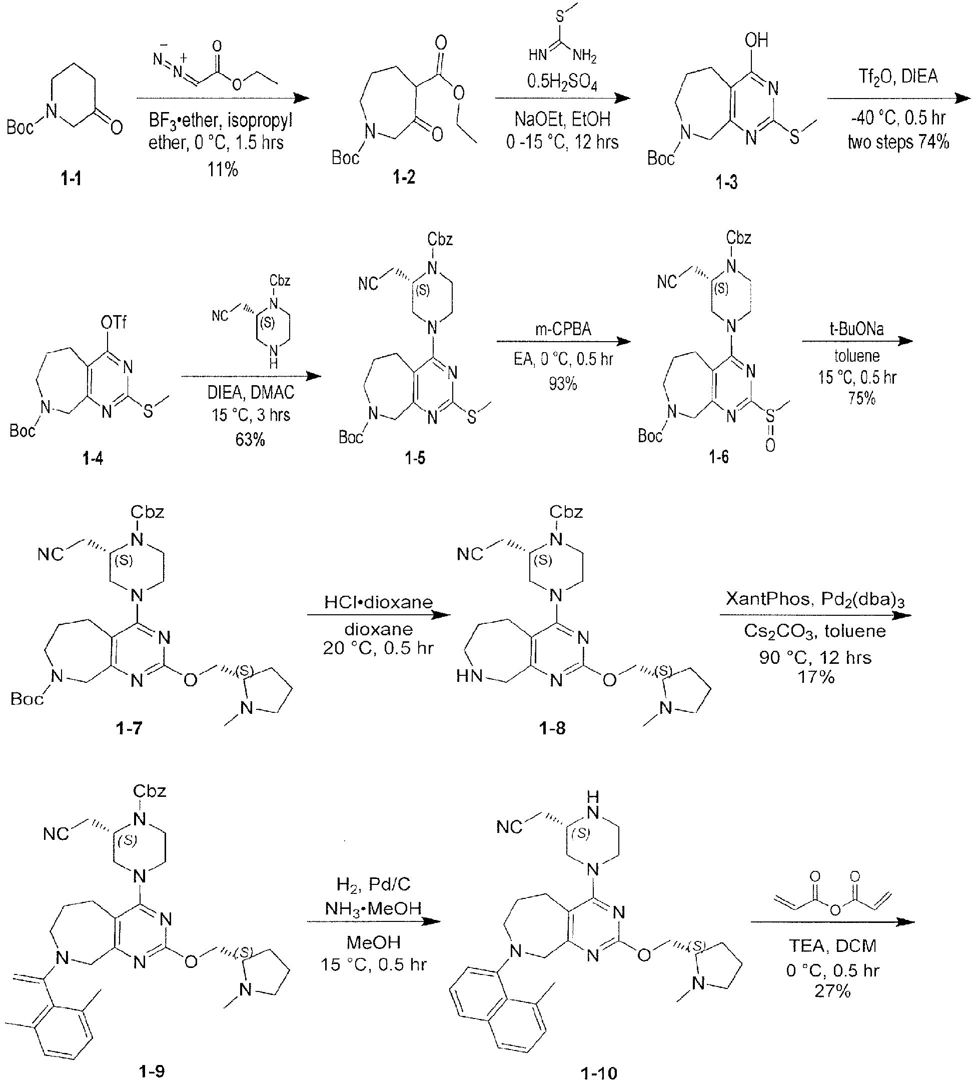

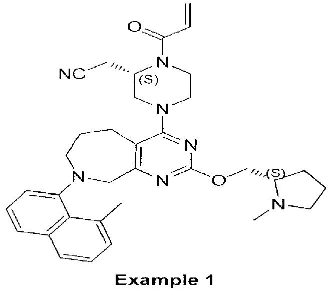

Compound **1-2:** To a solution of *tert-*butyl 3-oxopiperidine-1-carboxylate (20.0 g, 100 mmol, 1.0 *eq*) in isopropyl ether (160 mL) was added dropwise BF₃•Et₂O (17.1 g, 120 mmol, 15.0 mL, 1.2 *eq*) at 0 °C and then ethyl 2-diazoacetate (15.0 g, 130 mmol, 1.3 *eq*) dropwise over 0.5 hour at 0 °C. The resulting mixture was stirred at 0 °C for 1 hour. After completion, the reaction mixture was quenched by saturated aqueous NaHCO₃ (200 mL) and the solution was stirred for 1 hour, then extracted with ethyl acetate (2 × 150 mL). The combined organic layers were washed with saturated brine (1 × 200 mL), dried and concentrated to give a residue. The residue was purified by column chromatography (SiO₂, petroleum ether: ethyl acetate = 30:1 to 20:1) to give *O*1-*tert*-butyl *O*4-ethyl 3-oxoazepane-1,4-dicarboxylate (3.2 g, 11.2 mmol, 11% yield) as yellow oil.

Compound **1-3:** Na (774 mg, 33.6 mmol, 3.0 *eq*) was dissolved in methyl alcohol (45.0 mL) in portions at 0 °C for 0.5 hour, then *O*1-tert-butyl *O*4-ethyl 3-oxoazepane-1,4-dicarboxylate (3.2 g, 11.2 mmol, 1.0 *eq*) and 2-methylisothiourea (2.02 g, 14.5 mmol, 1.3 *eq,* 0.5 H₂SO₄) was added to the reaction mixture, the reaction mixture was stirred at 15 °C for 12 hours. After completion, the reaction mixture was adjusted with 1N HCl to pH ~ 6, then the precipitated solid was filtered and washed with methyl alcohol (20.0 mL), the filtrate was dried and concentrated to give *tert*-butyl 4-hydroxy-2-methylsulfanyl-5,6,7,9-tetrahydropyrimido[4,5-c]azepine -8-carboxylate (3.4 g, crude) as yellow solid which was used for the next step without further purification.

Compound **1-4:** To a solution of *tert-*butyl 4-hydroxy-2-methylsulfanyl-5,6,7,9-tetrahydropyrimido[4,5-*c*]azepine-8-carboxylate (3.4 g, 10.9 mmol, 1.0 *eq*) and TEA (3.87 g, 38.2 mmol, 5.3 mL, 3.5 *eq*) in dichloromethane (35.0 mL) was added Tf₂O (6.16 g, 21.8 mmol, 3.6 mL, 2.0 *eq*) at -40 °C and stirred for 0.5 hour. After completion, the reaction mixture was quenched by addition of water (30.0 mL) at -40 °C and extracted with ethyl acetate (3 × 30 mL). The combined organic layers were dried over Na₂SO₄, filtered and concentrated under reduced pressure to give a residue. The residue was purified by column chromatography (SiO₂, petroleum ether: ethyl acetate= 15:1 to 10:1) to give *tert*-butyl 2-methylsulfanyl-4-(trifluoromethylsulfonyloxy)-5,6,7,9-tetrahydropyrimido[4,5-*c*]azepine-8-carboxylate (3.7 g, 8.13 mmol, 74% yield, two steps, 98% purity) as yellow oil. LCMS [ESI, M-55]: 388.

Compound **1-5:** A mixture of *tert*-butyl 2-methylsulfanyl-4-(trifluoromethylsulfonyloxy)-5,6,7,9-tetrahydropyrimido[4,5-*c*]azepine-8-carboxylate (3.7 g, 8.34 mmol, 1.0 *eq*)*,* benzyl (2*S*)-2-(cyanomethyl)piperazine-1-carboxylate (2.27 g, 8.76 mmol, *1.05 eq*) and DIEA (3.24 g, 25.0 mmol, 4.36 mL, 3.0 *eq*) in DMAC (35.0 mL) was degassed and purged with N₂ for 3 times, and then the mixture was stirred at 15 °C for 3 hours under N₂ atmosphere. After completion, the reaction mixture was quenched by addition H₂O (100.0 mL), and then extracted with ethyl acetate (3 × 30 mL). The combined organic layers were washed with saturated brine (4 × 30 mL), dried over Na₂SO₄, filtered and concentrated under reduced pressure to give a residue. The residue was purified by column chromatography (SiO₂, petroleum ether: ethyl acetate = 5:1 to 1:1) to give *tert*-butyl 4-[(3*S*)-4-benzyloxycarbonyl-3-(cyanomethyl) piperazin-1-yl]-2-methylsulfanyl-5,6,7,9-tetrahydropyrimido[4,5-*c*]azepine-8-carboxylate (3.0 g, 5.27 mmol, 63% yield, 97% purity) as white solid. LCMS [ESI, M+1]: 553.

¹H NMR (400 MHz, chloroform-d) δ 7.43 - 7.34 (m, 5H), 5.24 - 5.17 (m, 211), 4.69 - 4.35 (m, 3H), 4.13 - 4.11 (m, 1H), 3.81 - 3.74 (m, 2H), 3.57 - 3.50 (m, 2H), 3.28 - 3.20 (m, 2H), 2.94 - 2.85 (m, 2H), 2.77 - 2.65 (m, 3H), 2.52 (s, 3H), 1.99 - 1.95 (m, 1H), 1.36 (s, 9H).

Compound **1-6:** To a solution of *tert*-butyl 4-[(3*S*)-4-benzyloxycarbonyl-3-(cyanomethyl)piperazin-1-yl]-2-methylsulfanyl-5,6,7,9-tetrahydropyrimido[4,5-*c*]azepine-8-carboxylate (3.0 g, 5.43 mmol, 1.0 *eq*) in ethyl acetate (10.0 mL) was added m-CPBA (1.10 g, 5.43 mmol, 1.0 *eq*)*.* The mixture was stirred at 0 °C for 0.5 hour. After completion, the reaction mixture was quenched with saturated aq. Na₂SO₃ (15.0 mL) and diluted with H₂O (20.0 mL). The crude mixture was extracted with ethyl acetate (3 × 30 mL). The combined extracts were washed with saturated aq. NaHCO₃ (30.0 mL), dried with Na₂SO₄ the solvent was then removed under vacuum. The residue was purified by column chromatography (SiO₂, Ethyl acetate: Methanol 30:1 to 10:1). Compound *tert*-butyl 4-[(3*S*)-4-benzyloxycarbonyl-3-(cyanomethyl)piperazin-1-yl]-2-methylsulfinyl-5,6,7,9-tetrahydropyrimido[4,5-*c*]azepine-8-carboxylate (2.9 g, 5.05 mmol, 93% yield, 99% purity) was obtained as white solid. LCMS [ESI, M+1]: 569.

Compound **1-7:** To a solution of [(2*S*)-1-methylpyrrolidin-2-yl]methanol (705 mg, 6.12 mmol, 727 uL, 1.2 *eq*) and *t*-BuONa (833 mg, 8.67 mmol, 1.7 *eq*) in toluene (20.0 mL) was added a solution of *tert*-butyl 4-[(3*S*)-4-benzyloxycarbonyl-3- (cyanomethyl)piperazin-1-yl]-2-methylsulfinyl-5,6,7,9-tetrahydropyrimido[4,5-c]azepine-8-carboxylate (2.9 g, 5.10 mmol, 1.0 *eq*) in toluene (10.0 mL) dropwise at 0 °C. The reaction mixture was stirred at 0 °C for 0.5 hour. After completion, the reaction was quenched with water (40.0 mL). The crude mixture was extracted with ethyl acetate (2 × 50 mL). The combined extracts were washed with saturated brine (100 mL), dried with Na₂SO₄, the solvent was removed under vacuum. The residue was purified by column chromatography (Al₂O₃, Petroleum ether: Ethyl acetate = 3:1 to 0:1). Compound *tert*-butyl 4-[(3*S*)-4-benzyloxycarbonyl-3-(cyanomethyl)piperazin-1-yl]-2- [[(2*S*)-1-methylpyrrolidin-2-yl]methoxy]-5,6,7,9-tetrahydropyrimido[4,5-*c*]azepine-8-carboxylate (2.4 g, 3.84 mmol, 75% yield, 99% purity) was obtained as white solid. LCMS [ESI, M+1]: 620.

Compound **1-8:** To a solution of *tert*-butyl 4-[(3*S*)-4-benzyloxycarbonyl-3-(cyanomethyl)piperazin-1-yl]-2-[[(2*S*)-1-methylpyrrolidin-2-yl]methoxy]-5,6,7,9-tetrahydropyrimido[4,5-*c*]azepine-8-carboxylate (400 mg, 645.42 µmol, 1.0 *eq*) in dioxane (5.0 mL) was added HCl•dioxane (4.0 M, 5.0 mL, 31.0 *eq*)*.* The mixture was stirred at 20 °C for 0.5 hour under N₂ atmosphere. After completion, the reaction mixture was quenched by addition saturated aq. Na₂CO₃ at 0 °C until pH ~ 8, and then extracted with ethyl acetate (3 × 20 mL). The combined organic layers were dried over Na₂SO₄, filtered and concentrated under reduced pressure to give a residue. Benzyl(2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (330 mg, crude) was obtained as white solid which was used to the next step without further purification. LCMS [ESI, M+1]: 520.

Compound **1-9:** A mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azcpin-4-yl]piperazine-1-carboxylate (330 mg, 540 µmol, 1.0 *eq*)*,* 1-bromo-8-methyl-naphthalene (239 mg, 1.08 mmol, *2.0 eq*)*,* Xantphos (93.7 mg, 162 µmol, 0.3 *eq*)*,* Pd₂(dba)₃ (74.2 mg, 81.0 µmol, 0.15 *eq*) and Cs₂CO₃ (528 mg, 1.62 mmol, 3.0 *eq*) in toluene (3.0 mL) was degassed and purged with N₂ for 3 times, and then the mixture was stirred at 90 °C for 12 hours under N₂ atmosphere. After completion, the reaction was washed with HCl (1 N, 2 × 5.0 mL). The aqueous phase was treated with solid NaHCO₃ to pH ~ 7 and extracted with ethyl acetate (3 × 5.0 mL). The organic layer was dried over Na₂SO₄, filtered and concentrated. The residue was purified by column chromatography (basic Al₂O₃, Petroleum ether: Ethyl acetate = 3:1 to Ethyl acetate : Methanol = 50:1) and then residue was purified by reverse phase flash [C18, 0.1% FA in water , 0-65% MeCN] and was treated with NaHCO₃ solid to pH ~ 7, and extracted with ethyl acetate (2 × 50 mL). The organic layers were dried over Na₂SO₄, filtered and concentrated under vacuum. Benzyl(2*S*)-2-(cyanomethyl)-4-[8-(8-methyl-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (60.0 mg, 91.0 µmol, 17% yield, 100% purity) was obtained as white solid. LCMS [ESI, M+1]: 660.

¹H NMR (400 MHz, chloroform-d) δ 7.55 - 7.46 (m, 2H), 7.31 - 7.27 (m, 2H), 7.27 - 7.10 (m, 6H), 7.06 (t, *J* = 5.8 Hz, 1H), 5.09 (s, 2H), 4.58 (br s, 1H), 4.28 - 4.20 (m, 2H), 4.19 - 4.11 (m, 1H), 3.99 - 3.92 (m, 1H), 3.71 - 3.59 (m, 1H), 3.49 (t, *J* = 12.6 Hz, 1H), 3.36 - 3.03 (m, 4H), 2.98 - 2.76 (m, 4H), 2.73 - 2.47 (m, 7H), 2.31 (d, *J* = 3.2 Hz, 3H), 2.18 - 2.10 (m, 1H), 1.92 - 1.79 (m, 3H), 1.73 - 1.54 (m, 3H).

Compound **1-10:** To a solution of benzyl (2*S*)-2-(cyanomethyl)-4-[8-(8-methyl-1-naphthyl)-2-[[(2*S*)-1-methylpynolidin-2-yl]methoxy]-5,6,7,9-tetrahydropyrimido[4,5-*c*]azepin-4-yl]piperazine-1-carboxylate (60 mg, 90.9 µmol, 1.0 *eq*) in MeOH (1.0 mL) and NH3•MeOH (1.0 mL, 15% purity) was added Pd/C (20 mg, 10% purity) under N₂ atmosphere. The suspension was degassed and purged with H₂ for 3 times and the mixture was stirred under H₂ (15 Psi) at 20 °C for 0.5 hour. After completion, the crude mixture was filtered through a pad of celite. The cake was washed with MeOH (2 × 5.0 mL) and the filtrate was concentrated. Compound 2-[(2*S*)-4-[8-(8-methyl -1-naphtliyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (45.0 mg, 75.3 µmol, 83% yield, 88% purity) was obtained as yellow solid and used into the next step without further purification. LCMS [ESI, M+1]: 526.

**Example 1:** To a solution of 2-[(2*S*)-4-[8-(8-methyl-1-naphthyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (45.0 mg, 75.3 µmol, 1.0 *eq*) in dichloromethane (1.0 mL) was added TEA (30.5 mg, 301 µmol, 41.9 µL, 4.0 *eq*) and prop-2-enoyl prop-2-enoate (9.50 mg, 75.3 µmol, 1.0 *eq*) at 0 °C. The mixture was stirred at 0 °C for 0.5 hour under N₂ atmosphere. After completion, the organic solvent was washed with water (5.0 mL). The aqueous phase was extracted with dichloromethane (3 × 5.0 mL). The combined extracts were washed with brine (10.0 mL), dried with Na₂SO₄ the solvent was then removed under vacuum. The residue was purified by column chromatography (basic Al₂O₃, Petroleum ether : Ethyl acetate = 3:1 to Ethyl acetate : Methanol = 50:1) and then purified by prep-HPLC (column: Xtimate C18 10µ 250 mm × 50 mm; mobile phase: [water (0.05% ammonium hydroxide v/v)-ACN]; B%: 63% - 93%, 10 min) and lyophilization to give 2-[(2*S*)-4-[8-(8-methyl-1-naphthyl) -2-[[(2*S*)-1-Methylpyrrolidin-2-yl]methoxy]|-5,6,7,9-tetrahydropyrimido[4,5-*c*]azepin-4-yl]-1-prop-2-enoyl-piperazin-2-yl]acetonitrile (10.5 mg, 17.8 µmol, 24% yield, 99% purity) as yellow solid. LCMS [ESI, M+1]: 580.

¹H NMR (400 MHz, chloroform-d) δ 7.71 - 7.60 (m, 2H), 7.41 - 7.28 (m, 3H), 7.23 - 7.17 (m, 1H), 6.67 - 6.53 (m, 1H), 6.45 - 6.36 (m, 1H), 5.83 (br d, *J* = 10.4 Hz, 1H), 5.25 - 4.97 (m, 1H), 4.43 - 4.23 (m, 3H), 4.18 - 4.08 (m, 1H), 4.05 - 3.54 (m, 3H), 3.51 - 3.36 (m, 1H), 3.35 - 3.16 (m, 2H), 3.14 - 2.89 (m, 4H), 2.87 - 2.59 (m, 7H), 2.44 (br d, *J* = 2.4 Hz, 3H), 2.34 - 2.21 (m, 1H), 2.11 - 1.91 (m, 3H), 1.89 - 1.67 (m, 3H).

### EXAMPLE 2

### 2-[(2S)-1-(2-fluoroprop-2-enoyl)-4-[8-(8-methyl-1-naphthyl)-2-[[(2S)-1-methylpyrrolidin-2-yl]methoxy]-5,6,7,9-tetrahydropyrimido[4,5-c]azcpin-4-yl]piperazin-2-yl]acetonitrile

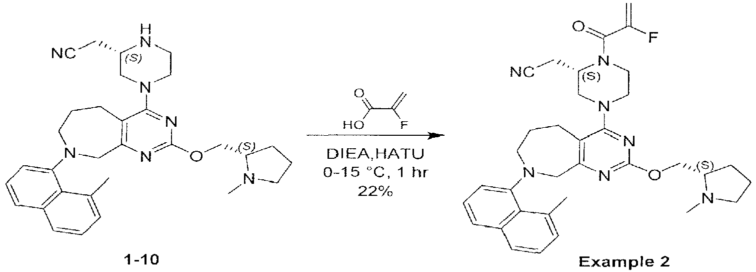

**Example 2:** To a solution of 2-[(2*S*)-4-[8-(8-methyl-1-naphthyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (120 mg, 201 µmol, 1.0 *eq*)*,* 2-fluoroprop-2-enoic acid (41.1 mg, 457 µmol, 2.27 *eq*) in dichloromethane (2.0 mL) was added DIEA (118 mg, 913 µmol, 159 uL, 4.55 *eq*) and HATU (174 mg, 457 µmol, 2.27 *eq*) at 0 °C. The mixture was stirred at 15 °C for 1 hour under N₂ atmosphere. After completion, the water was added (10.0 mL). The aqueous phase was extracted with dichloromethane (2 × 10 mL). The combined extracts were washed with brine (15.0 mL), dried with Na₂SO₄, the filtrate was removed under vacuum. The residue was purified by column chromatography (Base Al₂O₃, Petroleum ether: Ethyl acetate = 3:1 to Ethyl acetate: Methanol = 50:1) and then purified by prep-HPLC (column: Waters Xbridge 150 × 255µ; mobile phase: [water (0.05% ammonium hydroxide v/v)-ACN]; B%: 60%-84%,10 min) and lyophilized to give 2-[(2*S*)-1-(2-fluoroprop-2-enoyl)- 4-[8-(8-methyl-1-naphthyl)-2-[[(2*S*-1-methylpyrrolidin-2-yl]methoxy]-5,6,7,9-tetrahydropyrimido[4,5-*c*]azepin-4-yl]piperazin-2-yl]acetonitrile (26.6 mg, 44.2 µmol, 22% yield, 99% purity) as while solid. LCMS [ESI, M+1]: 598.

¹H NMR (400 MHz, chloroform-d) δ 7.69 - 7.60 (m, 2H), 7.41 - 7.28 (m, 3H), 7.22 - 7.18 (m, 1H), 5.51 - 5.33 (m, 1H), 5.26 (dd, *J* = 3.6, 16.8 Hz, 1H), 5.05 - 4.65 (m, 1H), 4.42 - 4.25 (m, 3H), 4.17 - 4.09 (m, 1H), 3.87 - 3.78 (m, 1H), 3.69 (br t, *J* = 13.8 Hz, 1H), 3.55 - 3.15 (m, 4H), 3.13 - 2.92 (m, 4H), 2.89 - 2.54 (m, 7H), 2.44 ( d, *J* = 2.4 Hz, 3H), 2.32 - 2.20 (m, 1H), 2.13 - 1.93 (m, 3H), 1.83 - 1.64 (m, 3H).

### EXAMPLE 3

### 2-[(2S)-4-[8-(2-methyl-1-naphthyl)-2-[[(2S)-1-methylpyrrolidin-2-yl]methoxy]-5,6,7,9-tetrahyciropyrimido[4,5-cJazepin-4-yl]-1-prop-2-enoyl-piperazin-2-yl]acetonitrile

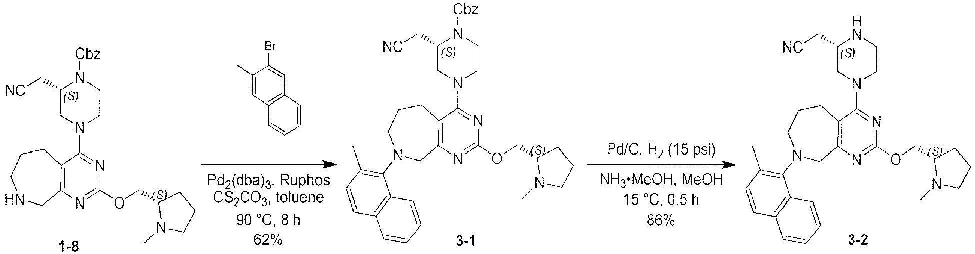

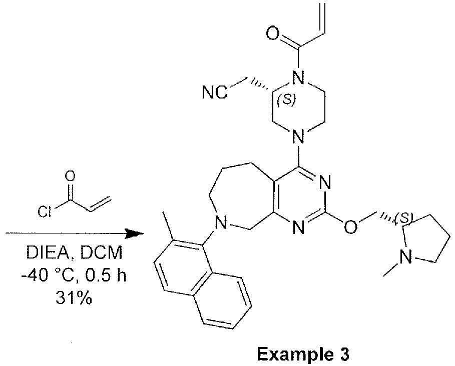

Compound **3-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (300 mg, 577 µmol, 1.0 *eq*)*,* 1-bromo-2-methyl-naphthalene (191 mg, 866 µmol, 1.5 *eq*)*,* Cs₂CO₃ (564 mg, 1.73 mmol, 3.0 *eq*) and RuPhos (108 mg, 231 µmol, 0.4 *eq*) in toluene (9 mL) was added Pd₂(dba)₃ (106 mg, 115 µmol, 0.2 *eq)* under N₂. The suspension was degassed under vacuum and purged with N₂ several times. The mixture was stirred under N₂ at 90 °C for 8 hours. Water (20 mL) was added into the mixture. The mixture was diluted with EtOAc (10 mL) and extracted with EtOAc (3 × 15 mL). The combined organic layers were washed with brine (20 mL). The combined organic layers were dried over anhydrous Na₂SO₄, filtered and concentrated under vacuum. The residue was purified by reverse-phase flash [water (0.1% FA)/acetonitrile] to give benzyl (2*S*)-2-(cyanomethyl)-4-[8-(2-methyl-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (250 mg, 360 µmol, 62 % yield, 95 % purity) as a yellow solid. LCMS [ESI, M+1]: 660.

¹H NMR (400 MHz, chloroform-d) δ = 8.14 - 7.96 (m, 1H), 7.82 - 7.75 (m, 1H), 7.60 (d, *J* = 8.4 Hz, 1H), 7.45 - 7.34 (m, 7H), 7.30 (s, 1H), 5.27 - 5.16 (m, 2H), 4.73 (br s, 1H), 4.63 - 4.52 (m, 1H), 4.49 - 4.38 (m, 1H), 4.35 (br d, *J* = 12.8 Hz, 1H), 4.11 - 4.04 (m, 1H), 3.84 (br s, 1H), 3.78 - 3.64 (m, 1H), 3.62 - 3.48 (m, 1H), 3.43 - 3.23 (m, 3H), 3.13 - 2.88 (m, 5H), 2.84 - 2.74 (m, 1H), 2.63 (br s, 1H), 2.49 - 2.36 (m, 6H), 2.26 (br s, 1H), 2.15 - 2.08 (m, 1H), 2.04 - 1.93 (m, 1H), 1.86 - 1.67 (m, 3H).

Compound **3-2:** To a solution of benzyl (2*S*)-2-(cyanomethyl)-4-[8-(2-methyl-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (250 mg, 379 µmol, 1 *eq*) and NH₃•MeOH (2 mL, 20 % purity) in MeOH (4 mL) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum to give 2-[(2*S*)-4-[8-(2-methyl-1-naphthyl)-2-[[(2*S*)-1-methylpyrrolidin-2-yl]methoxy]-5,6,7,9-tetrahydropyrimido[4,5-c]azepin-4-yl]piperazin-2-yl]acetonitrile (190 mg, 325 µmol, 86 % yield, 90 % purity) as a yellow solid which was used for next step without further purification.

**Example 3:** To a solution of 2-[(2*S*)-4-[8-(2-methyl-1-naphthyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (190 mg, 361 µmol, 1 *eq*) and DIEA (93.4 mg, 723 µmol, 126 µL, 2 *eq*) in DCM (4 mL) was added prop-2-enoyl chloride (49.1 mg, 542 µmol, 44.2 uL, 1.5 *eq*) at -40 °C. The reaction mixture was stirred at -40 °C for 0.5 hour. The reaction mixture was quenched by water (5 mL). The mixture was extracted with DCM (3 × 10 mL). The combined organic layers were dried over anhydrous Na₂SO₄, filtered and concentrated under vacuum. The residue was purified by prep-HPLC (column: Xtimate C18 150^{∗}25mm^{∗}5µm;mobile phase: [water (0.05 % ammonium hydroxide v/v)-ACN];B %: 55 % - 85 %, 8 min) to give 2-[(2S)-4-(8-(2-methyl-1-naphthyl)-2-[[(2S)-1-methylpyrrolidin-2-yl]methoxy]-5,6,7,9-tetrahydropyrimido[4,5-c]azepin-4-yl]-1-prop-2-enoyl-piperazin-2-yl]acetonitrile (65.0 mg, 111 µmol, 31 % yield, 98.8 % purity) as a white solid. LCMS [ESI, M+1]: 580.

¹H NMR (400 MHz, chloroform-d) δ = 8.13 - 7.97 (m, 1H), 7.83 - 7.76 (m, 1H), 7.60 (d, *J* = 8.4 Hz, 1H), 7.45 - 7.35 (m, 2H), 7.29 (d, *J* = 8.4 Hz, 1H), 6.69 - 6.55 (m, 1H), 6.40 (br d, *J* = 16.8 Hz, 1H), 5.84 (br d, *J* = 10.4 Hz, 1H), 5.15 (br s, 1H), 4.63 - 4.52 (m, 1H), 4.50 - 4.36 (m, 1H), 4.33 (br dd, *J* = 4.4, 10.0 Hz, 1H), 4.16 - 3.87 (m, 3H), 3.86 - 3.71 (m, 1H), 3.55 (ddd, *J* = 5.6, 11.6, 17.2 Hz, 2H), 3.46 - 3.36 (m, 1H), 3.34 - 3.25 (m, 1H), 3.14 - 2.90 (m, 5H), 2.83 (br s, 1H), 2.61 (br d, *J* = 3.2 Hz, 1H), 2.50 - 2.38 (m, 6H), 2.29 - 2.19 (m, 1H), 2.17 - 2.05 (m, 2II), 2.04 - 1.94 (m, 1H), 1.86 - 1.71 (m, 3H).

### EXAMPLE 4

### 2-[(2S)-4-[2-[[(2S)-1-methylpyrrolidin-2-yl]methoxy]-8-(o-tolyl)-5,6,7,9-tetrahydropyrimido[4,5-c]azepin-4-yl]-1-prop-2-enoyl-piperazin-2-yl]acetonitrile

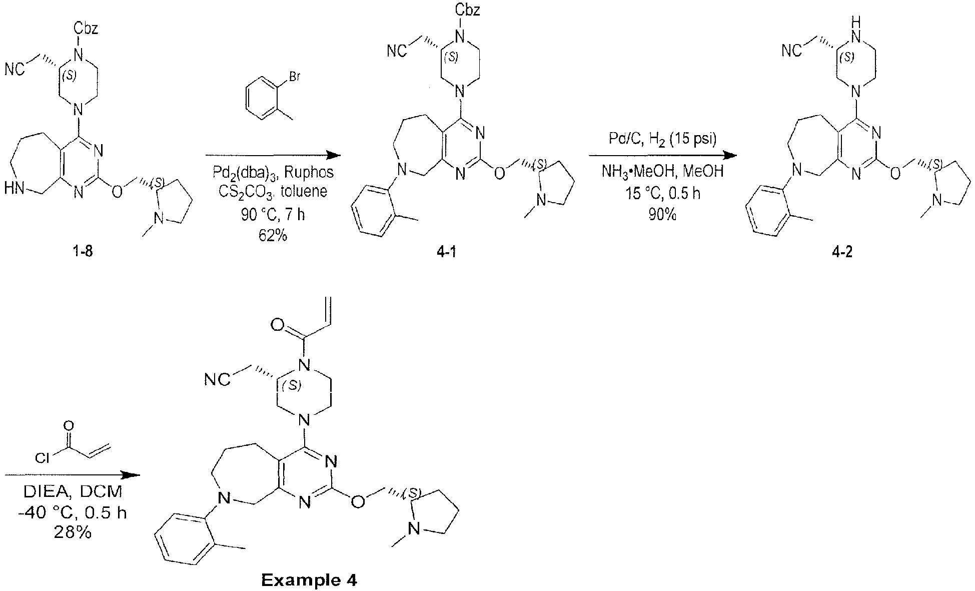

Compound **4-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (300 mg, 577 µmol, 1.0 *eq*)*,* 1-bromo-2-methyl-benzene (148 mg, 866 µmol, 104 uL, 1.5 *eq*)*,* Cs₂CO₃ (564 mg, 1.73 mmol, 3.0 *eq*) and RuPhos (108 mg, 231 µmol, 0.4 *eq*) in toluene (9 mL) was added Pd₂(dba)₃ (106 mg, 115 µmol, 0.2 *eq*) under N₂. The suspension was degassed under vacuum and purged with N₂ several times. The mixture was stirred under N₂ at 90 °C for 7 hours. Water (20 mL) was added into the mixture. The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1 % FA)/acetonitrile] to give benzyl (2*S*)-2-(cyanomethyl)-4-[2-[[(2*S*)-1-methylpyrrolidin-2-yl]methoxy]-8-(o-tolyl)-5,6,7,9-tetrahydropyrimido[4,5-*c*]azepin-4-yl]piperazine-1-carboxylate (230 mg, 358 µmol, 62 % yield, 95 % purity) as a white solid. LCMS [ESI, M+1]: 610.

¹H NMR (400 MHz, chloroform-d) δ = 7.45 - 7.32 (m, 5H), 7.14 (br t, *J* = 6.0 Hz, 2H), 7.07 - 7.02 (m, 1H), 6.98 - 6.92 (m, 1H), 5.20 (s, 2H), 4.68 (br s, 1H), 4.39 (dd, *J* = 4.8, 10.8 Hz, 1H), 4.20 - 4.08 (m, 4H), 3.82 (br d, *J* = 13.2 Hz, 1H), 3.65 (br d, *J* = 12.4 Hz, 1H), 3.39 - 3.18 (m, 4H), 3.11 (br t, *J* = 7.6 Hz, 1H), 2.99 - 2.64 (m, 6H), 2.48 (s, 3H), 2.35 - 2.24 (m, 1H), 2.14 (s, 3H), 2.11 - 2.06 (m, 1H), 2.05 - 1.93 (m, 2H), 1.87 - 1.71 (m, 3H).

Compound **4-2:** To a solution of benzyl (2*S*)-2-(cyanomethyl)-4-[2-[[(2*S*)-1-methylpyrrolidin-2-yl]methoxy]-8-(o-tolyl)-5,6,7,9-tetrahydropyrimido[4,5-*c*]azepin-4-yl]piperazine-1-carboxylate (230 mg, 377 µmol, 1.0 *eq*) and NH₃•MeOH (1 mL, 20 % purity) in MeOH (4 mL) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum. 2-[(2*S*)-4-[2-[[(2*S*)-1-methylpyrrolidin-2-yl]methoxy]-8-(o-tolyl)-5,6,7,9-tetrahydropyrimido[4,5-c]azepin-4-yl]piperazin-2-yl]acetonitrile (180 mg, 341 µmol, 90 % yield, 90 % purity) was obtained as a yellow solid which was used for the next step without further purification.

**Example 4:** To a solution of 2-[(2*S*)-4-[2-[[(2*S*)-1-methylpyrrolidin-2-yl]methoxy]-8-(o-tolyl)-5,6,7,9-tetrahydropyrimido[4,5-*c*]azepin-4-yl]piperazin-2-yl]acetonitrile (180 mg, 378 µmol, 1 *eq*) and DIEA (97.8 mg, 757 µmol, 132 uL, 2 *eq*) in DCM (4 mL) was added prop-2-enoyl chloride (51.4 mg, 567 µmol, 46.3 uL, 1.5 *eq*) at -40 °C. The reaction mixture was stirred at -40 °C for 0.5 hour. The reaction mixture was quenched by water (5 mL). The mixture was extracted with DCM (3 × 10 mL). The combined organic layers were dried over anhydrous Na₂SO₄, filtered and concentrated under vacuum. The residue was purified by prep-HPLC (column: Xtimate C18 150^{∗}25mm^{∗}5µm;mobile phase: [water (0.05 % ammonium hydroxide v/v)-ACN];B %: 51 % - 81 %, 8 min) to give 2-[(2*S*)-4-[2-[[(2*S*)-1-methylpyrrolidin-2-yl]methoxy]-8-(o-tolyl)-5,6,7,9-tetrahydropyrimido[4,5-*c*]azepin-4-yl]-1-prop-2-enoyl-piperazin-2-yl]acetonitrile (56.6 mg, 104 µmol, 28 % yield, 97.5 % purity) as a white solid. LCMS [ESI, M+1]: 530.

¹H NMR (400 MHz, chloroform-d) δ = 7.18 - 7.11 (m, 2H), 7.04 (d, *J* = 7.6 Hz, 1H), 6.99 - 6.91 (m, 1H), 6.67 - 6.52 (m, 1H), 6.38 (dd, *J* = 1.6, 16.8 Hz, 1H), 5.82 (br d, *J* = 10.8 Hz, 1H), 5.09 (br s, 1H), 4.38 (dd, *J* = 4.8, 10.8 Hz, 1H), 4.24 - 4.09 (m, 3H), 3.87 (br d, *J* = 13.6 Hz, 2H), 3.73 (brd, *J* = 12.4 Hz, 1H), 3.57 (br s, 1H), 3.39 - 3.31 (m, 1H), 3.31 - 3.16 (m, 2H), 3.09 (br t, *J* = 7.6 Hz, 1H), 2.94 (br dd, *J* = 8.4, 16.4 Hz, 2H), 2.82 (br d, *J* = 4.4 Hz, 3H), 2.67 (td, *J* = 6.8, 13.2 Hz, 1H), 2.47 (s, 3H), 2.33 - 2.22 (m, 1H), 2.15 (s, 3H), 2.10 - 1.92 (m, 3H), 1.89 - 1.75 (m, 3H).

### EXAMPLE 5

### 2-[(2S)-4-[2-[[(2S)-1-methylpyrrolidin-2-yl]methoxy]-8-phenyl-5,6,7,9-tetrahydropyrimido[4,5-c]azepin-4-yl]-1-prop-2-enoyl-piperazin-2-yl] acetonitrile

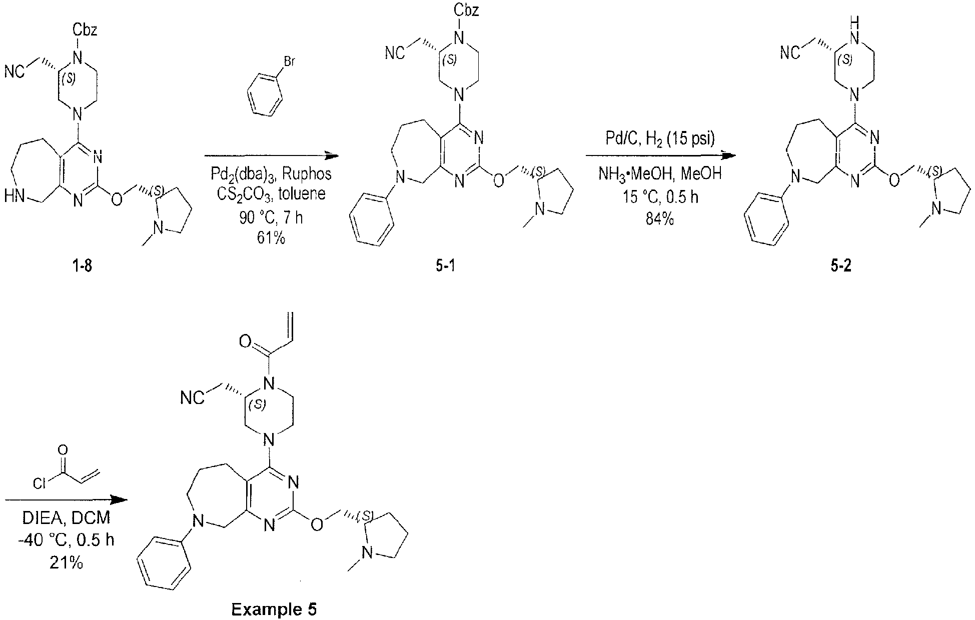

Compound **5-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (300 mg, 577 µmol, 1.0 *eq*)*,* iodobenzene (177 mg, 866 µmol, 96.5 µL, 1.5 *eq*)*,* Cs₂CO₃ (564 mg, 1.73 mmol, 3.0 *eq*) and RuPhos (108 mg, 231 µmol, 0.4 *eq*) in toluene (9 mL) was added Pd₂(dba)₃ (106 mg, 115 µmol, 0.2 *eq*) under N₂. The suspension was degassed under vacuum and purged with N₂ several times. The mixture was stirred under N₂ at 90 °C for 7 hours. Water (20 mL) was added into the mixture. The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1 % FA)/ acetonitrile] to give benzyl (2*S*)-2-(cyanomethyl)-4-[2-[[(2*S*)-1-methylpyrrolidin-2-yl]methoxy]-8-phenyl-5,6,7,9-tetrahydropyrimido[4,5-*c*]azepin-4-yl]piperazine-1-carboxylate (220 mg, 351 µmol, 61 % yield, 95 % purity) as a yellow solid. LCMS [ESI, M+1]: 596.

¹H NMR (400 MHz, chloroform-d) δ = 7.41 - 7.31 (m, 5H), 7.20 - 7.14 (m, 2H), 6.82 (d, *J* = 8.0 Hz, 2H), 6.68 (t*, J* = 7.2 Hz, 1H), 5.22 - 5.12 (m, 2H), 4.62 (br s, 1H), 4.57 (s, 2H), 4.41 (br s, 1H), 4.22 - 4.15 (m, 1H), 4.07 (br s, 1H), 3.83 - 3.59 (m, 3H), 3.46 (br d, *J* = 11.2 Hz, 1H), 3.24 (br s, 1H), 3.12 (br dd, *J* = 3.6, 13.6 Hz, 2H), 2.91 - 2.62 (m, 7H), 2.51 (s, 3H), 2.31 (br d, *J* = 8.0 Hz, 1H), 2.09 (br d, *J* = 8.4 Hz, 1H), 1.97 (br s, 2H), 1.86 - 1.70 (m, 3H).

Compound **5-2:** To a solution of benzyl (2*S*)-2-(cyanomethyl)-4-[2-[[(2*S*)-1-methylpyrrolidin-2-yl]methoxy]-8-phenyl-5,6,7,9-tetrahydropyrimido[4,5-*c*]azepin-4-yl]piperazine-1-carboxylate (220 mg, 369 µmol, 1.0 *eq*) and NH₃•MeOH (1 mL, 20 % purity) in MeOH (4 mL) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum to. 2-[(2*S*)-4-[2-[[(2*S*)-1-methylpyrrolidin-2-yl]methoxy]-8-phenyl-5,6,7,9-tetrahydropyrimido[4,5-*c*]azepin-4-yl]piperazin-2-yl]acetonitrile (160 mg, 312 µmol, 84 % yield, 90 % purity) was obtained as a white solid which was used for next step without further purification.

**Example 5:** To a solution of 2-[(2*S*)-4-[2-[[(2S-methylpyrrolidin-2-yl]methoxy]-8-phenyl-5,6,7,9-tetrahydropyrimido[4,5-*c*]azepin-4-yl]piperazin-2-yl]acetonitrile (160 mg, 347 µmol, 1.0 *eq*) and DIEA (89.6 mg, 693 µmol, 121 µL, 2.0 *eq*) in DCM (4 mL) was added prop-2-enoyl chloride (47.1 mg, 520 µmol, 42.4 µL, 1.5 *eq*) at -40 °C. The reaction mixture was stirred at -40 °C for 0.5 hour. The reaction mixture was quenched by water (5 mL). The mixture was extracted with DCM (3 × 10 mL). The combined organic layers were dried over anhydrous Na₂SO₄, filtered and concentrated under vacuum. The residue was purified by prep-HPLC (column: Xtimate C18 150*25mm*5pm;mobile phase: [water (0.05 % ammonium hydroxide v/v)-ACN];B%: 40 %-70 %, 8 min) to give 2-[(2*S*)-4-[2-[[(2*S*)-1-methylpyrrolidin-2-yl]methoxy]-8-phenyl-5,6,7,9-tetrahydropyrimido[4,5-*c*]azepin-4-yl]-1-prop-2-enoyl-piperazin-2-yl]acetonitrile (38.6 mg, 73.4 µmol, 21 % yield, 98.1 % purity) as a white solid. LCMS [ESI, M+1]: 516.

¹H NMR (400 MHz, chloroform-d) δ = 7.18 (t, *J* = 8.0 Hz, 2H), 6.85 - 6.81 (m, 2H), 6.69 (t, *J* = 7.2 Hz, 1H), 6.54 (br d, *J* = 10.4 Hz, 1H), 6.36 (dd, *J=* 1.6, 16.8 Hz, 1H), 5.80 (br d, *J* = 10.4 Hz, 1H), 5.03 (brs, 1H), 4.58 (s, 2H), 4.41 (dd, *J* = 4.8, 10.4 Hz, 1H), 4.17 (dd, *J* = 7.2, 10.4 Hz, 1H), 4.03 - 3.66 (m, 4H), 3.55 (br d, *J* = 11.2 Hz, 2H), 3.19 - 3.06 (m, 2H), 2.88 (br dd, *J* = 8.0, 16.4 Hz, 2H), 2.82 - 2.63 (m, 4H), 2.50 (s, 3H), 2.35 - 2.26 (m, 1H), 2.14 - 2.04 (m, 1H), 2.02 - 1.89 (m, 2H), 1.89 - 1.72 (m, 3H).

### EXAMPLE 6

### 2-[(2S)-4-[2-[[(2S)-1-methylpyrrolidin-2-yl]methoxy]-8-(1-naphthyl)-5,6,7,9-tetrahydropyrimido[4,5-c]azepin-4-yl]-1-prop-2-enoyl-piperazin-2-yl]acetonitrile

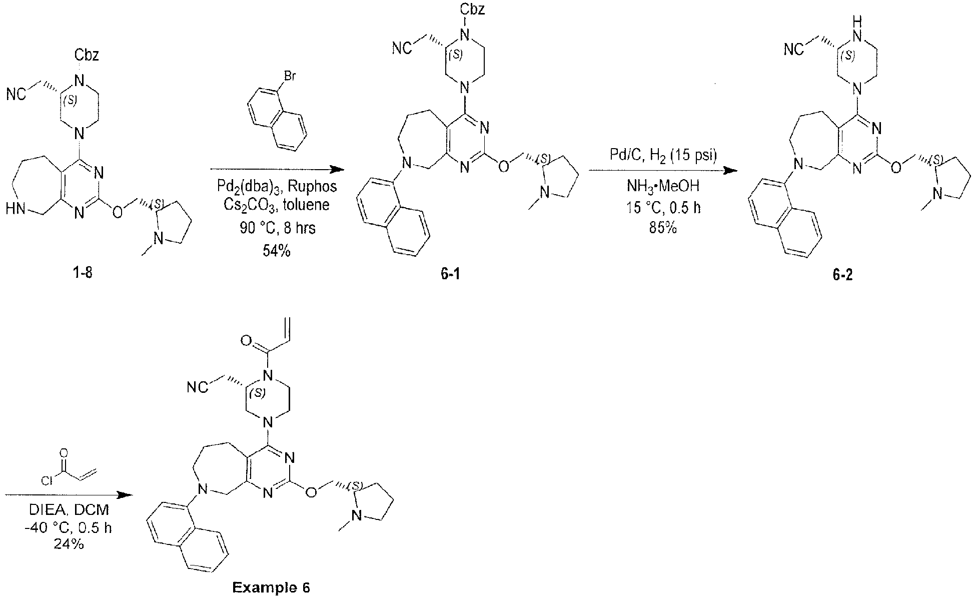

Compound **6-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (200 mg, 385 µmol, 1.0 *eq*), 1-bromonaphthalene (120 mg, 577 µmol, 80.2 uL, 1.5 *eq*)*,* Cs₂CO₃ (376 mg, 1.15 mmol, 3.0 *eq*) and RuPhos (71.8 mg, 154 µmol, 0.4 *eq*) in toluene (6 mL) was added Pd₂(dba)₃ (70.5 mg, 77.0 µmol, 0.2 *eq*) under N₂. The suspension was degassed under vacuum and purged with N₂ several times. The mixture was stirred under N₂ at 90 °C for 8 hours. Water (15 mL) was added into the mixture. The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1 % FA)/acetonitrile] to give benzyl (2*S*)-2-(cyanomethyl)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazine-1-carboxylate (140 mg, 206 µmol, 54 % yield, 95 % purity) as a yellow solid. LCMS [ESI, M+1]: 646.

¹H NMR (400 MHz, chloroform-d) δ = 7.95 (d, *J* = 8.4 Hz, 1H), 7.81 (d, *J* = 8.0 Hz, 1H), 7.54 (d, *J* = 8.4 Hz, 1H), 7.46 - 7.34 (m, 8H), 7.13 (d, *J* = 7.6 Hz, 1H), 5.21 (s, 2H), 4.70 (br s, 1H), 4.44 - 4.35 (m, 3H), 4.20 - 4.15 (m, 1H), 3.87 (br d, *J* = 12.4 Hz, 1H), 3.70 (br d, *J* = 12.4 Hz, 1H), 3.57 - 3.41 (m, 2H), 3.40 - 3.23 (m, 2H), 3.09 (br s, 1H), 2.99 (dt, *J* = 3.2, 12.4 Hz, 1H), 2.91 (br s, 3H), 2.81 - 2.73 (m, 1H), 2.68 (br s, 1H), 2.47 (s, 3H), 2.28 (br d, *J* = 8.0 Hz, 1H), 2.19 - 2.06 (m, 2H), 2.04 - 1.99 (m, 1H), 1.89 - 1.69 (m, 4H).

Compound 6-2: To a solution of benzyl (2*S*)-2-(cyanomethyl)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazine-1-carboxylate (140 mg, 217 µmol, 1.0 *eq*) and NH₃•MeOH (0.5 mL, 30 % purity) in MeOH (4 mL) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum to give 2-[(2*S*)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazin-2-yl]acetonitrile (105 mg, 185 µmol, 85 % yield, 90 % purity) as a yellow solid which was used for next step without further purification.

**Example *6:*** To a solution of 2-[(2*S*)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazin-2-yl]acetonitrile (105 mg, 205 µmol, 1.0 *eq*) and DIEA (53.0 mg, 410 µmol, 71.5 µL, 2.0 *eq)* in DCM (2 mL) was added prop-2-enoyl chloride (27.9 mg, 308 µmol, 25.1 µL, 1.5 *eq)* at -40 °C. The reaction mixture was stirred at -40 °C for 0.5 hour. The reaction mixture was quenched with water (8 mL). The mixture was extracted with DCM (3 × 10 mL). The combined organic layers were dried over anhydrous Na₂SO₄, filtered and concentrated under vacuum. The residue was purified by reverse-phase flash [water (0.1% FA)/acetonitrile]. The residue was purified by prep-HPLC (column: Xtimate C18 150^{∗}25mm^{∗}5µm;mobile phase: [water (0.05 % ammonium hydroxide v/v)-ACN];B %: 54 % - 84 %, 10 min) to give 2-[(2*S*)-4-[2-[[(2*S*)-1-methylpyrrolidin-2-yl]methoxy]-8- (1-naphthyl)-5,6,7,9-tetrahydropyrimido[4,5-*c*]azepin-4-yl]-1-prop-2-enoyl-piperazin-2-yl]acetonitrile (27.9 mg, 49.4 µmol, 24 % yield, 100 % purity) as a white solid. LCMS [ESI, M+1J: 566.

¹H NMR (400 MHz, chloroform-d) δ = 7.95 (d, *J* = 8.4 Hz, 1H), 7.81 (d, *J* = 8.0 Hz, 1H), 7.54 (d, *J* = 8.4 Hz, 1H), 7.47 - 7.34 (m, 3H), 7.13 (d, *J* = 6.8 Hz, 1H), 6.59 (br d, J= 11.2 Hz, 1H), 6.40 (dd, *J* = 1.6, 16.8 Hz, 1H), 5.83 (br d, *J* = 10.8 Hz, 1H), 5.11 (br s, 1H), 4.45 - 4.32 (m, 3H), 4.17 (dd, *J* = 6.8, 10.8 Hz, 1H), 3.93 (br d, *J* = 13.6 Hz, 2H), 3.78 (br d, J= 12.0 Hz, 1H), 3.69 - 3.39 (m, 3H), 3.30 (dd, *J* = 4.0, 13.6 Hz, 1H), 3.13 - 2.74 (m, 6H), 2.72 - 2.61 (m, 1H), 2.46 (s, 3H), 2.33 - 2.23 (m, 1H), 2.21 - 1.97 (m, 3H), 1.89 - 1.70 (m, 3H).

### EXAMPLE 7

### 2-((S)-1-acryloyl-4-(8-(8-chloronaphthalen-1-yl)-2-(((S)-1-methylpyrrolidin-2-yl)methoxy)-6,7,8,9-tetrahydro-5H-pyrimido[4,5-c]azepin-4-yl)piperazin-2-yl)acetonitrile

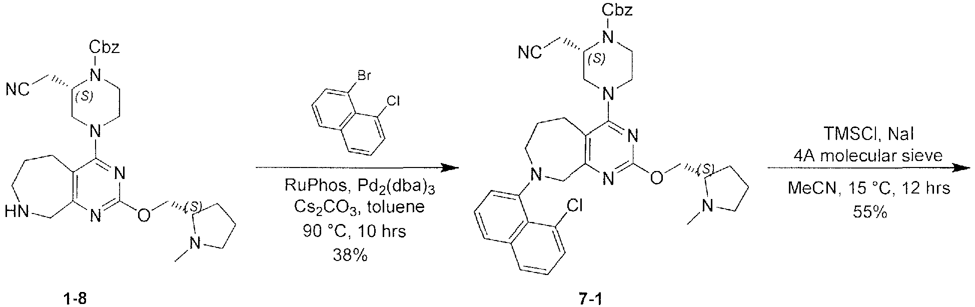

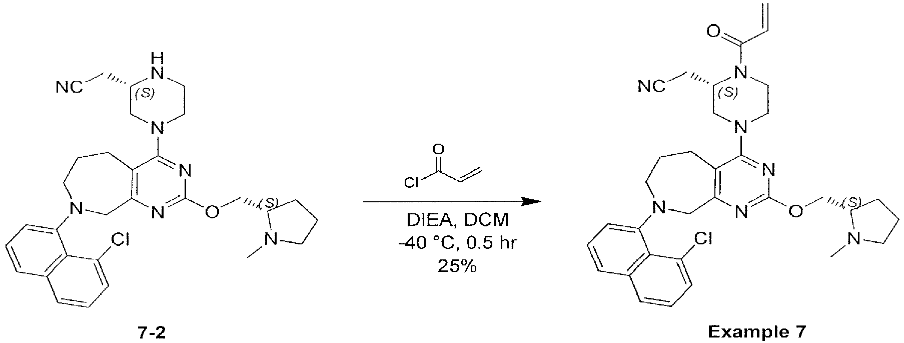

Compound **7-1:** To a solution of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-c]azepin-4-yl]piperazine-1-carboxylate (250 mg, 481 µmol, 1.0 *eq)* and 1-bromo-8-chloro- naphthalene (232 mg, 962 µmol, *2.0 eq)* in toluene (4.0 mL) was added RuPhos (89.8 mg, 192 µmol, 0.4 *eq*)*,* Cs₂CO₃ (392 mg, 1.20 mmol, 2.5 *eq*) and Pd₂(dba)₃ (88.1 mg, 96.2 µmol, 0.2 *eq*)*,* the reaction mixture was stirred at 90 °C for 10 hours under N₂. After completion, the reaction mixture was filtered through a celite, the filter cake was washed with ethyl acetate (10 mL), and adjusted with 1N HCl aqueous to pH~3, the organic layer was separated, and the aqueous was adjusted with Na₂CO₃ solid to pH~8, extracted with ethyl acetate (2 × 10 mL), the organic layer was washed with saturated brine (1 × 20 mL), dried over Na₂SO₄, filtered and concentrated. The residue was purified by reverse phase flash (C18, 0.1%FA in water, 30%-50% MeCN). The product benzyl (2*S*)-4-[8-(8-chloro-1-naphthyl)-2-[[(2*S*)-1-methylpyrrolidin-2-yl] methoxy]-5,6,7,9-tetrahydropyrimido[4,5-*c*]azepin-4-yl]-2-(cyanomethyl)piperazine-1-carboxylate (130 mg, 185 µmol, 38% yield, 97% purity) was obtained as yellow solid. LCMS [ESI, M+1]: 680.

¹H NMR (400 MHz, chloroform-d) δ 7.74 - 7.68 (m, 1H), 7.59 - 7.51 (m, 1H), 7.48 - 7.15 (m, 9H), 5.21 (s, 2H), 4.74 - 4.63 (m, 1H), 4.40 - 4.29 (m, 211), 4.21 - 4.05 (m, 211), 3.84 - 3.71 (m, 1H), 3.69 - 3.46 (m, 2H), 3.44 - 3.15 (m, 3H), 3.13 - 2.60 (m, 8H), 2.46 (s, 3H), 2.33 - 2.21 (m, 1H), 2.17 - 2.02 (m, 2H), 1.96 - 1.72 (m, 4H). LCMS [ESI, M+1]: 546.

Compound **7-2:** To a solution of TMSCI (239 mg, 2.21 mmol, 280 µL, 15.0 *eq*) in MeCN (2 mL) containing 4A molecular sieves (100 mg) at 0 °C was added NaI (353 mg, 2.35 mmol, *16.0 eq)* in portions. Stirring was continued for a period of 1 hour at 15 °C. Then a solution of benzyl (2*S*)-4-[8-(8-chloro-1-naphthyl)-2-[[(2*S*)-1- methylpyrrolidin-2-yl]methoxy]-5,6,7,9-tetrahydropyrimido[4,5-*c*]azepin-4-yl]-2-(cyanomethyl)piperazine-1-carboxylate (100 mg, 147 µmol, 1.0 *eq*) in MeCN (1 mL) was added to the reaction, and the reaction mixture was stirred at 15 °C for 11 hours. After completion, the reaction mixture was concentrated, then the reaction mixture was added 1N HCl aqueous (8 mL), extracted with methyl *tert*-butyl ether (2 × 5 mL), the organic layer was discarded, and the aqueous phase was adjusted to pH~8 with saturated Na₂CO₃ aqueous, and then extracted with ethyl acetate (2 × 8 mL), the organic layer was dried over Na₂SO₄, filtered and concentrated. The product 2-[(2*S*)-4-[8-(8-chloro-1-naphthyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (45 mg, 81.0 µmol, 55% yield, 98% purity) was obtained as brown oil.

**Example 7**: To a solution of 2-[(2*S*)-4-[8-(8-chloro-1-naphthyl)-[[(2*S*)-1-methylpyrrolidin-2-yl]methoxy]-5,6,7,9-tetrahydropyrimido[4,5-*c*]azepin-4-yl]piperazin-2-yl]acetonitrile (45 mg, 82.4 µmol, 1.0 *eq*) in dichloromethane (1.0 mL) was added DIEA (42.6 mg, 329 µmol, 57.4 uL, 4.0 *eq*) and prop-2-enoyl chloride (11.2 mg, 124 µmol, 10.1 µL, 1.5 *eq*) in portions at -40 °C, the reaction mixture was stirred at -40 °C for 0.5 hour. After completion, the reaction mixture was quenched with water (1.0 mL) at -40 °C, the reaction mixture was warmed to 20 °C, and diluted with water (5 mL), then extracted with dichloromethane (2 × 5 mL), the combined organic layer was dried over Na₂SO₄, filtered and concentrated. The residue was purified column chromatography (base Al₂O₃, petroleum ether/ethyl acetate=3/1 to petroleum ether/ethyl acetate/methanol=3/1/0.1), the crude product was repurified by prep-HPLC (column: Xtimate C18 150^{∗}25mm^{∗}5µm; mobile phase: [water (0.05% ammonium hydroxide v/v)-ACN]; B%: 56%-86%, 10 min). The product 2-((*S*)-1- acryloyl-4-(8-(8-chloronaphthalen-1-yl)-2-(((*S*)-1-methylpyrrolidin-2-yl)methoxy)-6,7,8,9-tetrahydro-5*H*-pyrimido[4,5-*c*]azepin-4-yl)piperazin-2-yl)acetonitrile (14.8 mg, 24.1 µmol, 29 % yield, 97% purity) was obtained as white solid. LCMS [ESI, M+1]: 600.

¹H NMR (400 MHz, chloroform-d) δ 7.74 - 7.68 (m, 1H), 7.58 - 7.52 (m, 1H), 7.47 - 7.43 (m, 1H), 7.41 - 7.33 (m, 1H), 7.32 - 7.16 (m, 2H), 6.71 - 6.53 (m, 1H), 6.39 (d, *J* = 16.8 Hz, 1H), 5.83 (brd, *J* = 10.4 Hz, 1H), 5.18 - 5.05 (m, 1H), 4.52 - 4.29 (m, 3H), 4.16 - 4.08 (m, 1H), 4.02 - 3.78 (m, 2H), 3.75 - 3.47 (m, 3H), 3.43 - 3.32 (m, 1H), 3.30 - 3.16 (m, 1H), 3.08 (br t, *J* = 7.6 Hz, 1H), 3.04 - 2.87 (m, 3H), 2.84 - 2.60 (m, 3H), 2.45 (s, 3H), 2.34 - 2.21 (m, 1H), 2.19 - 1.97 (m, 2H), 1.96 - 1.72 (m, 4H).

### EXAMPLE 8

### 2-[(2S)-4-[8-(3-isopropylphenyl)-2-[[(2S)1l-methylpyrrolidin-2-yl]methoxy]-5,6,7,9-tetrahydropyrimido[4,5-c]azepin-4-yl]-1-prop-2-enoyl-piperazin-2-yl]acetonitrile

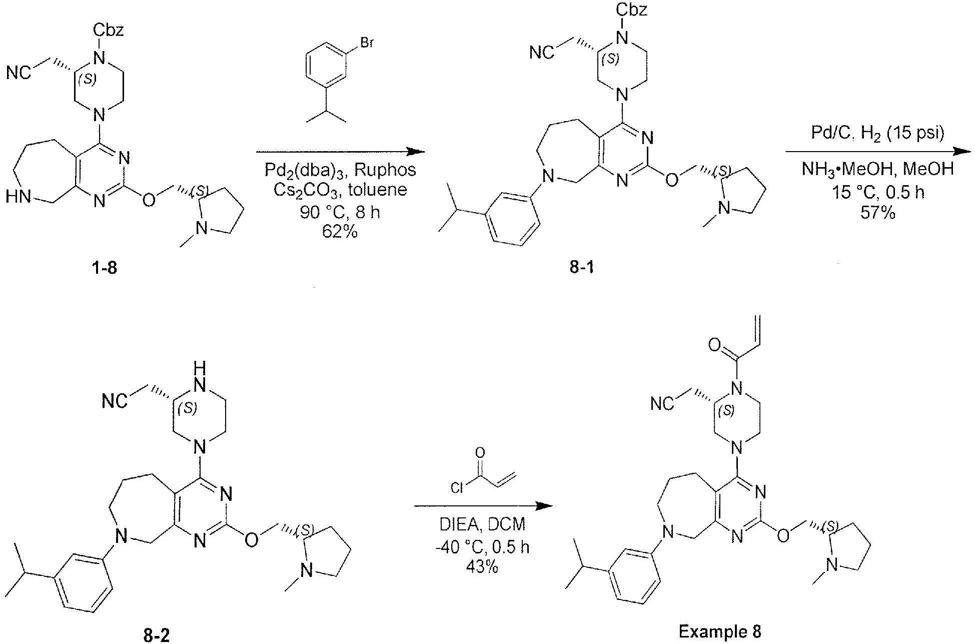

Compound 8-1: To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (150 mg, 289 µmol, 1.0 *eq),* 1-bromo-3-isopropyl-benzene (86.2 mg, 433 µmol, 12.9 µL, 1.5 *eq),* Cs₂CO₃ (282 mg, 866 µmol, 3.0 *eq)* and RuPhos (53.9 mg, 115 µmol, 0.4 *eq)* in toluene (4 mL) was added Pd₂(dba)₃ (52.9 mg, 57.7 µmol, 0.2 *eq*) under N₂. The suspension was degassed under vacuum and purged with N₂ several times. The mixture was stirred under N₂ at 90 °C for 8 hours. Water (15 mL) was added into the mixture. The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1 % FA)/acetonitrile] to give benzyl (2*S*)-2-(cyanomethyl)-4-[8-(3-isopropylphenyl)-2-[[(2*S*)-1-methylpyrrolidin-2-yl]methoxy]-5,6,7,9-tetrahydropyrimido[4,5-*c*]azepin-4-yl]piperazine-1-carboxylate (120 mg, 179 µmol, 62 % yield, 95 % purity) as a yellow solid. LCMS [ESI, M+1]: 638.

¹H NMR (400 MHz, chloroform-d) δ = 7.42 - 7.32 (m, 5H), 7.11 (t, *J* = 8.0 Hz, 1H), 6.72 (s, 1H), 6.65 (dd, *J* = 2.4, 8.0 Hz, 1H), 6.58 (d, *J* = 7.6 Hz, 1H), 5.24 - 5.13 (m, 2H), 4.71 - 4.55 (m, 3H), 4.41 (dd, *J* = 4.8, 10.8 Hz, 1H), 4.21 - 3.99 (m, 2H), 3.84 - 3.60 (m, 3H), 3.46 (br d, *J*= 12.8 Hz, 1H), 3.24 (br s, 1H), 3.12 (br dd, *J* = 3.6, 13.2 Hz, 2H), 2.93 - 2.63 (m, 7H), 2.50 (s, 3H), 2.34 - 2.24 (m, 1H), 2.13 - 2.06 (m, 1H), 1.96 (br s, 2H), 1.88 - 1.74 (m, 3H), 1.21 (d, *J*= 6.8 Hz, 6H).

Compound **8-2:** To a solution of benzyl (2*S*)-2-(cyanomethyl)-4-[8-(3-isopropylphenyl)-2-[[(2*S*)-1-methylpyrrolidin-2-yl]methoxy]-5,6,7,9-tetrahydropyrimido[4,5-*c*]azepin-4-yl]piperazine-1-carboxylate (120 mg, 188 µmol, 1.0 *eq*) and NH₃•MeOH (1 mL, 20 % purity) in MeOH (4 mL) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum to give 2-[(2*S*)-4-[8-(3-isopropylphenyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (60 mg, 107 µmol, 57 % yield, 90 % purity) as a yellow solid which was used for next step without further purification.

**Example 8:** To a solution of 2-[(2*S*)-4-[8-(3-isopropylphenyl)-2-[[(2*S*)-1-methylpyrrolidin-2-yl]methoxy]-5,6,7,9-tetrahydropyrimido[4,5-c]azepin-4-yl]piperazin-2-yl]acetonitrile (60 mg, 119 µmol, 1.0 *eq*) and DIEA (30.8 mg, 238 µmol, 41.5 µL, 2.0 *eq)* in DCM (1.5 mL) was added prop-2-enoyl chloride (16.2 mg, 179 µmol, 14.6 µL, 1.5 *eq)* at -40 °C. The reaction mixture was stirred at -40 °C for 0.5 hour. The reaction mixture was quenched by water (8 mL). The mixture was extracted with DCM (3 × 10 mL). The combined organic layers were dried over anhydrous Na₂SO₄, filtered and concentrated under vacuum. The residue was purified by reverse-phase flash [water (0.1 % FA)/acetonitrile]. The residue was purified by prep-HPLC (column: Waters Xbridge 150^{∗}25 5u;mobile phase: [water (0.05 % ammonium hydroxide v/v)-ACN];B %: 55 % - 76 %, 10 min) to give 2-[(2*S*)-4-[8-(3-isopropylphenyl)-2-[[(2*S*)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (28.8 mg, 51.3 µmol, 43 % yield, 99.4 % purity) as a white solid. LCMS [ESI, M+1]: 558.

¹H NMR (400 MHz, chloroform-d) δ = 7.11 (t, *J* = 8.0 Hz, 1H), 6.72 (s, 1H), 6.66 (dd, *J* = 2.4, 8.4 Hz, 1H), 6.59 (d, *J* = 7.6 Hz, 1H), 6.53 (br s, 1H), 6.40 - 6.32 (m, 1H), 5.80 (br d, *J* = 10.4 Hz, 1H), 5.05 (br s, 1H), 4.58 (s, 2H), 4.42 (br s, 1H), 4.18 (br dd, *J* = 7.2, 10.4 Hz, 1H), 4.07 - 3.65 (m, 4H), 3.55 (br d, *J* = 13.2 Hz, 2H), 3.14 (br dd, *J* = 3.6, 13.6 Hz, 2H), 2.98 - 2.62 (m, 7H), 2.52 (s, 3H), 2.32 (br d, *J* = 8.8 Hz, 1H), 2.16 - 1.91 (m, 3H), 1.89 - 1.70 (m, 3H), 1.21 (d, *J* = 6.8 Hz, 6H).

### EXAMPLE 9

### 2-((S)-1-acryloyl-4-(8-(3-fluoro-2-methylphenyl)-2-(((S)-1-methylpyrrolidin-2-yl)methoxy)-6,7,8,9-tetrahydro-5H-pyrimido[4,5-c]azepin-4-yl)piperazin-2-yl)acetonitrile

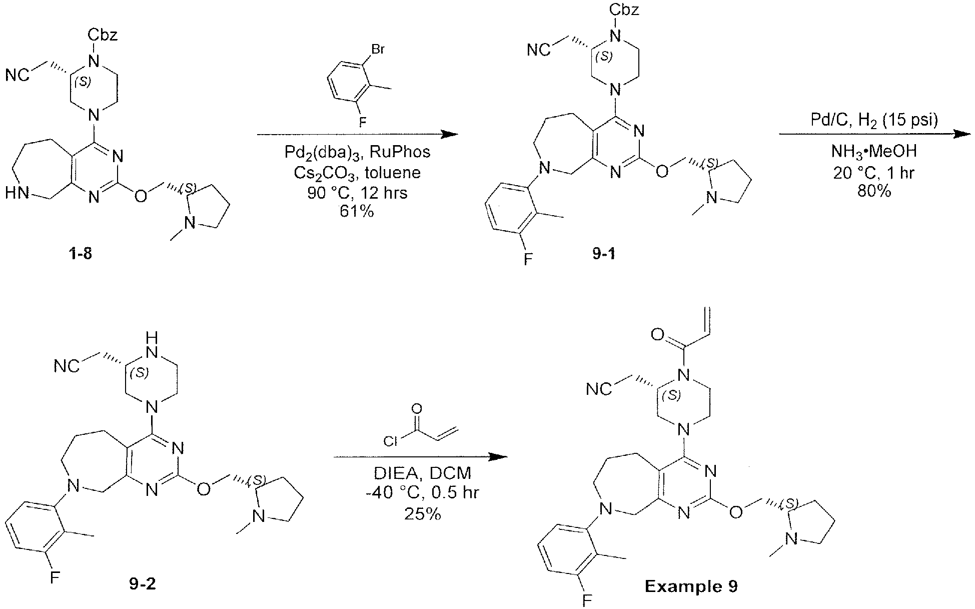

Compound **9-1:** To a solution of benzyl (2*S*)-2-(cyanomethyl)-4-[2-[[(2*S*) -1-methylpyrrolidin-2-yl]methoxy]-6,7,8,9-tetrahydro-5*H*-pyrimido[4,5-*c*]azepin-4-yl]piperazine-1-carboxylate (150 mg, 289 µmol, 1.0 *eq*) and 1-bromo-3-fluoro-2- methyl-benzene (109 mg, 577 µmol, 2.0 *eq)* in toluene (3.0 mL) was added RuPhos (53.9 mg, 115 µmol, 0.4 *eq),* Cs₂CO₃ (235 mg, 722 µmol, 2.5 *eq)* and Pd₂(dba)₃ (52.9 mg, 57.7 µmol, 0.2 *eq),* the reaction mixture was stirred at 90 °C for 12 hours under N₂. After completion, the reaction mixture was filtered through a celite, the filter cake was washed with ethyl acetate (10 mL), and adjusted with 1N HCl aqueous to pH~3, then the organic layer was separated, and the aqueous was adjusted with Na₂CO₃ solid to pH~8, extracted with ethyl acetate (2 × 10 mL), the organic layer was washed with saturated brine (1 × 20 mL), dried over Na₂SO₄, filtered and concentrated. The residue was purified by reverse phase flash (Cl 8, 0.1%FA in water, 30%-50% MeCN). The product benzyl (2*S*)-2-(cyanomethyl)-4-[8-(3-fluoro-2-methyl-phenyl)-2-[[(2*S*)-1-methylpyrrolidin-2-yl]methoxy]-5,6,7,9-tetrahydropyrimido[4,5-c]azepin-4-yl]piperazine-1-carboxylate (110 mg, 175 µmol, 61% yield, 100% purity) was obtained as brown oil. LCMS [ESI, M+1]: 628.

¹H NMR (400 MHz, chloroform-d) δ 7.44 - 7.31 (m, 5H), 7.11 - 7.02 (m, 1H), 6.81 (d, *J* = 8.4 Hz, 1H), 6.73 (t, *J* = 8.6 Hz, 1H), 5.20 (s, 2H), 4.73 - 4.61 (m, 1H), 4.45 - 4.32 (m, 1H), 4.18 - 4.13 (m, 4H), 3.83 (br d, *J* = 13.2 Hz, 1H), 3.65 (br d, J= 12.4 Hz, 1H), 3.41 - 3.18 (m, 4H), 3.14 - 3.05 (m, 1H), 3.00 - 2.63 (m, 6H), 2.48 (s, 3H), 2.36 - 2.21 (m, 1H), 2.13 - 1.94 (m, 6H), 1.89 - 1.73 (m, 3H).

Compound **9-2:** To a solution of benzyl (2*S*)-2-(cyanomethyl)-4-[8-(3- fluoro-2-methylphenyl)-2-[[(2*S*)-1-methylpyrrolidin-2-yl]methoxy]-5,6,7,9-tetrahydropyrimido[4,5-*c*]azepin-4-yl]piperazine-1-carboxylate (110 mg, 175 µmol, 1.0 *eq)* in methanol (1.5 mL) was added Pd/C (30 mg, 175 µmol, 10% purity, 1.0 *eq)* and NH₃•MeOH (1.5 mL, 20% purity, 1.0 *eq),* the suspension was degassed under vacuum and purged with H₂ several times. The mixture was stirred under H₂ (15 psi) at 20 °C for 1 hour. After completion, the reaction mixture was filtered through a pad of celite, and the filter cake was washed with dichloromethane (2 × 5 mL), the filtrate was concentrated. The product 2-[(2*S*)-4-[8-(3-fluoro-2-methyl-phenyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (70 mg, 140 µmol, 80% yield, 99% purity) was obtained as white solid which was used for the next step without further purification. LCMS [ESI, M+1]: 494.

**Example 9:** To a solution of 2-[(2*S*)-4-[8-(3-fluoro-2-methyl-phenyl) -2-[[(2*S*)-1-methylpyrrolidin-2-yl]methoxy]-5,6,7,9-tetrahydropyrimido[4,5-*c*]azepin-4-yl]piperazin-2-yl]acetonitrile (70 mg, 142 µmol, 1.0 *eq*) in dichloromethane (1.5 mL) was added DIEA (73.3 mg, 567 µmol, 98.8 µL, 4.0 *eq)* and prop-2-enoyl chloride (19.3 mg, 213 µmol, 17.3 µL, 1.5 *eq)* in portions at -40 °C, the reaction mixture was stirred at -40 °C for 0.5 hour. After completion, the reaction mixture was quenched with water (1.0 mL) at -40 °C, then the reaction mixture was warmed to 20 °C, and diluted with water (5 mL), extracted with dichloromethane (2 × 5 mL), the combined organic layer was dried over Na₂SO₄, filtered and concentrated. The residue was purified column chromatography (base Al₂O₃, petroleum ether/ethyl acetate=3/1 to petroleum ether/ethyl acetate/methanol=3/1/0.1), the crude product was repurified by prep-IIPLC (column: Xtimate C18 150^{∗}25mm^{∗}5pm; mobile phase: [water (0.05% ammonium hydroxide v/v)-ACN]; B%: 51%-81%, 10 min). The product 2-((*S*)-1-acryloyl-4-(8-(3-fluoro-2-methylphenyl)-2-(((*S*)-1-methylpyrrolidin-2-yl)methoxy)-6,7,8,9-tetrahydro-5*H*-pyrimido[4,5-*c*]azepin-4-yl)piperazin-2-yl)acetonitrile (19.7 mg, 35.7 µmol, 25% yield, 99% purity) was obtained as white solid. LCMS [ESI, M+1]: 548.

¹H NMR (400 MHz, chloroform-d) δ 7.12 - 7.03 (m, 1H), 6.81 (d, *J* = 8.0 Hz, 1H), 6.73 (t, *J* = 8.6 Hz, 1H), 6.65 - 6.51 (m, 1H), 6.40 (dd, *J* = 1.6, 16.8 Hz, 1H), 5.83 (br d, *J* = 10.8 Hz, 1H), 5.16 - 5.07 (m, 1H), 4.38 (dd, *J* = 4.4, 10.4 Hz, 1H), 4.21 - 4.08 (m, 3H), 4.03 - 3.85 (m, 2H), 3.82 - 3.46 (m, 2H), 3.41 - 3.19 (m, 3H), 3.16 - 2.90 (m, 3H), 2.87 - 2.61 (m, 4H), 2.48 (s, 3H), 2.35 - 2.23 (m, 1H), 2.17 - 1.93 (m, 6H), 1.89 - 1.70 (m, 3H).

### EXAMPLE 10

### 2-((S)-1-acryloyl-4-(8-(2-fluoronaphthalen-1-yl)-2-(((S)-1-methylpyrrolidin-2-yl)methoxy)-6,7,8,9-tetrahydro-5H-pyrimido[4,5-c]azepin-4-yl)piperazin-2-yl)acetonitrile

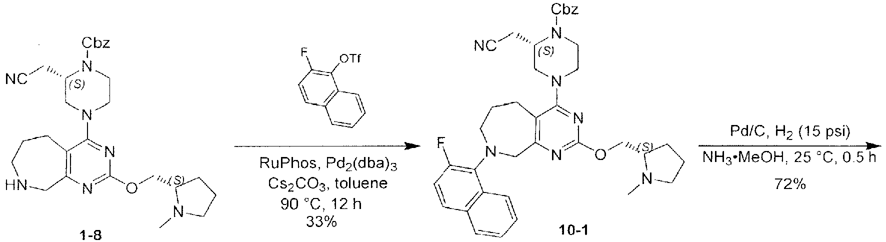

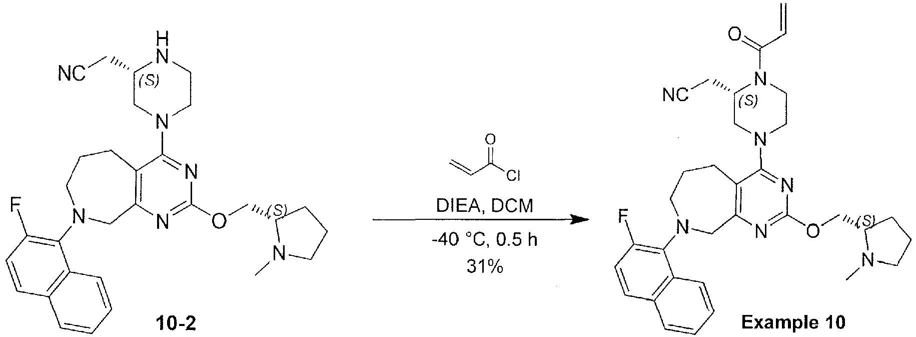

Compound **10-1:** A mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-c]azepin-4-yl]piperazine-1-carboxylate (300 mg, 577 µmol, 1 *eq),* (2-fluoro-1-naphthyl) trifluoromethanesulfonate (339 mg, 1.15 mmol, 2 *eq),* RuPhos (107 mg, 231 µmol, 0.4 *eq*)*,* Pd₂(dba)₃ (105 mg, 115 µmol, 0.2 *eq*) and Cs₂CO₃ (564 mg, 1.73 mmol, 3 *eq*) in toluene (5 mL) was degassed and purged with N₂ for 3 times, and then the mixture was stirred at 90 °C for 12 hours under N₂ atmosphere. The reaction mixture was diluted with water (20 mL) and extracted with ethyl acetate (20 mL × 3). The combined organic layers were washed with brine (20 mL), dried over Na₂SO₄, filtered and concentrated under reduced pressure to give a residue. The residue was purified by reverse phase flash [water (0.1% formic acid)/acetonitrile)]. The collected desired fractions were neutralized with saturated NaHCO₃ aqueous solution to pH = 7 and extracted with ethyl acetate (20 mL × 3). The combined organic layers were washed with brine (20 mL), dried over Na₂SO₄, filtered and concentrated under reduced pressure to give the product. benzyl (2*S*)-2-(cyanomethyl)-4-[8-(2-fluoro-1-naphthyl)- 2-[[(2*S*)-1-methylpyrrolidin-2-yl]methoxy]-5,6,7,9-tetrahydropyrimido[4,5-c]azepin-4-yl]piperazine-1-carboxylate (140 mg, 192 µmol, 33% yield, 91% purity) was obtained as a yellow oil. LCMS [ESI, M+1]: 664. Compound **10-2:** To a solution of benzyl (2S)-2-(cyanomethyl)-4-[8-(2-fluoro- 1-naphthyl)-2-[[(2*S*)-1-methylpyrrolidin-2-yl]methoxy]-5,6,7,9-tetrahydropyrimido[4,5-c]azepin-4-yl]piperazine-1-carboxylate (140 mg, 210 µmol, 1 *eq)* in methanol (5 mL) was added NH₃•MeOH (2 mL, 20% purity) and Pd/C (50 mg, 10% purity) under N₂. The suspension was degassed under vacuum and purged with H₂ several times. The mixture was stirred under H₂ (15 psi) at 25°C for 0.5 hour. The Pd/C was filtered off and the filtrate was concentrated under vacuum. 2-[(2*S*)-4-[8-(2-fluoro-1-naphthyl)-2-[[(2*S*)-1-methylpyrrolidin-2-yl]methoxy]-5,6,7,9-tetrahydropyrimido[4,5-c]azepin-4-yl]piperazin-2-yl]acetonitrile (80 mg, 151 µmol, 72% yield) was obtained as a yellow oil and used into next step without further purification. LCMS [ESI, M+1]: 530.

**Example 10:** To a solution of 2-[(2*S*)-4-[8-(2-fluoro-1-naphthyl)-2- [[(2*S*)-1-methylpyrrolidin-2-yl]methoxy]-5,6,7,9-tetrahydropyrimido[4,5-c]azepin-4-yl]piperazin-2-yl]acetonitrile (60 mg, 113 µmol, 1 *eq*) and DIEA (73.2 mg, 566 µmol, 98.7 µL, 5 *eq)* in DCM (1 mL) was added a solution of prop-2-enoyl chloride (15.4 mg, 169 µmol, 13.9 µL, 1.5 *eq)* in DCM (1 mL) at - 40 °C. After stirred at - 40 °C for 0.5 hour, the reaction mixture was quenched with water (20 mL) and extracted with DCM (20 mL × 3). The combined organic layers were washed with brine (10 mL), dried over Na₂SO₄, filtered and concentrated under reduced pressure to give a residue. The residue was purified by column chromatography (SiO₂, Ethyl acetate/Methanol = 100/1 to 10/1), followed by prep-HPLC (column: Waters Xbridge 150*25 5µ; mobile phase: [water (0.05% ammonium hydroxide v/v) - ACN]; B%: 52% - 76%, 10 min). The desired fraction was collected and lyophilized. 2-[(2*S*)-4-[8-(2-fluoro-1-naphthyl)-2-[[(2*S*)-1-methylpyrrolidin-2-yl]methoxy]-5,6,7,9-tetrahydropyrimido[4,5-c]azepin-4-yl]-1-prop-2-enoyl-piperazin-2-yl]acetonitrile (21 mg, 35.1 µmol, 31% yield, 97% purity) was obtained as a white solid. LCMS [ESI, M+1]: 584.

¹H NMR (400MHz, chloroform-d) δ = 8.18 (d, *J* = 7.6 Hz, 1H), 7.80 (dd, *J* = 1.6, 7.6 Hz, 1H), 7.65 (dd, *J* = 5.2, 8.8 Hz, 1H), 7.49 - 7.38 (m, 2H), 7.27 - 7.20 (m, 1H), 6.75 - 6.53 (m, 1H), 6.41 (dd, *J* = 1.6, 16.8 Hz, 1H), 5.84 (d, *J* = 10.4 Hz, 1H), 5.31 - 4.47 (m, 2H), 4.42 - 4.16 (m, 2H), 4.20 - 4.08 (m, 1H), 4.05 - 3.80 (m, 2H), 3.79 - 3.48 (m, 2H), 3.45 - 2.54 (m, 10H), 2.44 (s, 3H), 2.34 - 2.19 (m, 1H), 2.19 - 1.92 (m, 3H), 1.90 - 1.72 (m, 3H).

### EXAMPLE 11

### 2-((S)-1-acryloyl-4-(8-(5-methylnaphthalen-1-yl)-2-(((S)-1-methylpyrrolidin-2-yl)methoxy)-6,7,8,9-tetrahydro-5Hpyrimido[4,5-c]azepin-4-yl)piperazin-2-yl)acetonitrile

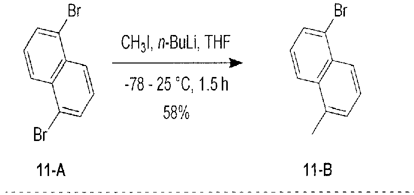

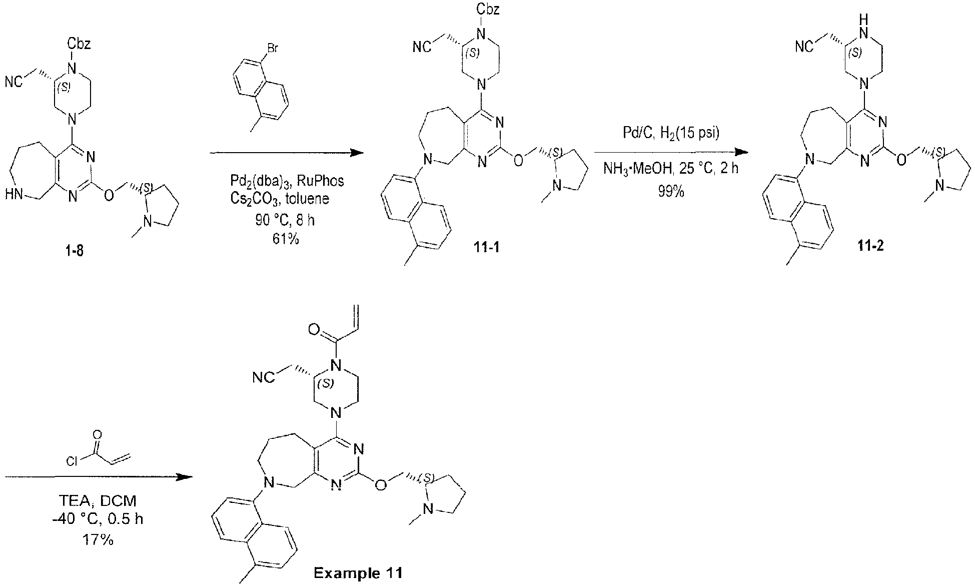

Compound **11-B:** To a mixture of 1,5-dibromonaphthalene (1.00 g, 3.50 mmol, 1.00 eq) in THF (10.0 mL) was added *n*-BuLi (2.5 M, 1.82 mL, 1.3 eq) in portion at -78 °C under N₂. The mixture was stirred at -78 °C for 30 min, then CH₃I (4.58 g, 32.3 mmol, 2.01 mL, 9.23 eq) was added dropwise and warmed to 25 °C and stirred for 1 hour. The reaction mixture was quenched with water (15.0 mL), then extracted with ethyl acetate(30.0 mL × 3). The combined organic layers were washed with brine (50.0 mL × 1), dried over Na₂SO₄, filtered and concentrated under reduced pressure to give a residue. The residue was purified by column chromatography (SiO₂, Petroleum ether/Ethyl acetate=1/0 to 10/1). Compound 1-bromo-5-methyl-naphthalene (450 mg, 2.01 mmol, 58% yield) was obtained as a yellow solid.

¹H NMR (400MHz, chloroform-d) δ = 8.15 (d, *J* = 8.4 Hz, 1H), 7.99 (d, *J* = 8.4 Hz, 1H), 7.81 (dd, *J* = 0.8, 7.2 Hz, 1H), 7.49 (dd, *J*= 7.2, 8.8 Hz, 1H), 7.41 - 7.33 (m, 2H), 2.72 (s, 3H).

Compound **11-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (400 mg, 770 µmol, 1.00 eq) and 1-bromo-5-methyl- naphthalene (204 mg, 924 µmol, 1.20 eq) in toluene (10.0 mL) was added Pd₂(dba)₃ (141 mg, 154 µmol, 0.20 eq), RuPhos (144 mg, 308 µmol, 0.4 eq), Cs₂CO₃ (752 mg, 2.31 mmol, 3.00 eq) under N₂. The mixture was degassed and purged with N₂ for 3 times, then heated to 90 °C and stirred for 8 hours. The reaction mixture was diluted with water (20.0 mL) and extracted with ethyl acetate (30 mL × 3). The combined organic layers were washed with brine (50 mL × 1), dried over sodium sulfate, filtered and concentrated under reduced pressure to give a residue. The residue was purified by reverse phase flash [water (0.1% FA)/acetonitrile]. The desired fractions were collected and neutralized with saturated NaHCO₃ solution and extracted with ethyl acetate (50 mL × 3). The separated organic layer was dried over sodium sulfate, filtered and concentrated under vacuum. Compound benzyl (2*S*)-2-(cyanomethyl)-4- [8-(5-methyl-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (310 mg, 470 µmol, 61% yield, 100% purity) was obtained as a yellow solid. LCMS [ESI, M+1]: 660.

¹H NMR (400MHz, chloroform-d) δ = 7.86 (d, *J* = 7.6 Hz, 1H), 7.71 (d, *J* = 8.4 Hz, 1H), 7.46 - 7.34 (m, 6H), 7.31 - 7.27 (m, 1H), 7.26 - 7.22 (m, 1H), 7.15 (d, *J* = 7.2 Hz, 1H), 5.27 - 5.14 (m, 2H), 4.70 (br s, 1H), 4.43 - 4.33 (m, 3H), 4.22 - 4.06 (m, 2H), 3.87 (br d, *J* = 11.6 Hz, 1H), 3.70 (br d, *J=* 12.8 Hz, 1H), 3.57 - 3.39 (m, 2H), 3.39 - 3.20 (m, 2H), 3.09 (br t, *J* = 7.6 Hz, 1H), 3.05 - 2.83 (m, 4H), 2.82 - 2.73 (m, 1H), 2.68 (s, 4H), 2.46 (s, 3H), 2.33 - 2.23 (m, 1H), 2.17 - 2.00 (m, 3H), 1.90 - 1.72 (m, 3H).

Compound **11-2:** To a solution of benzyl (2*S*)-2-(cyanomethyl)-4-[8-(5-methyl-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (160 mg, 242 µmol, 1.00 eq) in methanol (3.00 mL) was added Pd/C (50.0 mg, 10% purity), NH₃•MeOH (3.00 mL, 20% purity) under N₂. The suspension was degassed under vacuum and purged with H₂ several times. The mixture was stirred under H₂ (15 psi) at 25 °C for 2 hours. The reaction mixture was concentrated under reduced pressure to give a residue. The crude product was used in the next step directly without further purification. Compound 2-[(2*S*)-4-[8-(5-methyl-1-naphthyl)-2-[[(2*S*)-]-methylpyrrolidin-2-yl]methoxy]-5,6,7,9-tetrahydropyrimido[4,5-*c*]azepin-4-yl]piperazin-2-yl]acetonitrile (150 mg, 282 µmol, 99% yield, 99% purity) was obtained as a white solid. LCMS [ESI, M+1]: 526.

**Example 11:** To a mixture of 2-[(2*S*-4-[8-(5-methyl-1-naphthyl)-2-[[(2*S*)-1 - methylpyrrolidin-2-yl]methoxy]-5,6,7,9-tetrahydropyrimido[4,5-*c*]azepin-4-yl]piperazin-2-yl]acetonitrile (120 mg, 228 µmol, 1.00 eq) in dichloromethane (3.00 mL) was added TEA (115 mg, 1.14 mmol, 159 µL, 5.00 eq) and prop-2-enoyl chloride (31.0 mg, 342 µmol, 27.9 µL, 1.50 eq) in portion at -40 °C under N₂. Then the mixture was stirred at -40 °C for 0.5 hours. The reaction mixture was diluted with ice-water (5.00 mL) and extracted with dichloromethane (30.0 mL × 2). The combined organic layers were washed with brine (10.0 mL × 1), dried over sodium sulfate, filtered and concentrated under reduced pressure to give a residue. The residue was purified by prep-HPLC (column: Xtimate C18 150 × 25 mm × 5 µm;mobile phase: [water (0.05% ammonium hydroxide v/v)-ACN];B%: 55%-85%,10 min). Compound 2-[(2*S*)-4-[8-(5-methyl-1-naphthyl)-2-[[(2*S*)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (22.3 mg, 38.4 µmol, 17% yield, 99.8% purity) was obtained as a off-white solid. LCMS [ESI, M+1]: 580.

¹H NMR (400MHz, chloroform-d) δ = 7.86 (br d, *J* = 7.2 Hz, 1H), 7.72 (d. *J* = 8.4 Hz, 1H), 7.43 (t, *J* = 7.6 Hz, 1H), 7.30 - 7.28 (m, 1H), 7.27 - 7.23 (m, 1H), 7.16 (d, *J* = 7.2 Hz, 1H), 6.59 (br d, *J* = 11.2 Hz, 1H), 6.40 (dd, *J* = 1.6, 16.4 Hz, 1H), 5.83 (br d, *J* = 10.6 Hz, 1H), 5.11 (br s, 1H), 4.45 - 4.30 (m, 3H), 4.16 (dd, *J* = 6.8, 10.4 Hz, 1H), 3.92 (br d, *J* = 13.6 Hz, 1H), 3.78 (br d, *J* = 12.0 Hz, 1H), 3.70 - 3.38 (m, 3H), 3.29 (dd, *J=* 4.0, 13.6 Hz, 1H), 3.16 - 2.72 (m, 7H), 2.71 - 2.59 (m, 4H), 2.45 (s, 3H), 2.27 (dt, *J* = 7.2, 9.2 Hz, 1H), 2.18 - 1.96 (m, 3H), 1.89 - 1.73 (m, 3H).

### EXAMPLE 12

### 2-((S)-1-acryloyl-4-(8-(5-fluoronaphthalen-1-yl)-2-(((S)-1-methylpyrrolidin-2-yl)methoxy)-6,7,8,9-tetrahydro-5H-pyrimido[4,5-c]azepin-4-yl)piperazin-2-yl)acetonitrile

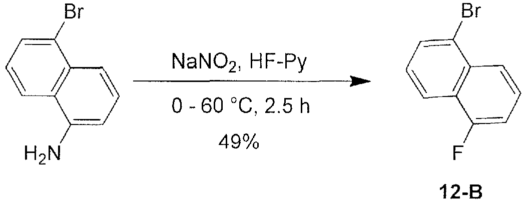

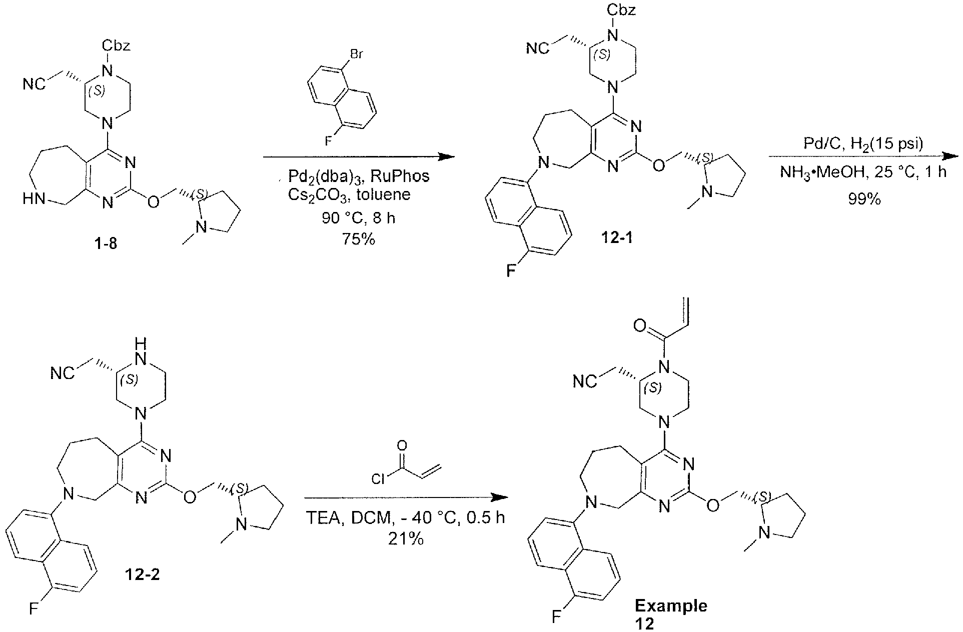

Compound **12-B:** To a mixture of 5-bromonaphthalen-1-amine (2.00 g, 9.01 mmol, 1.00 eq) in pyridine•hydrofluoride (29.8 g, 180 mmol, 27.1 mL, 20.0 eq) was added NaNO₂ (2.49 g, 36.0 mmol, 4.00 eq) at 0 °C under N₂. The mixture was stirred at 25 °C for 30 min, and then heated to 60 °C and stirred for 2 hours. The reaction mixture was diluted with water (20.0 mL) and extracted with ethyl acetate (30.0 mL × 3). The combined organic layers were washed with brine (50.0 mL × 1), dried over sodium sulfate, filtered and concentrated under reduced pressure to give a residue. The residue was purified by column chromatography (SiO₂, Petroleum ether/Ethyl acetate = 1/0 to 1/0). Compound 1-bromo-5-fluoro-naphthalene (1.30 g, 4.45 mmol, 49% yield) was obtained as a yellow oil.

Compound **12-1:** To a mixture of benzyl (2*S*)-2-(cyanomethyl)-4-[2-[[(2*S*)-1-methylpyirolidin-2-yl]methoxy]-6,7,8,9-tetrahydro-5*H*-pyrimido[4,5-*c*]azepin-4-yl]piperazin-1-carboxylate (500 mg, 962 µmol, 1.00 eq) and 1-bromo-5-fluoro- naphthalene (433 mg, 1.92 mmol, 2.00 eq) in toluene (15.0 mL) was added Pd₂(dba)₃ (176 mg, 192 µmol, 0.20 eq), RuPhos (180 mg, 385 µmol, 0.40 eq) and Cs₂CO₃ (941 mg, 2.89 mmol, 3.00 eq) under N₂. The mixture was degassed and purged with N₂ for 3 times, then heated to 90 °C and stirred for 8 hours. The reaction mixture was diluted with water (20.0 mL) and extracted with ethyl acetate (30.0 mL × 3). The combined organic layers were washed with brine (50.0 mL × 1), dried over sodium sulfate, filtered and concentrated under reduced pressure to give a residue. The residue was purified by reverse phase flash [water (0.1% TA)/acetonitrile]. The desired fractions were collected and neutralized with saturated NaHCO₃ solution (12 mL), and then extracted with ethyl acetate (50.0 mL × 3). The separated organic layer was dried over sodium sulfate, filtered and concentrated under vacuum. Compound benzyl (2*S*)-2- (cyanomethyl)-4-[8-(5-fluoro-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (480 mg, 723 µmol, 75% yield, 100% purity) was obtained as a yellow solid. LCMS [ESI, M+1]: 664.

¹H NMR (400MHz, chloroform-d) δ = 7.80 (d, *J* = 8.4 Hz, 1H), 7.71 (d, *J* = 8.4 Hz, 1H), 7.46 - 7.33 (m, 6H), 7.30 (s, 1H), 7.17 (d, *J* = 7.2 Hz, 1H), 7.14 - 7.07 (m, 1H), 5.25 - 5.17 (m, 2H), 4.70 (br s, 1H), 4.43 - 4.33 (m, 3H), 4.17 (d, *J* = 6.8 Hz, 1H), 3.87 (br d, *J* = 12.0 Hz, 1H), 3.70 (br d, J= 12.8 Hz, 1H), 3.58 - 3.50 (m, 1H), 3.47 - 3.22 (m, 3H), 3.09 (br t, *J* = 7.6 Hz, 1H), 3.05 - 2.83 (m, 4H), 2.81 - 2.72 (m, 1H), 2.72 - 2.60 (m, 1H), 2.46 (s, 3H), 2.28 (dt, *J* = 7.2, 9.2 Hz, 1H), 2.20 - 2.06 (m, 2H), 2.04 - 1.94 (m, 1H), 1.92 - 1.78 (m, 4H). LCMS [ESI, M+1]: 530.

Compound **12-2:** To a solution of benzyl (2*S*)-2-(cyanomethyl)-4-[8-(5-fluoro-1-naphthyl)-2-[[(2*S*)-1-methylpyrrolidin-2-yl]methoxy]-5,6,7,9-tetrahydropyrimido[4,5-*c*]azepin-4-yl]piperazine-1-carboxylate (330 mg, 497 µmol, 1.00 eq) in methanol (6.00 mL) was added Pd/C (80.0 mg, 10% purity) and NH₃•MeOH (3.00 mL, 20% purity) under N₂. The suspension was degassed under vacuum and purged with H₂ several times. The mixture was stirred under H₂ (15 psi) at 25 °C for 1 hour. The reaction mixture was concentrated under reduced pressure to give a residue. 2-[(2*S*)-4-[8-(5-fluoro-1-naphthyl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (262 mg, 490 µmol, 99% yield, 99% purity) was obtained as a yellow solid and used in the next step directly without further purification.

**Example 12:** To a mixture of 2-[(2*S*)-4-[8-(5-fluoro-1-naphthyl)-2-[[(2*S*)-1-methylpymolidin-2-yl]methoxy]-5,6,7,9-tetrahydropyrimido[4,5-*c*]azepin-4-yl]piperazin-2-yl]acetonitrile (150 mg, 283 µmol, 1.00 eq) in dichloromethane (3.00 mL) was added TEA (143 mg, 1.42 mmol, 197 µL, 5.00 eq), prop-2-enoyl chloride (38.5 mg, 425 µmol, 34.6 µL, 1.50 eq) in portion at -40 °C under N₂. The mixture was stirred at -40 °C for 30 min. The reaction mixture was quenched by adding water (3.00 mL) at -40 °C, and then extracted with dichloromethane (10.0 mL × 3). The combined organic layers were washed with brine (10.0 mL × 1), dried over sodium sulfate, filtered and concentrated under reduced pressure to give a residue. The residue was purified by prep-HPLC (column: Xtimate C18 150 × 25 mm × 5 µm;mobile phase: [water (0.05% ammonium hydroxide v/v)-ACN];B%: 53%-83%,10 min). Compound 2-[(2*S*)-4-[8-(5-fluoro-1-naphthyl)-2-[[(2*S*)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (34.2 mg, 58.5 µmol, 21% yield) was obtained as a white solid. LCMS [ESI, M+1]: 584.

¹H NMR (400MHz, chloroform-d) δ = 7.81 (d, *J* = 8.0 Hz, 1H), 7.72 (d, *J* = 8.4 Hz, 1H), 7.44 (t, *J* = 7.6 Hz, 1H), 7.32 - 7.27 (m, 1H), 7.18 (d, *J* = 7.2 Hz, 1H), 7.14 - 7.07 (m, 1H), 6.69 - 6.53 (m, 1H), 6.40 (dd, *J* = 2.0, 16.8 Hz, 1H), 5.83 (br d, *J* = 10.4 Hz, 1H), 5.10 (br s, 1H), 4.44 - 4.32 (m, 3H), 4.17 (dd, *J* = 6.8, 10.4 Hz, 1H), 3.93 (br d, *J* = 14.0 Hz, 2H), 3.78 (br d, *J* = 12.0 Hz, 1H), 3.68 - 3.40 (m, 3H), 3.30 (dd, *J* = 3.6, 13.6 Hz, 1H), 3.18 - 2.86 (m, 5H), 2.80 (br s, 1H), 2.71 - 2.61 (m, 1H), 2.46 (s, 311), 2.35 - 2.23 (m, 1H), 2.22 - 1.97 (m, 3H), 1.92 - 1.72 (m, 3H).

### EXAMPLE 13

### 2-[(2S)-4-[2-[[(2S)-1-methylpyrrolidin-2-yl]methoxy]-8-[2-(trifluoromethyl)phenyl]-5,6,7,9-tetrahydropyrimido[4,5-c]azepin-4-yl]-1-prop-2-enoyl-piperazin-2-yl]acetonitrile

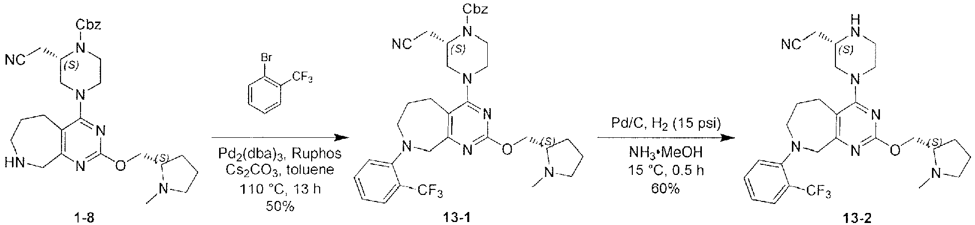

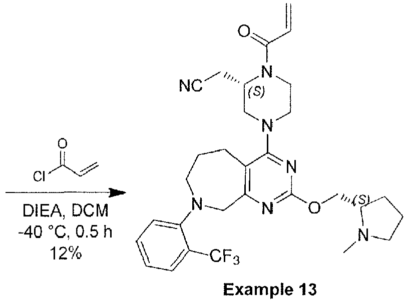

Compound **13-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (200 mg, 385 µmol, 1.0 *eq*), 1-bromo-2-(trifluoromethyl)benzene (173 mg, 770 µmol, 105 µL, 2.0 *eq),* Cs₂CO₃ (376 mg, 1.15 mmol, 3.0 *eq)* and RuPhos (71.8 mg, 154 µmol, 0.4 *eq*) in toluene (6 mL) was added Pd₂ (dba)₃ (70.5 mg 77.0 µmol, 0.2 *eq*) under N₂. The suspension was degassed under vacuum and purged with N₂ several times. The mixture was stirred under N₂ at 110 °C for 13 hours. Water (15 mL) was added into the mixture. The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1 % FA)/acetonitrile] to give benzyl (2S)-2-(cyanomethyl)-4-[2-[[(2S)-1-methylpyrrolidin-2-yl]methoxy]-8- [2-(trifluoromethyl)phenyl]-5,6,7,9-tetrahydropyrimido[4,5-c]azepin-4-yl]piperazine-1-carboxylate (140 mg, 194 µmol, 50 % yield, 92 % purity) as a yellow solid. LCMS [ESI, M+1]: 664.

¹H NMR (400 MHz, chloroform-d) δ = 7.63 - 7.58 (m, 1H), 7.55 - 7.49 (m, 1H), 7.42 - 7.33 (m, 6H), 7.22 (t, *J* = 7.6 Hz, 1H), 5.26 - 5.16 (m, 2H), 4.70 (br s, 1H), 4.37 (br dd, *J*= 4.8, 10.4 Hz, 1H), 4.16 - 4.08 (m, 2H), 3.81 (br d, *J* = 10.0 Hz, 1H), 3.66 (brd, *J*= 12.4 Hz, 1H), 3.52 - 3.14 (m, 5H), 3.09 (br t, *J* = 7.6 Hz, 1H), 3.02 - 2.72 (m, 6H), 2.66 (br s, 1H), 2.47 (s, 3H), 2.33 - 2.22 (m, 1H), 2.05 - 1.91 (m, 3H), 1.89 - 1.74 (m, 3H)..

Compound **13-2:** To a solution of benzyl (2*S*)-2-(cyanomethyl)-4-[2-[[(2*S*)-1-methylpyrrolidin-2-yl]methoxy]-8-[2-(trifluoromethyl)phenyl]-5,6,7,9-tetrahydropyrimido[4,5-*c*]azepin-4-yl]piperazine-1-carboxylate (140 mg, 211 µmol, 1.0 *eq)* and NH₃•MeOH (2 mL, 15 % purity) was added Pd/C (60 mg, 10 % purity) under N₂. The suspension was degassed under vacuum and purged with H₂ several times. The mixture was stirred under H₂ (15 psi) at 15 °C for 0.5 hour. The reaction mixture was filtered and the filtrate was concentrated under vacuum to give 2-[(2*S*)-4-[2-[[(2*S*)-1-methylpyrrolidin-2-yl]methoxy]-8-[2-(trifluoromethyl) phenyl]-5,6,7,9-tetrahydropyrimido[4,5-*c*]azepin-4-yl]piperazin-2-yl]acetonitrile (75 mg, 127 µmol, 60 % yield, 90 % purity) as a yellow solid which was used for next step without further purification.

**Example 13:** To a solution of 2-[(2*S*)-4-[2-[[(2*S*)-1-methylpyrrolidin-2-yl]methoxy]-8-[2-(trifluoromethyl)phenyl]-5,6,7,9-tetrahydropyrimido[4,5-*c*]azepin-4-yl]piperazin-2-yl]acetonitrile (75 mg, 142 µmol, 1.0 *eq*) and DIEA (36.6 mg, 283 µmol, 49.3 uL, 2.0 *eq*) in DCM (1.5 mL) was added prop-2-enoyl chloride (19.2 mg, 212 µmol, 17.3 µL, 1.5 *eq*) at -40 °C. The reaction mixture was stirred at -40 °C for 0.5 hour. The reaction mixture was quenched by water (8 mL). The mixture was extracted with DCM (3 × 10 mL). The combined organic layers were dried over anhydrous Na₂SO₄, filtered and concentrated under vacuum. The residue was purified by reverse-phase flash [water (0.1 % FA)/acetonitrile]. The residue was purified by prep-HPLC (column: Waters Xbridge 150^{∗}25 5µ:mobile phase: [water (0.05 % ammonium hydroxide v/v)-ACN];B %: 55 % - 73 %, 10 min) to give 2-[(2*S*)-4-[2-[[(2*S*)-1-methylpyrrolidin-2-yl]methoxy]-8-[2- (trifluoromethyl)phenyl]-5,6,7,9-tetrahydropyrimido[4,5-*c*]azepin-4-yl]-1-prop-2-enoyl-piperazin-2-yl]acetonitrile (10.3 mg, 17.4 µmol, 12 % yield, 98.8 % purity) as a white solid. LCMS [ESI, M+1]: 584.

¹H NMR (400 MHz, chloroform-d) δ = 7.61 (d, *J* = 7.6 Hz, 1H), 7.56 - 7.49 (m, 1H), 7.35 (d, *J* = 8.0 Hz, 1H), 7.23 (t, *J* = 7.6 Hz, 1H), 6.58 (br d, *J* = 10.0 Hz, 1H), 6.39 (dd, *J* = 1.6, 16.8 Hz, 1H), 5.82 (br d, *J* = 10.4 Hz, 1H), 5.13 (br s, 1H), 4.36 (dd, *J* = 4.8, 10.6 Hz, 1H), 4.22 (q, *J* = 17.2 Hz, 2H), 4.11 (dd, *J* = 6.8, 10.4 Hz, 1H), 4.04 - 3.81 (m, 2H), 3.80 - 3.38 (m, 2H), 3.34 - 3.17 (m, 3H), 3.08 (br t, *J* = 7.6 Hz, 1H), 3.04 - 2.72 (m, 5H), 2.71 - 2.60 (m, 1H), 2.46 (s, 3H), 2.32 - 2.22 (m, 1H), 2.12 - 1.90 (m, 3H), 1.89 - 1.72 (m, 3H).

### EXAMPLE 14

### 2-[(2S)-4-[8-[3-fluoro-2-(trifluoromethyl)phenyl]-2-[[(2S)-1-methylpyrrolidin-2-yl]methoxy]-5,6,7,9-tetrahydropyrimido[4,5-c]azepin-4-yl]-1-prop-2-enoyl-piperazin-2-yl]acetonitrile

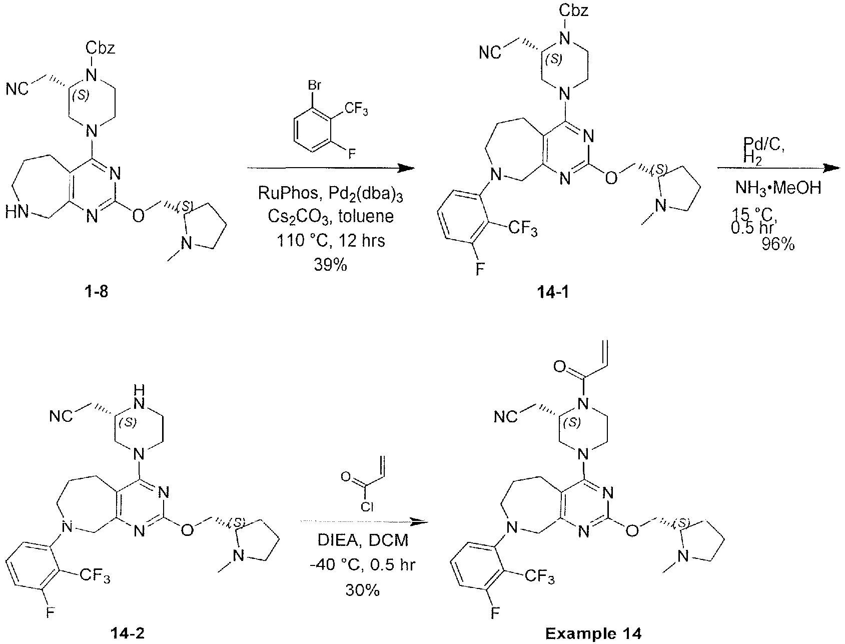

Compound **14-1:** To a solution of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (250 mg, 481 µmol, 1.0 *eq),* 1-bromo-3-fluoro-2- (trifluoromethyl)benzene (234 mg, 962 µmol, 2.0 *eq),* RuPhos (89.8 mg, 192 µmol, 0.40 *eq)* and Cs₂CO₃ (392 mg, 1.20 mmol, 2.50 *eq)* in toluene (3.0 mL) was added Pd₂(dba)₃ (88.1 mg, 96.2 µmol, 0.20 *eq).* The mixture was stirred at 110 °C for 12 hours. After completion, the reaction mixture was added water (10.0 mL) and extracted with ethyl acetate (3 × 10.0 mL). The organic layer was dried over Na₂SO₄, filtered and concentrated. The residue was purified by reverse phase flash (C18, 0.1% FA in water, 0-60 % MeCN) to give the compound benzyl (2*S*)-2-(cyanomethyl)-4-[8-[3-fluoro-2-(trifluoromethyl)phenyl]-2-[[(2*S*)-1-methylpyrrolidin-2-yl]methoxy]-5,6,7,9-tetrahydropyrimido[4,5-*c*]azepin-4-yl]piperazine-1-carboxylate (130 mg, 190 µmol, 39% yield, 99% purity) as yellow solid. LCMS [ESI, M+1]: 682.

¹H NMR (400 MHz, chloroform-d) δ 7.42 - 7.36 (m, 5H), 6.94 (d, *J* = 8.4 Hz, 1H), 6.88 - 6.81 (m, 1H), 5.20 (s, 2H), 4.74 - 4.62 (m, 1H), 4.40 - 4.35 (m, 1H), 4.17 - 4.10 (m, 4H), 3.85 - 3.75 (m, 1H), 3.66 - 3.56 (m, 1H), 3.43 - 3.17 (m, 4H), 3.13 - 3.05 (m, 1H), 2.99 - 2.81 (m, 2H), 2.79 - 2.54 (m, 5H), 2.47 (s, 3H), 2.33 - 2.24 (m, 1H), 2.04 - 1.94 (m, 3H), 1.87 - 1.73 (m, 3H).

Compound **14-2:** To a solution of benzyl (2*S*)-2-(cyanomethyl)-4-[8-[3-fluoro- 2-(trifluoromethyl)phenyl]-2-[[(2*S*)-1-methylpyrrolidin-2-yl]methoxy]-5,6,7,9-tetrahydropyrimido[4,5-*c*]azepin-4-yl]piperazine-1-carboxylate (130 mg, 191 µmol, 1.0 *eg*) in MeOH (2.0 mL) and NH₃•MeOH (2.0 mL, 20% purity) was added Pd/C (40.0 mg, 10% purity). The mixture was stirred under H₂ (15 Psi) atmosphere at 15 °C for 0.5 hour. After completion, the reaction mixture was filtered and washed with tetrahydrofuran (20.0 mL). The filtrate was concentrated to give the compound 2-[(2*S*)-4-[8-[3-fluoro-2-(trifluoromethyl)phenyl]-2-[[(2*S*)-1-methylpyrrolidin-2-yl]methoxy]-5,6,7,9-tetrahydropyrimido[4,5-c]azepin-4-yl]piperazin-2-yl]acetonitrile (100 mg, 183 µmol, 96% yield) as yellow solid. The product was used for the next step without further purification. LCMS [ESI, M+1]: 548.

**Example 14:** To a solution of 2-[(2*S*)-4-[8-[3-fluoro-2- (trifluoromethyl) phenyl]-2-[[(2*S*)-1-methylpyrrolidin-2-yl]methoxy]-5,6,7,9-tetrahydropyrimido[4,5-*c*]azepin-4-yl]piperazin-2-yl]acetonitrile (90.0 mg, 164 µmol, 1.0 *eq)* and DIEA (42.5 mg, 329 µmol, 57.3 µL, 2.0 *eq)* in dichloromethane (2.0 mL) was added prop-2-enoyl chloride (22.3 mg, 247 µmol, 20.1 µL, 1.50 *eq)* at -40 °C. The mixture was stirred at -40 °C for 0.5 hour. After completion, the reaction mixture was quenched with H₂O (2.50 mL) at -40 °C, then warmed to 15 °C and extracted with dichloromethane (2 × 10 mL). The combined organic layers were dried over Na₂SO₄, filtered and concentrated under reduced pressure to give the residue. The residue was purified by prep-HPLC (column: Xtimate C18 150*25mm*5um; mobile phase: [water (0.05% ammonium hydroxide v/v)-ACN]; B%: 51%-81%, 10 min) to give the compound 2-[(2*S*)-4-[8-[3-fluoro-2-(trifluoromethyl)phenyl]-2-[[(2*S*)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (30.2 mg, 50.1 µmol, 30% yield, 99% purity) as white solid. LCMS [ESI, M+1]: 602.

¹H NMR (400 MHz, Chloroform-d) δ 7.40 - 7.33 (m, 1H), 6.95 (d, *J* = 8.4 Hz, 1H). 6.89 - 6.82 (m, 1H), 6.67 - 6.50 (m, 1H), 6.38 (dd, *J* = 1.6, 16.8 Hz, 1H), 5.82 (br d, *J* = 10.8 Hz, 1H), 5.20 - 4.90 (m, 1H), 4.42 - 4.26 (m, 3H), 4.18 - 4.11 (m, 1H), 4.08 - 3.77 (m, 2H), 3.75 - 3.50 (m, 2H), 3.42 - 3.21 (m, 3H), 3.14 - 3.05 (m, 1H), 3.03 - 2.87 (m, 2H), 2.81 - 2.62 (m, 4H), 2.48 (s, 3H), 2.33 - 2.24 (m, 1H), 2.10 - 1.94 (m, 3H), 1.88 - 1.68 (m, 3H).

### EXAMPLE 15

### 2-[(2S)-4-[2-[[(2S)-1-methylpyrrolidin-2-yl]methoxy]-8-[3-(trifluoromethyl)-2-pyridyl]-5,6,7,9-tetrahydropyrimido[4,5-c]azepin-4-yl]-1-prop-2-enoyl-piperazin-2-yl]acetonitrilc

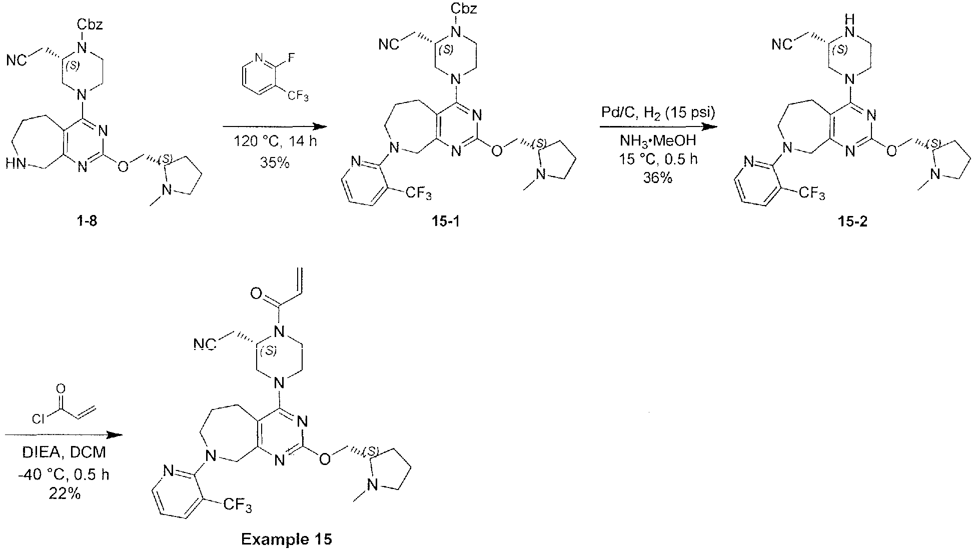

Compound 15-1: A mixture of benzyl (2*S*)-2-(cyanomethyl)-4-[2-[[(2*S*)-1-methylpyrrolidiu-2-yl]methoxy]-6,7,8,9-tetrahydro-5*H*-pyrimido[4,5-c]azepin-4-yl]piperazine-1-carboxylate (300 mg, 577 µmol, 1.0 *eq)* and 2-fluoro-3-(trifluoromethyl) pyridine (953 mg, 5.77 mmol, 10 *eq)* was heated to 120 °C and stirred for 14 hours. The mixture was diluted with EtOAc (10 mL) and CuSO4 (4 %, 15 mL). Then the mixture was extracted with EtOAc (2 × 20 mL). The combined organic layers were washed with CuSO4 (4 %, 3 × 15 mL) and brine (30 mL). The combined organic layers were dried over anhydrous Na₂SO₄, filtered and concentrated under vacuum. The residue was purified by reverse-phase flash [water (0.1% FA)/acetonitrile] to give benzyl (2*S*)-2-(cyanomethyl)-4-[2-[[(2*S*)-1-methylpyrrolidin-2-yl]methoxy]-8-[3-(trifluoromethyl)-2-pyridyl]-5,6,7,9-tetrahydropyrimido[4,5-*c*]azepin-4-yl]piperazine-1-carboxylate (140 mg, 200 µmol, 35 % yield, 95 % purity) as a yellow solid. LCMS [ESI, M+1]: 665.

¹H NMR (400 MHz, chloroform-d) δ = 8.29 (dd, *J* = 1.6, 4.8 Hz, 1H), 7.83 (dd, *J* = 1.6, 7.6 Hz, 1H), 7.42 - 7.32 (m, 5H), 6.89 (dd, *J* = 4.8, 7.6 Hz, 1H), 5.24 - 5.13 (m, 2H), 4.65 (br s, 1H), 4.62 - 4.45 (m, 2H), 4.41 (dd, *J* = 4.8, 10.4 Hz, 1H), 4.16 - 4.00 (m, 2H), 3.75 (br d, *J* = 13.2 Hz, 1H), 3.67 - 3.51 (m, 3H), 3.27 (br s, 1H), 3.19 (dd, *J* = 3.6, 13.6 Hz, 1H), 3.11 (br t, *J* = 7.6 Hz, 1H), 2.88 (dt, *J* = 3.6, 12.4 Hz, 1H), 2.82 - 2.64 (m, 4H), 2.62 - 2.54 (m, 1H), 2.49 (s, 3H), 2.35 - 2.25 (m, 1H), 2.15 - 2.06 (m, 2H), 2.04 - 1.97 (m, 1H), 1.93 - 1.76 (m, 3H).

Compound **15-2:** To the solution of benzyl (2*S*)-2-(cyanomethyl)-4-[2-[[(2*S*)-1-methylpyrrolidin-2-yl]methoxy]-8-[3-(trifluoromethyl)-2-pyridyl]-5,6,7,9-tetrahydropyrimido[4,5-*c*]azepin-4-yl]piperazine-1-carboxylate (140 mg, 211 µmol, 1.0 *eq)* and NH₃•MeOH (1 mL, 20 % purity) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filter cake was washed with MeOH (3 × 8 mL). The filtrate was concentrated under vacuum to give 2-[(2*S*)-4-[2-[[(2*S*)-1-methylpyrrolidin-2-yl] methoxy]-8-[3-(trifluoromethyl)-2-pyridyl]-5,6,7,9-tetrahydropyrimido[4,5-*c*]azepin-4-yl]piperazin-2-yl]acetonitrile (45 mg, 76.3 µmol, 36 % yield, 90 % purity) as a yellow solid which was used for next step without further purification.

**Example 15:** To a solution of 2-[(2*S*)-4-[2-[[(2*S*)-1-methylpyrrolidin-2-yl]methoxy]-8-[3-(trifluoromethyl)-2-pyridyl]-5,6,7,9-tetrahydropyrimido[4,5-*c*]azepin-4-yl]piperazin-2-yl]acetonitrile (45 mg, 84.8 µmol, 1.0 *eq*) and DIEA (32.9 mg, 254 µmol, 44.3 µL, 3.0 *eq)* in DCM (1 mL) was added 2-oxoacetyl chloride (11.8 mg, 127 µmol, 1.5 *eq)* at -40 °C, and the mixture was stirred at -40 °C for 0.5 hour. Water (8 mL) was added into the mixture. The mixture was diluted with EtOAc (8 mL) and extracted with EtOAc (2 × 10 mL). The combined organic layers were washed with brine (20 mL). The combined organic layers were dried over anhydrous Na₂SO₄, filtered and concentrated under vacuum. The residue was purified by reverse-phase flash [water (0.1 % FA)/acetonitrile]. The residue was purified by prep-HPLC (column: Waters Xbridge 150^{∗}25 5µ:mobile phase: [water (0.05 % ammonium hydroxide v/v)-ACN]; B %: 45 % - 69 %, 10 min) to give 2-[(2*S*)-4-[2-[[(2*S*)-1-methylpyrrolidin-2-yl]methoxy]-8-[3-(trifluoromethyl)-2-pyridyl]-5,6,7,9-tetrahydropyrimido[4,5-*c*]azepin-4-yl]-1-prop-2-enoyl-piperazin-2-yl]acetonitrile (10.9 mg, 18.5 µmol, 22 % yield, 99.7 % purity) as a white solid. LCMS [ESI, M+1]: 585.

¹H NMR (400 MHz, chloroform-d) δ = 8.29 (dd, *J* = 1.6, 4.8 Hz, 1H), 7.84 (dd, *J* = 1.6, 7.6 Hz, 1H), 6.90 (dd, *J* = 4.8, 7.6 Hz, 1H), 6.55 (br d, *J* = 10.4 Hz, 1H), 6.37 (dd, *J* = 1.6, 16.8 Hz, 1H), 5.80 (br d, *J* = 10.4 Hz, 1H), 5.07 (br s, 1H), 4.63 - 4.46 (m, 2H), 4.39 (dd, J= 4.8, 10.8 Hz, 1H), 4.14 (dd, *J* = 7.2, 10.4 Hz, 1H), 4.03 - 3.74 (m, 2H), 3.72 - 3.42 (m, 4H), 3.21 (dd, *J* = 3.6, 13.6 Hz, 1H), 3.09 (br t, J= 7.6 Hz, 1H), 3.00 - 2.82 (m, 2H), 2.80 - 2.56 (m, 4H), 2.48 (s, 3H), 2.34 - 2.23 (m, 1H), 2.17 - 1.99 (m, 3H), 1.90 - 1.76 (m, 3H).

### EXAMPLE 16

### 2-[(2S)-4-[8-[2-(hydroxymethyl)phenyl]-2-[[(2S)-1-methylpyroolidin-2-yl]methoxy]-5,6,7,9-tetrahydropyrimido[4,5-c]azepin-4-yl]-1-prop-2-enoyl-piperazin-2-yl]acetonitrile

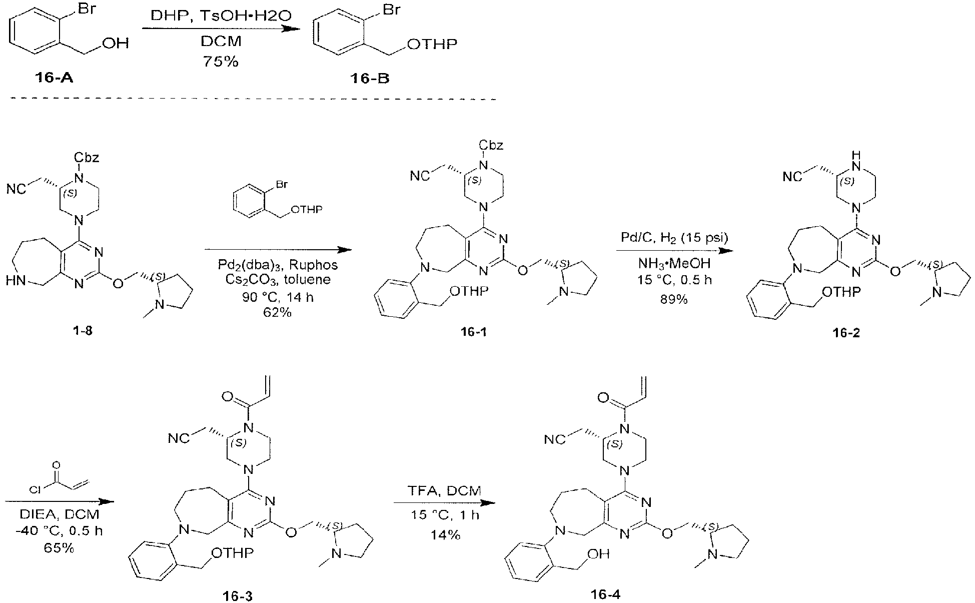

Compound **16-B:** To a solution of (2-bromophenyl)methanol (1 g, 5.35 mmol, 1.0 *eq)* and TsOH.H₂O (102 mg, 535 µmol, 0.1 *eq*) in DCM (20 mL) was added DHP (899 mg, 10.7 mmol, 978 µL, 2.0 *eq),* the mixture was stirred at 18 °C for 14 hours. To the mixture was added saturated NaHCO₃ (20 mL). The mixture was extracted with DCM (2 × 15 mL). The combined organic layers were dried over anhydrous Na₂SO₄, filtered and concentrated under vacuum. The residue was purified by silica gel chromatography (Petroleum ether: Ethyl acetate = 150:1) to give 2-[(2-bromophenyl)methoxy] tetrahydropyran (1.15 g, 4.03 mmol, 75 % yield, 95 % purity) as a colorless oil.

¹H NMR (400 MHz, chloroform-d) δ = 7.57 - 7.50 (m, 2H), 7.33 (dt, *J* = 1.2, 7.6 Hz, 1H), 7.18 - 7.12 (m, 1H), 4.84 (d, *J* = 13.2 Hz, 1H), 4.79 (t, *J* = 3.6 Hz, 1H), 4.59 (d, *J* = 13.2 Hz, 1H), 3.99 - 3.90 (m, 1H), 3.63 - 3.53 (m, 1H), 1.98 - 1.85 (m, 1H), 1.84 - 1.59 (m, 4H), 1.58 - 1.53 (m, 1H).

Compound **16-1:** To a mixture of benzyl (2*S*)-2-(cyanomelhyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (400 mg, 770 µmol, 1.0 *eq),* 2-[(2-bromophenyl)methoxy]tetrahydropyran (313 mg, 1.15 mmol, 1.5 *eq),* Cs₂CO₃ (752 mg, 2.31 mmol, 3.0 *eq)* and RuPhos (144 mg, 308 µmol, 0.4 *eq)* in toluene (8 mL) was added Pd₂(dba)₃ (141 mg, 154 µmol, 0.2 *eq)* under N₂. The suspension was degassed under vacuum and purged with N₂ several times. The mixture was stirred under N₂ at 90 °C for 14 hours. Water (20 mL) was added into the mixture. The mixture was diluted with EtOAc (10 mL) and extracted with EtOAc (2 × 20 mL). The combined organic layers were washed with brine (20 mL). The combined organic layers were dried over anhydrous Na₂SO₄, filtered and concentrated under vacuum. The residue was purified by reverse-phase flash [water (0.1% FA)/acetonitrile] to give benzyl (2*S*-2-(cyanomethyl)-4-[2-[[(2*S*)-1-methylpyrrolidin-2-yl]methoxy]-8-[2-(tetrahydropyran-2-yloxymethyl)phenyl]-5,6,7,9-tetrahydropyrimido[4,5-*c*]azepin-4-yl]piperazine-1-carboxylate (360 mg, 482 µmol, 62 % yield, 95 % purity) as a yellow solid. LCMS [ESI, M+1]: 710.

¹H NMR (400 MHz, chloroform-d) δ = 7.46 (dd, *J* = 2.8, 7.6 Hz, 1H), 7.42 - 7.32 (m, 5H), 7.26 - 7.20 (m, 1H), 7.11 - 7.05 (m, 2H), 5.27 - 5.16 (m, 2H), 4.67 (br dd, *J* = 4.4, 12.4 Hz, 2H), 4.54 - 4.41 (m, 2H), 4.37 (ddd, *J* = 2.0, 4.8, 10.4 Hz, 1H), 4.28 - 4.15 (m, 2H), 4.12 - 4.07 (m, 1H), 3.88 - 3.74 (m, 2H), 3.65 (br d, *J* = 12.4 Hz, 1H), 3.45 - 3.16 (m, 5H), 3.08 (br t, J= 7.2 Hz, 1H), 2.99 - 2.71 (m, 5H), 2.69 - 2.60 (m, 1H), 2.46 (s, 3H), 2.27 (dt, *J* = 7.2, 9.2 Hz, 1H), 2.04 - 1.89 (m, 2H), 1.88 - 1.69 (m, 5H), 1.66 - 1.43 (m, 5H).

Compound **16-2:** To a solution of benzyl (2*S*)-2-(cyanomethyl)-4-[2-[[(2*S*)-1-methylpyrrolidin-2-yl]methoxy]-8-[2-(tetrahydropyran-2-yloxymethyl)phenyl]-5,6,7,9-tetrahydropyrimido[4,5-*c*]azepin-4-yl]piperazine-1-carboxylate (200 mg, 282 µmol, 1.0 *eq*) and NH₃•MeOH (2 mL, 20 % purity) was added Pd/C (50 mg, 10 % purity) under N₂. The suspension was degassed under vacuum and purged with H₂ several times. The mixture was stirred under H₂ (15 psi) at 15 °C for 0.5 hour. The reaction mixture was filtered and the filter cake was washed with MeOH (3 × 8 mL). The filtrate was concentrated under vacuum to give 2-[(2*S*)-4-[2-[[(2*S*)-1-methylpyrrolidin-2-yl]methoxy]-8-[2-(tetrahydropyran-2-yloxymethyl)phenyl]-5,6,7,9-tetrahydropyrimido[4,5-*c*]azepin-4-yl]piperazin-2-yl]acetonitrile (160 mg, 250 µmol, 89 % yield, 90 % purity) as a yellow solid which was used for next step without further purification.

Compound **16-3:** To a solution of 2-[(2*S*)-4-[2-[[(2*S*)-1-methylpyrrolidin-2-yl]methoxy]-8-[2-(tetrahydropyran-2-yloxymethyl)phenyl]-5,6,7,9-tetrahydropyrimido[4,5-*c*]azepin-4-yl]piperazin-2-yl]acetonitrile (160 mg, 278 µmol, 1.0 *eq*) and DIEA (71.8 mg, 556 µmol, 96.8 µL, 2.0 *eq)* in DCM (3 mL) was added prop-2-enoyl chloride (37.7 mg, 417 µmol, 34.0 µL, 1.5 *eq)* at -40 °C, the mixture was stirred at -40 °C for 0.5 hour. The reaction mixture was quenched with water (10 mL). The mixture was diluted with EtOAc (10 mL) and extracted with EtOAc (2 × 15 mL). The combined organic layers were washed with brine (20 mL). The combined organic layers were dried over anhydrous Na₂SO₄, filtered and concentrated under vacuum. The residue was purified by reverse-phase flash [water (0.1% FA)/acetonitrile] to give 2-[(2*S*)-4-[2-[[(2*S*)-1-methylpyrrolidin-2-yl]methoxy]-8-[2-(tetrahydropyran-2-yloxymethyl)phenyl]-5,6,7,9-tetrahydropyrimido[4,5-*c*]azepin-4-yl]-1-prop-2-enoyl-piperazin-2-yl]acetonitrile (120 mg, 181.01 µmol, 65.14% yield, 95% purity) as a yellow solid. LCMS [ESI, M+1]: 630.

**Example 16:** To the solution of 2-[(2*S*)-4-[2-[[(2*S*)-1-methylpyrrolidin-2-yl]methoxy]-8 [2-(tetrahydropyran-2-yloxymethyl)phenyl]-5,6,7,9-tetrahydropyrimido[4,5-*c*]azepin-4-yl]-1-prop-2-enoyl-piperazin-2-yl]acetonitrile (100 mg, 159 µmol, 1.0 *eq)* in DCM (0.1 mL) was added TFA (308 mg, 2.70 mmol, 0.2 mL, 17 *eq),* the mixture was stirred at 15 °C for 1 hour. The reaction mixture was concentrated under vacuum. The residue was diluted with EtOAc (10 mL) and saturated NaHCO₃ (10 mL). Then the mixture was extracted with EtOAc (2 × 15 mL). The combined organic layers were washed with brine (15 mL). The combined organic layers were dried over anhydrous Na₂SO₄, filtered and concentrated under vacuum. The residue was purified by reverse-phase flash [water (0.1 % FA)/acetonitrile]. The residue was purified by prep-HPLC (column: Xtimate C18 150^{∗}25mm^{∗}5µm;mobile phase: [water (0.05 % ammonium hydroxide v/v)-ACN];B %: 32 % - 62 %,10 min) to give 2-[(2*S*)-4-[8-[2-(hydroxymethyl) phenyl]-2-[[(2S)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (12.0 mg, 22.0 µmol, 14 % yield, 99.7 % purity) as a white solid. LCMS [ESI, M+1): 546.

¹H NMR (400 MHz, chloroform-d) δ = 7.32 (dd, *J* =1.2, 7.6 Hz, 1H), 7.30 - 7.27 (m, 1H), 7.18 (d. *J* = 7.2 Hz, 1H), 7.10 (dt, *J* = 1.2, 7.6 Hz, 1H), 6.66 - 6.52 (m, 1H), 6.39 (dd, *J* = 1.6, 16.8 Hz, 1H), 5.82 (br d, *J* = 11.6 Hz, 1H), 5.07 (br s, 111), 4.65 (d, *J* = 1.2 Hz, 2H), 4.37 (dd, *J* = 5.2, 10.4 Hz, 1H), 4.29 - 4.12 (m, 3H), 3.87 (br d, *J* = 13.6 Hz, 2H), 3.82 - 3.46 (m, 2H), 3.44 - 3.23 (m, 3H), 3.14 - 2.98 (m, 2H), 2.97 - 2.89 (m, 1H), 2.88 - 2.79 (m, 2H), 2.78 - 2.60 (m, 2H), 2.47 (s, 3H), 2.28 (dt, *J* = 7.2, 9.2 Hz, 1H), 2.13 - 1.92 (m, 3H), 1.90 - 1.75 (m, 3H).

### EXAMPLE 17

### 4-[(3S)-3-(cyanomethyl)piperazin-1-yl]-7-(3-hydroxy-1-naphthyl)-2-[[(2S)-1-methylpyrrolidin-2-yl]methoxy]-6,8-dihydro-5H-1,7-naphthyridine-3-carbonitrile

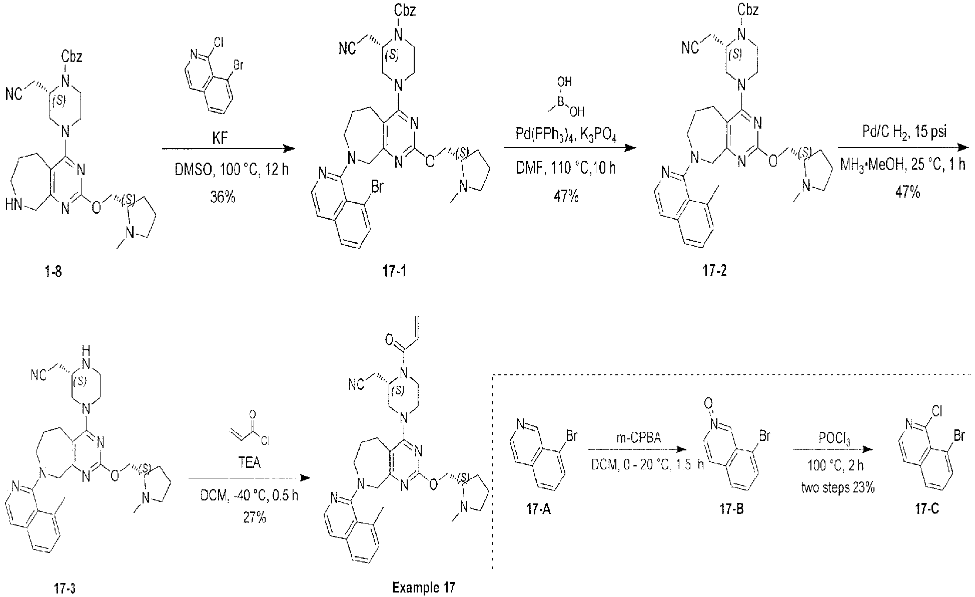

Compound **17-B:** To a mixture of 8-bromoisoquinoline (1.00 g, 4.81 mmol, 1.00 eq) in dichloromethane (10.0 mL) was added *m*-CPBA (1.17 g, 5.77 mmol, 85% purity, 1.20 eq) at 0 °C. After stirring at 0 °C for 0.5 h and 20 °C for 1 hour, the mixture was washed with saturated Na₂CO₃ (3 × 10.0 mL) and brine (1 × 10.0 mL), dried over Na₂SO₄, filtered and concentrated under vacuum to give 8-bromo-2-oxido-isoquinolin-2-ium (1.20 g, crude) as a yellow solid and used into next step without further purification. LCMS [ESI, M+1]: 226.

Compound **17-C:** A mixture of 8-bromo-2-oxido-isoquinolin-2-ium (2.20 g, crude) in POCl₃ (20.0 g, 130 mmol, 12.1 mL) was stirred at 100 °C for 2 hours. The mixture was concentrated under vacuum. The residue was diluted with EA (10.0 mL) and adjusted pH > 7 by saturated Na₂CO₃. The organic layer was washed brine (1 × 5.00 mL), dried over Na₂SO₄, filtered and concentrated under vacuum. The residue was purified by column chromatography (SiO₂, PE/EA=5/1) to give 8-bromo-1-chloro-isoquinoline (550 mg, 2.22 mmol, 23 yield, 98% purity) as a yellow solid. LCMS [ESI, M+1]: 244.

¹H NMR (400MHz, chloroform-d) δ = 8.35 - 8.27 (m, 1H), 8.09 - 7.99 (m, 1H), 7.80 (br d, *J*=8.4 Hz, 1H), 7.64 - 7.58 (m, 1H), 7.53 - 7.44 (m, 1H).

Compound **17-1:** A mixture of benzyl (2*S*)-2-(cyanomethyl)-4-[2-[[(2*S*)-1-methylpyrrolidin-2-yl]methoxy]-6,7,8,9-tetrahyciro-5*H*-pyrimido[4,5-*c*]azepin-4-yl]piperazine-1-carboxylate (400 mg, 770 µmol, 1.00 eq), 8-bromo-1-chloro-isoquinoline (224 mg, 924 µmol, 1.2 eq), KF (89.4 mg, 1.54 mmol, 36.1 µL, 2.00 eq) in DMSO (4.00 mL) was stirred at 100 °C for 12 hours. The mixture was diluted with ethyl acetate (10.0 mL), washed with brine (3 × 10.0 mL), the organic layer was dried over Na₂SO₄, filtered and concentrated under vacuum. The residue was purified by reverse phase flash [water (FA, 0.1 %)/acetonitrile]. The desired fraction was collected and treated with NaHCO₃ (3.00 g). The mixture was concentrated under vacuum to removed acetonitrile. The mixture was extracted with ethyl acetate (3 × 10.0 mL), the organic layers were washed brine (1 × 10.0 mL), dried over Na₂SO₄, filtered and concentrated under vacuum to give benzyl (2*S*)-4-[8-(8-bromo-1-isoquinolyl)-2- [[(2*S*)-1-methylpyrrolidin-2-yl]methoxy]-5,6,7,9-tetrahydropyrimido[4,5-*c*]azepin-4-yl]-2-(cyanomethyl)piperazine-1-carboxylate (240 mg, 278 µmol, 36% yield, 84% purity) as a yellow solid. LCMS [ESI, M+1]: 725.

¹H NMR (400MHz, chloroform-d) δ = 7.92 - 7.76 (m, 1H), 7.71 (d, *J* = 7.6 Hz, 1H), 7.65 - 7.57 (t, *J* = 8.8 Hz 1H), 7.41 - 7.30 (m, 6H), 7.09 - 6.97 (dt, *J* = 5.2 Hz, *J* = 30.4 Hz, 1H), 5.24 - 5.14 (m, 2H), 4.92 - 4.69 (m, 1H), 4.66 (br s, 1H), 4.58 - 4.49 (m, 1H), 4.40 - 4.30 (m, 1H), 4.08 - 3.45 (m, 5H), 3.41 - 3.03 (m, 4H), 3.00 - 2.82 (m, 1H), 2.81 - 2.50 (m, 5H), 2.48 - 2.43 (d, *J* = 8.4 Hz, 3H), 2.33 - 2.22 (m, 1H), 2.12 - 2.06 (m, 1H), 2.03 - 1.93 (m, 2H), 1.89 - 1.75 (m, 3H).

Compound **17-2:** A mixture of benzyl (2*S*)-4-[8-(8-bromo-1-isoquinolyl)-2-[[(2*S*)-1-methylpyrrolidin-2-yl]methoxy]-5,6,7,9-tetrahydropyrimido[4,5-c]azepin-4-yl]-2-(cyanomethyl)piperazine-1-carboxylate (0.10 g, 138 µmol, 1.00 eq), methylboronic acid (165 mg, 2.76 mmol, 11.6 µL, 20.0 eq), Pd(PPh₃)₄ (15.9 mg, 13.8 µmol, 0.10 eq) and K₃PO₄ (87.8 mg, 413 µmol, 3.00 eq) in DMF (3.00 mL) was stirred at 110 °C for 10 h under N₂. The mixture was diluted with ethyl acetate (5.00 mL), washed with brine (3 × 3.00 mL). The combined organic layer dried over Na₂SO₄, filtered and concentrated under vacuum. The residue was purified by reverse phase flash [water (FA, 0.1 %)/acetonitrile]. The desired fraction was collected and solid NaHCO₃ (1.00 g) was added. The mixture was concentrated under vacuum to remove acetonitrile. The residue was extracted with ethyl acetate (3 ×5.00 mL), the organic layers were washed brine (1 × 5.00 mL), dried over Na₂SO₄, filtered and concentrated under vacuum to give benzyl (2*S*)-2-(cyanomethyl)-4-[8-(8-methyl-1-isoquinolyl)-2-[[(2*S*)-1-methylpyrrolidin-2-yl]methoxy]-5,6,7,9-tetrahydropyrimido[4,5-c]azepin-4-yl]piperazine-1-carboxylate (60.0 mg, 64.5 µmol, 47% yield, 71% purity) as a yellow oil. LCMS [ESI, M+1]: 661.

Compound **17-3:** A mixture of benzyl (2*S*)-2-(cyanomethyl)-4-[8-(8-methyl-1-isoquinolyl)-2-[[(2*S*)-1-methylpyrrolidin-2-yl]methoxy]-5,6,7,9-tetrahydropyrimido[4,5-c]azepin-4-yl]piperazine-1-carboxylate (0.05 g, 75.7 µmol, 1.0 eq) and Pd/C (5.0 mg, 10% purity) in NH₃•MeOH (1.0 mL, 20% purity) and methanol (1.0 mL) was stirred at 25 °C for 1 hour under H₂ at 15 psi. The mixture was filtered and concentrated under vacuum. The residue was purified by prep-HPLC (column: Waters Xbridge 150*25 5µ; mobile phase: [water (0.05% ammonium hydroxide v/v) - ACN]; B%: 40% - 64%, 10 min). The desired fractions were collected. The mixture was concentrated under vacuum to remove acetonitrile. The residue was lyophilized to give 2-[(2*S*)-4-[8-(8-methyl-1-isoquinolyl)-2-[[(2*S*)-1-methylpyrrolidin-2-yl]methoxy]-5,6,7,9-tetrahydropyrimido[4,5-c]azepin-4-yl]piperazin-2-yl]acetonitrile (18.7 mg, 35.5 µmol, 47% yield, 100% purity) as a white solid. LCMS [ESI, M+1]: 527.

¹H NMR (400MHz, CHLOROFORM-d) δ = 7.92 (dd, *J*=5.6, 6.4 Hz, 1H), 7.56 - 7.51 (d, *J*=8.0 Hz, 1H), 7.44 (t, *J*=8.0 Hz, 1H), 7.25 (m, 1H), 7.16 (t, *J*=*5.6* Hz, 1H), 4.60 - 4.43 (m, 2H), 4.37 (m, 1H), 4.12 (dd, *J*=6.8, 10.4 Hz, 1H), 3.72 - 3.62 (m, 2H), 3.62 - 3.53 (m, 1H), 3.49 - 3.37 (m, 1H), 3.32 - 3.19 (m, 1H), 3.13 - 2.76 (m, 6H), 2.74 (d, *J*=2.0 Hz, 3H), 2.72 - 2.58 (m, 2H), 2.51 (d, *J*=6.4 Hz, 2H), 2.45 (d, *J*=1.6 Hz, 3H), 2.43 - 2.32 (m, 1H), 2.31 - 2.22 (m, 1H), 2.10 - 1.90 (m, 5H).

**Example 17:** To a solution of 2-[(2*S*)-4-[8-(8-methyl-1-isoquinolyl)-2-[[(2*S*)-1-methylpyrrolidin-2-yl]methoxy]-5,6,7,9-tetrahydropyrimido[4,5-c]azepin-4-yl]piperazin-2-yl]acetonitrile (50.0 mg, 94.9 µmol, 1.0 eq) and TEA (38.4 mg, 380 µmol, 52.9 µL, 4.0 eq) in dichloromethane (1.0 mL) was added prop-2-enoyl chloride (8.59 mg, 94.9 µmol, 7.74 µL, 1.0 eq) at -40 °C. After stirring at -40 °C for 0.5 h, the mixture was diluted with water (3.00 mL) dichloromethane (3.00 mL), the mixture was separated. The organic layer was dried over Na₂SO₄, filtered and concentrated under vacuum. The residue was purified by prep-HPLC (column: Waters Xbridge 150*25 5µ; mobile phase: [water (0.05% ammonium hydroxide v/v) - ACN]; B%: 40% - 67%, 10min). The desired fractions were collected and concentrated under vacuum to remove acetonitrile. The residue was lyophilized to give 2-[(2*S*)-4-[8-(8-methyl-1-isoquinolyl)-2-[[(2*S*)-1-methylpyrrolidin-2-yl]methoxy]-5,6,7,9-tetrahydropyrimido[4,5-*c*]azepin-4-yl]-1-prop-2-enoyl-piperazin-2-yl]acetonitrile (15.3 mg, 25.5 µmol, 27% yield, 97% purity) as white solid. LCMS [ESI, M+1]: 581.

¹H NMR (400MHz, CHLOROFORM-d) δ = 7.96 (dd, *J*=*5.2* Hz, *J*=48.4 Hz, 1H), 7.57 - 7.51 (t, *J*=4.0 Hz, 1H), 7.45 (t, *J*=7.6 Hz, 1H), 7.28 - 7.25 (m, 1H), 7.16 (dd, *J*=*5.6, J*=31.2 Hz, 1H), 6.57 (m, 1H), 6.42 - 6.33 (m, 1H), 5.81 (m, 1H), 5.09 (br s, 1H), 4.71 - 4.54 (m, 1H), 4.51 - 4.48 (m, 1H), 4.36 (m, 1H), 4.14 (m, 1H), 3.86 (br s, 1H), 3.78 - 3.50 (m, 5H), 3.21 (m, 1H), 3.09 (m, 211), 2.92 - 2.81 (m, 2H), 2.79 - 2.71 (m, 4H), 2.69 - 2.51 (m, 2H), 2.46 (d, *J*=5.6 Hz, 3H), 2.34 - 2.21 (m, 2H), 2.15 - 1.92 (m, 3H), 1.90 - 1.82 (m, 2H).

### EXAMPLE 18

### 2-[(2S)-4-[8-(2-fluoro-6-methyl-phcnyl)-2-[[(2S)-1-methylpyrrolidin-2-yl]methoxy]-5,6,7,9-tetrahydropyrimido[4,5-c]azepin-4-yl]-1-prop-2-enoyl-piperazin-2-yl]acetonitrile

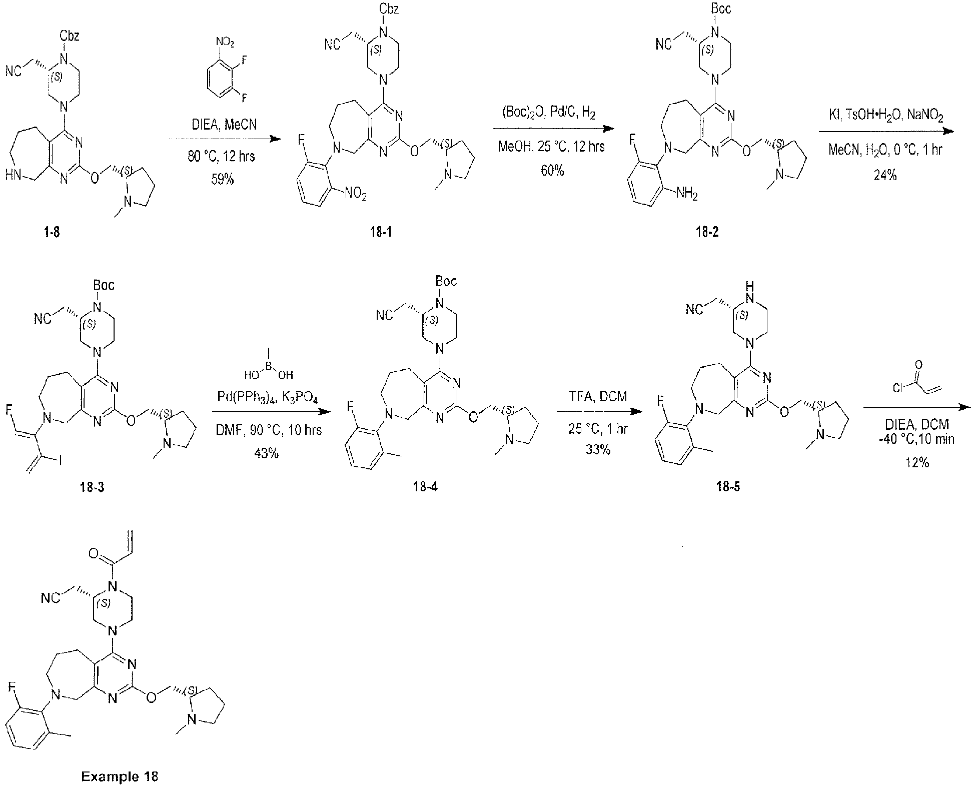

Example 18 was prepared from compound Example 1-8.

Compound 18-1: To a solution of benzyl (2*S*)-2-(cyanomethyl)-4-[2-[[(2*S*)-1-methyl pyrrolidin-2-yl]methoxy]-6,7,8,9-tetrahydro-5*H*-pyrimido[4,5-*c*]azepin-4-yl]piperazine-1-carboxylate (3.0 g, 5.77 mmol, 1.0 *eq)* and 1,2-difluoro-3-nitro-benzene (1.29 g, 8.08 mmol, 1.4 *eq)* in MeCN (60 mL)was added DIEA (1.49 g, 11.6 mmol, 2.01 mL, 2.0 *eq)* and the reaction was stirred for 12 hours at 80 °C. Upon completion, the mixture was concentrated under vacuum, diluted with water (40 mL) and extracted with Ethyl acetate (2 × 60 mL). The organic layers were dried over Na₂SO₄ and concentrated under vacuum. The residue was purified by reversed phase flash [water (0.1% FA)/acetonitrile]. The desired fractions were collected and neutralized with solid NaHCO₃, concentrated under vacuum to remove MeCN and extracted with ethyl acetate (2 × 80 mL). The organic layers were dried over Na₂SO₄ and concentrated under vacuum to give benzyl (2*S*)-2-(cyanomethyl)-4-[8-(2-fluoro-6-nitro- phenyl)-2-[[(2*S*)-1-methylpyrrolidin-2-yl]methoxy]-5,6,7,9-tetrahydropyrimido[4,5-c]azepin-4-yl]piperazine-1-carboxylate (2.3 g, 3.42 mmol, 59% yield, 98% purity) as a yellow solid.

¹H NMR (400MHz, chloroform-d) δ = 7.48 - 7.32 (m, 6H), 7.26 - 7.22 (m, 1H), 7.19 - 7.11 (m, 1H), 5.25 - 5.15 (m, 2H), 4.65 (br s, 1H), 4.40 - 4.30 (m, 2H), 4.28 - 4.18 (m, 1H), 4.18 - 4.05 (m, 2H), 3.79 (br d, J=12.0 Hz, 1H), 3.67 (br d, J=11.6 Hz, 1H), 3.42 (td, J=5.2, 11.2 Hz, 1H), 3.37 - 3.18 (m, 3H), 3.08 (br t, J=7.2 Hz, 1H), 2.99 - 2.79 (m, 2H), 2.79 - 2.69 (m, 3H), 2.68 - 2.60 (m, 1H), 2.46 (s, 3H), 2.27 (dt, *J*=7.2, 9.2 Hz, 1H), 2.10 - 1.92 (m, 3H), 1.89 - 1.63 (m, 3H).

Compound **18-2:** To a solution of benzyl (2*S*)-2-(cyanomethyl)-4-[8-(2-fluoro-6-nitrophenyl)-2-[[(2*S*)-1-methylpyrrolidin-2-yl]methoxy]-5,6,7,9-tetrahydropyrimido[4,5-c]azepin-4-yl]piperazine-1-carboxylate (2.0 g, 3.04 mmol, 1.0 *eq),* (Boc)₂O (1.33 g, 6.07 mmol, 1.40 mL, *2.0 eq)* in MeOH (40 mL) was added Pd/C (1.0 g, 10% purity) under N₂. The suspension was degassed under vacuum and purged with H₂ several times. The mixture was stirred under H₂ (15 psi) at 25 °C for 12 hours. Upon completion, the catalyst was removed by filtering through a plug of celite. The solvent was removed under reduced pressure to give tert-butyl (2*S*)-4-[8-(2-amino-6-fluoro-phenyl)-2-[[(2*S*)-1-methylpyrrolidin-2-yl]methoxy]-5,6,7,9-tetrahydropyrimido[4,5-c]azepin-4-yl]-2-(cyanomethyl)piperazine-1-carboxylate (1.55 g, 1.82 mmol, 60% yield, 70% purity) as a yellow solid which was used directly in the next step without further purification.

Compound **18-3:** *tert*-butyl (2*S*)-4-[8-(2-amino-6-fluoro-phenyl)-2-[[(2*S*)-1-methyl pyrrolidin-2-yl]methoxy]-5,6,7,9-tetrahydropyrimido[4,5-c]azepin-4-yl]-2-(cyanomethyl)piperazine-1-carboxylate (1.45 g, 2.44 mmol, 1.0 *eq)* was dissloved in MeCN (14.5 mL) and H₂O (7.25 mL) followed by the addition of TsOH•H₂O (1.86 g, 9.75 mmol, 4.0 *eq).* A solution of KI (1.21 g, 7.31 mmol, 3.0 *eq)* and NaNO₂ (336 mg, 4.88 mmol, 2.0 *eq)* in H₂O (3 mL) was added to the reaction mixture of slowly at 0 °C. The mixture was stirred at 0 °C for 1 hour. Upon completion, the mixture was concentrated under vacuum to remove acetonitrile, added water (10 mL) and extracted with Ethyl acetate (2 × 30 mL). Combined organic layers were dried over Na₂SO₄, filtered and concentrated under vacuum. The residue was purified by silica gel chromatography (Ethyl acetate/Methanol 50/1 to 5/1) followed by prep-HPLC (column: Phenomenex luna C18 250^{∗}50mm^{∗}10 um; mobile phase: [water (0.225%FA)-ACN]; B%: 30%-60%, 23MIN; 30%min). The desired fractions were collected and neutralized with solid NaHCO₃, concentrated under vacuum to remove MeCN and extracted with ethyl acetate (2 × 50 mL). The organic layers were dried over Na₂SO₄ and concentrated under vacuum to give *tert*-butyl(2*S*)-2-(cyanomethyl)-4-[8-(2-fluoro-6-iodo-phenyl)-2-[[(2*S*)-1-methyl pyrrolidin-2-yl]methoxy]-5,6,7,9-tetrahydropyrimido[4,5-c]azepin-4-yl]piperazine-1-carboxylate (420 mg, 587 µmol, 24% yield, 98.6% purity) as a yellow solid.

Compound **18-4:** A mixture of tert-butyl (2*S*)-2-(cyanomethyl)-4-[8-(2-fluoro-6-iodophenyl)-2-[[(2*S*)-1-methylpyrrolidin-2-yl]methoxy]-5,6,7,9-tetrahydropyrimido[4,5-*c*]azepin-4-yl]piperazine-1-carboxylate (320 mg, 454 µmol, 1.0 *eq),* methylboronic acid (543 mg, 9.07 mmol, 20.0 *eq*), Pd(PPh₃)₄ (52.4 mg, 45.4 µmol, 0.1 *eq)* and K₃PO₄ (289 mg, 1.36 mmol, 3.0 *eq)* in DMF (6 mL) was stirred at 90 °C for 10 hours under N₂. Upon completion, the mixture was diluted with ethyl acetate (10 mL) and extracted with water (3 × 5 mL). The organic layers were dried over Na₂SO₄ and concentrated under vacuum. The residue was purified by reversed phase flash [water (0.1% NH₃•H₂O)/acetonitrile]. The desired fractions were collected and concentrated under vacuum. The residue was further purified by prep-HPLC (column: Xtimate C18 150*25mm*5um; mobile phase: [water (0.05% ammonium hydroxide v/v)-ACN]; B%:68%-98%). The desired fractions were collected and concentrated under vacuum to remove MeCN, extracted with ethyl acetate (2 × 10 mL). The organic layers were dried over Na₂SO₄ and concentrated under vacuum to give tert-butyl (2*S*)-2-(cyanomethyl)-4- [8-(2-fluoro-6-methylphenyl)-2-[[(2*S*)-1-methylpyrrolidin-2-yl]methoxy]-5,6,7,9-tetrahydropyrimido[4,5-*c*]azepin-4-yl]piperazine-1-carboxylate (115 mg, 194 µmol, 43% yield, 100% purity) as a yellow oil.

¹H NMR (400MHz, chloroform-d) δ = 7.03 - 6.95 (m, 1H), 6.94 - 6.85 (m, 2H), 4.62 (br s, 1H), 4.38 - 4.23 (m, 2H), 4.22 - 3.95 (m, 3H), 3.77 (br d, *J*=13.6 Hz, 1H), 3.62 (br d, J=12.8 Hz, 1H), 3.45 - 3.12 (m, 4H), 3.08 (br t, J=7.2 Hz, 1H), 2.98 - 2.71 (m, 5H), 2.64 (td, .7=6.4, 13.2 Hz, 1H), 2.45 (s, 3H), 2.33 - 2.24 (m, 1H), 2.23 (s, 3H), 2.04 - 1.90 (m, 3H), 1.88 - 1.76 (m, 3H), 1.52 (s, 9H).

Compound **18-5:** To a solution of tert-butyl (2*S*)-2-(cyanomethyl)-4-[8-(2-fluoro-6-methyl-phenyl)-2-[[(2*S*)-1-methylpyrrolidin-2-yl]methoxy]-5,6,7,9-tetrahydropyrimido[4,5-*c*]azepin-4-yl]piperazine-1-carboxylate (30.0 mg, 50.5 µmol, 1.0 *eq)* in dichloromethane (0.03 mL) was added TFA (86.4 mg, 758 µmol, 56.1 µE, 15.0 *eq*). The mixture was stirred at 25 °C for 1 hour. Upon completion, the mixture was diluted with dichloromethane (1 mL) and neutralized with saturated NaHCO₃ solution at 0 °C. The separated aqueous layer was extracted with dichloromethane (3 × 2 mL). Combined organic layers were dried over Na₂SO₄ and concentrated under vacuum. The residue was purified by prep-HPLC (column: Xtimate C18 150^{∗}25mm^{∗}5µm; mobile phase: [water (0.05% ammonium hydroxide v/v)-ACN]; B%: 49%-79%, 1min). The desired fractions were collected and lyophilized to give 2-[(2*S*)-4-[8-(2-fluoro-6-methyl-phenyl)-2-[[(2*S*)-1-methylpyrrolidin-2-yl]methoxy]-5,6,7,9-tetrahydropyrimido[4,5-c]azepin-4-yl]piperazin-2-yl]acetonitrile (8.18 mg, 16.5 µmol, 33% yield, 99.8% purity) as a yellow solid. LCMS [ESI, M+1]: 494.

¹H NMR (400MHz, chloroform-d) δ = 7.02 - 6.95 (m, 1H), 6.94 - 6.84 (m, 2H), 4.46 - 4.03 (m, 4H), 3.74 (br d, *J*=12.4 Hz, 1H), 3.56 (br d, J=9.6 Hz, 1H), 3.28 (br s, 3H), 3.15 - 2.97 (m, 4H), 2.91 - 2.73 (m, 3H), 2.69 - 2.59 (m, 1H), 2.54 (d, *J*=6.8 Hz, 2H), 2.45 (s, 3H), 2.30 - 2.24 (m, 1H), 2.23 (s, 3H), 2.09 - 2.00 (m, 1H), 1.99 - 1.91 (m, 2H), 1.78 - 1.67 (m, 3H).

SFC condition: Column: Cellucoat 50 × 4.6mm I.D., 3µm, Mobile phase: Phase A for CO₂, and Phase B for MeOH (0.05% DEA); Gradient elution: MeOH (0.05% DEA) in CO₂ from 5% to 40%, Flow rate: 3mL/min; Wavelength: 220nm, Column Temp: 35C; Back Pressure: 100Bar.

**Example 18:** To a solution of 2-[(2*S*)-4-[8-(2-fluoro-6-methyl-phenyl)-2-[[(2*S*)-1-methylpyrrolidin-2-yl]methoxy]-5,6,7,9-tetrahydropyrimido[4,5-c]azepin-4-yl]piperazin-2-yl]acetonitrile (66 mg, 109 µmol, 1.0 *eq,* TFA) and DIEA (562 mg, 4.34 mmol, 757 uL, 40.0 *eq)* in dichloromethane (1.5 mL) was added prop-2-enoyl chloride (19.7 mg, 217 µmol, 17.7 uL, 2.0 *eq*) dropwise at -40 °C. The mixture was stirred at -40 °C for 10 minutes. Upon completion, the mixture was quenched with saturated aqueous sodium bicarbonate (0.5 mL) and layers were separated. The aqueous phase was extracted with dichloromethane (5 mL). Combined organic layers were dried over Na₂SO₄, filtered and concentrated under vacuum. The residue was purified by prep-HPLC (column: Waters Xbridge 150*25 5u; mobile phase: [water (0.05% ammonium hydroxide v/v)-ACN]; B%: 55%-74%, 10min). The desired fractions were collected and lyophilized to give 2-[(2*S*)-4-[8-(2-fluoro-6-methyl-phenyl)-2-[[(2*S*)-1- methylpyrrolidin-2-yl]methoxy]-5,6,7,9-tetrahydropyrimido[4,5-*c*]azepin-4-yl]-1-prop-2-enoyl-piperazin-2-yl]acetonitrile (7.15 mg, 13.0 µmol, 12% yield, 99.5% purity) as a white solid. LCMS [ESI, M+1]: 548.

¹H NMR (400MHz, chloroform-d) δ = 7.03 - 6.96 (m, 1H), 6.95 - 6.84 (m, 2H), 6.59 (br s, 1H), 6.39 (dd, *J*=1.6, 16.8 Hz, 1H), 5.83 (br d, *J*=10.4 Hz, 1H), 5.11 (br s, 1H), 4.46 - 4.08 (m, 4H), 4.08 - 3.79 (m, 2H), 3.79 - 3.44 (m, 2H), 3.43 - 3.14 (m, 3H), 3.09 (br t, *J*=7.6 Hz, 1H), 3.04 - 2.72 (m, 5H), 2.70 - 2.60 (m, 1H), 2.46 (s, 3H), 2.34 - 2.25 (m, 1H), 2.23 (s, 3H), 2.09 - 1.94 (m, 3H), 1.89 - 1.73 (m, 3H).

SFC condition: Column: Cellucoat 50×4.6mm I.D., 3um, Mobile phase: Phase A for CO₂, and Phase B for MeOH (0.05%DEA); Gradient elution: MeOH (0.05% DEA) in CO₂ from 5% to 40%, Flow rate: 3mL/min; Wavelength: 220nm, Column Temp: 35C; Back Pressure: 100Bar.

### EXAMPLE 19

### 2-[(2S)-4-[8-(5-methyl-1H-indazol-4-yl)-2-[[(2S)-1-methylpyrrolidin-2-yl]methoxy]-5,6,7,9-tetrahydropyrimido[4,5-c]azepin-4-yl]-1-prop-2-enoyl-piperazin-2-yl]acetonitrile

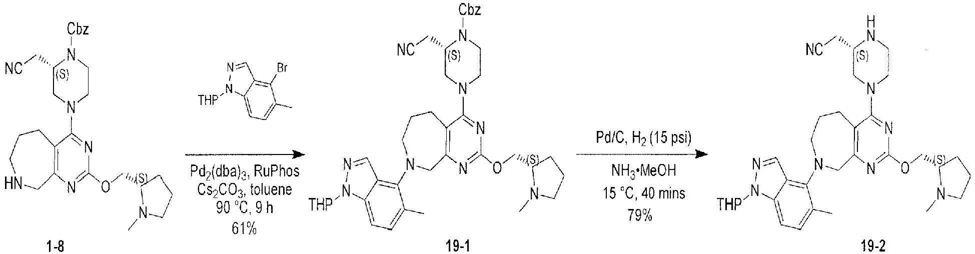

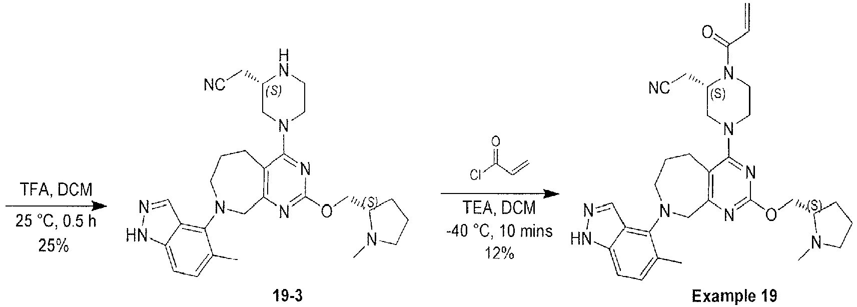

Compound **19-1:** To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-c]azepin-4-yl]piperazine-1-carboxylate (800 mg, 1.54 mmol, 1.0 *eq*), 4-bromo-5-methyl-1- tetrahydropyran-2-yl-indazole (545 mg, 1.85 mmol, 6.31 µL, 1.2 *eq),* Cs₂CO₃ (1.50 g, 4.62 mmol, 3.0 *eq)* an RuPhos (287 mg, 616 µmol, 0.4 *eq)* in toluene (20 mL) was added Pd₂(dba)₃ (282 mg, 308 µmol, 0.2 *eq)* under N₂. The suspension was degassed under vacuum and purged with N₂ several times. The mixture was stirred under N₂ at 90 °C for 8 hours. The reaction mixture was filtered and the filtrate was concentrated under vacuum. The residue was purified by reverse-phase flash [water (0.1% FA)/acetonitrile]. The desired fractions were collected and NaHCO₃ added to pH ~7, concentrated under vacuum to remove MeCN and extracted with ethyl acetate (2 × 40 mL). The organic layers were dried over Na₂SO₄ and concentrated under vacuum to give benzyl (2*S*)-2-(cyanomethyl)-4-[2-[[(2*S*)-1-methylpyrrolidin-2- yl]methoxy]-8-(5-methyl-1-tetrahydropyran-2-yl-indazol-4-yl)-5,6,7,9-tetrahydropyrimido[4,5-c]azepin-4-yl]piperazine-1-carboxylate (700 mg, 935 µmol, 61% yield, 98% purity) as a yellow solid. LCMS [ESI, M+1]: 734.

¹H NMR (400MHz, chloroform-d) δ = 8.04 (d, J=2.4 Hz, 1H), 7.43 - 7.33 (m, 5H), 7.26 - 7.16 (m, 2H), 5.67 (dd, J=2.4, 9.6 Hz, 1H), 5.26 - 5.17 (m, 2H), 4.70 (br s, 1H), 4.54 - 4.38 (m, 2H), 4.35 (dd, J=4.8, 10.8 Hz, 1H), 4.20 - 4.08 (m, 2H), 4.03 (br d, *J*=10.0 Hz, 1H), 3.82 (br d, 7=12.4 Hz, 1H), 3.77 - 3.70 (m, 1H), 3.66 (br d, J=13.2 Hz, 1H), 3.61 - 3.53 (m, 1H), 3.50 - 3.40 (m, 1H), 3.33 (br s, 1H), 3.23 (br d, *J*=11.6 Hz, 1H), 3.07 (br t, J=7.6 Hz, 1H), 3.01 - 2.82 (m, 4H), 2.81 - 2.73 (m, 111), 2.68 - 2.51 (m, 2H), 2.45 (s, 3H), 2.26 (d, 7=1.2 Hz, 4H), 2.19 - 2.13 (m, 1H), 2.08 (br s, 1H), 2.04 - 1.96 (m, 2H), 1.86 - 1.78 (m, 2H), 1.73 - 1.60 (m, 3H).

Compound **19-2:** To the solution of benzyl (2*S*)-2-(cyanomethyl)-4-[2-[[(2*S*)-1-methylpyrrolidin-2-yl]methoxy]-8-(5-methyl-1-tetrahydropyran-2-yl-indazol-4-yl)-5,6,7,9-tetrahydropyrimido[4,5-*c*]azepin-4-yl]piperazine-1-carboxylate (670 mg, 913 µmol, 1.0 *eq)* and NH₃•MeOH (5 mL, 30% purity) in MeOH (10 mL) was added Pd/C (300 mg, 10% purity) under N₂. The suspension was degassed under vacuum and purged with H₂ several times. The mixture was stirred under H₂ (15 psi) at 25 °C for 40 mins. The reaction mixture was filtered and the filtrate was concentrated under vacuum to give 2-[(2*S*)-4-[2-[[(2*S*)-1-methylpyrrolidin-2-yl]methoxy]-8-(5-methyl-1- tetrahydropyran-2-yl-indazol-4-yl)-5,6,7,9-tetrahydropyrimido[4,5-c]azepin-4-yl]piperazin-2-yl]acetonitrile (480 mg, 720 µmol, 79% yield, 90% purity) as a white solid which was used for next step without further purification. LCMS [ESI, M+1]: 600.

Compound **19-3:** To a solution of 2-[(2*S*)-4-[2-[[(2*S*)-1-methylpyrrolidin-2- yl]methoxy]-8-(5-methyl-1-tetrahydropyran-2-yl-indazol-4-yl)-5,6,7,9-tetrahydropyrimido[4,5-c]azepin-4-yl]piperazin-2-yl]acetonitrile (40 mg, 66.7 µmol, 1.0 *eq)* in DCM (0.05 mL) was added TFA (308 mg, 2.70 mmol, 0.2 mL, 40 *eq*), the mixture was stirred at 25 °C for 0.5 hour. The reaction mixture was concentrated under vacuum. The residue was diluted with DCM (4 mL) and saturated aqueous NaHCO₃ was added to pH = 7 ~ 8. Then the mixture was extracted with DCM (2 × 5 mL). The combined organic layers were dried over anhydrous Na₂SO₄, filtered and concentrated under vacuum. The residue was purified by prep-HPLC (column: Waters Xbridge 150*25 5µ;mobile phase: [water (0.05% ammonium hydroxide v/v)-ACN];B%: 25% - 53%,10min). The desired fractions were collected and lyophilized to give 2-[(2S)-4-[8-(5-methyl-1*H*-indazol-4-yl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (8.46 mg, 16.4 µmol, 25% yield, 100% purity) as a white solid. LCMS [ESI, M+11: 516.

¹H NMR (400MHz, chloroform-d) δ = 8.11 (d, *J*=0.8 Hz, 1H), 7.21 - 7.11 (m, 2H), 4.93 - 4.56 (m, 1H), 4.46 (s, 2H), 4.38 (dd, J=4.8, 10.4 Hz, 1H), 4.14 (dd, J=6.8, 10.4 Hz, 1H), 3.78 (br d, .7=12.0 Hz, 1H), 3.65 - 3.47 (m, 311), 3.34 - 3.26 (m, 1H), 3.17 - 2.99 (m, 4H), 2.92 - 2.81 (m, 3H), 2.66 (td, J=6.8, 13.2 Hz, 1H), 2.54 (d, *J*=6.4 Hz, 2H), 2.46 (s, 3H), 2.31 - 2.24 (m, 4H), 2.10 - 1.99 (m, 3H), 1.89 - 1.81 (m, 2H).

**Example 19:** To the solution of 2-[(2*S*)-4-[8-(5-methyl-1*H*-indazol-4-yl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (100 mg, 194 µmol, 1.0 *eq)* and TEA (58.9 mg, 582 µmol, 81.0 uL, 3.0 *eq)* in DCM (2 mL) was added prop-2-enoyl chloride (17.6 mg, 194 µmol, 15.8 µL, 1.0 *eq)* at -40 °C, the mixture was stirred at -40 °C for 10 mins. The reaction mixture was quenched by water (4 mL). The mixture was extracted with DCM (3 × 5 mL). The combined organic layers were dried over anhydrous Na₂SO₄, filtered and concentrated under vacuum. The residue was purified by reverse-phase flash [water (0.1% FA)/acetonitrile]. Then the residue was purified by prep-HPLC (column: Xtimate C18 150^{∗}25mm^{∗}5µm;mobile phase: [water (0.05% ammonium hydroxide v/v)-ACN];B%: 40%-70%,1min). The desired fractions were collected and lyophilized to give 2-[(2*S*)-4-[8-(5-methyl-1*H*-indazol-4-yl)-2-[[(2*S*)-1-methylpyrroildin-2-yl]methoxy]-5,6,7,9-tetrahydropyrimido[4,5-*c*]azepin-4-yl]-1-prop-2-enoyl-piperazin-2-yl]acetonitrile (16.8 mg, 29.1 µmol, 12% yield, 98.6% purity) as a white solid. LCMS [ESI, M+1]: 570.

¹H NMR (400 MHz, chloroform-d) δ = 10.34 (br s, 1H), 8.11 (s, 1H), 7.21 - 7.11 (m, 2H), 6.67 - 6.52 (m, 1H), 6.39 (dd, J=1.6, 16.8 Hz, 1H), 5.82 (br d, *J*=10.4 Hz, 1H), 5.11 (br s, 1H), 4.55 - 4.43 (m, 2H), 4.36 (dd, *J*=4.8, 10.4 Hz, 1H), 4.14 (dd, J=6.8, 10.8 Hz, 1H), 4.07 - 3.82 (m, 2H), 3.75 (br d, J=11.6 Hz, 1H), 3.66 - 3.41 (m, 3H), 3.26 (dd, J=3.9, 13.6 Hz, 1H), 3.08 (br t, J=8.0 Hz, 1H), 3.04 - 2.88 (m, 4H), 2.80 (br s, 1H), 2.69 - 2.61 (m, 1H), 2.46 (s, 3H), 2.33 - 2.22 (m, 4H), 2.17 - 1.98 (m, 3H), 1.90 - 1.78 (m, 3H).

### EXAMPLE 20

### 2-[(2S)-1-(2-fluoroprop-2-enoyl)-4-[8-(5-methyl-1H-indazol-4-yl)-2-[[(2S)-1-methylpyrrolidin-2-yl]methoxy]-5,6,7,9-tetrahydropyrimido[4,5-c]azepin-4-yl]piperazin-2-yl]acetonitrile

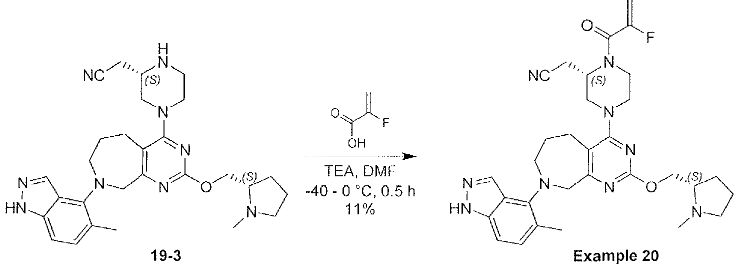

**Example 20:** To a solution of 2-[(2*S*)-4-[8-(5-methyl-1*H*-indazol-4-yl)-2- [[(2*S*)-1-methylpyrrolidin-2-yl]methoxy]-5,6,7,9-tetrahydropyrimido[4,5-*c*]azepin-4-yl]piperazin-2-yl]acetonitrile (100 mg, 194 pmol, 1.0 *eq),* 2-fluoroprop-2-enoic acid (26.2 mg, 291 µmol, 3.16 µL, 1.5 *eq)* and TEA (118 mg, 1.16 mmol, 162 µL, 6.0 *eq)* in DMF (2 mL) was added T3P (617 mg, 970 µmol, 577 µL, 50% purity, 5.0 *eq)* at -40 °C, the mixture was stirred at -40 °C for 10 mins. The mixture was stirred at 0 °C for 20 mins. Water (20 mL) was added into the mixture. The mixture was diluted with DCM (10 mL) and extracted with DCM (3 × 10 mL). The combined organic layers were dried over anhydrous Na₂SO₄, filtered and concentrated under vacuum. The residue was purified by prep-HPLC (column: Xtimate C18 150^{∗}25mm^{∗}5µm;mobile phase: [water (0.05% ammonium hydroxide v/v)-ACN];B%: 44%-74%,1min). The desired fractions were collected and lyophilized to give 2-[(2*S*)-1-(2-fluoroprop-2- enoyl)-4-[8-(5-methyl-1*H*-indazol-4-y1)-2-[[(2*S*)-1-methylpyrrolidin-2-yl]methoxy]-5,6,7,9-tetrahydropyrimido[4,5-*c*]azepin-4-yl]piperazin-2-yl]acetonitrile (13.2 mg, 22.2 µmol, 11% yield, 98.5% purity) as an off-white solid. LCMS [ESI, M+1]: 588.

¹H NMR (400MHz, chloroform-d) δ = 10.10 (br s, 1H), 8.11 (s, 1H), 7.21 - 7.14 (m, 2H), 5.52 - 5.32 (m, 1H), 5.25 (dd, *J*=3.6, 16.8 Hz, 1H), 4.88 (br s, 1H), 4.56 - 4.42 (m, 2H), 4.35 (dd, J=4.8, 10.4 Hz, 1H), 4.13 (dd, *J*=6.8, 10.4 Hz, 1H), 3.88 (br d, *J*=13.6 Hz, 1H), 3.74 (br d, *J*=13.6 Hz, 1H), 3.64 - 3.42 (m, 3H), 3.29 (dd, *J*=3.6, 13.6 Hz, 1H), 3.11 - 2.78 (m, 7H), 2.70 - 2.59 (m, 1H), 2.45 (s, 3H), 2.31 - 2.23 (m, 4H), 2.15 - 1.99 (m, 3H), 1.84 - 1.69 (m, 3H).

### EXAMPLE 21

### 2-((S)-1-acryloyl-4-(2-(((S)-1-methylpyrrolidin-2-yl)methoxy)-8-(8-(trifluoromethyl)naphthalen-1-yl)-6,7,8,9-tetrahydro-5H-pyrimido[4,5-c]azepin-4-yl)piperazin-2-yl)acetonitrile

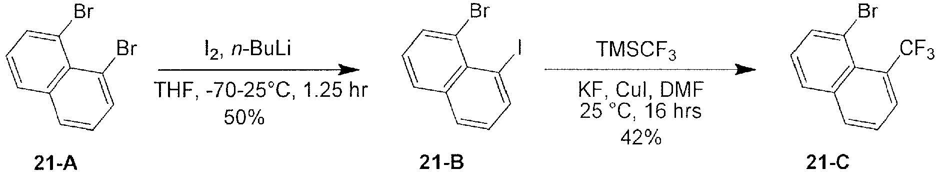

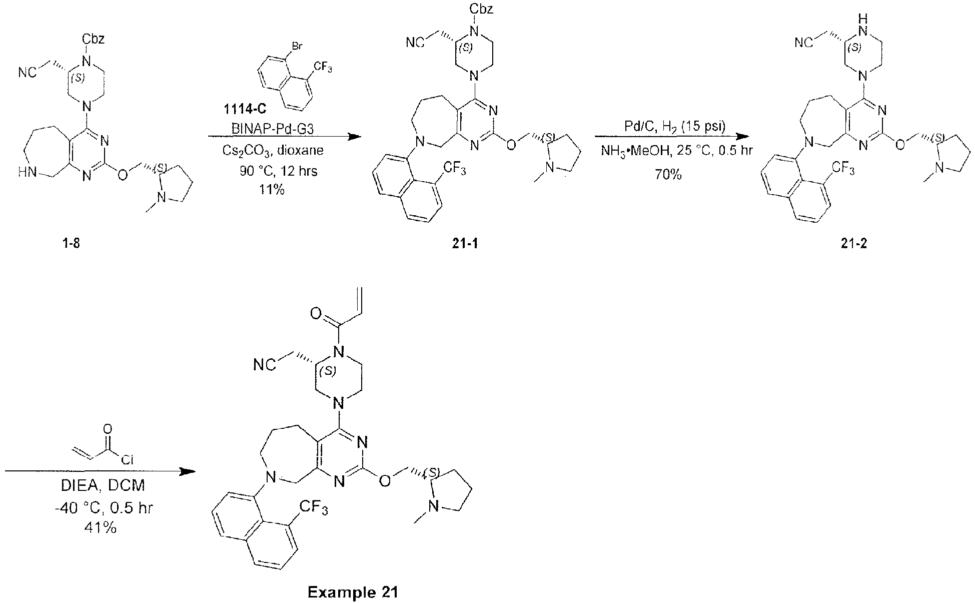

Compound **21-B:** 1,8-dibromonaphthalene (5 g, 17.5 mmol, 1.0 *eq)* was dissolved in THF (40 mL). The mixture was cooled down to - 70 °C and *n*-BuLi (2.5 M in hexane, 6.99 mL, 1.0 *eq)* was added dropwise. After 15 minutes, I₂ (4.44 g, 17.5 mmol, 3.52 mL, 1.0 *eq*) dissolved in THF (40 mL) was added. The mixture was allowed to reach 25 °C and stirred for 1 hour. The reaction was quenched with 30 mL of 1 M sodium thiosulfate solution in water. The reaction mixture was diluted with ethyl acetate (50 mL) and washed with water (20 mL). The organic layer was washed with brine (20 mL), dried over Na₂SO₄, filtered and concentrated under reduced pressure to give a residue. The residue was purified by column chromatography (SiO₂, Petroleum ether/Ethyl acetate=100/1 to 10:1). 1-bromo-8-iodo-naphthalene (3 g, 8.74 mmol, 50% yield, 97% purity) was obtained as a yellow solid.

¹H NMR (400MHz, chloroform-d) δ = 8.43 (dd, *J* = 1.2, 7.6 Hz, 1H), 7.96 (dd, *J=* 1.2, 7.6 Hz, 1H), 7.89 - 7.77 (m, 2H), 7.28 (t, J= 8.0 Hz, 1H), 7.08 (t, J= 7.6 Hz, 1H).

Compound **21-C**: A mixture of CuI (1.57 g, 8.26 mmol, 1.1 *eq)* and KF (479 mg, 8.26 mmol, 193 µL, 1.1 *eq)* was thoroughly mixed and heated to 150 °C under vacuum by using oil pump with heat gun with gentle shaking until an homogemeous greenish color was obtained. To the mixture was added DMSO (50 mL), trimethyl(trifluoromethyl)silane (3.20 g, 22.5 mmol, 3.0 *eq)* and 1-bromo-8-iodo-naphthalene (2.5 g, 7.51 mmol, 1.0 *eq)* was added and the slurry was heated to 25 °C for 16 h. The reaction mixture was diluted with ethyl acetate (50 mL) and washed with water (3 × 30 mL). The combined organic layers were washed with brine (20 mL), dried over Na₂SO₄, filtered and concentrated under reduced pressure to give a residue. The residue was purified by prep-HPLC (column: Phenomenex Synergi C18 150^{∗}25^{∗}10µm; mobile phase: [water (0.225% FA) - ACN]; B%: 66%-86%, 10min). 1-bromo-8-(trifluoromethyl)naphthalene (900 mg, 3.14 mmol, 42% yield, 96% purity) was obtained as a white solid.

¹H NMR (400MHz, chloroform-d) δ = 8.12 (d, J= 7.6 Hz, 1H), 8.10 - 8.02 (m, 2H), 7.90 (dd, *J* = 1.2, 8.0 Hz, 1H), 7.54 (t, *J=* 7.6 Hz, 1H), 7.37 (t, J= 7.6 Hz, 1H).

Compound **21-1:** A mixture of benzyl (2*S*)-2-(cyanomethyl)-4-[2-[[(2*S* -1-methylpyrrolidin-2-yl]methoxy]-6,7,8,9-tetrahydro-5*H*-pyrimido[4,5-c]azepin-4-yl]piperazine-1-carboxylate (500 mg, 962 µmol, 1.0 *eq),* 1-bromo-8- (trifluoromethyl)naphthalene (529 mg, 1.92 mmol, 2.0 *eq),* Cs₂CO₃ (940 mg, 2.89 mmol, 3.0 *eq),* BINAP-Pd-G3 (95.5 mg, 96.2 µmol, 0.1 *eq)* in dioxane (10 mL) was degassed and purged with N₂ for 3 times, and then the mixture was stirred at 90 °C for 12 hours under N₂ atmosphere. The reaction mixture was diluted with water (20 mL) and extracted with ethyl acetate (3 × 20 mL). The combined organic layers were washed with brine (20 mL), dried over Na₂SO₄, filtered and concentrated under reduced pressure to give a residue. The residue was purified by reverse phase flash [water (0.1% formic acid)/acetonitrile)]. The mixture was adjusted pH ~ 7 with saturated NaHCO₃ aqueous solution and extracted with ethyl acetate (3 × 20 mL). The combined organic layers were washed with brine (20 mL), dried over Na₂SO₄, filtered and concentrated under reduced pressure to give the product. benzyl (2*S*)-2-(cyanomethyl)-4-[2-[[(2*S*)-1-methylpyrrolidin-2-yl]methoxy]-8-[8-(trifluoromethyl)-1-naphthyl]-5,6,7,9-tetrahydropyrimido[4,5-*c*]azepin-4-yl]piperazine-1-carboxylate (80 mg, 105 µmol, 11% yield, 94% purity) was obtained as a yellow solid. LCMS [ESI, M+1]: 714.

Compound **21-2:** To a solution of benzyl (2*S*)-2-(cyanomethyl)-4-[2-[[(2*S*)-1-methylpyrrolidin-2-yl]methoxy]-8-[8-(trifluoromethyl)-1-naphthyl]-5,6,7,9-tetrahydropyrimido[4,5-*c*]azepin-4-yl]piperazine-1-carboxylate (70 mg, 98.1 µmol, 1.0 *eq)* in MeOH (5 mL) was added NH₃/MeOH (2 mL, 20% purity) and Pd/C (20 mg, 10% purity) under N₂ atmosphere. The suspension was degassed under vacuum and purged with H₂ several times. The mixture was stirred under H₂ (15 psi) at 25 °C for 0.5 hour. The reaction mixture was concentrated under vacuum. 2-[(2*S*)-4-[2-[[(2*S*)-1-methylpyrrolidin-2-yl]methoxy]-8-[8-(trifluoromethyl) -1-naphthyl]-5,6,7,9-tetrahydropyrimido[4,5-c]azepin-4-yl]piperazin-2-yl]acetonitrile (40 mg, 69.0 µmol, 70% yield) was obtained as a yellow oil and used next step without purification. LCMS [ESI, M+1]: 580.

**Example 21:** To a solution of 2-[(2*S*)-4-[2-[[(2*S*)-1-methylpyrrolidin-2-yl]methoxy]-8-[8-(trifluoromethyl)-1-naphthyl]-5,6,7,9-tetrahydropyrimido[4,5-*c*]azepin-4-yl]piperazin-2-yl]acetonitrile (40 mg, 69.0 µmol, 1.0 *eq)* and DIEA (44.6 mg, 345 µmol, 60.1 µL, 5.0 *eq)* in dichloromethane (1 mL) was added a solution of prop-2-enoyl chloride (9.37 mg, 103 µmol, 8.44 µL, 1.5 *eq)* in dichloromethane (1 mL) at - 40 °C. The mixture was stirred at - 40 °C for 0.5 hour. The reaction mixture was quenched with water (20 mL) and extracted with dichloromethane (3 × 20 mL). The combined organic layers were washed with brine (10 mL), dried over Na₂SO₄, filtered and concentrated under reduced pressure to give a residue. The residue was purified by column chromatography (SiO₂, Ethyl acetate/Methanol=100/1 to 10:1) and further purification by prep-HPLC (column: Waters Xbridge 150 * 25 5µ; mobile phase: [water (0.05% ammonia hydroxide v/v) - ACN]; B%: 55% - 79%, 10min). The desired fraction was collected and lyophilized. 2-[(2*S*)-4-[2-[[(2*S*)-1-methylpyrrolidin-2-yl]methoxy]-8-[8-(trifluoromethyl)-1-naphthyl]-5,6,7,9-tetrahydropyrimido[4,5-*c*]azepin-4-yl]-1-prop-2-enoyl-piperazin-2-yl]acetonitrile (18 mg, 28.4 µmol, 41% yield, 99.9% purity) was obtained as a white solid. LCMS [ESI, M+1]: 634.

¹H NMR (400MHz, chloroform-d) δ = 8.07 - 7.88 (m, 2H), 7.83 - 7.70 (m, 1H), 7.62 - 7.40 (m, 3H), 6.71 - 6.51 (m, 1H), 6.39 (dd, J= 1.6, 16.8 Hz, 111), 5.83 (d, J= 10.8 Hz, 1H), 5.30 - 4.47 (m, 1H), 4.43 - 4.27 (m, 2H), 4.22 - 3.89 (m, 3H), 3.88 - 3.45 (m, 3H), 3.42 - 3.16 (m, 3H), 3.14 - 2.70 (m, 6H), 2.69 - 2.57 (m, 1H), 2.42 (d, *J* = 5.2 Hz, 3H), 2.32 - 2.19 (m, 1H), 2.09 - 1.90 (m, 3H), 1.88 - 1.75 (m, 3H). 14).

### EXAMPLE 22

### 2-((S)-1-(2-fluoroacryloyl)-4-(2-(((S)-1-methylpyrrolidin-2-yl)methoxy)-8-(naphthalen-1-yl)-6,7,8,9-tetrahydro-5H-pyrimido[4,5-c]azepin-4-yl)piperazin-2-yl)acetonitrile

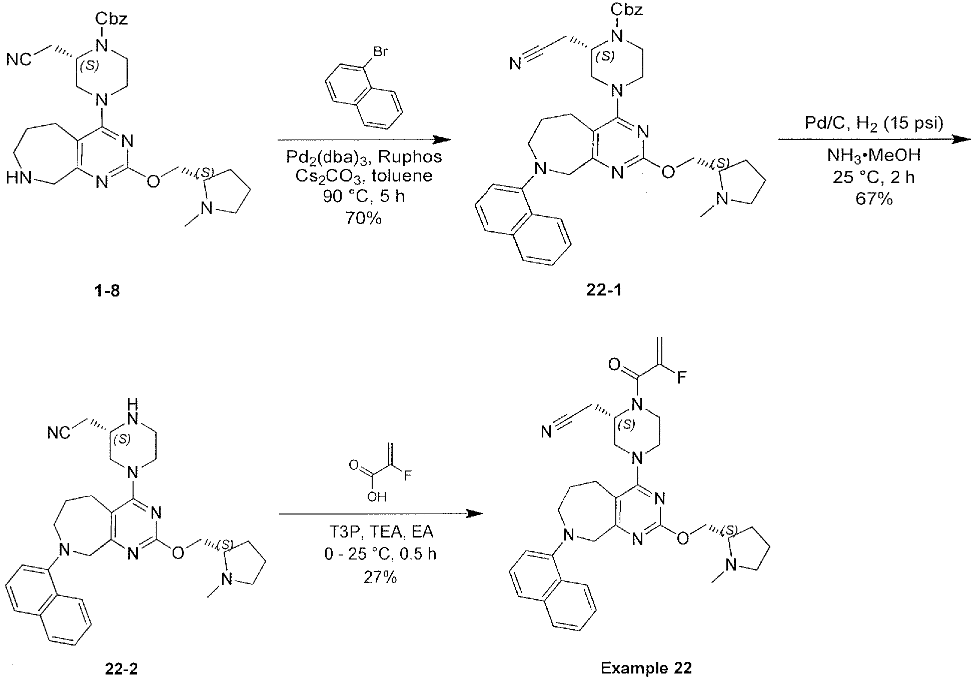

Compound 22-1: To a mixture of benzyl (2*S*)-2-(cyanomethyl)-4-[2-[[(2*S*)-1-methylpyrrolidin-2-yl]methoxy]-6,7,8,9-tetrahydro-5*H*-pyrimido[4,5-*c*]azepin-4-yl]piperazine-1-carboxylate (300 mg, 577 µmol, 1.00 eq) and 1-bromonaphthalene (239 mg, 1.15 mmol, 160 µL, 2.00 eq) in toluene (25.0 mL) was added Pd₂(dba)₃ (52.9 mg, 57.7 µmol, 0.10 eq), RuPhos (53.9 mg, 115 µmol, 0.20 eq), Cs₂CO₃ (564 mg, 1.73 mmol, 3.00 eq) in one portion under N₂. The mixture was degassed and purged with N₂ for 3 times and stirred at 90 °C for 5 hours. The reaction mixture was diluted with water (20.0 mL) and extracted with ethyl acetate (30 mL × 3). The combined organic layers were washed with brine (30 mL × 1), dried over sodium sulfate, filtered and concentrated under reduced pressure to give a residue. The residue was purified by reverse phase flash [water (0.1% FA)/acetonitrile]. The desired fractions were collected and neutralized with saturated NaHCO₃ solution (5.00 mL) and extracted with ethyl acetate (50.0 mL × 2). The separated organic layer was dried over sodium sulfate, filtered and concentrated under vacuum. Compound of benzyl (2*S*)-2- (cyanomethyl)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazine-1-carboxylate (260 mg, 403 µmol, 70% yield) as a yellow solid was obtained.

¹H NMR (400MHz, chloroform-d) δ = 7.95 (d, *J* = 8.4 Hz, 1H), 7.81 (d, J= 8.0 Hz, 1H), 7.54 (d, J= 8.4 Hz, 1H), 7.46 - 7.33 (m, 8H), 7.12 (dd, *J=* 0.4, 7.2 Hz, 1H), 5.29 - 5.14 (m, 2H), 4.70 (br s, 1H), 4.45 - 4.31 (m, 3H), 4.21 - 4.14 (m, 2H), 3.87 (br d, *J* = 13.2 Hz, 1H), 3.70 (br d, *J=* 13.2 Hz, 1H), 3.56 - 3.42 (m, 2H), 3.40 - 3.21 (m, 2H), 3.08 (br t, *J=* 7.2 Hz, 1H), 3.04 - 2.84 (m, 4H), 2.83 - 2.74 (m, 1H), 2.72 - 2.60 (m, 1H), 2.45 (s, 3H), 2.33 - 2.23 (m, 1H), 2.21 - 2.06 (m, 2H), 2.05 - 1.96 (m, 2H), 1.89 - 1.76 (m, 2H).

Compound **22-2:** To a solution of benzyl (2*S*)-2-(cyanomethyl)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazine-1-carboxylate (150 mg, 232 µmol, 1.00 eq) in MeOH (3.00 mL) was added Pd/C (50.0 mg, 10% purity), NH₃•MeOH (1.00 mL, 20% purity) under N₂. The suspension was degassed under vacuum and purged with H₂ several times. The mixture was stirred under H₂ (15 psi) at 25 °C for 2 hours. The reaction mixture was filtered and concentrated under reduced pressure to give a residue. Compound of 2-[(2*S*)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazin-2-yl]acetonitrile (86.0 mg, 155 µmol, 67% yield, 92% purity) as a yellow solid was obtained, which was used in the next step directly without further purification. LCMS [ESI, M+1]: 512.

**Example 22:** To a mixture of 2-[(2*S*)-4-[2-[[(2*S*)-1-methylpyrrolidin-2- yl]methoxy]-8-(1-naphthyl)-5,6,7,9-tetrahydropyrimido[4,5-*c*]azepin-4-yl]piperazin-2-yl]acetonitrile (76.0 mg, 149 µmol, 1.00 eq) in ethyl acetate (2 mL) was added TEA (120 mg, 1.19 mmol, 165 µL, 8.00 eq), 2-fluoroprop-2-enoic acid (26.8 mg, 297 µmol, 2.00 eq), T3P (284 mg, 446 µmol, 265 µL, 50% purity, 3.00 eq) in portion at 0 °C under N₂. The mixture was stirred at 25 °C for 30 min. The reaction mixture was quenched by addition of water (2.00 mL) at 0 °C, and then diluted with water (3 mL) and extracted with ethyl acetate (20 mL × 3). The combined organic layers were washed with brine (20 mL × 1), dried over sodium sulfate, filtered and concentrated under reduced pressure to give a residue. The residue was purified by prep-HPLC (column: Waters Xbridge 150 × 25 5µ; mobile phase: [water (0.05% ammonia hydroxide v/v)-ACN]; B%: 55%-82%, 10 min). Compound of 2-[(2*S*)-1-(2-fluoroprop -2-enoyl)-4-[2-[[(2*S*)-1-methylpyrrolidin-2-yl]methoxy]-8-(1-naphthyl)-5,6,7,9-tetrahydropyrimido[4,5-*c*]azepin-4-yl]piperazin-2-yl]acetonitrile (23.7 mg, 39.9 µmol, 27% yield, 98.3% purity) as a white solid was obtained. LCMS [ESI, M+1]: 584.

¹H NMR (400MHz, chloroform-d) δ = 7.95 (d, *J* = 8.4 Hz, 1H), 7.81 (d, *J* = 8.0 Hz, 1H), 7.54 (d, *J=* 8.0 Hz, 1H), 7.47 - 7.41 (m, 1H), 7.40 - 7.33 (m, 2H), 7.13 (d, *J=* 6.8 Hz, 1H), 5.53 - 5.31 (m, 1H), 5.25 (dd, *J* = 3.6, 16.8 Hz, 1H), 4.88 (br s, 1H), 4.44 - 4.33 (m, 3H), 4.17 (dd, *J =* 6.8, 10.8 Hz, 2H), 3.91 (br d, *J* = 13.6 Hz, 1H), 3.76 (br d, *J* = 12.8 Hz, 1H), 3.59 - 3.38 (m, 3H), 3.31 (dd, J= 3.6, 14.0 Hz, 1H), 3.12 - 2.80 (m, 6H), 2.73 - 2.61 (m, 1H), 2.46 (s, 3H), 2.32 - 2.22 (m, 1H), 2.20 - 1.93 (m, 5H), 1.89 - 1.77 (m, 1H).

### EXAMPLE 23

### 2-[(2S)-1-(2-fluoroprop-2-enoyl)-4-[8-(8-methyl-1-naphthyl)-5,6,7,9-tetrahydropyrimido[4,5-c]azepin-4-yl]piperazin-2-yl]acetonitrile

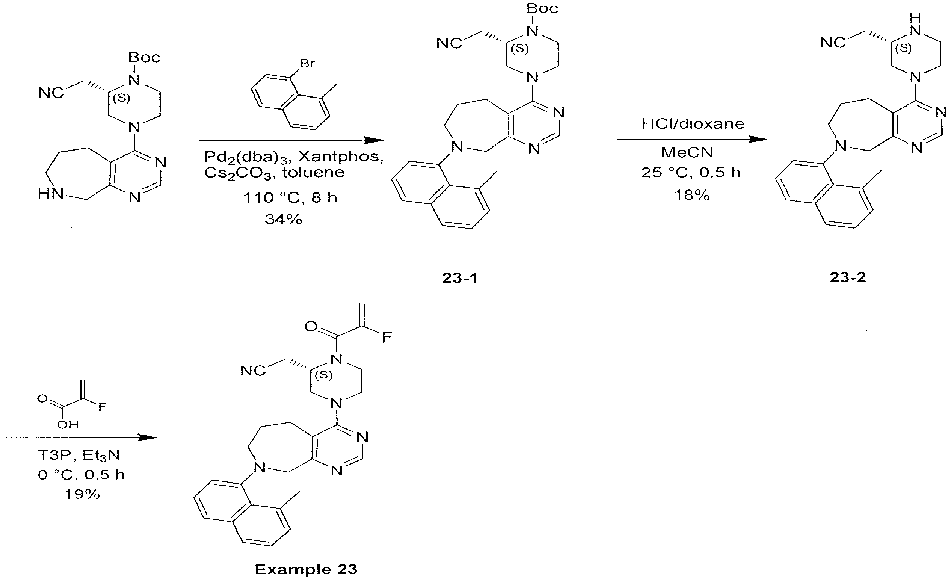

Compound 23-1: To a mixture of *tert-*butyl (2*S*)-2-(cyanomethyl)-4-(6,7,8,9-tetrahydro - 5*H*-pyrimido[4,5-*c*]azepin-4-yl)piperazine-1-carboxylate (2 g, 5.37 mmol, 1 *eq)* and 1-bromo-8-methyl-naphthalene (1.78 g, 8.05 mmol, 1.5 *eq)* in toluene (30 mL) was added Pd₂(dba)₃ (983 mg, 1.07 mmol, 0.2 *eq),* Xantphos (1.24 g, 2.15 mmol, 0.4 *eq),* Cs₂CO₃ (5.25 g, 16.1 mmol, 3 *eq)* in one portion under N₂. The mixture was stirred at 110 °C for 4 hours. Upon completion, the mixture was diluted with water (30 mL) and extracted with ethyl acetate (1 × 30 mL). The organic layer was separated, dried over sodium sulfate, filtered and concentrated under vacuum. The residue was purified by column chromatography (SiO₂, Dichloromethane/Methanol = 100/1 to 10/1). After that, the residue was purified by reverse-phase HPLC (0.1% FA condition). The residue was basified with saturated aqueous NaHCO₃ solution to pH = 8 and extracted with ethyl acetate (3 × 50 mL). The organic layers were separated, dried over sodium sulfate, filtered and concentrated under vacuum. *tert*-butyl (2*S*)-2-(cyanomethyl)-4-[8-(8-methyl-1-naphthyl)-5,6,7,9-tetrahydropyrimido[4,5-*c*]azepin-4-yl]piperazine-1-carboxylate (1 g, 1.80 mmol, 34% yield, 92.4% purity) as a yellow oil was obtained. LCMS [ESI, M+1]: 513.

¹H NMR (400MHz, chloroform-d) δ = 8.58 (d, *J*=7.0 Hz, 1H), 7.69 - 7.60 (m, 2H), 7.42 - 7.35 (m, 1H), 7.35 - 7.31 (m, 1H), 7.30 - 7.27 (m, 1H), 7.22 - 7.16 (m, 1H), 4.65 (br s, 1H), 4.52 - 4.42 (m, 1H), 4.40 - 4.29 (m, 1H), 3.85 - 3.71 (m, 1H), 3.65 - 3.53 (m, 1H), 3.50 - 3.39 (m, 1H), 3.35 - 3.25 (m, 2H), 3.23 - 3.14 (m, 1H), 3.11 - 2.85 (m, 5H), 2.83 - 2.71 (m, 5H), 2.03 - 1.97 (m, 1H), 1.53 (d, J=1.4 Hz, 9H).

Compound **23-2:** To a solution of tert-butyl (2*S*)-2-(cyanomethyl)-4-[8-(8-methyl-1-naphthyl)-5,6,7,9-tetrahydropyrimido[4,5-*c*]azepin-4-yl]piperazine-1-carboxylate (100mg, 195 µmol, 1 *eq)* in MeCN (1 mL) was added HCl/dioxane (4 M, 2 mL, 41.0 *eq)* at 25 °C. The mixture was stirred at 25 °C for 0.5 hour. Upon completion, the mixture was concentrated under vacuum. The residue was diluted with saturated NaHCO₃ solution (3 mL) and extracted with ethyl acetate (2 × 3 mL). The combined organic layers were washed with brine (1 × 5 mL), dried over sodium sulfate, filtered and concentrated under vacuum. The residue was purified by prep-HPLC (column: Waters Xbridge 150 * 25 5µ; mobile phase: [water (0.05% ammonia hydroxide v / v) - ACN]; B%: 38% - 68%, 10min). The residue was concentrated under reduced pressure to remove ACN, and then lyophilization. 2-[(2*S*)-4-[8-(8-methyl-1- naphthyl)-5,6,7,9-tetrahydropyrimido[4,5-c]azepin-4-yl]piperazin-2-yl]acetonitrile (14.9 mg, 35.3 µmol, 18% yield, 97.5% purity) as a yellow solid was obtained. LCMS [ESI, M+1]: 413.

1H NMR (400MHz, chloroform-d) δ = 8.55 (br s, 1H), 7.72 - 7.58 (m, 2H), 7.47 - 7.14 (m, 4H), 4.77 - 4.20 (m, 2H), 3.91 - 2.72 (m, 13H), 2.56 (br d, J=5.0 Hz, 2H), 2.02 (br s, 3H).

**Example 23:** To a solution of 2-[(2*S*)-4-[8-(8-methyl-1-naphthyl)-5,6,7,9-tetrahydropyrimido[4,5-c]azepin-4-yl]piperazin-2-yl]acetonitrile (150 mg, 364 µmol, 1 *eq),* 2-fluoroprop-2-enoic acid (98.2 mg, 1.09 mmol, 3 *eq)* and Et₃N (331 mg, 3.27 mmol, 455 µL, 9 *eq)* in DMF (15 mL) was added T3P (926 mg, 1.45 mmol, 865 µL, 50% purity, 4 *eq)* at 0 °C. The mixture was stirred at 0 °C for 0.5 hour. The residue was diluted with water (15 mL) and extracted with ethyl acetate (2 × 20 mL). The combined organic layers were washed with brine (1 × 20 mL), dried over sodium sulfate, filtered and concentrated under vacuum. The mixture was purified by column chromatography (SiO₂, Dichloromethane/Methanol = 10/1). After that, the residue was purified by prep-HPLC (column: Waters Xbridge 150 * 25 5µ; mobile phase: [water (0.05% ammonia hydroxide v / v) - ACN]; B%: 48% - 78%, 10min). The residue was concentrated under reduced pressure to remove ACN, and then lyophilization. 2-[(2*S*)-1-(2-fluoroprop-2-enoyl)-4-[8-(8-methyl-1-naphthyl)-5,6,7,9-tetrahydropyrimido[4,5-*c*]azepin-4-yl]piperazin-2-yl]acetonitrile (33.8 mg, 68.3 µmol, 19% yield, 98.0% purity) as a white solid was obtained. LCMS [ESI, M+1]: 485.5.

¹H NMR (400MHz, chloroform-d) δ = 8.59 (d, *J*=7.0 Hz, 1H), 7.71 - 7.61 (m, 2H), 7.45 - 7.27 (m, 3H), 7.23 - 7.14 (m, 1H), 5.56 - 5.32 (m, 1H), 5.26 (dd, *J*=3.6, 16.9 Hz, 1H), 4.56 - 4.44 (m, 1H), 4.42 - 4.31 (m, 1H), 4.22 - 3.96 (m, 1H), 3.86 (br t, *J*=13.2 Hz, 1H), 3.68 (br t, *J*=11.4 Hz, 1H), 3.54 - 3.20 (m, 4H), 3.19 - 2.72 (m, 9H), 2.17 - 1.94 (m, 2H).

### EXAMPLE 24

### 2-((S)-4-(8-(1H-indazol-4-yl)-2-(((S)-1-methylpyrrolidin-2-yl)methoxy)-6,7,8,9-tetrahydro-5H-pyrimido[4,5-c]azepin-4-yl)-1-acryloylpiperazin-2-yl)acetonitrile

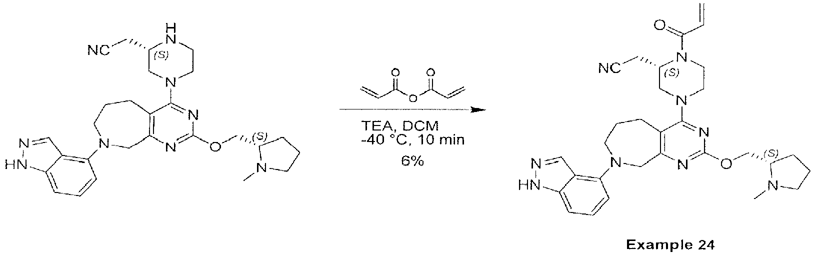

**Example 24:** To a mixture of 2-[(2*S*)-4-[8-(1*H*-indazol-4-yl)-2-[[(2*S*)-1-methylpyrrolidin-2-yl]methoxy]-5,6,7,9-tetrahydropyrimido[4,5-c]azepin-4-yl]piperazin-2-yl]acetonitrile (20.0 mg, 39.9 µmol, 1.0 eq) in dichloromethane (0.50 mL) was added TEA (12.1 mg, 120 µmol, 16.7 µL, 3.0 eq) and prop-2-enoyl prop-2-enoate (5.03 mg, 39.9 µmol, 1.0 eq) in portion at -40 °C under N₂. The mixture was stirred at -40 °C for 10 min. The reaction mixture was quenched by addition of methanol (0.05 mL) at -40 °C, and then diluted with water (0.50 mL) and extracted with dichloromethane (3.0 mL × 3). The combined organic layers were washed with brine (2.0 mL × 1), dried over sodium sulfate, filtered and concentrated under reduced pressure to give a residue. The residue was purified by prep-HPLC (column: Waters Xbridge 150 × 25 5 µ; mobile phase: [water (0.05% ammonia hydroxide v/v)-ACN]; B%: 27%-57%, 10 min). Compound 2-[(2*S*)-4-[8-(1*H*-indazol-4-yl)-2-[[(2*S*)-1- methylpyrrolidin-2-yl]methoxy]-5,6,7,9-tetrahydropyrimido[4,5-*c*]azepin-4-yl]-1-prop-2-enoyl-piperazin-2-yl]acetonitrile (1.60 mg, 2.56 µmol, 6% yield, 89% purity) as a white solid was obtained. LCMS [ESI, M+1]: 556.

¹H NMR (400MHz, chloroform-d) δ = 10.61 - 9.47 (m, 1H), 8.04 (s, 1H), 7.20 (t, *J*=8.0 Hz, 1H), 6.90 (d, J=8.0 Hz, 1H), 6.56 (br s, 1H), 6.43 - 6.29 (m, 2H), 5.80 (br d, J=10.8 Hz, 1H), 5.21 - 4.63 (m, 3H), 4.47 (dd, *J*=4.8, 10.6 Hz, 1H), 4.24 (dd, J=6.8, 10.8 Hz, 1H), 3.94 - 3.73 (m, 3H), 3.69 - 3.32 (m, 2H), 3.23 - 3.08 (m, 2H), 2.91 (br dd, J=8.0, 16.4 Hz, 2H), 2.84 - 2.66 (m, 4H), 2.53 (s, 3H), 2.40 - 2.26 (m, 1H), 2.24 - 2.03 (m, 3H), 1.95 - 1.73 (m, 4H).

### EXAMPLE 25

### 2-[(2S)-4-[8-(8-methyl-1-naphthyl)-5,6,7,9-tetrahydropyrimido[4,5-c]azepin-4-yl]-1-prop-2-enoyl-piperazin-2-yl]acetonitrile

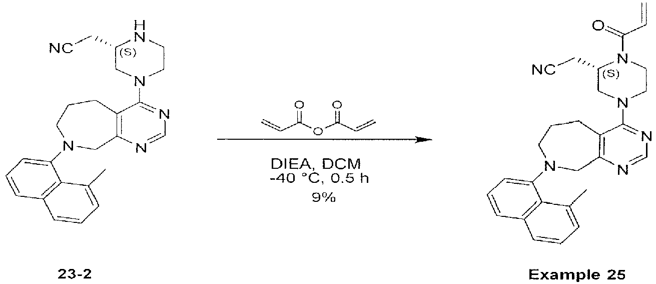

**Example 25:** To a mixture of 2-[(2*S*)-4-[8-(8-methyl-1-naphthyl)-5,6,7,9-tetrahydropyrimido[4,5-*c*]azepin-4-yl]piperazin-2-yl]acetonitrile (100 mg, 150 µmol, 1 *eq*, HCl) and DIEA (194 mg, 1.50 mmol, 261 µL, 10 *eq)* in DCM (10 mL) was added prop-2-enoyl prop-2-enoate (22.7 mg, 180 µmol, 1.2 *eq)* in portion at - 40 °C. The mixture was stirred at - 40 °C for 30 min. Upon completion, the mixture was diluted with water (5 mL). The organic layer was separated, washed with brine (1 × 10 mL), dried over sodium sulfate, filtered and concentrated under vacuum. The mixture was purified by column chromatography (SiO₂, Dichloromethane/Methanol = 1011). After that the residue was purified by prep-HPLC (column: Waters Xbridge 150 * 25 5µ; mobile phase: [water (0.05% ammonia hydroxide v / v) - ACN]; B%: 42% - 72%, 10min). The residue was concentrated under reduced pressure to remove ACN, and then lyophilization. 2-[(2*S*)-4-[8-(8-methyl -1-naphthyl)-5,6,7,9-tetrahydropyrimido[4,5-*c*]azepin-4-yl]-1-prop-2-enoyl-piperazin-2-yl]acetonitrile (6.2 mg, 12.9 µmol, 9% yield, 96.9% purity) as a white solid was obtained. LCMS [ESI, M+1]: 467.

¹H NMR (400MHz, chloroform-d) δ = 8.59 (d, J=6.2 Hz, 1H), 7.75 - 7.57 (m, 2H), 7.46 - 7.28 (m, 3H), 7.20 (br d, *J*=4.2 Hz, 1H), 6.61 (br s, 1H), 6.40 (brd, J=16.4 Hz, 1H), 5.84 (br d, *J*=10.6 Hz, 1H), 4.58 - 4.42 (m, 1H), 4.41 - 4.30 (m, 1H), 3.98 (br s, 1H), 3.92 - 3.79 (m, 1H), 3.70 (br s, 1H), 3.47 (br dd, J=7.2, 13.0 Hz, 1H), 3.37 - 2.62 (m, 12H), 2.04 (br d, J=5.2 Hz, 2H).

### EXAMPLE 26

### 2-((S)-4-(8-(1H-indazol-4-yl)-2-(((S)-1-methylpyrrolidin-2-yl)methoxy)-6,7,8,9-tetrahydro-5H-pyrimido[4,5-c]azepin-4-yl)-1-(2-fluoroacryloyl)piperazin-2-yl)acetonitrile

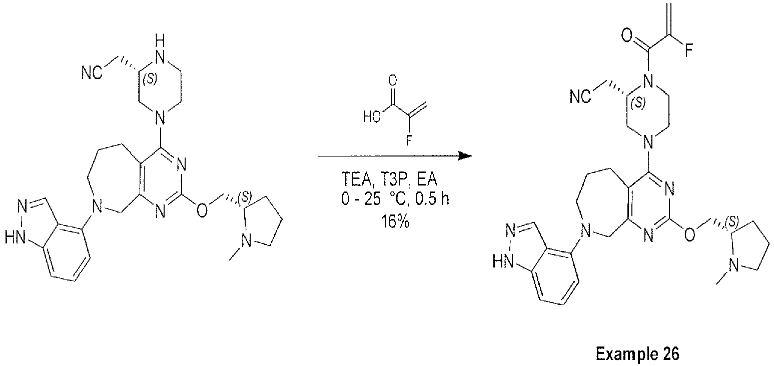

**Example 26:** To a mixture of 2-[(2*S*)-4-[8-(1*H*-indazol-4-yl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (30.0 mg, 59.8 µmol, 1.00 eq) in ethyl acetate (0.20 mL) was added TEA (96.8 mg, 957 µmol, 133 µL, 16.0 eq), 2-fluoroprop-2-enoic acid (5.39 mg, 59.8 µmol, 1.00 eq) and T3P (228 mg, 359 µmol, 213 µL, 50% purity, 6.00 eq) in portion at 0 °C under N₂. The mixture was stirred at 25 °C for 0.5 hour. The reaction mixture was quenched by addition of water (1.00 mL) at 0 °C, and then extracted with ethyl acetate (5 mL × 3). The combined organic layers were washed with brine (2 mL × 1), dried over sodium sulfate, filtered and concentrated under reduced pressure to give a residue. The residue was purified by prep-HPLC (column: Waters Xbridge 150 × 25 5 µ;mobile phase: [water (0.05% ammonia hydroxide v/v)-ACN];B%: 30%-60%,10 min). Compound 2-[(2*S*)-1-(2-fluoroprop-2-enoyl)-4-[8-(1*H*-indazol-4- yl)-2-[[(2*S*)-1-methylpyrrolidin-2-yl]methoxy]-5,6,7,9-tetrahydropyrimido[4,5-*c*]azepin-4-yl]piperazin-2-yl]acetonitrile (5.75 mg, 9.82 µmol, 16% yield, 98% purity) as a gray solid was obtained. LCMS [ESI, M+1): 574.

¹H NMR (400MHz, chloroform-d) δ = 10.69 - 9.24 (m, 1H), 8.04 (s, 1H), 7.20 (t, *J*=8.0 Hz, 1H), 6.91 (d, J=8.4 Hz, 1H), 6.34 (d, J=7.6 Hz, 1H), 5.52 - 5.29 (m, 1H), 5.23 (dd, *J*=3.6, 16.8 Hz, 1H), 5.01 - 4.54 (m, 4H), 4.44 (dd, J=4.8, 10.8 Hz, 1H), 4.23 (dd, *J*=7.2, 10.8 Hz, 1H), 3.87 - 3.74 (m, 3H), 3.63 (br d, *J*=13.2 Hz, 1H), 3.43 (br s, 1H), 3.21 (dd, *J*=4.0, 14.0 Hz, 1H), 3.12 (br t, J=7.2 Hz, 1H), 3.02 - 2.88 (m, 2H), 2.85 - 2.68 (m, 4H), 2.51 (s, 3H), 2.38 - 2.26 (m, 1H), 2.21 - 2.06 (m, 3H), 1.95 - 1.78 (m, 3H).

### EXAMPLE A

### KRas G12C Modification Assay

This Example illustrates that exemplary compounds of the present invention may be assayed using a LCMS assay to detect a covalent adduct of the exemplary compound and KRAS G12C.

The protein concentration of GDP-loaded K-Ras (1-169) G12C, C51S,C80L,C118S and GTP-loaded K-Ras (1-169) G12C,C51S,C80L,C118S,Q61H are adjusted to 2 µM in K-Ras Assay Buffer (25 mM HEPES,150 mM NaCl, 5 mM MgCl₂, and 10 mM Octyl β-glucopyranoside at pH 7.5). A 10 µL aliquot of each protein solution is transferred to a 384 well microtiter plate. Initial compound stocks are generated at fifty times their desired final assay concentration in DMSO.

Exemplary compounds of Formula (I) are diluted 25-fold into K-Ras Assay Buffer to a final of two times their final concentration. A 10 µL aliquot of each diluted compound solution is then added to each of the protein solutions in the microtiter plate to initiate reaction. Typical final compound concentrations are 3.0, 5.0 and 25.0 µM. At each time point, the reactions are quenched with 20 µL of a 25 mM acetic acid solution. Usual assay endpoints are 15, 180 and 1440 minutes. Once all reactions are quenched, the plates are heat sealed and the samples are injected into a LC/MS system for data acquisition.

Data collection may take place on an Agilent 6520 Q-TOF Accurate Mass Spectrometer. Samples are injected in their liquid phase onto a C-3 reverse phase column to remove assay buffer and prepare the samples for mass spectrometer. The proteins are eluted from the column using an acetonitrile gradient and fed directly into the mass analyzer. Initial raw data analysis may take place using Agilent MassHunter software immediately post data acquisition.

Raw data analysis of the intact protein is exclusively a deconvolution of the multiple charge states of each protein in solution using a maximum entropy deconvolution provided in Mass Hunter. To minimize complexity, only the data over limited mass ranges are considered for analysis, with a minimum of one Dalton mass step intervals. The heights of all masses identified during raw data analysis are exported to be further analyzed in Spotfire^{®} data analysis software.

Final data analysis is a multistep process in the Spotfire^{®} data analysis software package. Briefly, each protein mass is calculated as a percent of the total signal of that sample, that percentage is then normalized to the percentage of signal of the protein in the absence of reactive compounds. Those normalized signals are reported as normalized percent of control (POC). An increased POC value indicates a compound that displays a higher degree of modification at a given condition compared to other compounds under the same conditions.

### EXAMPLE B

### Inhibition of KRas G12C-dependent Cell Growth

This Example illustrates that exemplary compounds of the present invention inhibit the growth of tumor cell lines that express KRas G12C.

The cellular inhibition of KRAs G12C by exemplary compounds of the present invention was determined by measuring the amount of a downstream marker of KRas activity, phosphorylated ERK ("Phospho-ERK").

NCI-H358 cells (ATCC CRL-5807) express KRas G12C and were grown in RPMI medium supplemented with 10% fetal bovine serum, penicillin/streptomycin and 10 mM HEPES. Cells were plated in poly-D-Lysine coated 96-well plates at a concentration of 50,000 cells/well and allowed to attach for 8-12 hours. Diluted compounds were then added at a final concentration of 0.5 % DMSO. After 3 hours, the medium was removed, 150 µL of 4% formaldehyde was added and the plates were incubated for 20 minutes. The plates were washed with PBS, and permeabilized using 150 µL of ice cold 100% methanol for 10 minutes. Nonspecific antibody binding to the plates was blocked using 100 µL Licor Blocking Buffer (Li-Cor Biotechnology, Lincoln NE) for 1 hour at room temperature. Positive control samples and samples lacking cells were parallel processed with test samples as standards.

The amount Phospho-ERK was determined using an antibody specific for the phosphorylated form of ERK and compared to the amount of GAPDH. Primary antibodies used for detection were added as follows: Phospho-ERK (Cell Signaling cs9101) diluted 1:500 and GAPDH (Millipore MAB374) diluted 1:5000 in Licor block + 0.05% Tween 20. The plates were incubated for 2 hours at room temperature. The plates were washed with PBS + 0.05% Tween 20.

Secondary antibodies used to visualize primary antibodies were added as follows: Anti-rabbit-680 diluted 1:1000 and Anti-mouse-800 diluted 1:1000 in Licor Block + 0.05% Tween 20 and incubated for 1 hour at room temperature. The plates were washed with PBS + 0.05% Tween 20. A 100 µL aliquot of PBS was added to each well and the plates were read on a LICOR AERIUS plate reader.

The pERK(Thr202/Tyr204) signal was normalized with the GAPDH signal and percent of DMSO control values were calculated. IC₅₀ values were generated using a 4 parameter fit of the dose response curve. The results for exemplary compounds of Formula (I) are shown in Table 2. Key: "A" ≤ 500 nM; "B" > 500 nM and ND = not determined.

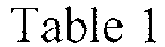

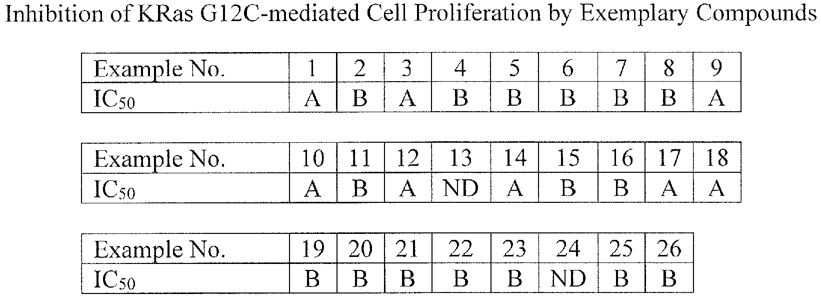

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A compound of formula (I): 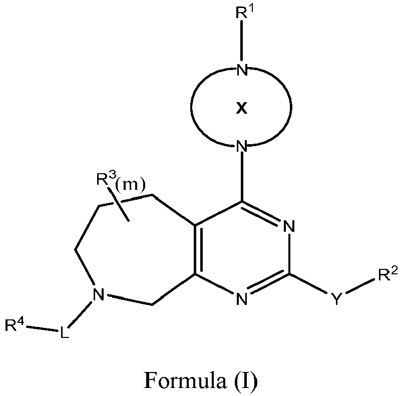 or a pharmaceutically acceptable salt thereof:
wherein:
R¹-X is: 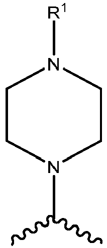
wherein the piperazinyl ring is optionally substituted with R⁸;
Y is a bond, O, S or NR⁵;
R¹ is -C(O)C(R^{A}) 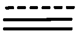 C(R^{B})ₚ or -SO₂C(R^{A}) 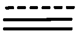 C(R^{B})ₚ;
R² is hydrogen, alkyl, hydroxyalkyl, dihydroxyalkyl, alkylaminylalkyl, dialkylaminylalkyl, -Z-NR⁵R¹⁰, heterocyclyl, heterocyclylalkyl, aryl, heteroaryl, or heteroarylalkyl, wherein each of the Z, heterocyclyl, heterocyclylalkyl, aryl, heteroaryl, and heteroarylalkyl may be optionally substituted with one or more R⁹;
each Z is C1 - C4 alkylene;
each R³ is independently C1 - C3 alkyl, halogen or -OR⁵;
L is a bond, -C(O)-, or C1 - C3 alkylene;
R⁴ is aryl or heteroaryl, wherein the aryl or heteroaryl may be optionally substituted with one or more R⁶, R⁷ or R⁸;
each R⁵ is independently hydrogen or C1 - C3 alkyl;
R⁶ is cycloalkyl, heterocyclyl, heterocyclylalkyl, aryl, or heteroaryl, wherein each of the cycloalkyl, heterocyclyl, aryl, or heteroaryl may be optionally substituted with one or more R⁷;
each R⁷ is independently halogen, hydroxyl, C1 - C6 alkyl, cycloalkyl, alkoxy, haloalkyl, amino, cyano, heteroalkyl, hydroxyalkyl or Q-haloalkyl, wherein Q is O or S;
R⁸ is oxo, C1 - C3 alkyl, C2 - C4 alkynyl, heteroalkyl, cyano, -C(O)OR⁵, -C(O)N(R⁵)₂, -N(R⁵)₂, wherein the C1 - C3 alkyl may be optionally substituted with cyano, halogen, -OR⁵, -N(R⁵)₂, or heteroaryl;
each R⁹ is independently hydrogen, oxo, acyl, hydroxyl, hydroxyalkyl, cyano, halogen, C1 - C6 alkyl, aralkyl, haloalkyl, heteroalkyl, cycloalkyl, heterocyclylalkyl, alkoxy, dialkylaminyl, dialkylamidoalkyl, or dialkylaminylalkyl, wherein the C1 - C6 alkyl may be optionally substituted with cycloalkyl;
each R¹⁰ is independently hydrogen, acyl, C1 - C3 alkyl, heteroalkyl or hydroxyalkyl;
R¹¹ is haloalkyl;
R^{A} is absent, hydrogen, deuterium, cyano, halogen, C1 - C-3 alkyl, haloalkyl, heteroalkyl, - C(O)N(R⁵)₂, or hydroxyalkyl;
each R^{B} is independently hydrogen, deuterium, cyano, C1 - C3 alkyl, hydroxyalkyl, heteroalkyl, C1 - C3 alkoxy, halogen, haloalkyl, -ZNR⁵R¹¹, -C(O)N(R⁵)₂, -NHC(O)C1 - C3 alkyl, - CH₂NHC(O)C1 - C3 alkyl, heteroaryl, heteroarylalkyl, dialkylaminylalkyl, or heterocyclylalkyl wherein the heterocyclyl portion is substituted with one or more substituents independently selected from halogen, hydroxyl, alkoxy and C1 - C3 alkyl, wherein the heteroaryl or the heteroaryl portion of the heteroarylalkyl is optionally substituted with one or more R⁷;
when 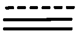 is a triple bond then R^{A} is absent, R^{B} is present and p equals one,
or when 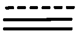 is a double bond then R^{A} is present, R^{B} is present and p equals two, or R^{A}, R^{B} and the carbon atoms to which they are attached form a 5-8 membered partially saturated cycloalkyl optionally substituted with one or more R⁷;
m is 0, 1, 2 or 3; and
p is one or two,
wherein
amino is -NH₂,
acyl is -C(O)CH₃,
alkyl is a straight or branched chain aliphatic group having from 1 to 12 carbon atoms,
haloalkyl is an alkyl chain in which one or more hydrogens has been replaced by a halogen, alkoxy is -OC1 - C6 alkyl,
cycloalkyl is a saturated or partially unsaturated cyclic hydrocarbon group having 3 to 12 carbon atoms,
heteroalkyl is an alkyl group wherein one or more carbon atoms in the chain are replaced by a heteroatom selected from the group consisting of O, S, and N,
hydroxyalkyl is -alkyl-OH,
dihydroxyalkyl is an alkyl group wherein two carbon atoms are each substituted with a hydroxyl group,
alkylaminylalkyl is -alkyl-NR^{x}-alkyl wherein R^{x} is hydrogen,
dialkylaminyl is N(R^{y})₂ wherein each R^{y} is Cl - C3 alkyl,
dialkylaminylalkyl is -alkyl-N(R^{y})₂ wherein each R^{y} is Cl - C4 alkyl,
aryl is a C₆-C₁₄ aromatic moiety comprising one to three aromatic rings,
aralkyl is a group comprising an aryl group covalently linked to an alkyl group,
heterocyclyl is a ring structure having from 3 to 12 atoms wherein one or more atoms are selected from the group consisting of N, O, and S, the remainder of the ring atoms being carbon, and excluding compounds having adjacent annular O and/or S atoms,
heterocyclylalkyl is a heterocyclyl group linked to the remaining portion of the molecule via an alkyl linker,
heteroaryl is a group having 5 to 14 ring atoms, having 6, 10, or 14 p electrons shared in a cyclic array, and having, in addition to carbon atoms, from one to three heteroatoms per ring selected from the group consisting of N, O, and S, and
heteroarylalkyl is a group comprising a heteroaryl group covalently linked to an alkyl group wherein the radical is on the alkyl group, and excluding compounds having adjacent annular O and/or S atoms.

2. The compound of claim 1, wherein R¹ is -C(O)C(R^{A}) 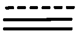 C(R^{B})ₚ, wherein 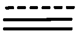 is a double bond and R^{A} is hydrogen, p is two and at least one R^{B} is independently hydrogen, deuterium, cyano, halogen, haloalkyl, hydroxyalkyl, heteroalkyl, heteroaryl, heteroarylalkyl, - ZNR⁵R¹¹, -C(O)N(R⁵)₂, -NHC(O)C1 - C3 alkyl or heterocyclylalkyl wherein the heterocyclyl portion is substituted with one or more substituents independently selected from halogen, hydroxyl, alkoxy or C1 - C3 alkyl.

3. The compound according to claim 1 or 2, wherein Y is O.

4. The compound according to any one of claims 1-3, wherein R² is heterocyclylalkyl optionally substituted with one or more R⁹, wherein the heterocyclyl of the heterocyclylalkyl is independently azetidinyl, methylazetidinyl, difluoroazetidinyl, tetrahydropyran, pyrrolidinyl, methylpyrrolidinyl, diemethylpyrrolidinyl, isopropylpyrrolidinyl, cycloalkylalkylpyrrolidinyl, hydroxypyrrolindinyl, fluoropyrrolidinyl, difluoropyrrolidinyl, (N-methyl)fluoropyrrolidinyl, (N-methyl)difluoropyrrolidinyl, methoxyethylpyrrolidinyl, (N-methyl)methoxypyrrolidinyl, piperazinyl, dimethylaminylpyrrolidinyl, morpholinyl, methylmorpholinyl, 1,4-oxazepanyl, piperdinyl, methylpiperidinyl acylpiperdinyl, cyanopiperdinyl, cycloalkylpiperdinyl, halopiperdinyl, dihalopiperdinyl, fluoropiperdinyl, difluoropiperdinyl, alkoxypiperdinyl, pyrrolidonyl, piperidinonyl, thiomorpholinyl-1,1-dioxide, 3-azabicyclo[3.1.0]hexanyl, oxa-5-azabicyclo[2.2.1]heptan-5-yl, or azabicyclo[2.2.1]heptan-2-yl.

5. The compound according to any one of claims 1-4, wherein R⁴ is aryl optionally substituted with one or more R⁷, and wherein the aryl is selected from the group consisting of phenyl and naphthyl optionally substituted with one or more R⁷, preferably wherein the phenyl and the naphthyl are each optionally substituted with one or more R⁷ selected from the group consisting of halogen, C1- C3 alkyl, haloalkyl, and hydroxyalkyl, preferably wherein R⁷ is selected from the group consisting of methyl, isopropyl, chloro, fluoro, and trifluoromethyl.

6. The compound of claim 1, wherein the compound is:

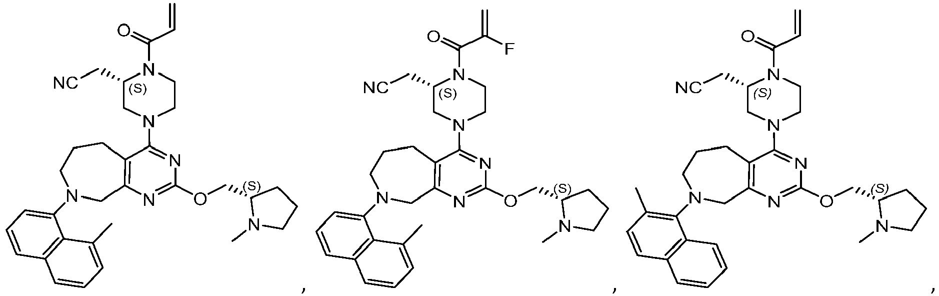

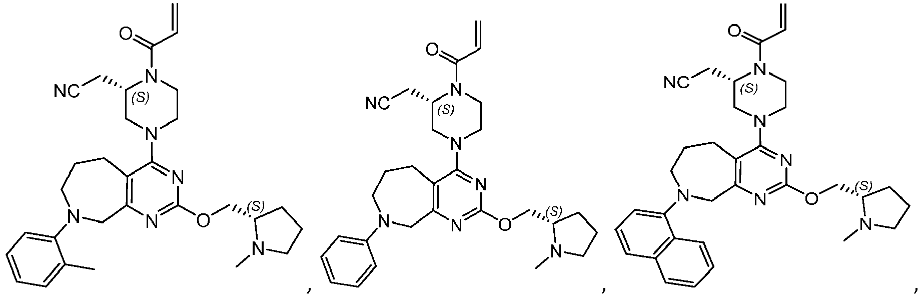

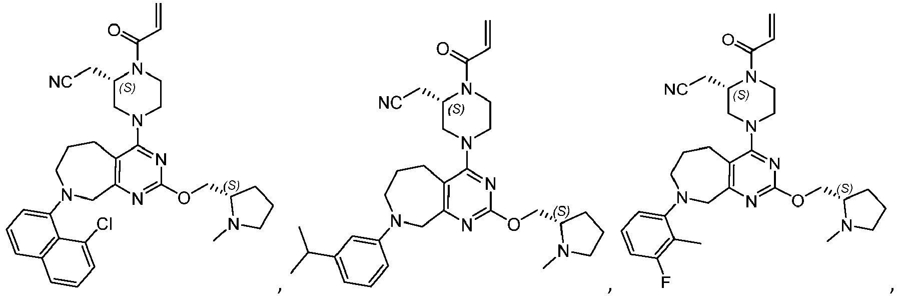

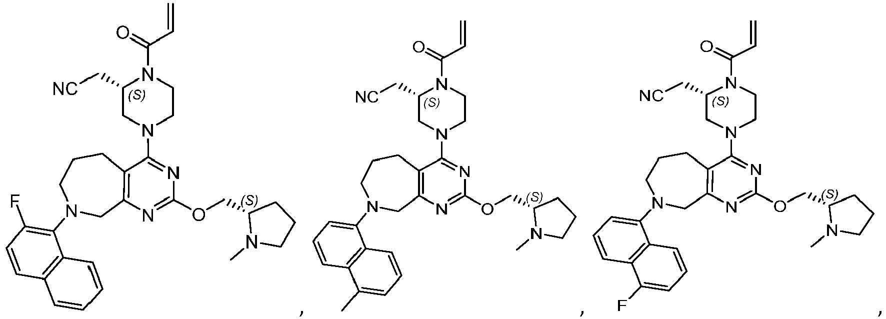

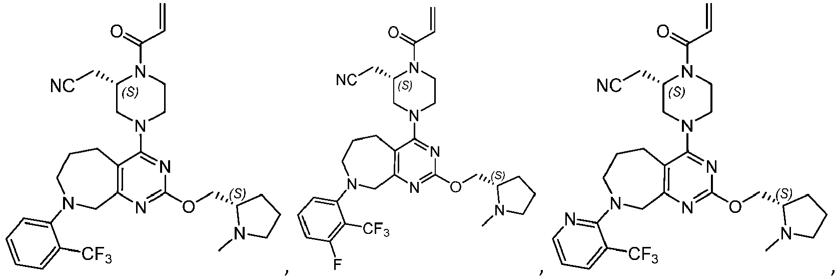

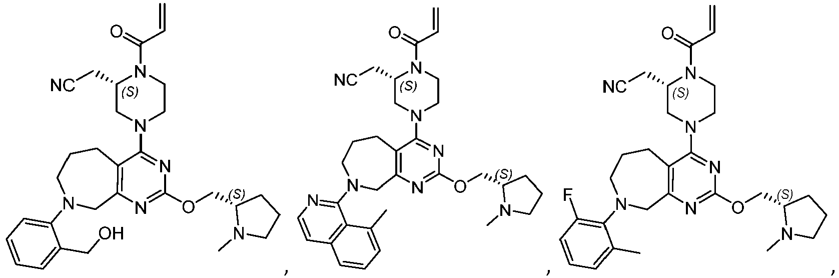

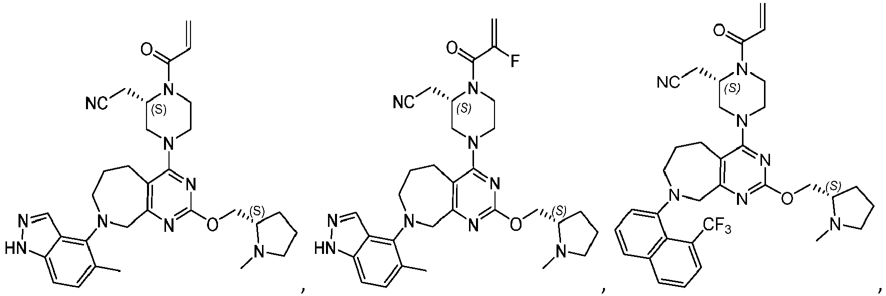

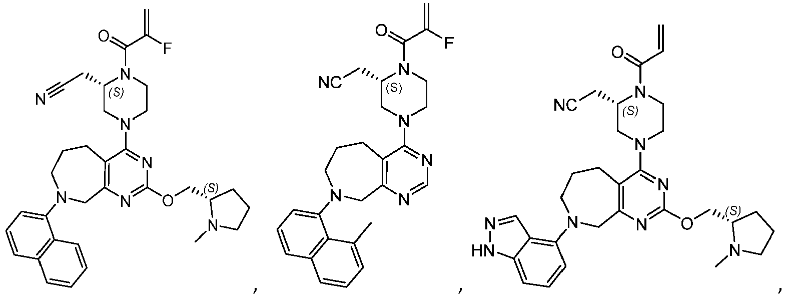

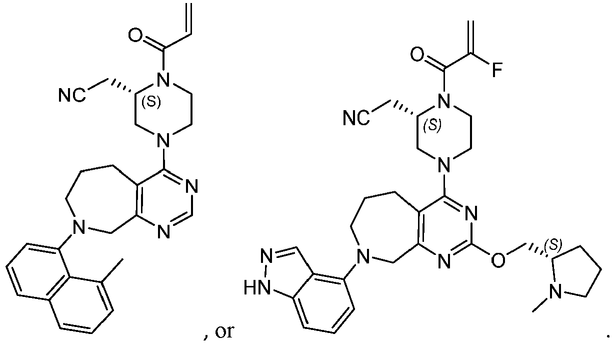

7. The compound of claim 1, wherein the compound is of Formula I-A: 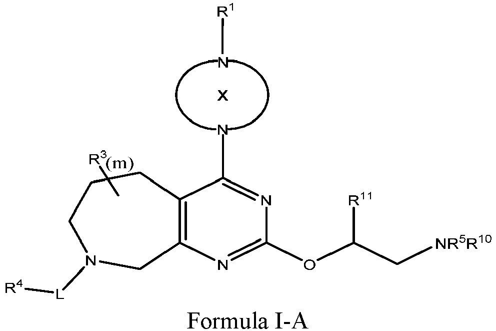 wherein X is a piperazinyl ring optionally substituted with R⁸, where R¹, R³, R⁴, R⁵, R⁸, R¹⁰, Land m are as defined in claim 1 and R¹¹ is hydrogen, methyl or hydroxyalkyl.

8. The compound of claim 1, wherein the compound is of Formula I-B: 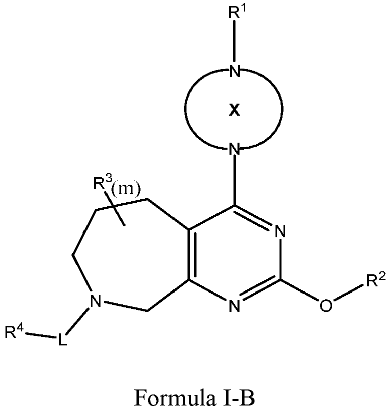 where X is a piperazinyl ring optionally substituted with R⁸, and R¹, R³, R⁴, R⁸, L and m are as defined in claim 1.

9. A pharmaceutical composition, comprising a therapeutically effective amount of a compound of Formula (I), Formula I-A or Formula I-B according to any one of claims 1-8, and a pharmaceutically acceptable excipient.

10. An in vitro method for inhibiting KRas G12C activity in a cell, comprising contacting the cell in which inhibition of KRas G12C activity is desired with an effective amount of a compound of Formula (I), Formula I-A or Formula I-B according to any one of claims 1-8, pharmaceutically acceptable salts thereof or pharmaceutical compositions containing the compound of Formula (I), Formula I-A or Formula I-B, or pharmaceutically acceptable salt thereof.

11. A compound of Formula (I), Formula I-A or Formula I-B according to any one of claims 1-8, pharmaceutically acceptable salts thereof or pharmaceutical compositions containing the compound of Formula (I), Formula I-A or Formula I-B, or pharmaceutically acceptable salt thereof for use in a method for inhibiting KRas G12C activity in a cell, comprising contacting the cell in which inhibition of KRas G12C activity is desired with an effective amount of the compound of Formula (I), Formula I-A or Formula I-B according to any one of claims 1-8, pharmaceutically acceptable salts thereof or pharmaceutical compositions containing the compound of Formula (I), Formula I-A or Formula I-B, or pharmaceutically acceptable salt thereof.

12. A compound of Formula (I), Formula I-A or Formula I-B, or a pharmaceutically acceptable salt thereof according to any one of claims 1-8, alone or combined with a pharmaceutically acceptable carrier, excipient or diluents for use in the treatment of a KRas G12C-associated cancer comprising administering to a patient having a KRas G12C-associated cancer a therapeutically effective amount of the compound of Formula (I), Formula I-A or Formula I-B, or the pharmaceutically acceptable salt thereof according to any one of claims 1-8, alone or combined with the pharmaceutically acceptable carrier, excipient or diluents.

13. The compound of Formula (I), Formula I-A or Formula I-B, or a pharmaceutically acceptable salt thereof, alone or combined with a pharmaceutically acceptable carrier, excipient or diluents for use according to claim 12, wherein the therapeutically effective amount of the compound is between about 0.01 to 100 mg/kg per day.

14. The compound of Formula (I), Formula I-A or Formula I-B, or a pharmaceutically acceptable salt thereof, alone or combined with a pharmaceutically acceptable carrier, excipient or diluents for use according to claim 12, wherein the KRas G12C-associated cancer is selected from the group consisting of Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial 'carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma.

15. The compound of Formula (I), Formula I-A or Formula I-B, or a pharmaceutically acceptable salt thereof, alone or combined with a pharmaceutically acceptable carrier, excipient or diluents for use according to any one of claims 12-14, wherein the KRas G12C-associated cancer is non-small cell lung cancer.

16. A compound of Formula (I), Formula I-A or Formula I-B or a pharmaceutically acceptable salt thereof according to any one of claims 1-8, or a pharmaceutical composition thereof for use in treating cancer in a patient in need thereof, comprising (a) determining that the cancer is associated with a KRas G12C mutation (e.g., a KRas G12C-associated cancer); and (b) administering to the patient a therapeutically effective amount of the compound of Formula (I), Formula I-A or Formula I-B or the pharmaceutically acceptable salt thereof according to any one of claims 1-8, or the pharmaceutical composition thereof.

## Patentansprüche

1. Verbindung der Formel (I): 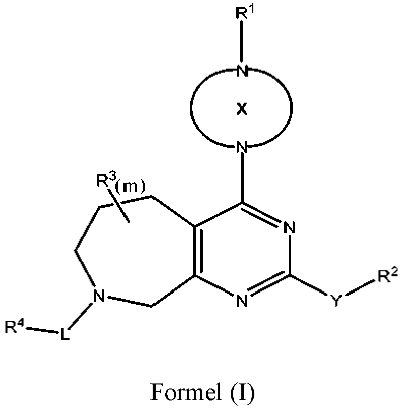 oder pharmazeutisch verträgliches Salz davon:
wobei:
R¹ -X ist: 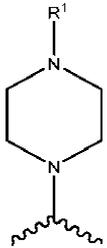
wobei der Piperazinylring optional mit R⁸ substituiert ist;
Y eine Bindung, O, S oder NR⁵ ist;
R¹ -C(O)C(R^{A}) 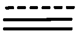 C(R^{B})ₚ oder -SO_{2C}(R^{A}) 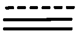 C(R^{B})ₚ ist;
R² Wasserstoff, Alkyl, Hydroxyalkyl, Dihydroxyalkyl, Alkylaminylalkyl, Dialkylaminylalkyl, -Z-NR⁵R¹⁰, Heterocyclyl, Heterocyclylalkyl, Aryl, Heteroaryl oder Heteroarylalkyl ist, wobei Z Heterocyclyl, Heterocyclylalkyl, Aryl, Heteroaryl und Heteroarylalkyl jeweils optional mit einem oder mehreren R⁹ substituiert sein kann;
Z jeweils C1 - C4-Alkylen ist;
R³ jeweils unabhängig C1 - C3-Alkyl, Halogen oder -OR⁵ ist;
L eine Bindung, -C(O)- oder C1 - C3-Alkylen ist;
R⁴ Aryl oder Heteroaryl ist, wobei das Aryl oder Heteroaryl optional mit einem oder mehreren R⁶, R⁷ oder R⁸ substituiert sein kann;
R⁵ jeweils unabhängig Wasserstoff oder C1 - C3-Alkyl ist;
R⁶ Cycloalkyl, Heterocyclyl, Heterocyclylalkyl, Aryl oder Heteroaryl ist, wobei jedes Cycloalkyl, Heterocyclyl, Aryl oder Heteroaryl optional mit einem oder mehreren R⁷ substituiert sein kann;
R⁷ jeweils unabhängig Halogen, Hydroxyl, C1 - C6-Alkyl, Cycloalkyl, Alkoxy, Halogenalkyl, Amino, Cyano, Heteroalkyl, Hydroxyalkyl oder Q-Halogenalkyl ist, wobei Q O oder S ist;
R⁸ Oxo, C1 - C3-Alkyl, C2 - C4-Alkinyl, Heteroalkyl, Cyano, -C(O)OR⁵, -C(O)N(R⁵)₂, -N(R⁵)₂ ist, wobei das C1 - C3-Alkyl optional mit Cyano, Halogen, -OR⁵, -N(R⁵)₂ oder Heteroaryl substituiert sein kann;
R⁹ jeweils unabhängig Wasserstoff, Oxo, Acyl, Hydroxyl, Hydroxyalkyl, Cyano, Halogen, C1 - C6-Alkyl, Aralkyl, Halogenalkyl, Heteroalkyl, Cycloalkyl, Heterocyclylalkyl, Alkoxy, Dialkylaminyl, Dialkylamidoalkyl oder Dialkylaminylalkyl ist, wobei das C1 - C6-Alkyl optional mit Cycloalkyl substituiert sein kann;
R¹⁰ unabhängig Wasserstoff, Acyl, C1 - C3-Alkyl, Heteroalkyl oder Hydroxyalkyl ist;
R¹¹ Halogenalkyl ist;
R^{A} fehlt, Wasserstoff, Deuterium, Cyano, Halogen, C1 - C-3-Alkyl, Halogenalkyl, Heteroalkyl, -C(O)N(R⁵)2 oder Hydroxyalkyl ist;
R^{B} jeweils unabhängig Wasserstoff, Deuterium, Cyano, C1 - C3-Alkyl, Hydroxyalkyl, Heteroalkyl, C1 - C3-Alkoxy, Halogen, Halogenalkyl, -ZNR⁵R¹¹, -C(O)N(R⁵)₂, -NHC(O)C1 - C3-Alkyl, -CH₂NHC(O)C1 - C3-Alkyl, Heteroaryl, Heteroarylalkyl, Dialkylaminylalkyl oder Heterocyclylalkyl ist, wobei der Heterocyclylanteil mit einem oder mehreren Substituenten substituiert ist, die unabhängig ausgewählt sind aus Halogen, Hydroxyl, Alkoxy und C1 - C3-Alkyl, wobei das Heteroaryl oder der Heteroarylanteil des Heteroarylalkyls optional mit einem oder mehreren R⁷ substituiert ist;
wenn 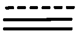 eine Dreifachbindung ist, dann R^{A} fehlt, R^{B} vorhanden ist und p gleich eins ist,
oder wenn 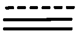 eine Doppelbindung ist, dann R^{A} vorhanden ist, R^{B} vorhanden ist und p gleich zwei ist, oder R^{A}, R^{B} und die Kohlenstoffatome, an die sie gebunden sind, ein 5-8-gliedriges, teilweise gesättigtes Cycloalkyl bilden, das optional mit einem oder mehreren R⁷ substituiert ist;
m 0, 1, 2 oder 3 ist; und
p eins oder zwei ist,
wobei
Amino -NH₂ ist,
Acyl -C(O)CH₃ ist,
Alkyl eine geradkettige oder verzweigte aliphatische Gruppe mit 1 bis 12 Kohlenstoffatomen ist, Halogenalkyl eine Alkylkette ist, bei der ein oder mehrere Wasserstoffe durch ein Halogen ersetzt sind,
Alkoxy -OC1 - C6-Alkyl ist,
Cycloalkyl eine gesättigte oder teilweise ungesättigte cyclische Kohlenwasserstoffgruppe mit 3 bis 12 Kohlenstoffatomen ist,
Heteroalkyl eine Alkylgruppe ist, bei der ein oder mehrere Kohlenstoffatome in der Kette durch ein Heteroatom, ausgewählt aus der Gruppe bestehend aus O, S und N, ersetzt sind,
Hydroxyalkyl -Alkyl-OH ist,
Dihydroxyalkyl eine Alkylgruppe ist, bei der zwei Kohlenstoffatome jeweils durch eine Hydroxylgruppe substituiert sind,
Alkylaminylalkyl -Alkyl-NR^{x}-Alkyl ist, wobei R^{x} Wasserstoff ist,
Dialkylaminyl N(R^{y})₂ ist, wobei R^{y} jeweils C1 - C3-Alkyl ist,
Dialkylaminylalkyl -Alkyl-N N(R^{y})₂ ist, wobei jedes R^{y} C1 - C4-Alkyl ist,
Aryl eine aromatische C₆-C₁₄-Einheit ist, die ein bis drei aromatische Ringe umfasst,
Aralkyl eine Gruppe ist, die eine Arylgruppe umfasst, die kovalent an eine Alkylgruppe gebunden ist,
Heterocyclyl eine Ringstruktur mit 3 bis 12 Atomen ist, wobei ein oder mehrere Atome aus der Gruppe bestehend aus N, O und S ausgewählt sind, wobei die übrigen Ringatome Kohlenstoff sind, und Verbindungen mit benachbarten O- und/oder S-Atomen im Ring ausgenommen sind,
Heterocyclylalkyl eine Heterocyclylgruppe ist, die über einen Alkyl-Linker mit dem restlichen Teil des Moleküls verbunden ist,
Heteroaryl eine Gruppe mit 5 bis 14 Ringatomen ist, die 6, 10 oder 14 in einer zyklischen Anordnung gemeinsam genutzte p-Elektronen aufweist, und zusätzlich zu Kohlenstoffatomen ein bis drei Heteroatome pro Ring, ausgewählt aus der Gruppe bestehend aus N, O, und S, aufweist und
Heteroarylalkyl eine Gruppe ist, die eine Heteroarylgruppe umfasst, die kovalent an eine Alkylgruppe gebunden ist, wobei sich der Rest an der Alkylgruppe befindet, und Verbindungen mit benachbarten O- und/oder S-Atomen im Ring ausgenommen sind.

2. Verbindung nach Anspruch 1, wobei R¹ -C(O)C(R^{A}) 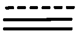 C(R^{B})ₚ ist, wobei 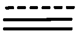 eine Doppelbindung ist und R^{A} Wasserstoff ist, p zwei ist und mindestens ein R^{B} unabhängig Wasserstoff, Deuterium, Cyano, Halogen, Halogenalkyl, Hydroxyalkyl, Heteroalkyl, Heteroaryl, Heteroarylalkyl, -ZNR⁵R¹¹, -C(O)N(R⁵)₂, -NHC(O)C1 - C3-Alkyl oder Heterocyclylalkyl ist, wobei der Heterocyclylanteil mit einem oder mehreren Substituenten substituiert ist, die unabhängig aus Halogen, Hydroxyl, Alkoxy oder C1 - C3-Alkyl ausgewählt sind.

3. Verbindung nach Anspruch 1 oder 2, wobei Y O ist.

4. Verbindung nach einem der Ansprüche 1-3, wobei R² Heterocyclylalkyl ist, das optional mit einem oder mehreren R⁹ substituiert ist, wobei das Heterocyclyl des Heterocyclylalkyls unabhängig Azetidinyl, Methylazetidinyl, Difluorazetidinyl, Tetrahydropyran, Pyrrolidinyl, Methylpyrrolidinyl, Diemethylpyrrolidinyl, Isopropylpyrrolidinyl, Cycloalkylalkylpyrrolidinyl, Hydroxypyrrolidinyl, Fluorpyrrolidinyl, Difluorpyrrolidinyl, (N-Methyl)fluorpyrrolidinyl, (N-Methyl)difluorpyrrolidinyl, Methoxyethylpyrrolidinyl, (N-Methyl)methoxypyrrolidinyl, Piperazinyl, Dimethylaminylpyrrolidinyl, Morpholinyl, Methylmorpholinyl, 1,4-Oxazepanyl, Piperdinyl, Methylpiperidinyl Acylpiperdinyl, Cyanopiperdinyl, Cycloalkylpiperdinyl, Halogenpiperdinyl, Dihalogenpiperdinyl, Fluorpiperdinyl, Difluorpiperdinyl, Alkoxypiperdinyl, Pyrrolidonyl, Piperidinonyl, Thiomorpholinyl-1,1-dioxid, 3-Azabicyclo[3.1.0]hexanyl, Oxa-5-azabicyclo[2.2.1]heptan-5-yl oder Azabicyclo[2.2.1]heptan-2-yl ist.

5. Verbindung nach einem der Ansprüche 1-4, wobei R⁴ Aryl ist, das optional mit einem oder mehreren R⁷ substituiert ist, und wobei das Aryl aus der Gruppe bestehend aus Phenyl und Naphthyl, optional substituiert mit einem oder mehreren R⁷, ausgewählt ist, vorzugsweise wobei das Phenyl und das Naphthyl jeweils optional mit einem oder mehreren R⁷ ausgewählt aus der Gruppe bestehend aus Halogen, C1 - C3-Alkyl, Halogenalkyl und Hydroxyalkyl substituiert sind, wobei vorzugsweise R⁷ aus der Gruppe bestehend aus Methyl, Isopropyl, Chlor, Fluor und Trifluormethyl ausgewählt ist.

6. Verbindung nach Anspruch 1, wobei die Verbindung Folgendes ist:

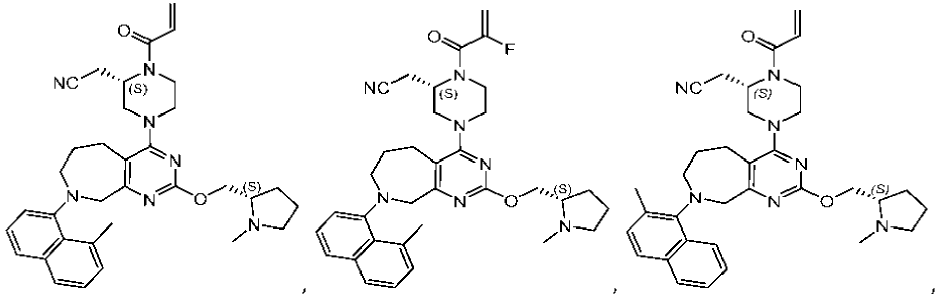

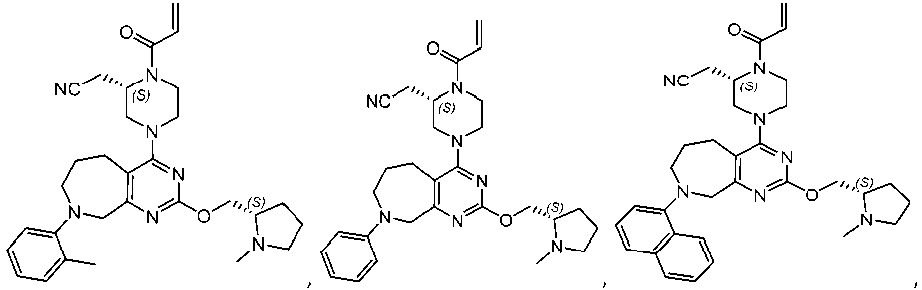

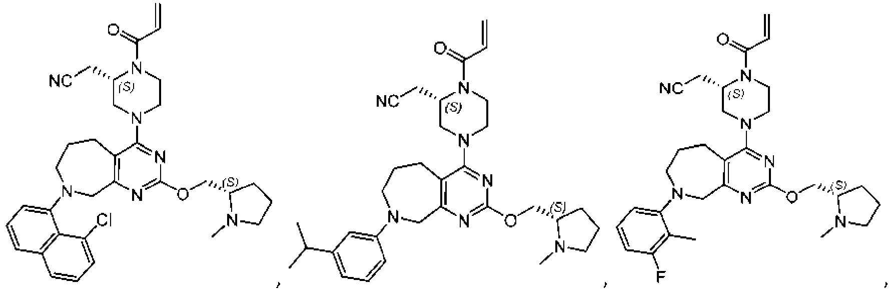

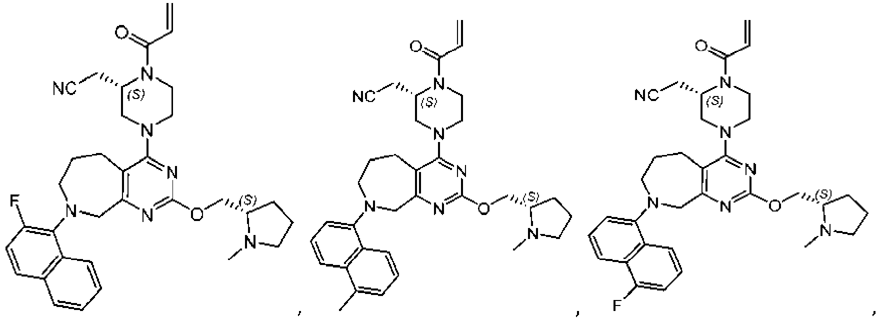

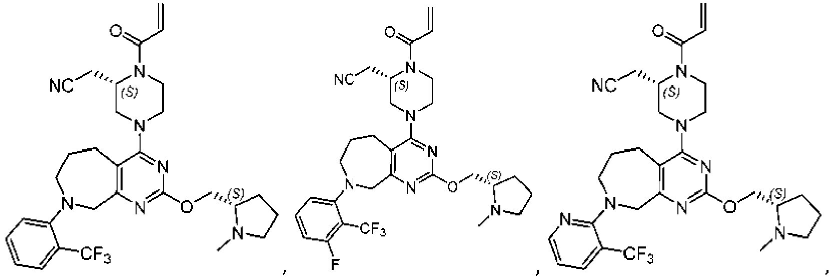

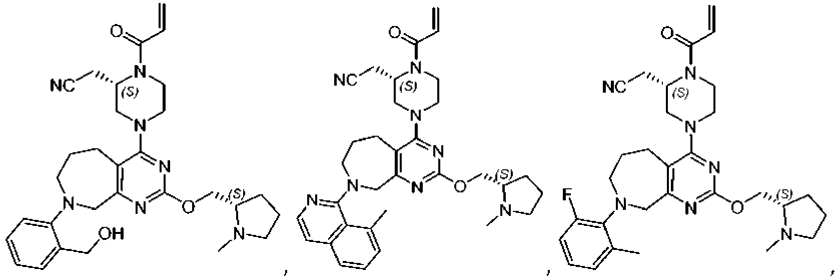

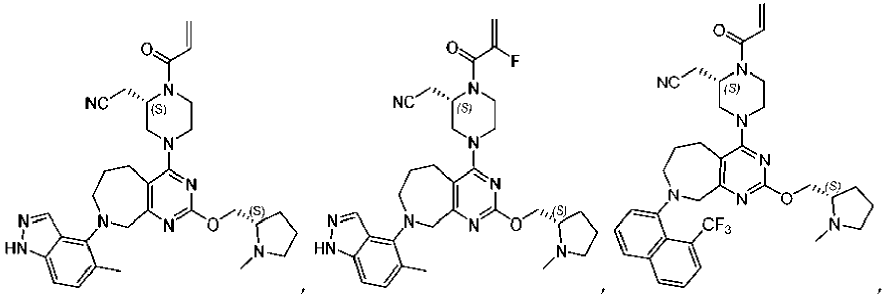

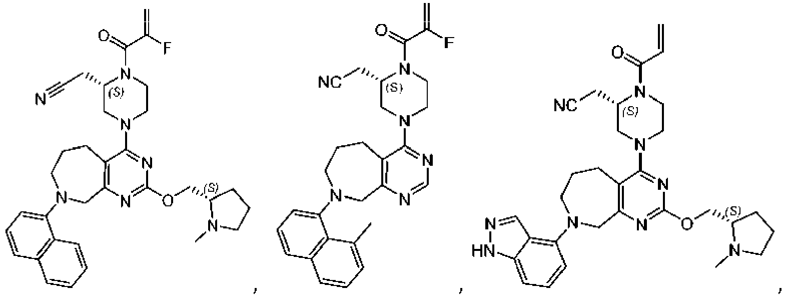

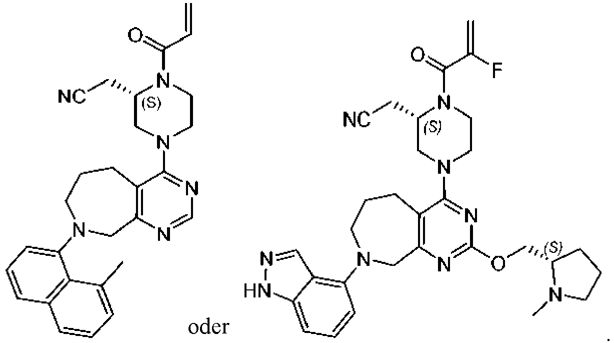

7. Verbindung nach Anspruch 1, wobei die Verbindung die Formel I-A aufweist: 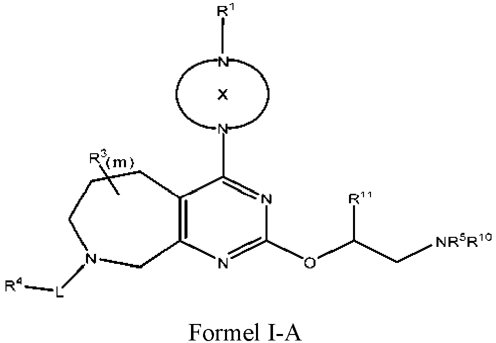 wobei X ein Piperazinylring ist, der optional mit R⁸ substituiert ist, wobei R¹, R³, R⁴, R⁵, R⁸, R¹⁰, L und m wie in Anspruch 1 definiert sind und R¹¹ Wasserstoff, Methyl oder Hydroxyalkyl ist.

8. Verbindung nach Anspruch 1, wobei die Verbindung die Formel I-B aufweist: 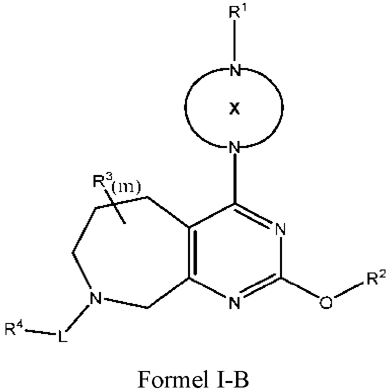 wobei X ein Piperazinylring ist, der optional mit R⁸ substituiert ist, und R¹, R³, R⁴, R⁸, L und m wie in Anspruch 1 definiert sind.

9. Pharmazeutische Zusammensetzung, umfassend eine therapeutisch wirksame Menge einer Verbindung der Formel (I), Formel I-A oder Formel I-B nach einem der Ansprüche 1-8, und einen pharmazeutisch verträglichen Hilfsstoff.

10. In-vitro-Verfahren zur Hemmen von KRas-G12C-Aktivität in einer Zelle, umfassend Inkontaktbringen der Zelle, in der eine Hemmung der KRas-G12C-Aktivität gewünscht wird, mit einer wirksamen Menge einer Verbindung der Formel (I), Formel I-A oder Formel I-B nach einem der Ansprüche 1-8, pharmazeutisch verträglichen Salzen davon oder pharmazeutischen Zusammensetzungen, welche die Verbindung der Formel (I), Formel I-A oder Formel I-B oder ein pharmazeutisch verträgliches Salze davon enthalten.

11. Verbindung der Formel (I), Formel I-A oder Formel I-B nach einem der Ansprüche 1-8, pharmazeutisch verträgliche Salze davon oder pharmazeutische Zusammensetzungen, welche die Verbindung der Formel (I), Formel I-A oder Formel I-B enthalten oder pharmazeutisch verträgliches Salz davon zur Verwendung in einem Verfahren zum Hemmen von KRas-G12C-Aktivität in einer Zelle, umfassend Inkontaktbringen der Zelle, in der eine Hemmung der KRas-G12C-Aktivität gewünscht wird, mit einer wirksamen Menge der Verbindung der Formel (I), Formel I-A oder Formel I-B nach einem der Ansprüche 1-8, pharmazeutisch verträglichen Salzen davon oder pharmazeutischen Zusammensetzungen, welche die Verbindung der Formel (I), Formel I-A oder Formel I-B oder ein pharmazeutisch verträgliches Salze davon enthalten.

12. Verbindung der Formel (I), Formel I-A oder Formel I-B oder pharmazeutisch verträgliches Salz davon nach einem der Ansprüche 1-8, allein oder in Kombination mit einem pharmazeutisch verträglichen Träger, Hilfsstoff oder Verdünnungsmitteln zur Verwendung bei der Behandlung eines KRas-G12C-assoziierten Krebs, umfassend Verabreichen einer therapeutisch wirksamen Menge der Verbindung der Formel (I), Formel I-A oder Formel I-B oder des pharmazeutisch verträglichen Salzes davon nach einem der Ansprüche 1-8, allein oder in Kombination mit dem pharmazeutisch verträglichen Träger, Hilfsstoff oder Verdünnungsmitteln an einen Patienten mit einem KRas-G12C-assoziierten Krebs.

13. Verbindung der Formel (I), Formel I-A oder Formel I-B oder pharmazeutisch verträgliches Salz davon, allein oder in Kombination mit einem pharmazeutisch verträglichen Träger, Hilfsstoff oder Verdünnungsmitteln zur Verwendung nach Anspruch 12, wobei die therapeutisch wirksame Menge der Verbindung zwischen etwa 0,01 bis 100 mg/kg pro Tag liegt.

14. Verbindung der Formel (I), Formel I-A oder Formel I-B oder pharmazeutisch verträgliches Salz davon, allein oder in Kombination mit einem pharmazeutisch verträglichen Träger, Hilfsstoff oder Verdünnungsmitteln zur Verwendung nach Anspruch 12, wobei der KRas-G12C-assoziierte Krebs ausgewählt ist aus der Gruppe bestehend aus Herz: Sarkom (Angiosarkom, Fibrosarkom, Rhabdomyosarkom, Liposarkom), Myxom, Rhabdomyom, Fibrom, Lipom und Teratom; Lunge: bronchogenem Karzinom (Plattenepithelkarzinom, undifferenziert kleinzellig, undifferenziert großzellig, Adenokarzinom), alveolärem (bronchiolärem) Karzinom, Bronchialadenom, Sarkom, Lymphom, chondromatösem Hamartom, Mesotheliom; Magen-Darm: Speiseröhre (Plattenepithelkarzinom, Adenokarzinom, Leiomyosarkom, Lymphom), Magen (Karzinom, Lymphom, Leiomyosarkom), Bauchspeicheldrüse (duktalem Adenokarzinom, Insulinom, Glucagonom, Gastrinom, Karzinoidtumoren, Vipom), Dünndarm (Adenokarzinom, Lymphom, Karzinoidtumoren, Kaposi-Sarkom, Leiomyom, Hämangiom, Lipom, Neurofibrom, Fibrom), Dickdarm (Adenokarzinom, tubuläres Adenom, Zottenadenom, Hamartom, Leiomyom); Urogenitaltrakt: Niere (Adenokarzinom, Wilm-Tumor (Nephroblastom), Lymphom, Leukämie), Blase und Harnröhre (Plattenepithelkarzinom, Übergangszellkarzinom, Adenokarzinom), Prostata (Adenokarzinom, Sarkom), Hoden (Seminom, Teratom, embryonalem Karzinom, Teratokarzinom, Choriokarzinom, Sarkom, interstitiellem Zellkarzinom, Fibrome, Fibroadenom, adenomatoiden Tumoren, Lipom); Leber: Hepatom (hepatozellulärem Karzinom), Cholangiokarzinom, Hepatoblastom, Angiosarkom, hepatozellulärem Adenom, Hämangiom; Gallenwege: Gallenblasenkarzinom, Ampullenkarzinom, Cholangiokarzinom; Knochen: osteogenem Sarkom (Osteosarkom), Fibrosarkom, malignem fibrösem Histiozytom, Chondrosarkom, Ewing-Sarkom, malignem Lymphom (Retikulumzellsarkom), multiplem Myelom, bösartigem Riesenzelltumor Chordom, Osteochronfroma (osteokartilaginären Exostosen), gutartigem Chondrom, Chondroblastom, Chondromyxofibrom, Osteoid-Osteom und Riesenzelltumoren; Nervensystem: Schädel (Osteom, Hämangiom, Granulom, Xanthom, Osteitis deformans), Hirnhäute (Meningeom, Meningiosarkom, Gliomatose), Gehirn (Astrozytom, Medulloblastom, Gliom, Ependymom, Germinom (Pinealom), Glioblastoma multiform, Oligodendrogliom, Schwannom, Retinoblastom, angeborenen Tumoren), Neurofibrom des Rückenmarks, Meningeom, Gliom, Sarkom); Gynäkologie: Gebärmutter (Endometriumkarzinom (serösem Zystadenokarzinom, muzinösem Zystadenokarzinom, nicht klassifiziertem Karzinom), Granulosa-Thekal-Zelltumoren, Sertoli-Leydig-Zelltumoren, Dysgerminom, malignem Teratom), Vulva (Plattenepithelkarzinom, intraepithelialem Karzinom, Adenokarzinom, Fibrosarkom, Melanom), Vagina (Klarzellkarzinom, Plattenepithelkarzinom, Botryoidsarkom (embryonalem Rhabdomyosarkom), Eileiter (Karzinom); Hämatologie: Blut (myeloischer Leukämie (akut und chronisch), akuter lymphatischer Leukämie, chronischer lymphatischer Leukämie, myeloproliferativen Erkrankungen, multiplem Myelom, myelodysplastischem Syndrom), Morbus Hodgkin, Non-Hodgkin-Lymphom (malignem Lymphom); Haut: malignem Melanom, Basalzellkarzinom, Plattenepithelkarzinom, Kaposi-Sarkom, Muttermale, dysplastische Nävi, Lipom, Angiom, Dermatofibrom, Keloiden, Psoriasis; und Nebennieren: Neuroblastom.

15. Verbindung der Formel (I), Formel I-A oder Formel I-B oder pharmazeutisch verträgliches Salz davon, allein oder in Kombination mit einem pharmazeutisch verträglichen Träger, Hilfsstoff oder Verdünnungsmitteln zur Verwendung nach einem der Ansprüche 12-14, wobei der KRas-G12C-assoziierte Krebs nicht-kleinzelliger Lungenkrebs ist.

16. Verbindung der Formel (I), Formel I-A oder Formel I-B oder pharmazeutisch verträgliches Salz davon nach einem der Ansprüche 1-8 oder pharmazeutische Zusammensetzung davon zur Verwendung beim Behandeln eines Krebs bei einem Patienten, der dessen bedarf, umfassend (a) Bestimmen, dass der Krebs mit einer KRas-G12C-Mutation assoziiert ist (z. B. ein KRas-G12C-assoziierter Krebs); und (b) Verabreichen einer therapeutisch wirksamen Menge der Verbindung der Formel (I), Formel I-A oder Formel I-B oder des pharmazeutisch verträglichen Salzes davon nach einem der Ansprüche 1-8 oder der pharmazeutischen Zusammensetzung davon an den Patienten.

## Revendications

1. Composé de formule (I) : 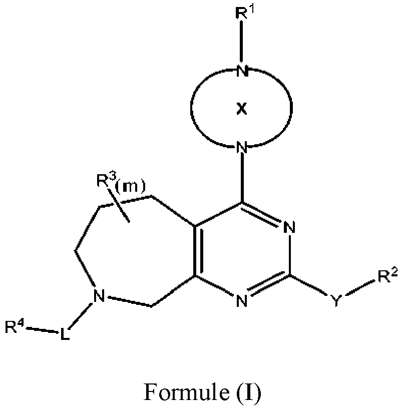 ou sel pharmaceutiquement acceptable de celui-ci :
avec :
R¹-X est : 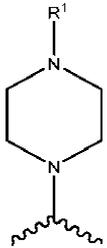
l'anneau pipérazinyle étant éventuellement substitué par R⁸ ;
Y est une liaison, O, S ou NR⁵ ;
R¹ est -C(O)C(R^{A}) 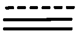 C(R^{B})ₚ ou -SO₂C(R^{A}) 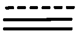 C(R^{B})ₚ ;
R² est un hydrogène, alkyle, hydroxyalkyle, dihydroxyalkyle, alkylaminylalkyle, dialkylaminylalkyle, -Z-NR⁵R¹⁰, hétérocyclyle, hétérocyclylalkyle, aryle, hétéroaryle, ou un hétéroarylalkyle, chacun des Z, hétérocyclyle, hétérocyclylalkyle, aryle, hétéroaryle, et hétéroarylalkyle pouvant être éventuellement substitué par un ou plusieurs R⁹ ;
chaque Z est un C1-C4 alkylène ;
chaque R³ est indépendamment un C1-C3 alkyle, halogène ou -OR⁵ ;
L est une liaison, -C(O)-, ou un C1-C3 alkylène ;
R⁴ est un aryle ou un hétéroaryle, l'aryle ou l'hétéroaryle pouvant être éventuellement substitué par un ou plusieurs R⁶, R⁷ ou R⁸ ;
chaque R⁵ est indépendamment un hydrogène ou un C1-C3 alkyle ;
R⁶ est un cycloalkyle, hétérocyclyle, hétérocyclylalkyle, aryle, ou hétéroaryle, chacun des cycloalkyle, hétérocyclyle, aryle, ou hétéroaryle pouvant être éventuellement substitué par un ou plusieurs R⁷ ;
chaque R⁷ est indépendamment un halogène, hydroxyle, C1-C6 alkyle, cycloalkyle, alcoxy, haloalkyle, amino, cyano, hétéroalkyle, hydroxyalkyle ou un Q-haloalkyle, Q étant O ou S ;
R⁸ est un oxo, C1-C3 alkyle, C2-C4 alcynyle, hétéroalkyle, cyano, -C(O)OR⁵, - C(O)N(R⁵)₂, -N(R⁵)₂, le C1-C3 alkyle pouvant éventuellement être substitué par un cyano, halogène, -OR⁵, -N(R⁵)₂, ou un hétéroaryle ;
chaque R⁹ est indépendamment un hydrogène, oxo, acyle, hydroxyle, hydroxyalkyle, cyano, halogène, C1-C6 alkyle, aralkyle, haloalkyle, hétéroalkyle, cycloalkyle, hétérocyclylalkyle, alcoxy, dialkylaminyle, dialkylamidoalkyle, ou un dialkylaminylalkyle, le C1-C6 alkyle pouvant être éventuellement substitué par un cycloalkyle ;
chaque R¹⁰ est indépendamment un hydrogène, acyle, C1-C3 alkyle, hétéroalkyle ou un hydroxyalkyle ;
R¹¹ est un haloalkyle ;
R^{A} est absent, un hydrogène, deutérium, cyano, halogène, C1-C3 alkyle, haloalkyle, hétéroalkyle, - C(O)N(R⁵)₂, ou un hydroxyalkyle ;
chaque R^{B} est indépendamment un hydrogène, deutérium, cyano, C1-C3 alkyle, hydroxyalkyle, hétéroalkyle, C1-C3 alcoxy, halogène, haloalkyle, -ZNR⁵R¹¹, -C(O)N(R⁵)₂, - NHC(O)C1-C3 alkyle, -CH₂NHC(O)C1-C3 alkyle, hétéroaryle, hétéroarylalkyle, dialkylaminylalkyle, ou un hétérocyclylalkyle dans lequel la partie hétérocyclyle est substituée par un ou plusieurs substituants choisis indépendamment parmi un halogène, hydroxyle, alcoxy et un C1-C3 alkyle, l'hétéroaryle ou la partie hétéroaryle de l'hétéroarylalkyle étant éventuellement substituée par un ou plusieurs R⁷ ;
lorsque 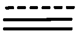 est une triple liaison alors R^{A} est absent, R^{B} est présent et p égale un,
ou lorsque 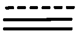 est une double liaison alors R^{A} est présent, R^{B} est présent et p égale deux, ou R^{A}, R^{B} et les atomes de carbone auxquels ils sont attachés forment un cycloalkyle partiellement saturé de 5 à 8 chaînons éventuellement substitué par un ou plusieurs R⁷ ;
m est 0, 1, 2 ou 3 ; et
p est un ou deux,
l'amino étant -NH₂,
l'acyle étant -C(O)CH₃,
l'alkyle est un groupe aliphatique à chaîne linéaire ou ramifiée ayant de 1 à 12 atomes de carbone, haloalkyle étant une chaîne alkyle dans laquelle un ou plusieurs hydrogènes ont été remplacés par un halogène, l'alcoxy étant un -OC1-C6 alkyle,
le cycloalkyle étant un groupe hydrocarbure cycliques saturé ou partiellement insaturé ayant 3 à 12 atomes de carbone,
l'hétéroalkyle étant un groupe alkyle dans lequel un ou plusieurs atomes de carbone dans la chaîne sont remplacés par un hétéroatome choisi parmi le groupe constitué de O, S, et N,
l'hydroxyalkyle étant un -alkyl-OH,
le dihydroxyalkyle étant un groupe alkyle dans lequel deux atomes de carbone sont chacun substitués par un groupe hydroxyle,
l'alkylaminylalkyle étant un -alkyl-NR^{x}-alkyle dans lequel R^{x} est l'hydrogène,
le dialkylaminyle étant N(R^{y})₂, chaque R^{y} étant un C1-C3 alkyle,
le dialkylaminylalkyle étant un -alkyl-N(R^{y})₂ dans lequel chaque R^{y} est un C1-C4 alkyle,
l'aryle étant un fragment C₆-C₁₄ aromatique comprenant de un à trois anneaux aromatiques,
l'aralkyle étant un groupe comprenant un groupe aryle lié de façon covalente à un groupe alkyle,
l'hétérocyclyle étant une structure en anneau ayant de 3 à 12 atomes dans laquelle un ou plusieurs atomes sont choisis parmi le groupe constitué de N, O et S, le reste des atomes de l'anneau étant le carbone,
et à l'exclusion des composés ayant des atomes O et/ou S annulaires adjacents,
l'hétérocyclylalkyle étant un groupe hétérocyclyle lié à la partie restante de la molécule par l'intermédiaire d'une séquence de liaison alkyle,
l'hétéroaryle étant un groupe ayant de 5 à 14 atomes d'anneau, ayant 6, 10 ou 14 électrons p partagés dans un réseau cyclique, et ayant, en plus des atomes de carbone, de un à trois hétéroatomes par anneau choisis parmi le groupe constitué de N, O et S, et
l'hétéroarylalkyle étant un groupe comprenant un groupe hétéroaryle covalent lié à un groupe alkyle dans lequel le radical est sur le groupe alkyle, et à l'exclusion des composés ayant des atomes adjacents annulaires O et/ou S.

2. Composé de la revendication 1, dans lequel R¹ est -C(O)C(R^{A}) 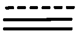 C(R^{B})ₚ, dans lequel 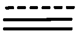 est une double liaison et R^{A} est un hydrogène, p est deux et au moins un R^{B} est indépendamment un hydrogène, deutérium, cyano, halogène, haloalkyle, hydroxyalkyle, hétéroalkyle, hétéroaryle, hétéroarylalkyle, - ZNR⁵R¹¹, -C(O)N(R⁵)₂, -NHC(O)C1-C3 alkyle ou hétérocyclylalkyle dans lequel la partie hétérocyclyle est substituée par un ou plusieurs substituants choisis indépendamment parmi un halogène, hydroxyle, alcoxy ou un C1-C3 alkyle.

3. Composé selon la revendication 1 ou 2, dans lequel Y est O.

4. Composé selon l'une quelconque des revendications 1 à 3, dans lequel R² est un hétérocyclylalkyle éventuellement substitué par un ou plusieurs R⁹, l'hétérocyclyle de l'hétérocyclylalkyle étant indépendamment un azétidinyle, méthylazétidinyle, difluoroazétidinyle, tétrahydropyrane, pyrrolidinyle, méthylpyrrolidinyle, diméthylpyrrolidinyle, isopropylpyrrolidinyle, cycloalkylalkylpyrrolidinyle, hydroxypyrrolindinyle, fluoropyrrolidinyle, difluoropyrrolidinyle, (N-méthyl)fluoropyrrolidinyle, (N- méthyl)difluoropyrrolidinyle, méthoxyéthylpyrrolidinyle, (N-méthyl)méthoxypyrrolidinyle, pipérazinyle, diméthylaminylpyrrolidinyle, morpholinyle, méthylmorpholinyle, 1,4-oxazépanyle, pipéridinyle, méthylpipéridinyle, acylpipéridinyle, cyanopipéridinyle, cycloalkylpipéridinyle, halopipéridinyle, dihalopipéridinyle, fluoropipéridinyle, difluoropipéridinyle, alcoxypipéridinyle, pyrrolidonyle, pipéridinonyle, thiomorpholinyl-1,1-dioxyde, 3-azabicyclo[3.1.0]hexanyle, oxa-5-azabicyclo[2.2.1]heptan-5-yle ou un azabicyclo[2.2.1]heptan-2-yle.

5. Composé selon l'une quelconque des revendications 1 à 4, dans lequel R⁴ est un aryle éventuellement substitué par un ou plusieurs R⁷, et dans lequel l'aryle est choisi parmi le groupe constitué de phényle et de naphtyle éventuellement substitué par un ou plusieurs R⁷, de préférence dans lequel le phényle et le naphtyle sont éventuellement substitués par un ou plusieurs R⁷ choisi parmi le groupe constitué d'halogène, C1-C3 alkyle, haloalkyle et hydroxyalkyle, de préférence R⁷ étant choisi parmi le groupe constitué de méthyle, isopropyle, chloro, fluoro et trifluorométhyle.

6. Composé de la revendication 1, le composé étant :

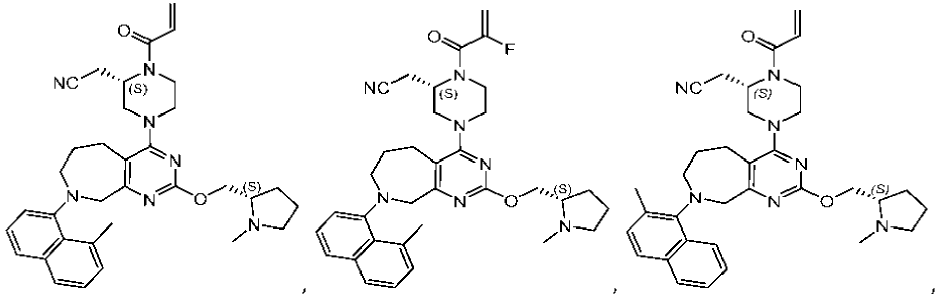

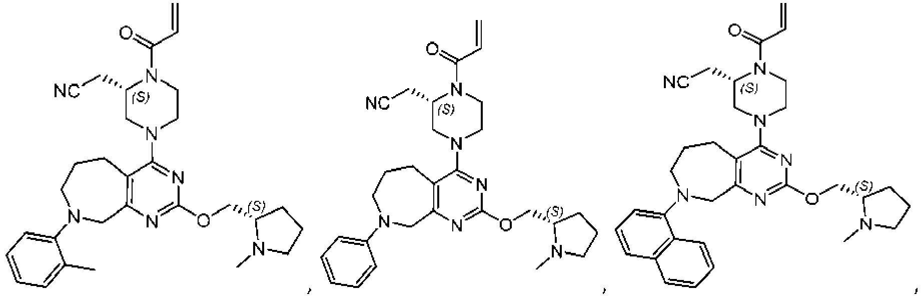

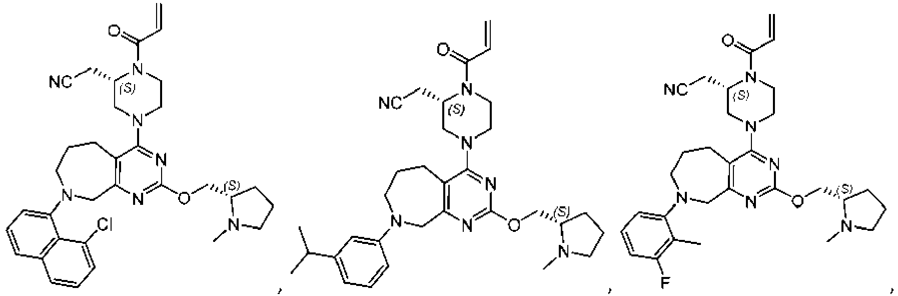

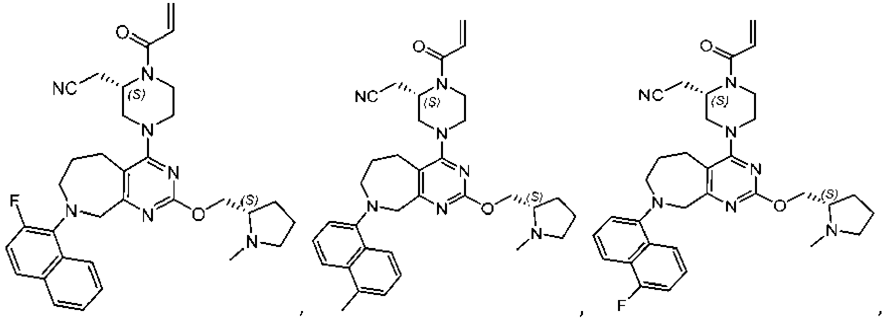

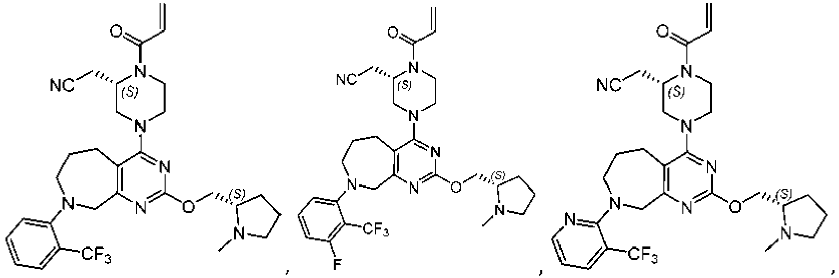

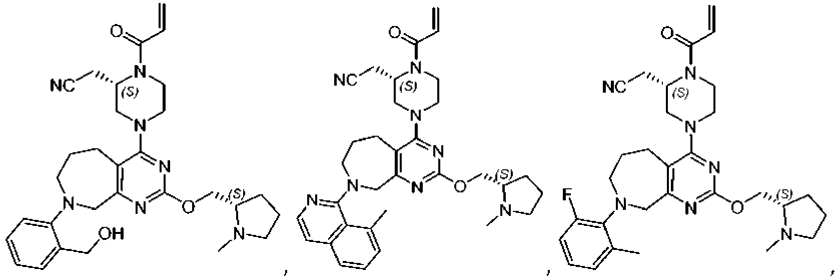

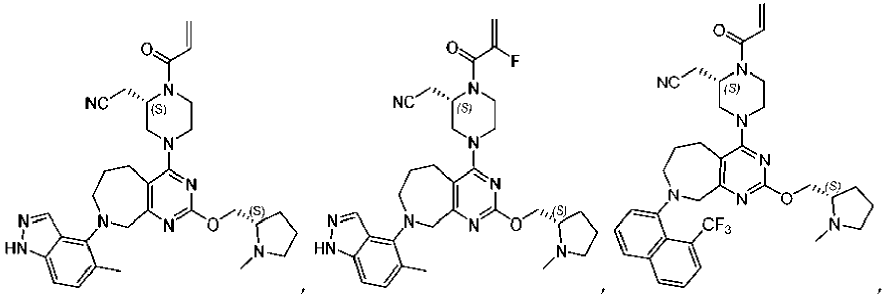

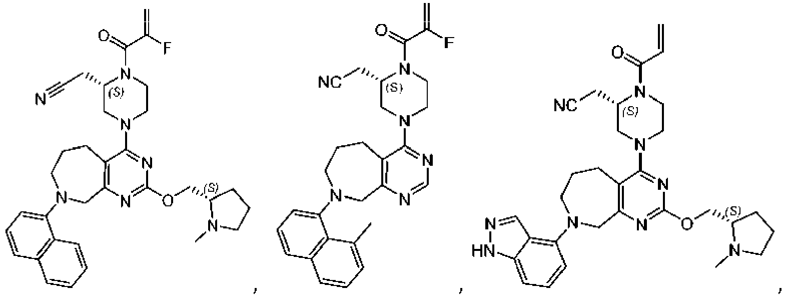

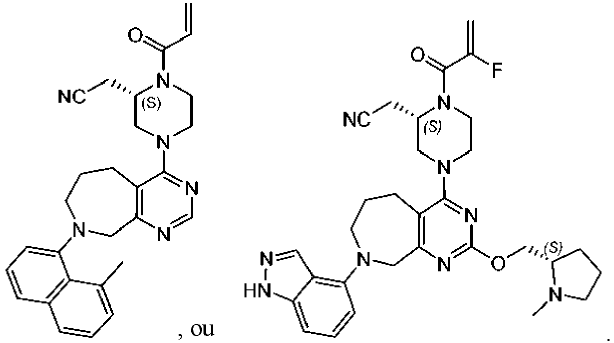

7. Composé de la revendication 1, le composé étant de formule I-A : 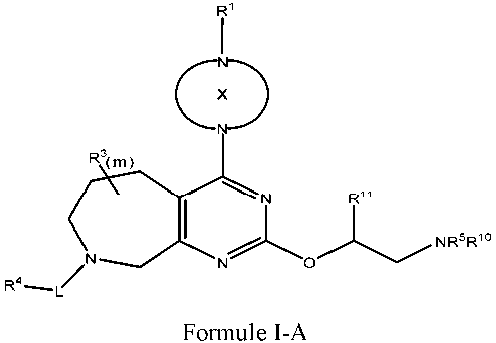 dans laquelle X est un anneau pipérazinyle éventuellement substitué par R⁸, R¹, R³, R⁴, R⁵, R⁸, R¹⁰, L et m sont tels que définis dans la revendication 1 et R¹¹ est un hydrogène, un méthyle ou un hydroxyalkyle.

8. Composé de la revendication 1, le composé étant de formule I-B : 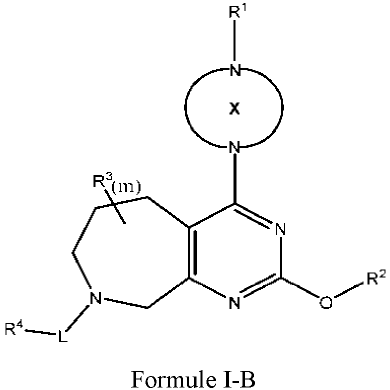 dans laquelle X est un anneau pipérazinyle éventuellement substitué par R⁸, et R¹, R³, R⁴, R⁸, L et m sont tels que définis dans la revendication 1.

9. Composition pharmaceutique, comprenant une quantité thérapeutiquement efficace d'un composé de formule (I), formule I-A ou formule I-B selon l'une quelconque des revendications 1 à 8, et un excipient pharmaceutiquement acceptable.

10. Procédé in vitro d'inhibition de l'activité KRas G12C dans une cellule, comprenant la mise en contact de la cellule dans laquelle l'inhibition de l'activité KRas G12C est souhaitée avec une quantité efficace d'un composé de formule (I), formule I-A ou formule I-B selon l'une quelconque des revendications 1 à 8, des sels pharmaceutiquement acceptables de celui-ci ou de compositions pharmaceutiques contenant le composé de formule (I), formule I-A ou formule I-B, ou un sel pharmaceutiquement acceptable de celui-ci.

11. Composé de formule (I), formule I-A ou de formule I-B selon l'une quelconque des revendications 1 à 8, sels pharmaceutiquement acceptables de celui-ci ou compositions pharmaceutiques contenant le composé de formule (I), formule I-A ou formule I-B, ou un sel pharmaceutiquement acceptable de celui-ci pour l'utilisation dans une méthode d'inhibition de l'activité KRas G12C dans une cellule, comprenant la mise contact de la cellule dans laquelle l'inhibition de l'activité KRas G12C est souhaitée avec une quantité efficace du composé de formule (I), formule I-A ou formule I-B selon l'une quelconque des revendications 1 à 8, des sels pharmaceutiquement acceptables de celui-ci ou des compositions pharmaceutiques contenant le composé de formule (I), formule I-A ou de formule I-B, ou un sel pharmaceutiquement acceptable celui-ci.

12. Composé de formule (I), formule I-A ou formule I-B, ou sel pharmaceutiquement acceptable de celui-ci selon l'une quelconque des revendications 1 à 8, seul ou combiné avec un véhicule, excipient ou diluant pharmaceutiquement acceptable pour le traitement d'un cancer associé au KRas G12C comprenant l'administration à un patient ayant un cancer associé au KRas G12C d'une quantité thérapeutiquement efficace du composé de formule (I), formule I-A ou formule I-B, ou du sel pharmaceutiquement acceptable de celui-ci selon l'une quelconque des revendications 1 à 8, seul ou combiné avec le véhicule, l'excipient ou les diluants pharmaceutiquement acceptables.

13. Composé de formule (I), formule I-A ou formule I-B, ou sel pharmaceutiquement acceptable de celui-ci, seul ou combiné avec un véhicule, excipient ou diluants pharmaceutiquement acceptables pour l'utilisation selon la revendication 12, la quantité thérapeutiquement efficace du composé étant comprise entre 0,01 et 100 mg/kg par jour.

14. Composé de formule (I), formule I-A ou formule I-B, ou sel pharmaceutiquement acceptable de celui-ci, seul ou combiné avec un véhicule, excipient ou diluants pharmaceutiquement acceptables pour l'utilisation selon la revendication 12, le cancer associé à KRas G12C étant choisi parmi le groupe constitué de Cardiaque : sarcome (angiosarcome, fibrosarcome, rhabdomyosarcome, liposarcome), myxome, rhabdomyome, fibrome, lipome et tératome ; Poumon : carcinome bronchogénique (cellules malpighiennes, petites cellules indifférenciées, grandes cellules indifférenciées, adénocarcinome), carcinome alvéolaire (bronchiolaire), adénome bronchique, sarcome, lymphome, hamartome chondromateux, mésothéliome ; Gastro-intestinal : oesophage (carcinome spinocellulaire, adénocarcinome, léiomyosarcome, lymphome ), estomac ( carcinome, lymphome, léiomyosarcome ), pancréas ( adénocarcinome canalaire, insulinome, glucagonome, gastrinome, tumeurs carcinoïdes, vipome), intestin grêle (adénocarcinome, lymphome, tumeurs carcinoïdes, sarcome de Kaposi, léiomyome, hémangiome, lipome, neurofibrome, fibrome), gros intestin (adénocarcinome, adénome tubulaire, adénome villeux, hamartome, léiomyome) ; Appareil génito-urinaire : rein (adénocarcinome, tumeur de Wilm (néphroblastome), lymphome, leucémie), vessie et urètre (carcinome spinocellulaire, carcinome à cellules transitionnelles, adénocarcinome), prostate (adénocarcinome, sarcome), testicules (séminome, tératome, carcinome embryonnaire, tératocarcinome, choriocarcinome, sarcome, carcinome à cellules interstitielles, fibrome, fibroadénome, tumeurs adénomatoïdes, lipome) ; Foie : hépatome (carcinome hépatocellulaire), cholangiocarcinome, hépatoblastome, angiosarcome, adénome hépatocellulaire, hémangiome ; Voies biliaires : carcinome de la vésicule biliaire, carcinome ampullaire, cholangiocarcinome ; Os : sarcome ostéogénique (ostéosarcome), fibrosarcome, histiocytome fibreux malin, chondrosarcome, sarcome d'Ewing, lymphome malin (sarcome à cellules réticulées), myélome multiple, tumeur maligne à cellules géantes chordome, ostéochondromes (exostoses ostéoblastiques), chondromes bénins, chondroblastome, chondromyxofibrome, ostéome ostéoïde et tumeurs à cellules géantes ; Système nerveux : crâne (ostéome, hémangiome, granulome, xanthome, ostéite déformée), méninges (méningiome, méningiosarcome, gliomatose), cerveau (astrocytome, médulloblastome, gliome, épendymome, germinome (pinéalome), glioblastome multiforme, oligodendrogliome, schwannome, rétinoblastome, tumeurs congénitales), neurofibrome de la moelle épinière, méningiome, gliome, sarcome) ; Gynécologiques : utérus (carcinome endométrial (cystadénocarcinome séreux, cystadénocarcinome muqueux, carcinome non classifié), tumeurs des cellules granulosa-thécales, tumeurs à cellules de Sertoli-Leydig, dysgerminome, tératome malin), vulve (carcinome spinocellulaire, carcinome intraépithélial, adénocarcinome, fibrosarcome, mélanome), vagin (carcinome à cellules claires, carcinome spinocellulaire, sarcome botryoïde (rhabdomyosarcome embryonnaire), trompes de Fallope (carcinome) ; Hématologique : sang (leucémie myéloïde (aiguë et chronique), leucémie lymphoblastique aiguë, leucémie lymphocytaire chronique, maladies myéloprolifératives, myélome multiple, syndrome myélodysplasique), maladie de Hodgkin, lymphome non hodgkinien (lymphome malin) ; Peau : mélanome malin, carcinome basocellulaire, carcinome spinocellulaire, sarcome de Kaposi, naevi nævi dysplasiques, lipome, angiome, dermatofibrome, chéloïdes, psoriasis ; et Glandes surrénales : neuroblastome.

15. Composé de formule (I), formule I-A ou formule I-B, ou sel pharmaceutiquement acceptable de celle-ci, seul ou combiné à un véhicule, excipient ou diluants pharmaceutiquement acceptables pour l'utilisation selon l'une des revendications 12 à 14, le cancer associé au KRas G12C étant le cancer du poumon non à petites cellules.

16. Composé de formule (I), formule I-A ou formule I-B ou sel pharmaceutiquement acceptable de celui-ci selon l'une quelconque des revendications 1 à 8, ou composition pharmaceutique de ceux-ci pour le traitement du cancer chez un patient qui le nécessite, comprenant (a) la détermination de l'association du cancer à une mutation KRas G12C (p. ex., un cancer associé à KRas G12C) 2 ; et (b) l'administration au patient d'une quantité thérapeutiquement efficace du composé de formule (I), formule I-A ou formule I-B ou du sel pharmaceutiquement acceptable de celui-ci selon l'une quelconque des revendications 1 à 8, ou de la composition pharmaceutique de celui-ci.
